# EUROPEAN PATENT APPLICATION

(11) **EP 4 800 016 A1**
(43) Date of publication of application: **02.09.2026**
(21) Application number: 24881726.4
(22) Date of filing: 25.10.2024
(51) Int. Cl.: C07D 487/04, C07D 471/04, C07D 471/12, A61K 31/519, A61P 35/00

(54) **METTL3 INHIBITOR**

(30) Priority: 26.10.2023 CN 202311399919; 16.11.2023 CN 202311529351; 23.01.2024 CN 202410091151; 26.03.2024 CN 202410349037; 03.07.2024 CN 202410883982
(71) Applicant: Hangzhou Bangshun Pharmaceutical Co., Ltd., Hangzhou, Zhejiang 31112 (CN)
(72) Inventor: WEN, Qiaodong, Hangzhou, Zhejiang 311112 (CN); YANG, Xin, Hangzhou, Zhejiang 311112 (CN); CUI, Rong, Hangzhou, Zhejiang 311112 (CN); ZHENG, Mei, Hangzhou, Zhejiang 311112 (CN); YIN, Jianming, Hangzhou, Zhejiang 311112 (CN); LV, Yubin, Hangzhou, Zhejiang 311112 (CN)
(74) Representative: Kador & Partner Part mbB
(86) International application number: PCT/CN2024/127250
(87) International publication number: WO 2025/087365

(57) **Abstract**

The present invention discloses a METTL3 inhibitor represented by general formula (I), its pharmaceutical composition, preparation method, and the use thereof in the preparation of drugs, prevention and/or treatment of drugs for indications associated with METTL3. The compound of the present invention is an ideal high-activity METTL3 inhibitor, which can be used for the treatment and/or prevention of diseases, including AML, myeloid leukemia, solid tumors such as hepatocellular carcinoma, colorectal cancer, and prostate cancer.

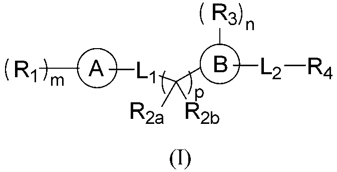

## Description

### Technical Field

The present invention belongs to the field of pharmaceutical chemistry, and relates to a METTL3 inhibitor, a pharmaceutical composition thereof, a preparation method thereof, and use thereof in the preparation of a medicament for prevention and/or treating METTL3-related indications.

### Background

m⁶A (N6-methyladenosine modification) is the most common post-transcriptional modification of mRNA in humans, involved in pre-mRNA splicing, translation, stability, structure, export, and decay (Stephanie Oerum et al 2021). RNA methylation is regulated by writers (methyltransferases), readers (RNA-binding proteins), and erasers (demethylases). The m⁶A modification of mRNA is carried out by a multi-component writer complex in the nucleus (Alex andra Maldonado Lopez et al 2021). METTL3 is a key m⁶A writer that forms a heterodimer with METTL14 and is the core component of the m⁶A methyltransferase complex (MTC), where METTL3 has catalytic function (Hanqi Liu et al 2023). Additionally, the WTAP protein binds to the METTL3-METTL14 dimer and is necessary for proper m⁶A modification (Schwartz S, et al 2014). Besides WTAP, MTC also includes other adaptor proteins such as VIRMA, RBM15, ZC3H13, etc. (Su S, et al 2022).

m⁶A is involved in various physiological functions. m⁶A can modify non-coding RNA functions, such as miRNA. Adenosine methylation modification can affect miRNA biogenes is, transport, processing, and functional activity on target mRNA (Alarcon, C. R., et al 2015). It can also influence lncRNA functions (Yuan S, et al 2014). Maintenance of normal m⁶A levels is crucial for cardiovascular homeostas is, and MTC dysregulation is associated with cardiovascular diseases such as hypertension, atheroscleros is, and heart failure (Li L, et al 2022). Furthermore, m⁶A is also linked to nervous system development, Alzheimer's disease, and viral infections (Francesco Fiorentino, et al 2023).

m⁶A plays an important role in tumorigenes is and development. METTL3 is overexpressed in breast cancer t issues and cells, promoting tumor development through mechan isms such as regulating Bcl-2 protein levels, methylating EZH2 mRNA to enhance EZH2 expression, and m⁶A modification of tumor suppressor protein LATS 1 to reduce its levels (Hong Wang et al 2020, Youqin Xu et al 2023, Hu S, et al 2022). It can also increase PD-1 expression to inhibit tumor immune surveillance (Weijun Wan et al 2022) and promote breast cancer lung metastas is by regulating KRT7 mRNA stability and translation efficiency (Feng Chen et al 2021).

METTL3 is also involved in the development of colorectal cancer. High METTL3 expression correlates with CRC metastas is and poor prognos is. METTL3 knockout significantly reduces the self-renewal ability of CRC cells and inhibits CRC tumorigenes is and metastas is in corresponding tumor models (Ting Li et al 2019). Involved regulatory pathways include immune suppression (Huarong Chen et al 2022), activation of the GLUT1-mTORC1 pathway (Huarong Chen et al 2021), and the miR-1246/MAPK pathway (Wen Peng et al 2019).

Other tumors where METTL3 plays an oncogenic role include solid tumors such as glioma, gastric cancer, hepatocellular carcinoma, lung cancer, melanoma, and hematological malignancies such as AML and CML. METTL3 is highly expressed in tumor t issues comp ared to normal t issues and is an independent prognostic factor for overall and disease-free survival in gastric cancer patients (Yoshinaga Okugawa et al 2022). High METTL3 expression has been found in AML cells and DLBCL cells. High METTL3 expression is essential for AML cell proliferation ( isaia Barbieri et al 2017).

Inhibition of METTL3 can effectively suppress tumor growth. Literature reports indicate that METTL3 inhibitors can be used to treat myeloid leukemia (Eliza Yankova et al 2021) and reduce the growth of lenvatinib-res istant hepatocellular carcinoma cells (Lina Wang et al 2023).

METTL3 inhibitors can also be combined with other drugs, such as BCL-2 inhibitors for treating AML (Lina Vasiliauskaite et al) and anti-PD-1 for treating CRC (Yaara Ofir-Rosenfeld et al).

### Summary

In view of the above technical problems, the present invention aims to provide a METTL3 inhibitor, a pharmaceutical composition thereof, a preparation method thereof, and use thereof in the preparation of a medicament for prevention and/or treating METTL3-related indications.

One aspect of the present invention provides a compound of formula (I): 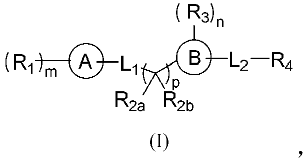
or a deuterated, a stereoisomer, a pharmaceutically acceptable salt, or a pharmaceutically acceptable solvate thereof;
wherein,
Ring A is a 5- to 15-membered heterocyclyl, preferably a 5- to 6-membered monocyclic heterocyclyl, 9- to 10-membered bicyclic heterocyclyl, or 13- to 14-membered tricyclic heterocyclyl, containing heteroatoms selected from O, N, S, P, and optionally 1, 2, or 3 atoms are oxo-substituted;
L₁ is 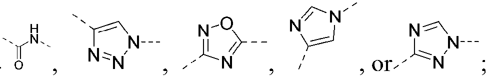
m is selected from 0, 1, 2, 3, 4, or 5;
R₁ is oxo, or
R₁ is -Z-Rₐ;
Z is selected from a chemical bond, C₁₋₃ alkylene, -NH-C₁₋₃ alkylene, -C(=O)-, -O-, -S-, -S(=O)-, - SO₂-;
Rₐ is selected from halogen, cyano, hydroxyl, C₁₋₃ alkyl, C₁₋₃ alkoxy, halo-C₁₋₃ alkyl, halo-C₁₋₃ alkoxy, -NH₂, nitro, formyl, -NH-C₁₋₃ alkyl, -N(-C₁₋₃ alkyl)₂, optionally substituted 3- to 6-membered cycloalkyl, optionally substituted 4- to 6-membered heterocycloalkyl, optionally substituted phenyl, optionally substituted 5- to 7-membered heteroaryl;
When two R₁ are both attached to the same carbon atom of Ring A, the two R₁ together with the carbon atom to which they are attached may form an optionally substituted 3- to 6-membered cycloalkyl or a 3- to 6-membered heterocyclyl containing 1-2 heteroatoms selected from N, O, S; Ring B is an 8- to 10-membered heterocyclyl, preferably an 8- to 10-membered heteroaryl;
n, p are selected from 0, 1, 2, or 3;
R₂ₐ and R_{2b} are independently selected from H, halogen, cyano, hydroxyl, C₁₋₃ alkyl, C₁₋₃ alkoxy, halo-C₁₋₃ alkyl, halo-C₁₋₃ alkoxy, nitro, formyl, or
R₂ₐ and R_{2b} together with the carbon atom to which they are attached form a 3- to 6-membered cycloalkyl or a 3- to 6-membered heterocycloalkyl containing 1-2 heteroatoms selected from N, O, S;
R₃ is selected from H, halogen, cyano, hydroxyl, C₁₋₃ alkyl, C₁₋₃ alkoxy, halo-C₁₋₃ alkyl, halo-C₁₋₃ alkoxy, nitro, formyl;
L₂ is selected from C₁₋₃ alkylene, haloC₁₋₃ alkylene;
R₄ is 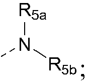
R₅ₐ and R_{5b} are selected from H or -L₃-R₆, where L₃ is selected from a chemical bond, C₁₋₃ alkylene, or halo-C₁₋₃ alkylene, and R₆ is selected from C₁₋₆ alkyl, C₂₋₆ alkynyl, optionally substituted phenyl, optionally substituted 5- to 10-membered heterocyclyl, optionally substituted 3- to 8-membered cycloalkyl, optionally substituted 4- to 8-membered heterocycloalkyl, or
R₅ₐ and R_{5b} together with the nitrogen atom to which they are attached form an optionally substituted 4- to 10-membered heterocyclyl, preferably a 4- to 10-membered heterocycloalkyl.
In some embodiments, R₁ is -Z-Rₐ;
In some embodiments, when two R₁ are substituted on the same carbon atom of Ring A, the two R₁ do not form a Ring;
In some embodiments, R₂ₐ and R_{2b} do not form a Ring with the attached carbon atom;
In some embodiments, L₂ is selected from C₁₋₃ alkylene;
In some embodiments, R₅ₐ and R_{5b} are selected from H or -L₃-R₆, where L₃ is selected from a chemical bond or C₁₋₃alkylene, and R₆ is selected from C₁₋₆alkyl, C₂₋₆alkynyl, optionally substituted phenyl, optionally substituted 3- to 8-membered cycloalkyl, optionally substituted 4- to 8-membered heterocycloalkyl, or
R₅ₐ and R_{5b} together with the nitrogen atom to which they are attached form an optionally substituted 4- to 10-membered heterocycloalkyl.

In some preferred embodiments, any hydrogen atom on the compound of formula (I) may be replaced by deuterium to form a deuterated derivative. In some more preferred embodiments, the deuteration sites may be R₂ₐ, R_{2b}, L₂ or L₃.

Preferably:
Ring A is selected from one of the following groups i), ii), iii):
   i) 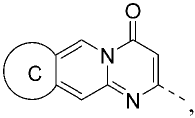 wherein Ring C is selected from 5- to 7-membered heterocyclyl containing 1-3 heteroatoms selected from N, O, S; more preferably 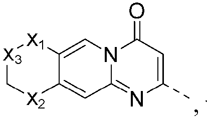 wherein X₁ and X₂ are independently selected from O, S, CH₂, S(=O)₂, C(=O), and X₃ is selected from CH₂, NH or a chemical bond;
      In this case, Ring A includes 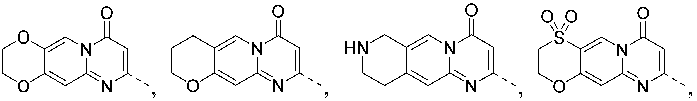 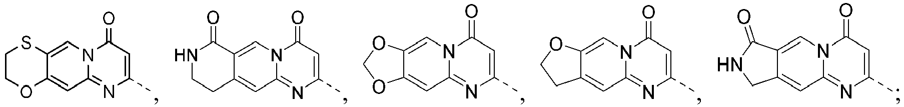
   ii) 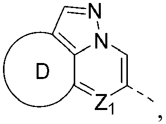 heteroatoms selected from N, O, S, and Z₁ is selected from N or CH; more preferably 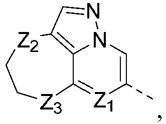 wherein Z₁ is selected from N or CH, and Z₂ and Z₃ are independently selected from CH₂, NH, O, S;
      In this case, Ring A includes 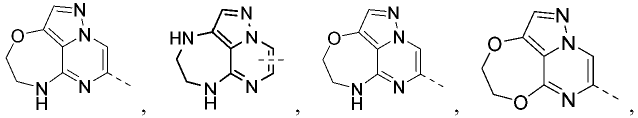 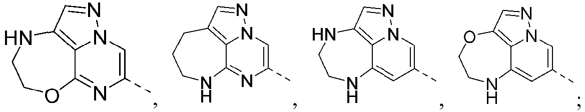
   iii) 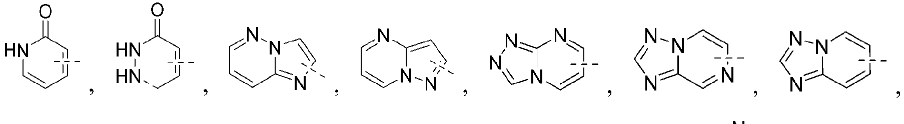 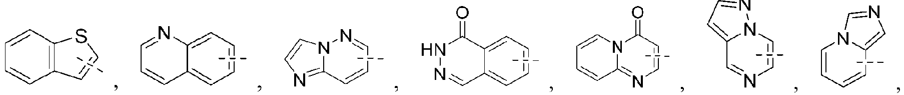 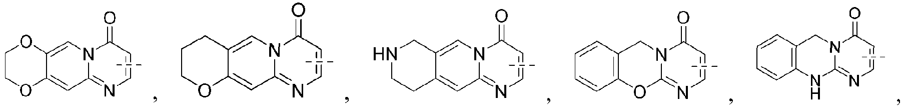 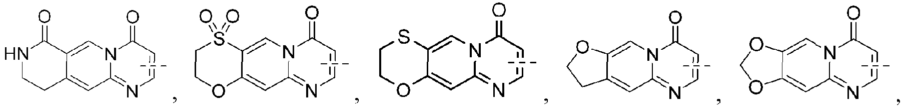 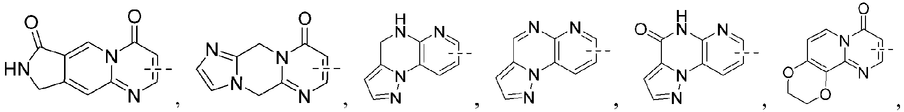 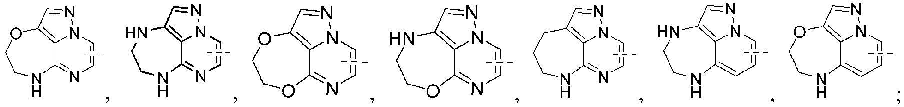
Ring A is preferably selected from: 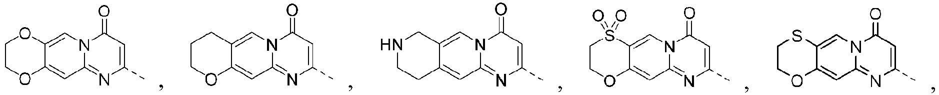 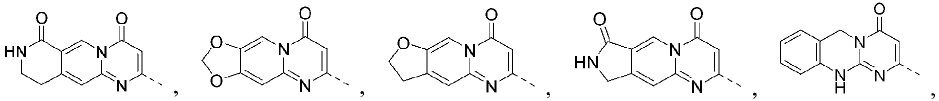 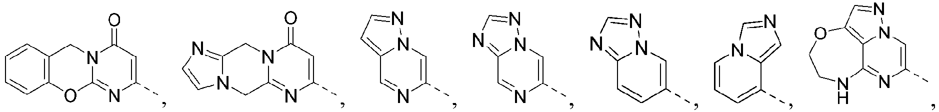 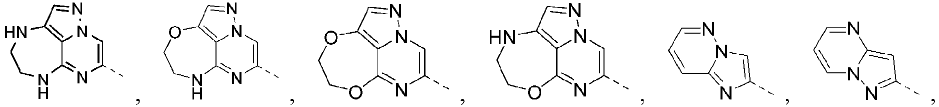 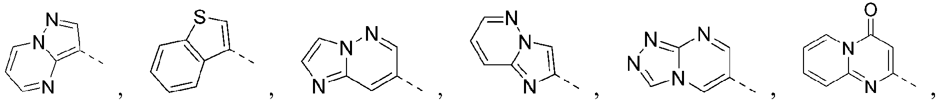 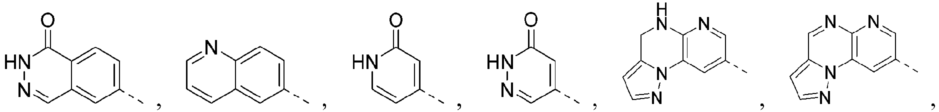 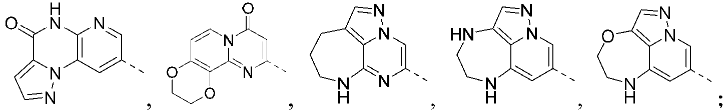 m is selected from 0, 1, 2 or 3;
R₁ is oxo, or
R₁ is -Z-Rₐ;
Z is selected from a chemical bond, methylene, -NH-CH₂-, -C(=O)-, -O-, -S-, -S(=O)-, -SO₂-;
Rₐ is selected from halogen, cyano, hydroxyl, C₁₋₃ alkyl, C₁₋₃ alkoxy, halo-C₁₋₃ alkyl, halo-C₁₋₃ alkoxy, -NH₂, nitro, formyl, -NH-C₁₋₃ alkyl, -N(-C₁₋₃ alkyl)₂, and the following groups optionally substituted by 1, 2 or 3 R_{b}: 3- to 6-membered cycloalkyl, 4- to 6-membered heterocycloalkyl, phenyl, 5- to 7-membered heteroaryl;
R_{b} is selected from halogen, cyano, hydroxyl, C₁₋₃ alkyl, C₁₋₃ alkoxy, halo-C₁₋₃ alkyl, halo-C₁₋₃ alkoxy, -NH₂, nitro, formyl, -NH-C₁₋₃ alkyl, -N(-C₁₋₃ alkyl)₂, preferably F, methyl, methoxy, amino, methylamino;
Rₐ is preferably selected from F, methyl, trifluoromethyl, difluoromethyl, methoxy, trifluoromethoxy, amino, methylamino, and the following groups optionally substituted by 1, 2 or 3 R_{b}: 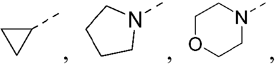 phenyl, 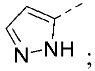 more preferably F, methyl, trifluoromethyl, difluoromethyl, methoxy, trifluoromethoxy, amino, methylamino, 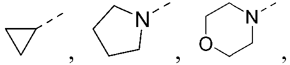 phenyl, 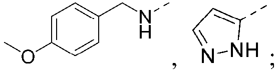
R₁ is more preferably selected from F, methyl, trifluoromethyl, difluoromethyl, methoxy, trifluoromethoxy, amino, methylamino,

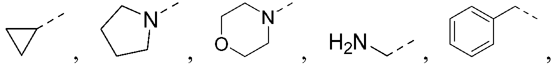

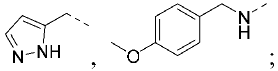

When two R₁ are both substituted on the same carbon atom of Ring A, the two R₁ together with the connected carbon atom may form an optionally substituted 3- to 6-membered cycloalkyl or a 3- to 6-membered heterocyclyl containing 1-2 heteroatoms selected from N, O, S, preferably cyclopropyl, cyclobutyl, oxiranyl, oxetanyl, azetidinyl.

In some embodiments, Ring A is selected from group iii). In some other embodiments, Ring A may also be selected from group i) or group ii).

In some embodiments, Ring A is selected from:

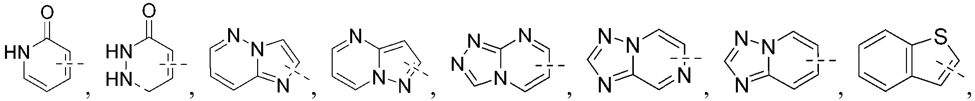

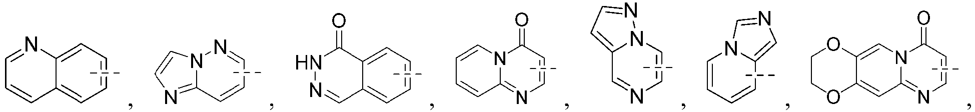

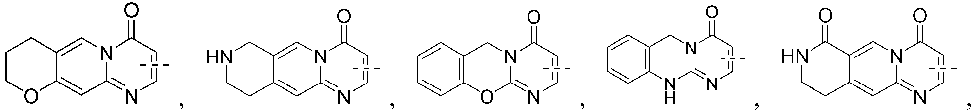

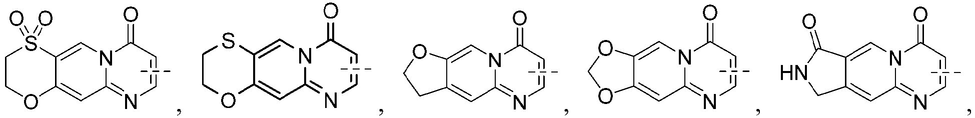

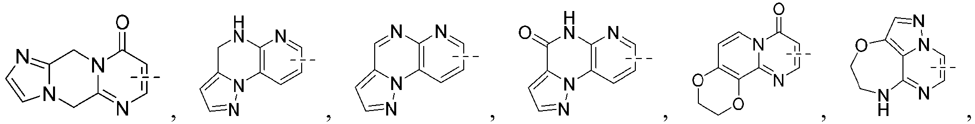

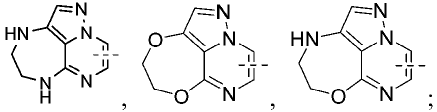

In some embodiments, Ring A is preferably selected from:

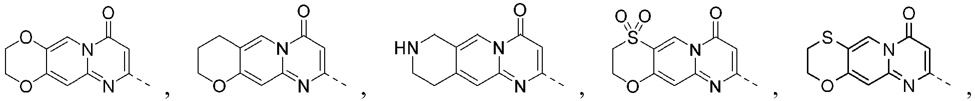

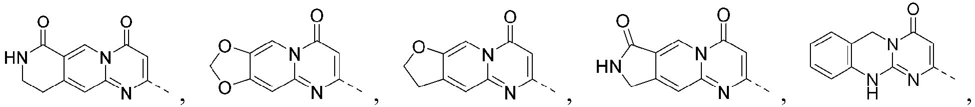

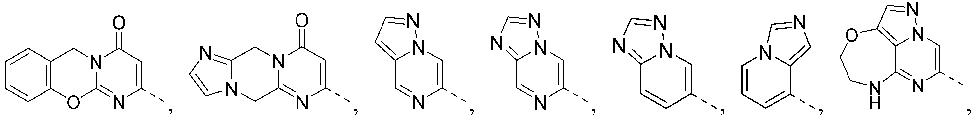

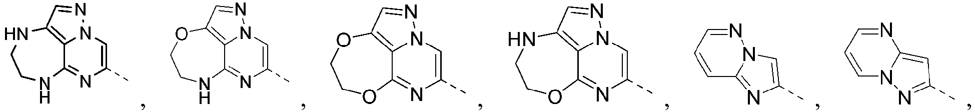

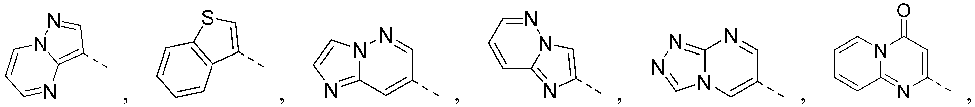

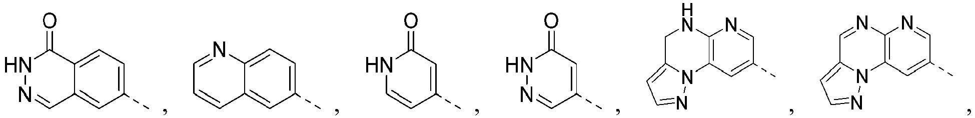

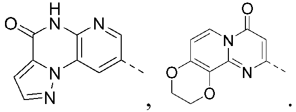

In some embodiments, R^{a} is preferably selected from F, methyl, amino, methylamino, and the following groups optionally substituted by 1, 2 or 3 R_{b}: 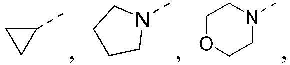 phenyl, 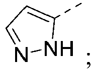 more preferably F, methyl, amino, methylamino, 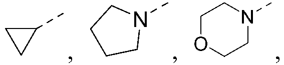 phenyl,

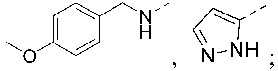

In some embodiments, R₁ is more preferably selected from F, methyl, amino, methylamino,

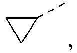

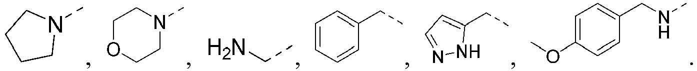

In some embodiments, when two R₁ are both substituted on the same carbon atom of Ring A, the two R₁ do not form a Ring.

Further preferably:
Ring A and R₁ form the following structure:

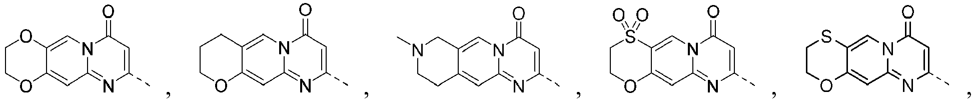

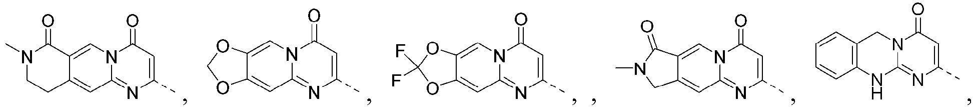

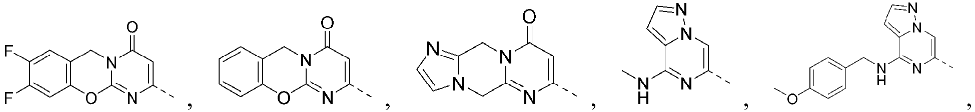

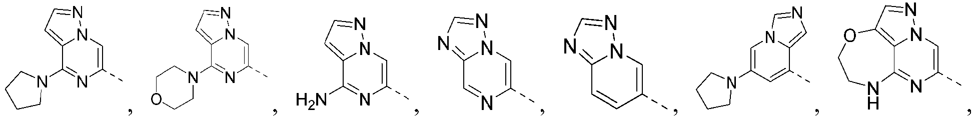

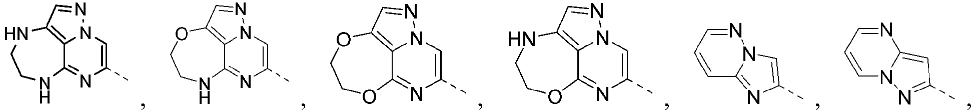

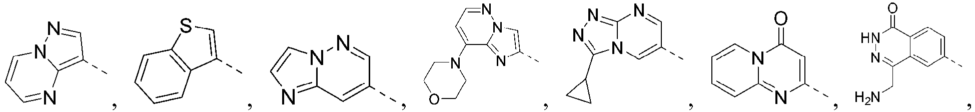

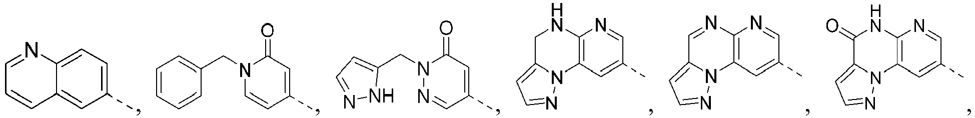

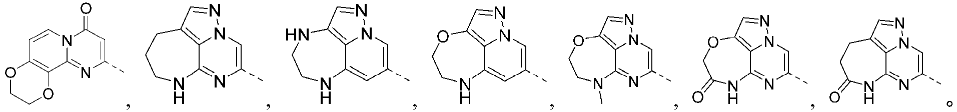

In some embodiments, Ring A and R₁ form the following structures:

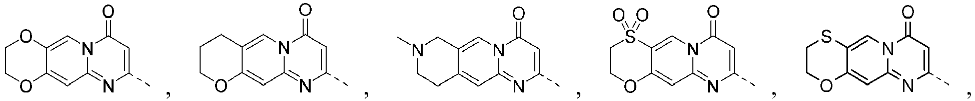

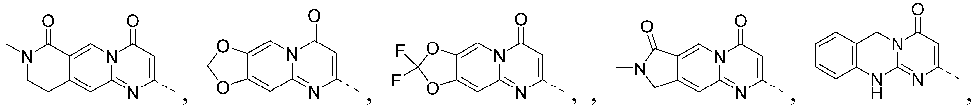

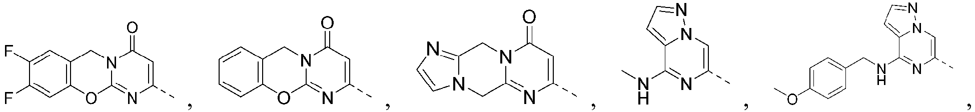

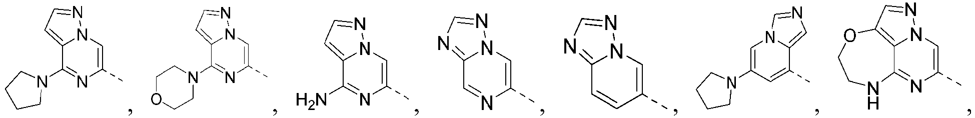

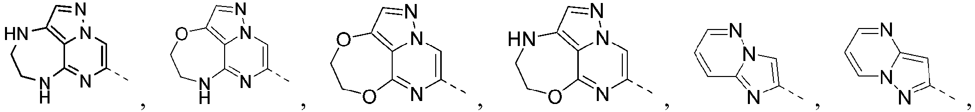

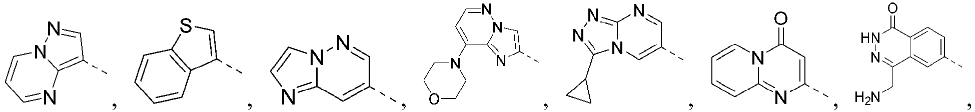

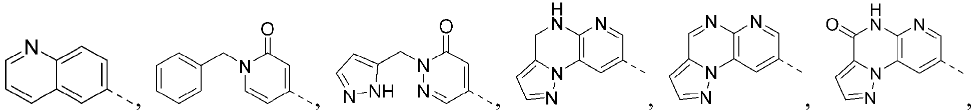

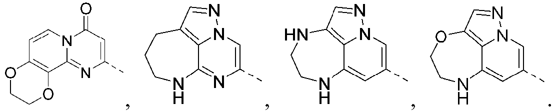

In some embodiments, Ring A and R₁ form the following structures:

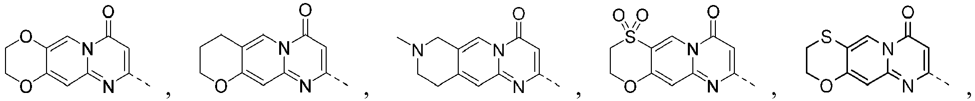

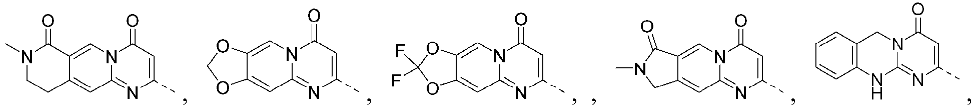

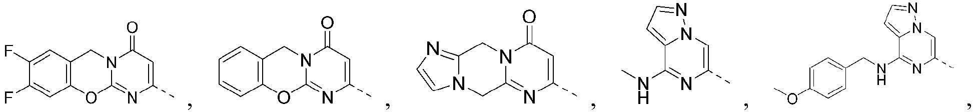

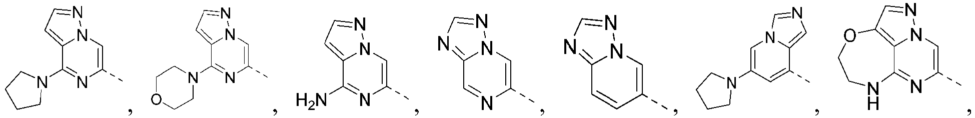

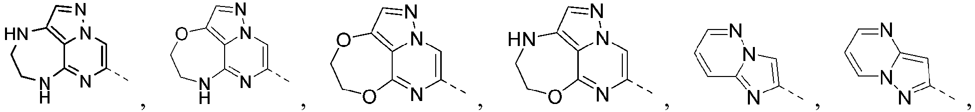

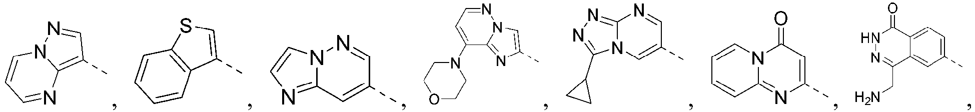

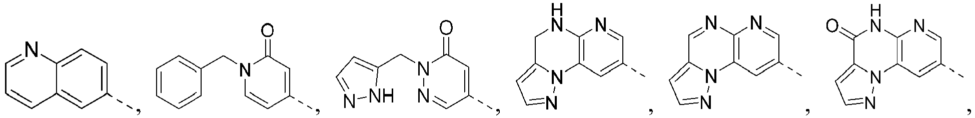

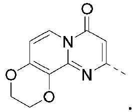

Preferably:
Ring B is selected from: 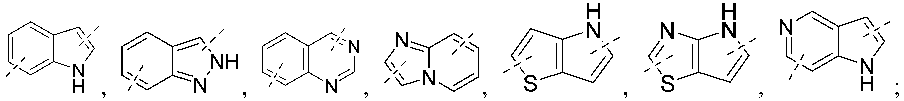
Ring B is preferably selected from: 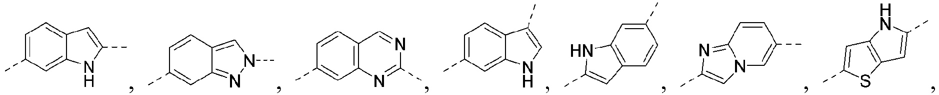 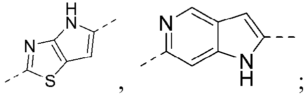
n is selected from 0, 1, 2 or 3;
R₃ is selected from H, halogen, C₁₋₃ alkyl, C₁₋₃ alkoxy, halo-C₁₋₃ alkyl, halo-C₁₋₃ alkoxy, preferably H, F, methyl, trifluoromethyl, difluoromethyl, methoxy, trifluoromethoxy.

In some embodiments, Ring B is selected from:

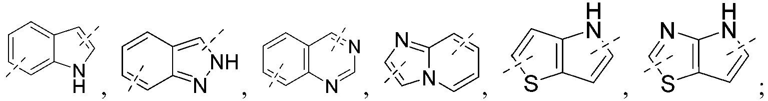

Ring B is preferably selected from:

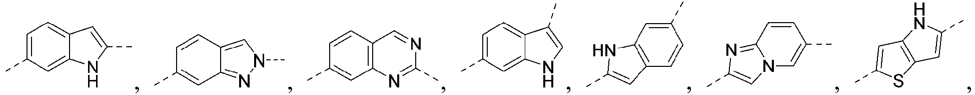

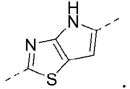

Preferably:
L₂ is selected from methylene, ethylidene;
R₄ is 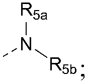
R₅ₐ, R_{5b} are selected from H or -L₃-R₆, where L₃ is selected from a chemical bond, methylene, ethylidene, propylidene, 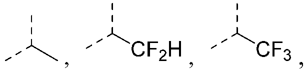 and R₆ is selected from the following groups optionally substituted by 1, 2 or 3 R₇: ethynyl, isopropyl, tert-butyl, phenyl, 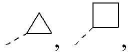 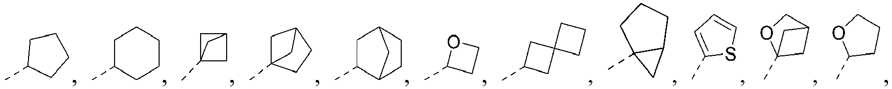 or
R₅ₐ and R_{5b} together with the nitrogen atom to which they are attached form, optionally substituted with 1, 2 or 3 R7, 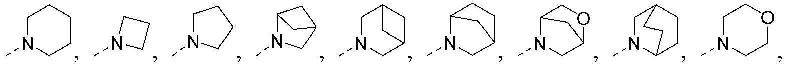 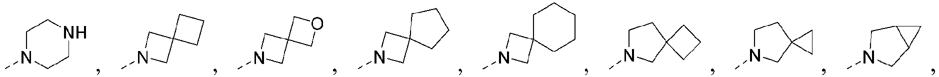 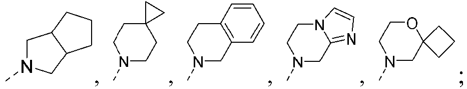
R₇ is selected from halogen, cyano, hydroxyl, C₁₋₃ alkyl, C₁₋₃ alkoxy, halo-C₁₋₃ alkyl, halo-C₁₋₃ alkoxy, =CF₂, hydroxy-C₁₋₃ alkyl, preferably F, hydroxyl, methyl, trifluoromethyl, difluoromethyl, methoxy, trifluoromethoxy, =CF₂, hydroxymethyl.

In some embodiments, R₅ₐ, R_{5b} are selected from H or -L₃-R₆, where L₃ is selected from a chemical bond, methylene, ethylidene, propylidene, 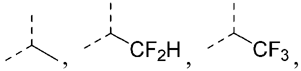 and R₆ is selected from the following groups optionally substituted by 1, 2 or 3 R₇: ethynyl, isopropyl, tert-butyl, phenyl, 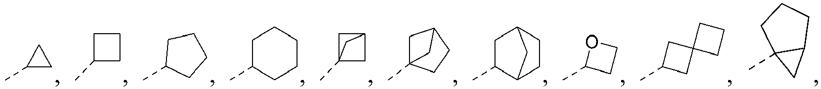 or
R₅ₐ and R_{5b} together with the attached N atom form, optionally substituted by 1, 2 or 3 R₇,

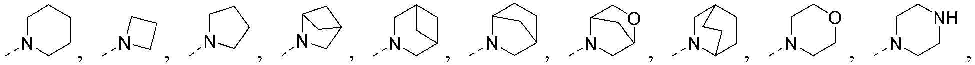

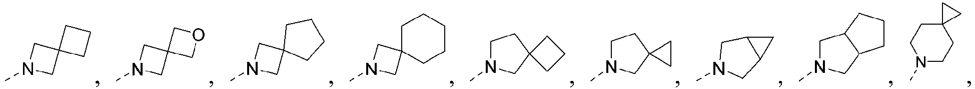

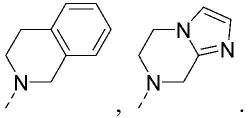

In some embodiments, R₅ₐ, R_{5b} are selected from H or -L₃-R₆, where L₃ is selected from a chemical bond, methylene, ethylidene, propylidene, and R₆ is selected from the following groups optionally substituted by 1, 2 or 3 R₇: ethynyl, isopropyl, tert-butyl, phenyl, 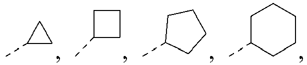 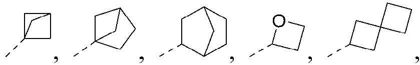 or
R₅ₐ and R_{5b} together with the attached N atom form, optionally substituted by 1, 2 or 3 R₇,

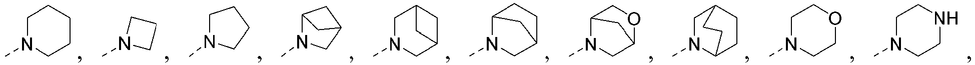

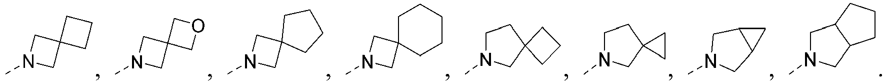

In some embodiments, R₇ is selected from F, hydroxyl, methyl, methoxy, =CF₂, hydroxymethyl.

Further preferably:
R₄ is selected from

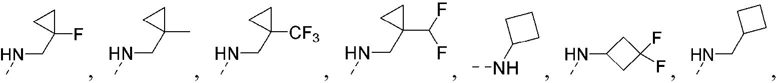

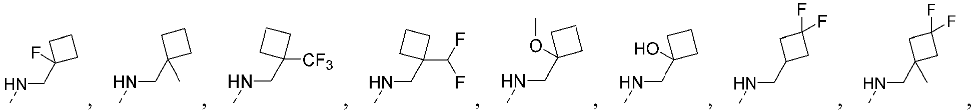

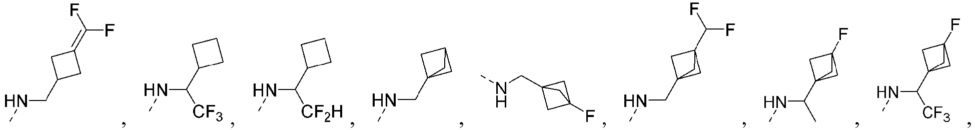

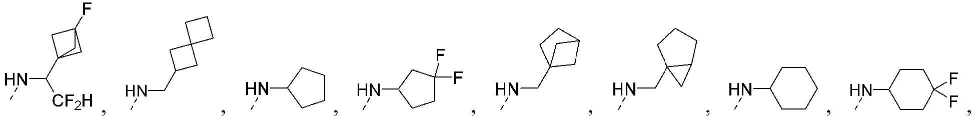

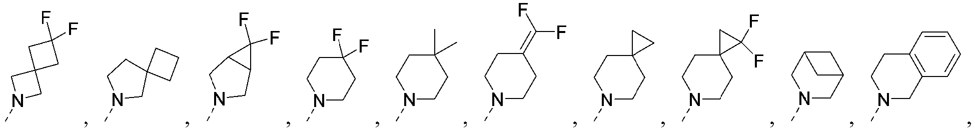

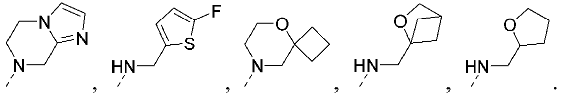

In some embodiments, R₄ is selected from 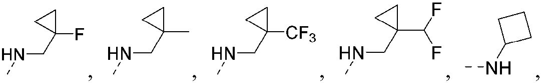 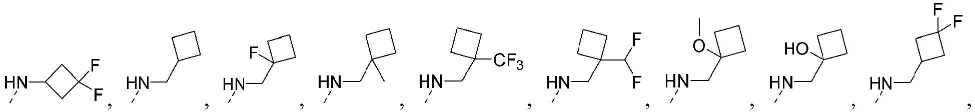 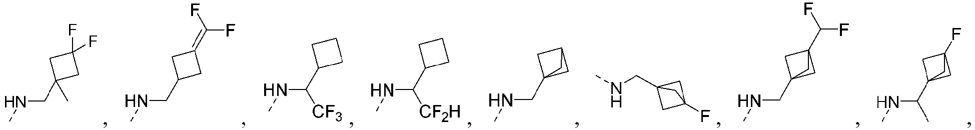 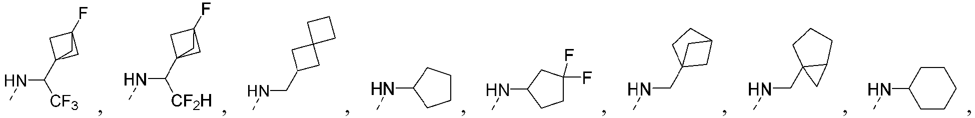 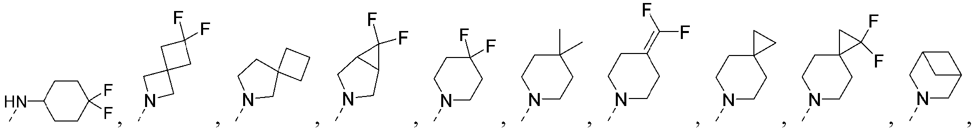 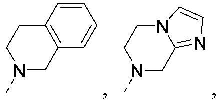 R₄ is selected from

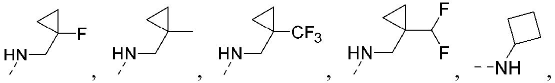

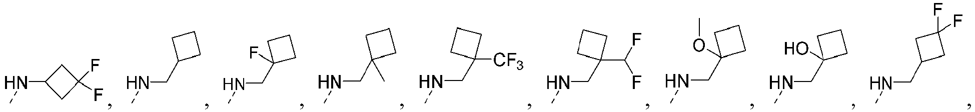

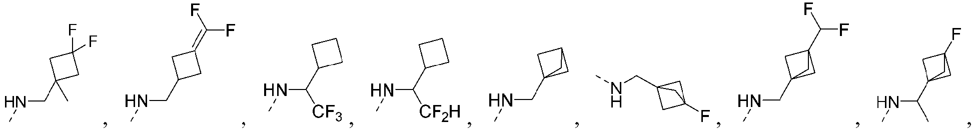

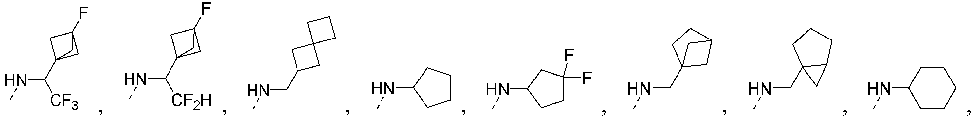

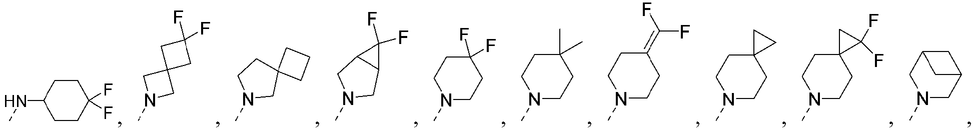

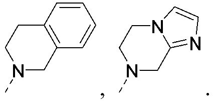

Further preferably:
L₂ and R₄ form the following structures:

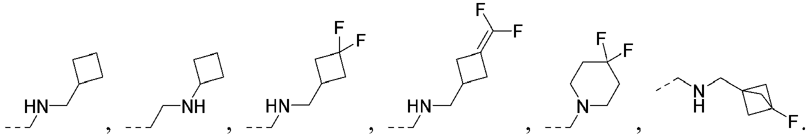

Preferably, L₁ is

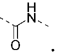

Preferably, n and p are 1; R₂ₐ, R_{2b}, and R₃ are H.

In some preferred embodiments, the compound,or the deuteride, the stereo isomer, or the pharmaceutically acceptable salt, or the pharmaceutically acceptable solvate thereof, has the structure of formula (II): 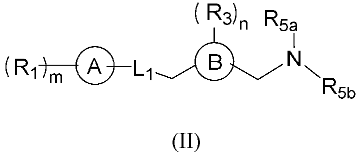 wherein,
Ring A is selected from one of the following:
   i) 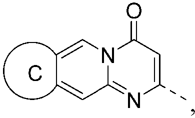 wherein Ring C is selected from 5- to 7-membered heterocyclyl containing 1-3 heteroatoms selected from N, O, S; more preferably 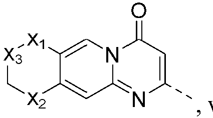 herein X₁ and X₂ are independently selected from O, S, CH₂, S(=O)₂, C(=O), and X₃ is selected from CH₂, NH or a chemical bond;
      In this case, Ring A includes 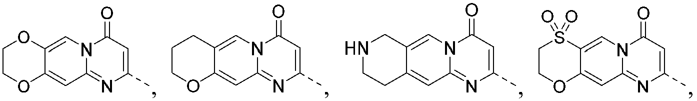 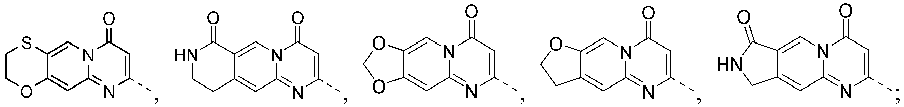
   ii) 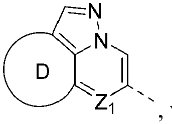 wherein Ring D is selected from 6- to 8-membered heterocyclyl containing 1-3 heteroatoms selected from N, O, S, and Z₁ is selected from N or CH; more preferably 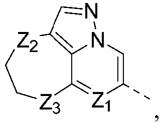 wherein Z₁ is selected from N or CH, and Z₂ and Z₃ are independently selected from CH₂, NH, O, S;
      In this case, Ring A includes 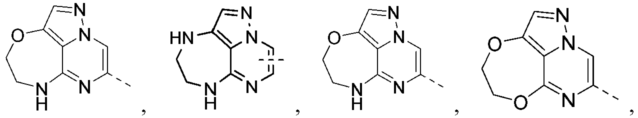 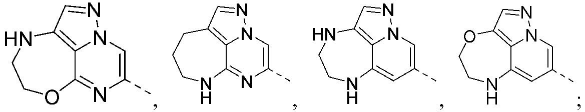
   iii) 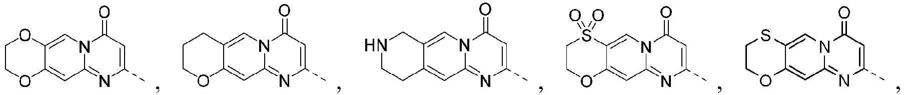 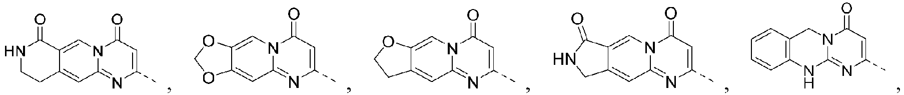 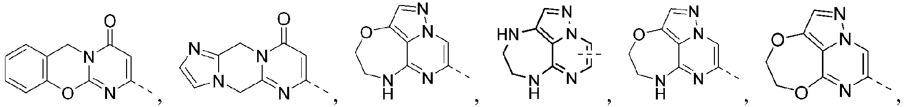 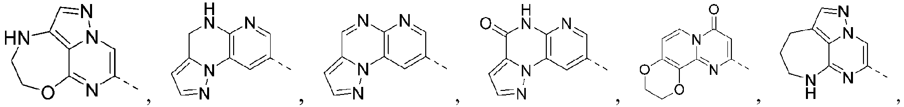 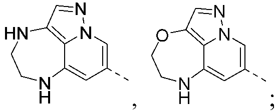
m is selected from 0, 1, 2 or 3;
R₁ is oxo, or
R₁ is -Z-Rₐ;
Z is selected from a chemical bond, C₁₋₃ alkylene, -NH-C₁₋₃ alkylene, -C(=O)-, -O-, -S-, -S(=O)-, - SO₂-, preferably methylene, -NH-CH₂-, -C(=O)-, -SO₂-;
Rₐ is selected from halogen, cyano, hydroxyl, C₁₋₃ alkyl, C₁₋₃ alkoxy, halo-C₁₋₃ alkyl, halo-C₁₋₃ alkoxy, -NH₂, nitro, formyl, -NH-C₁₋₃ alkyl, -N(-C₁₋₃ alkyl)₂, and the following groups optionally substituted by 1, 2 or 3 R_{b}: 3- to 6-membered cycloalkyl, 4- to 6-membered heterocycloalkyl, phenyl, 5- to 7-membered heteroaryl;
R_{b} is selected from halogen, cyano, hydroxyl, C₁₋₃ alkyl, C₁₋₃ alkoxy, halo-C₁₋₃ alkyl, halo-C₁₋₃ alkoxy, -NH₂, nitro, formyl, -NH-C₁₋₃ alkyl, -N(-C₁₋₃ alkyl)₂, preferably F, methyl, methoxy, amino, methylamino;
Rₐ is preferably selected from F, methyl, amino, methylamino;
When two R₁ are both substituted on the same carbon atom of Ring A, the two R₁ together with the connected carbon atom may form an optionally substituted 3- to 6-membered cycloalkyl or a 3- to 6-membered heterocyclyl containing 1-2 heteroatoms selected from N, O, S;
L₁ is 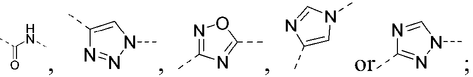
Ring B is selected from: 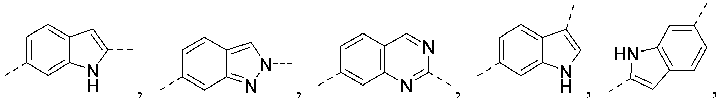 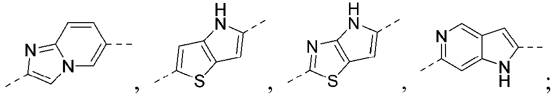
n is selected from 0, 1, 2 or 3;
R₃ is independently selected from halogen, cyano, hydroxyl, C₁₋₃ alkyl, C₁₋₃ alkoxy, halo-C₁₋₃ alkyl, halo-C₁₋₃ alkoxy;
R₅ₐ, R_{5b} are selected from H or -L₃-R₆; L₃ is selected from a chemical bond, C₁₋₃ alkylene, halo-C₁₋₃ alkylene, preferably a chemical bond, methylene, 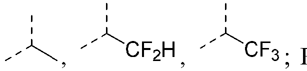 R₆ is selected from C₁₋₆ alkyl, C₂₋₆ alkynyl, optionally substituted phenyl, optionally substituted 5- to 10-membered heterocyclyl, optionally substituted 3- to 8-membered cycloalkyl, optionally substituted 4- to 8-membered heterocycloalkyl, preferably ethynyl, isopropyl, tert-butyl, the following groups optionally substituted by 1, 2 or 3 R₇: phenyl, 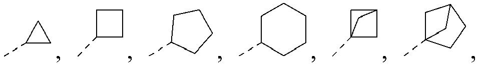 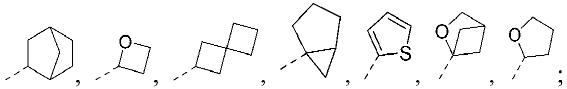 or
R₅ₐ, R_{5b} together with the attached N atom form an optionally substituted 4- to 10-membered heterocyclyl, preferably 4- to 10-membered heterocycloalkyl, preferably the following groups optionally substituted by 1, 2 or 3 R₇: 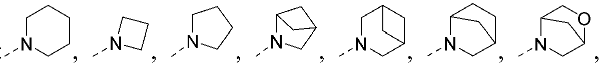 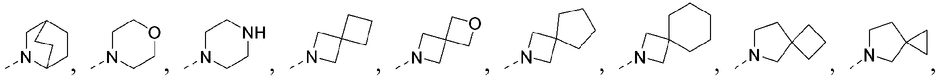 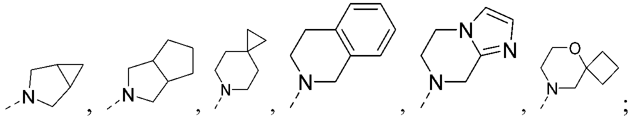
R₇ is selected from halogen, cyano, hydroxyl, C₁₋₃ alkyl, C₁₋₃ alkoxy, halo-C₁₋₃ alkyl, halo-C₁₋₃ alkoxy, =CF₂, hydroxyl C₁₋₃ alkyl, preferably F, hydroxyl, methyl, trifluoromethyl, difluoromethyl, methoxy, trifluoromethoxy, =CF₂, hydroxymethyl.

In some embodiments, Ring A is selected from group iii). In some other embodiments, Ring A may also be selected from group i) or group ii).

In some embodiments, when two R₁ are substituted on the same carbon atom of Ring A, the two R₁ do not form a Ring;
In some embodiments, Ring B is selected from:

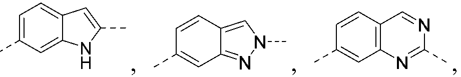

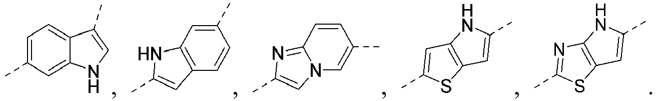

In some embodiments, L₃ is selected from a chemical bond, C₁₋₃ alkylene, preferably a chemical bond, methylene.

In some embodiments, R₆ is selected from C₁₋₆ alkyl, C₂₋₆ alkynyl, optionally substituted phenyl, optionally substituted 3- to 8-membered cycloalkyl, optionally substituted 4- to 8-membered heterocycloalkyl, preferably ethynyl, isopropyl, tert-butyl, the following groups optionally substituted by 1, 2 or 3 R₇: phenyl, 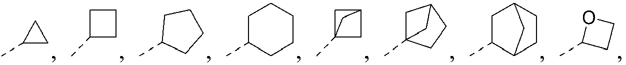 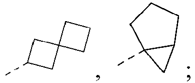 more preferably selected from ethynyl, isopropyl, tert-butyl, the following groups optionally substituted by 1, 2 or 3 R₇: phenyl,

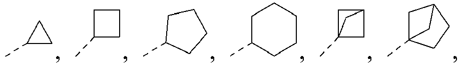

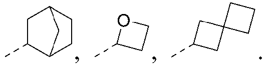

In some embodiments, preferably R₅ₐ, R_{5b} together with the attached N atom form the following groups optionally substituted by 1, 2 or 3 R₇:

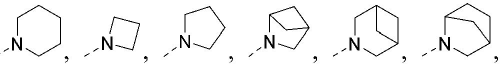

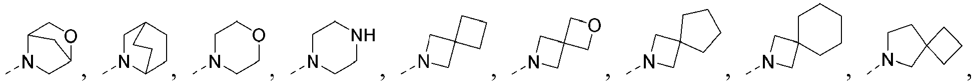

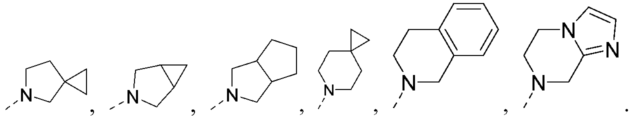

In some embodiments, R₅ₐ, R_{5b} together with the attached N atom form an optionally substituted 4- to 10-membered heterocycloalkyl, preferably the following groups optionally substituted by 1, 2 or 3 R₇:

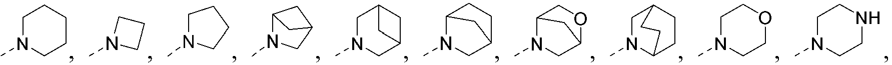

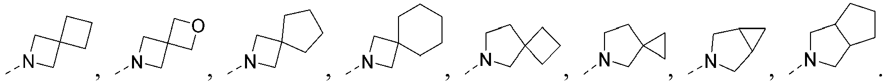

In some embodiments, R₇ is preferably selected from F, hydroxyl, methyl, methoxy, =CF₂, hydroxymethyl.

In some preferred embodiments, Ring A is selected from: 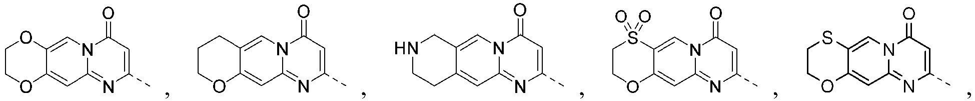 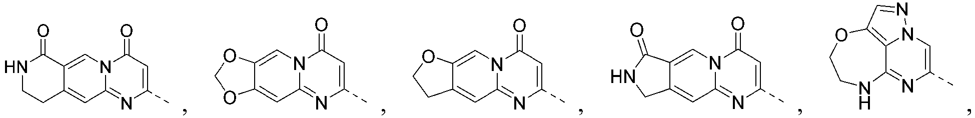 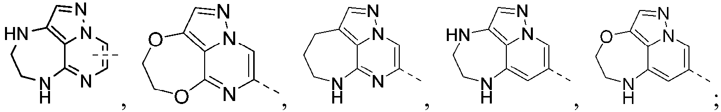 preferably 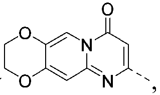 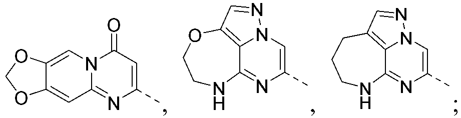 more preferably 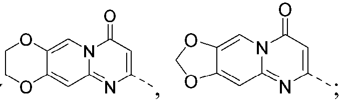
R₁ is oxo, or
R₁ is -Z-Rₐ;
Z is selected from a chemical bond;
Rₐ is selected from halogen, C₁₋₃ alkyl, C₁₋₃ alkoxy, halo-C₁₋₃ alkyl, halo-C₁₋₃ alkoxy; preferably F, methyl.

In some more preferred embodiments, L₁ is

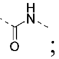

Preferably:
Ring B is selected from 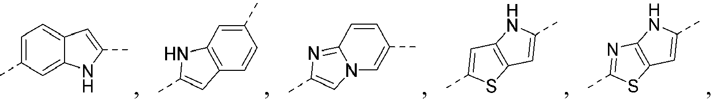 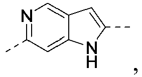 preferably 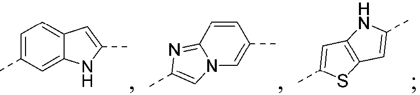
R₃ is independently selected from F, Cl, methyl, methoxy, trifluoromethyl, difluoromethyl, trifluoromethoxy, difluoromethoxy;
In some further preferred embodiments, Ring B is optionally substituted with 1, 2, or 3 F atoms;
Ring B and R₃ form 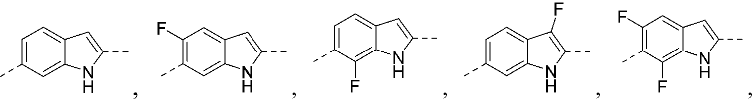 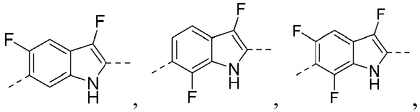 preferably

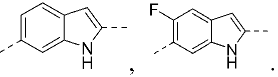

Preferably: R₆ is selected from the following groups optionally substituted by 1, 2 or 3 R₇:3- to 8-membered cycloalkyl, 4- to 8-membered heterocycloalkyl containing 1-2 heteroatoms selected from N, O, S, thienyl. The cycloalkyl or heterocycloalkyl may be in the form of monocyclic, bicyclic, fused, bridged, spiro, etc.

R₆ is more preferably selected from 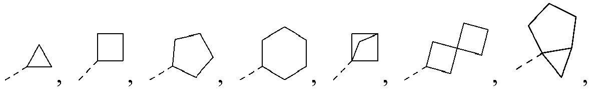 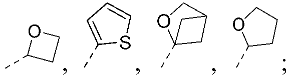
R₇ is selected from halogen, hydroxyl, C₁₋₃ alkyl, C₁₋₃ alkoxy, halo-C₁₋₃ alkyl, halo-C₁₋₃ alkoxy, =CF₂, preferably F, hydroxyl, methyl, trifluoromethyl, difluoromethyl, methoxy, trifluoromethoxy, =CF₂, more preferably F, methyl, trifluoromethyl, difluoromethyl, methoxy, trifluoromethoxy;
Preferably:
   R₅ₐ, R_{5b} together with the attached N atom preferably form the following groups optionally substituted by 1, 2 or 3 R₇: 4- to 10-membered heterocycloalkyl containing1-3 heteroatoms selected from N, O, S, 4- to 10-membered heterocyclyl containing1-3 heteroatoms selected from N, O, S;
   preferably 4- to 10-membered heterocycloalkyl containing1-3 heteroatoms selected from N, O, S, 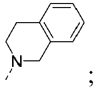 the cycloalkyl, heterocyclyl may be in the form of monocyclic, bicyclic, fused, bridged, spiro, etc;
   More preferably, R₅ₐ and R_{5b} together with the attached N atom form the following groups preferably optionally substituted by 1, 2 or 3 R₇: 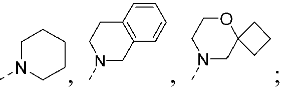
   R₇ is selected from halogen, hydroxyl, C₁₋₃ alkyl, C₁₋₃ alkoxy, halo-C₁₋₃ alkyl, halo-C₁₋₃ alkoxy, =CF₂, preferably F, hydroxyl, methyl, trifluoromethyl, difluoromethyl, methoxy, trifluoromethoxy, =CF₂, more preferably F, methyl, trifluoromethyl, difluoromethyl, methoxy, trifluoromethoxy.

In some preferred embodiments, the compound, or the deuteride, the stereo isomer, or the pharmaceutically acceptable salt, or the pharmaceutically acceptable solvate thereof, has the structure of formula (III): 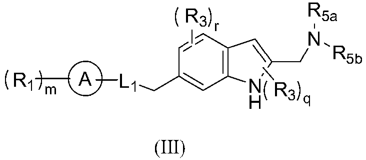 r and q are selected from 0, 1 or 2.

In some embodiments, the above compound may be a deuterated derivative, and the deuteration site may be any atom substituted by H atom. In some more preferred embodiments, the deuteration site is located at the C atom marked with "*", "**" or L₃; more preferably, the deuteration site is located at L₃.

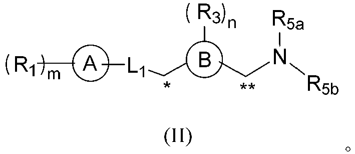

The compound of formula (I) includes the following specific compounds, or the deuteride, the stereo isomer, or the pharmaceutically acceptable salt, or the pharmaceutically acceptable solvate thereof:

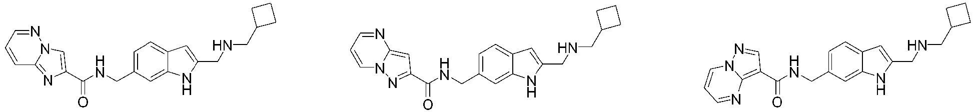

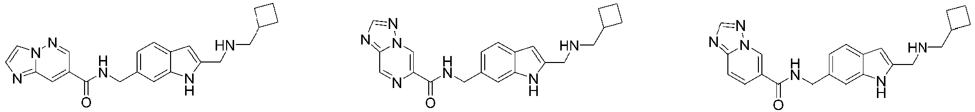

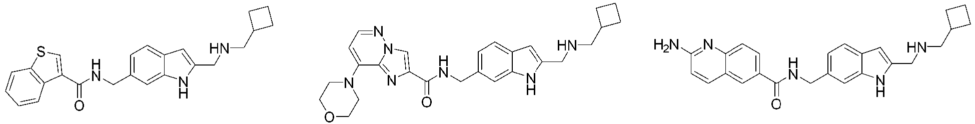

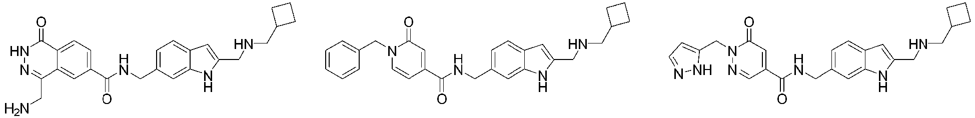

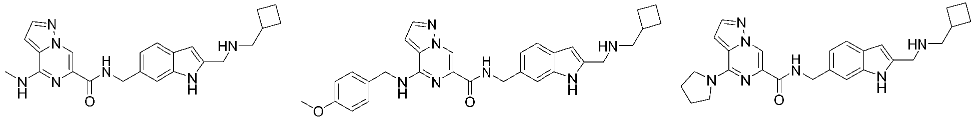

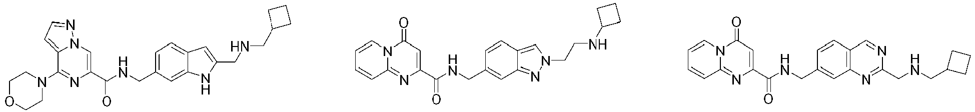

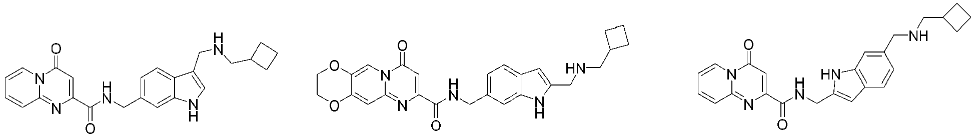

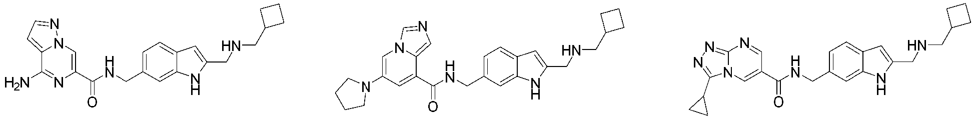

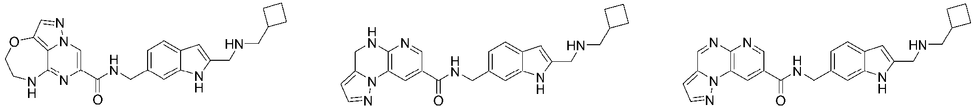

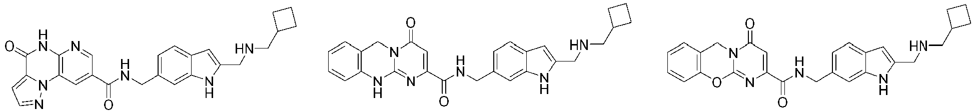

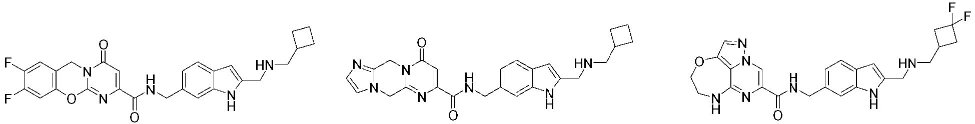

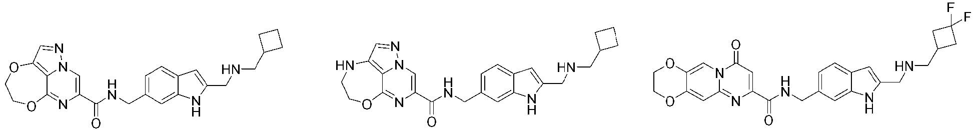

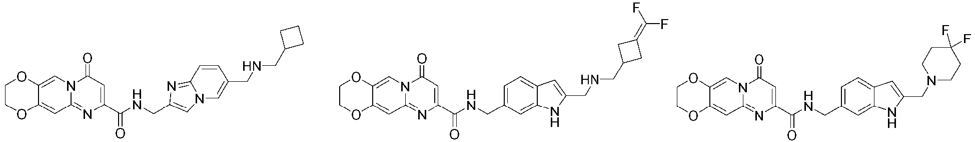

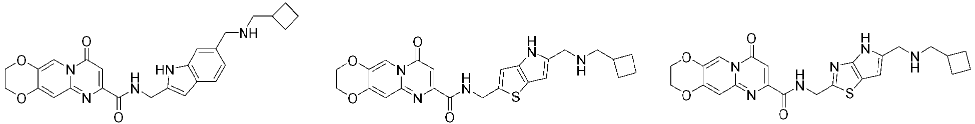

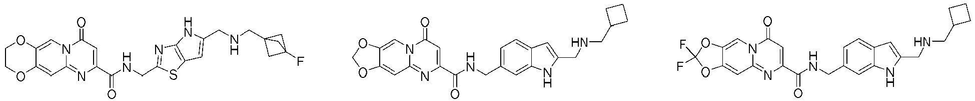

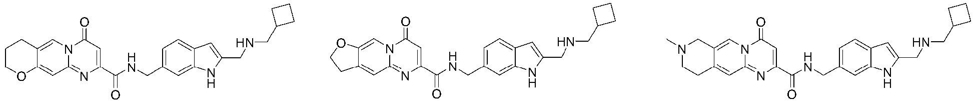

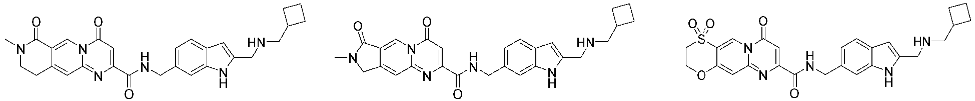

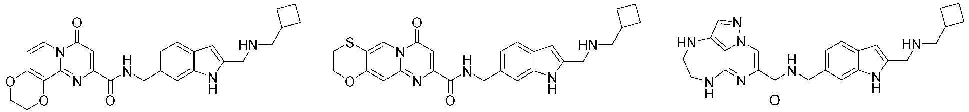

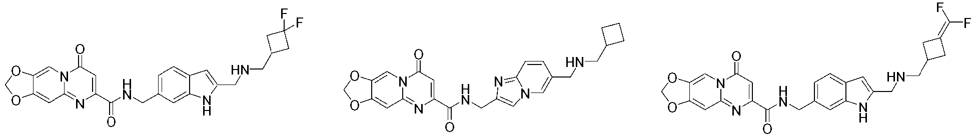

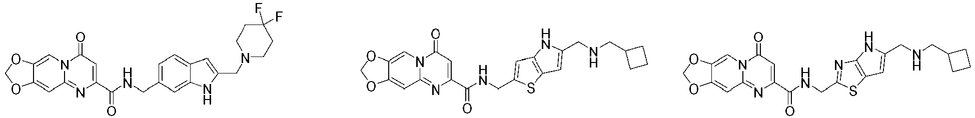

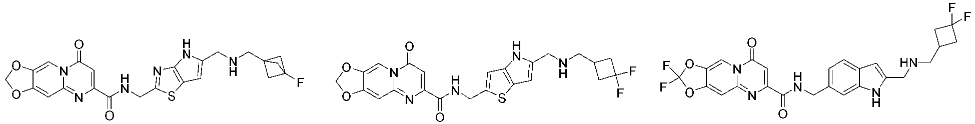

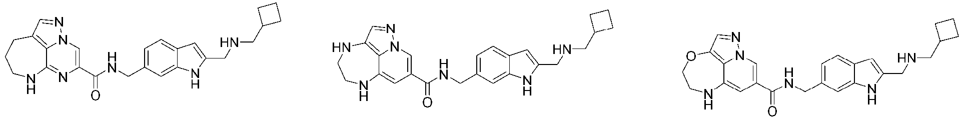

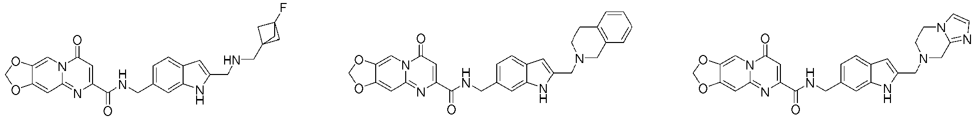

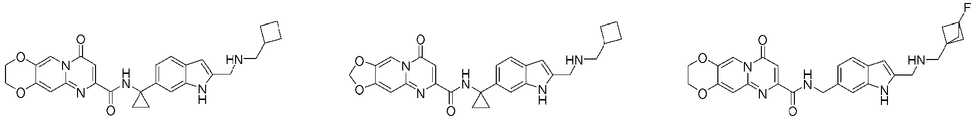

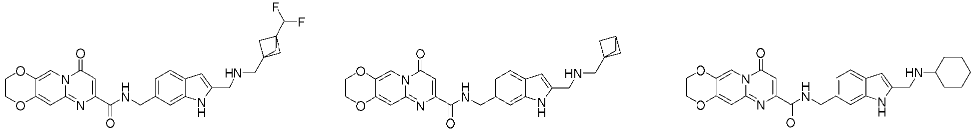

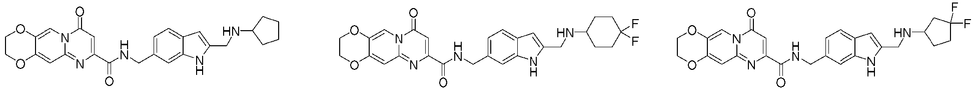

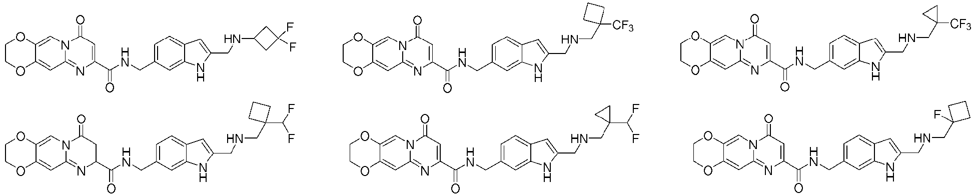

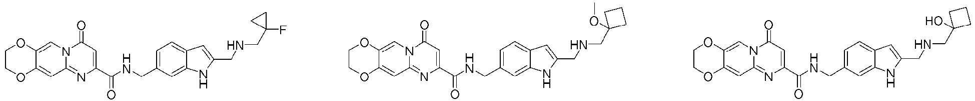

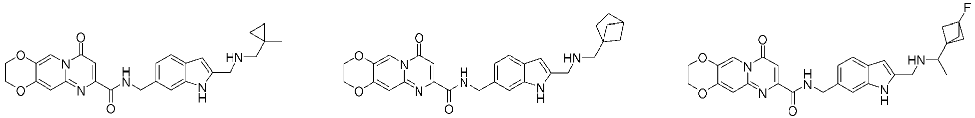

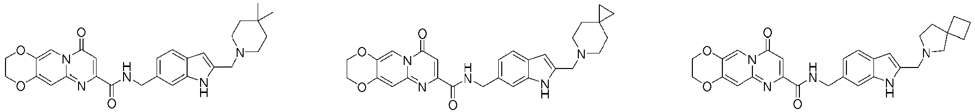

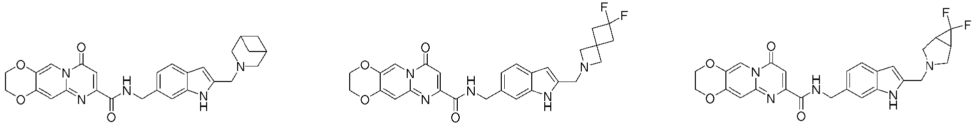

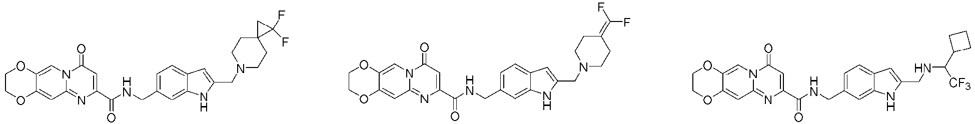

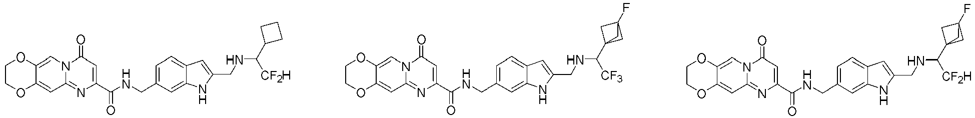

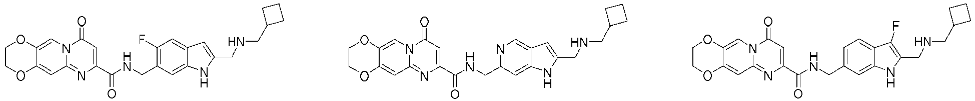

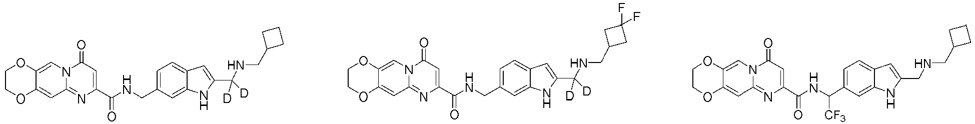

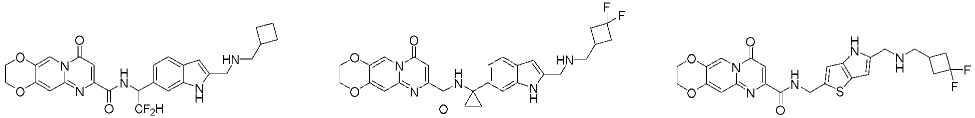

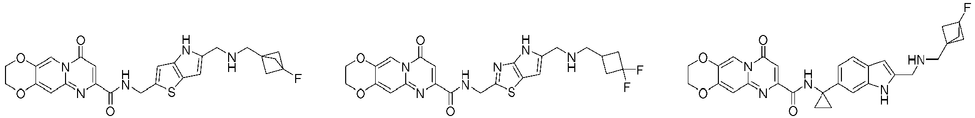

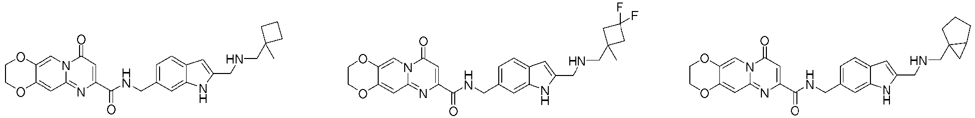

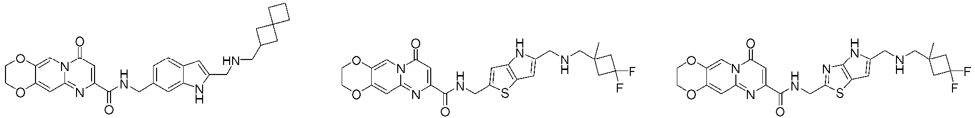

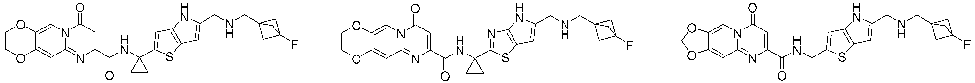

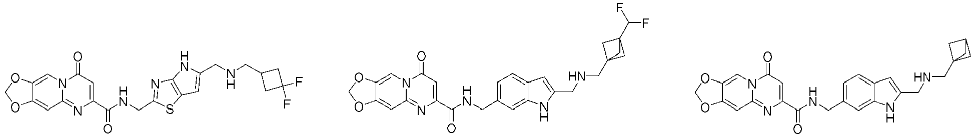

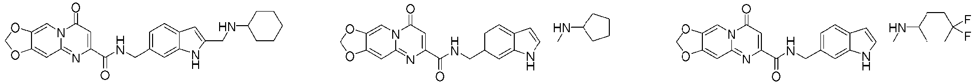

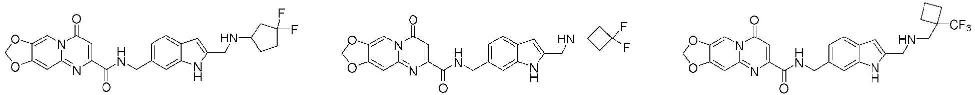

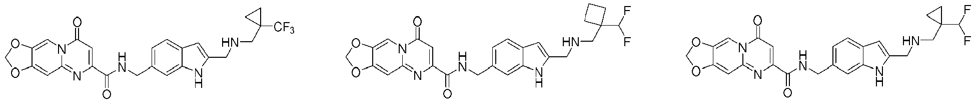

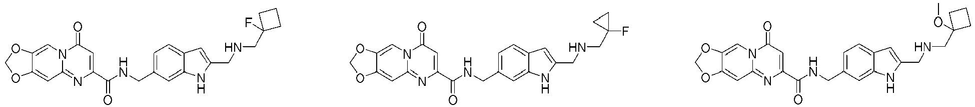

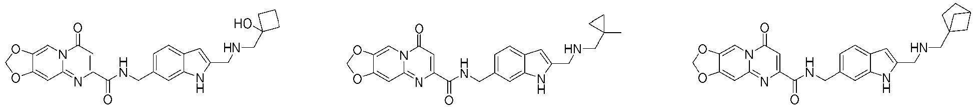

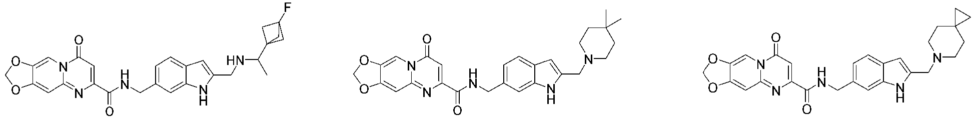

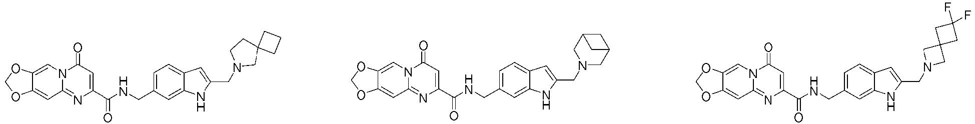

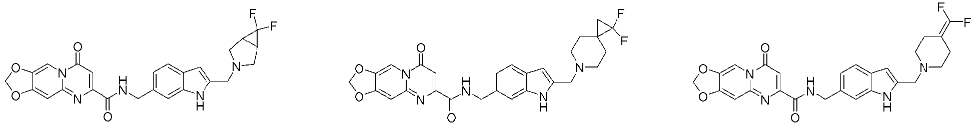

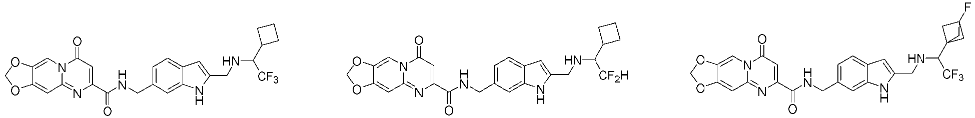

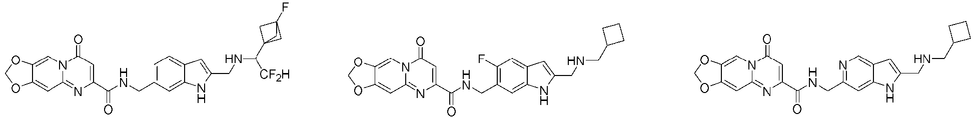

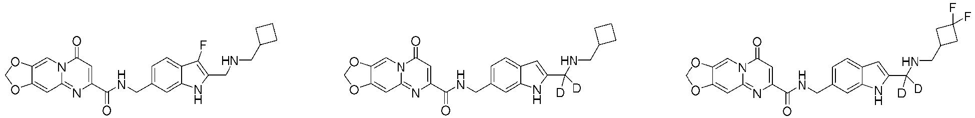

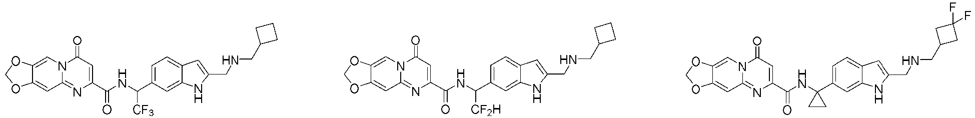

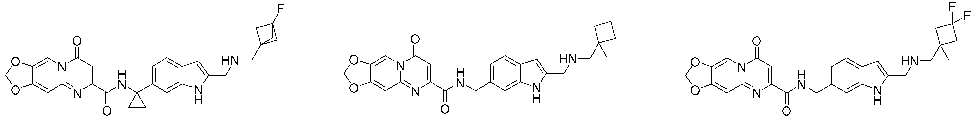

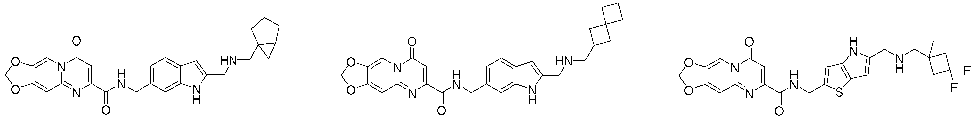

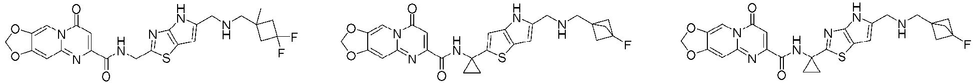

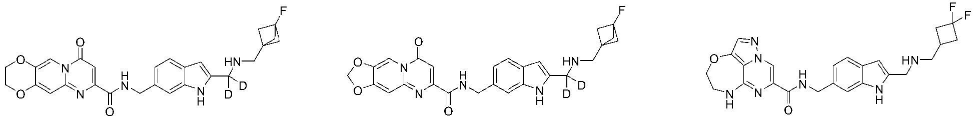

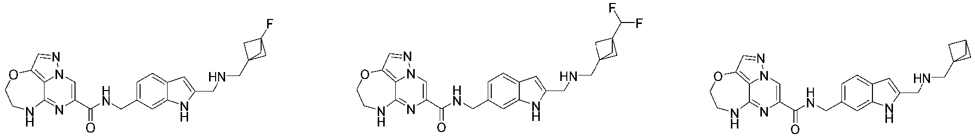

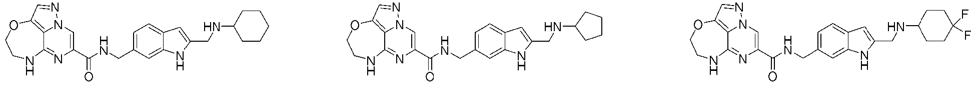

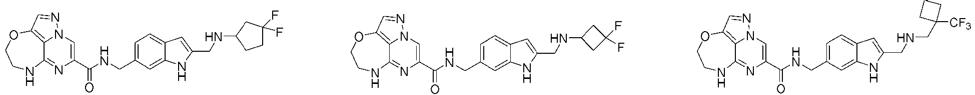

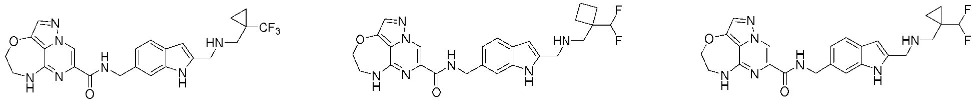

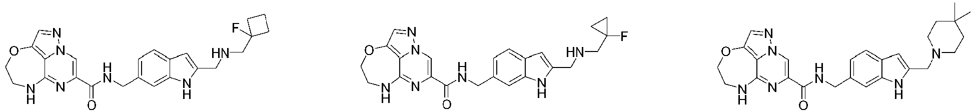

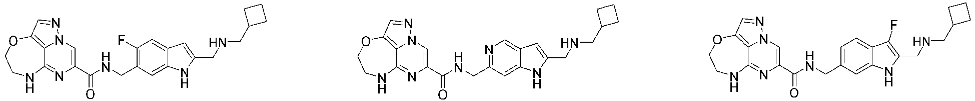

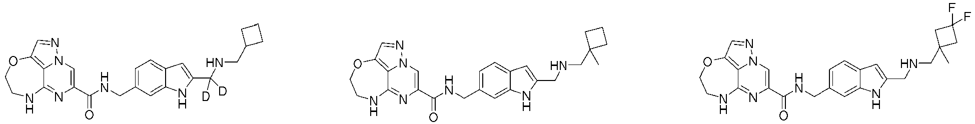

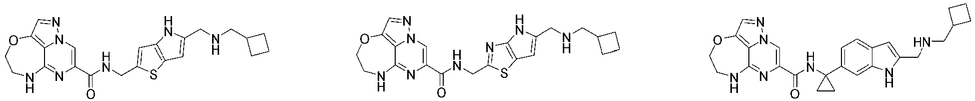

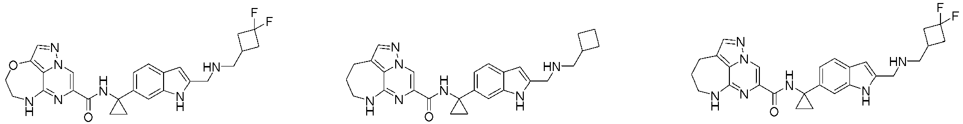

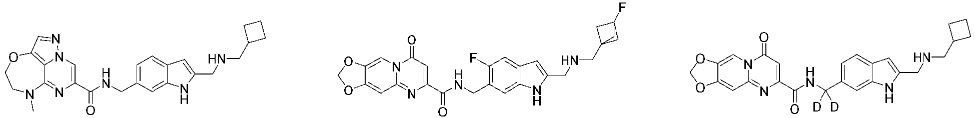

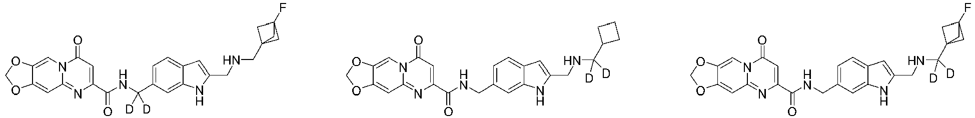

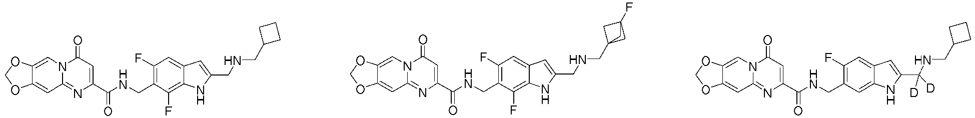

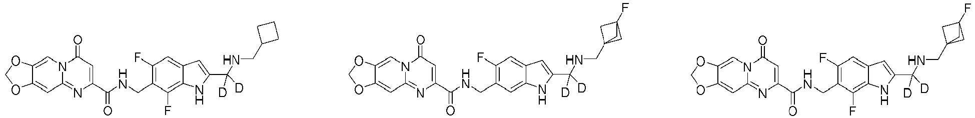

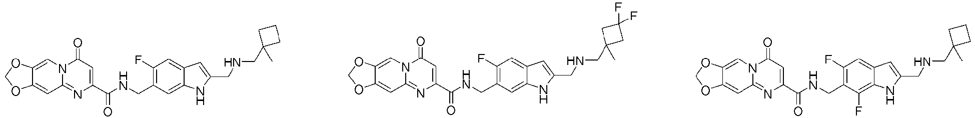

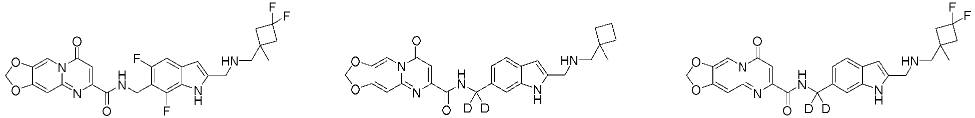

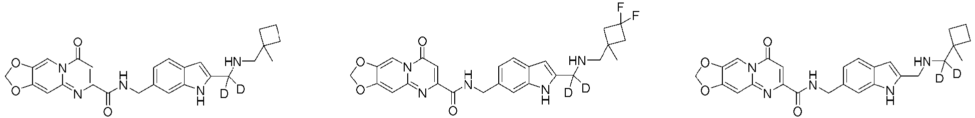

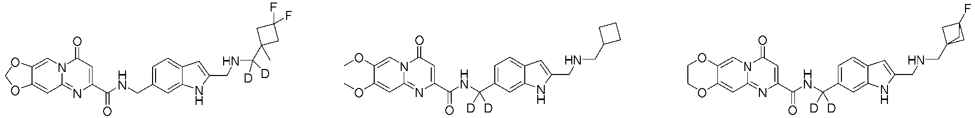

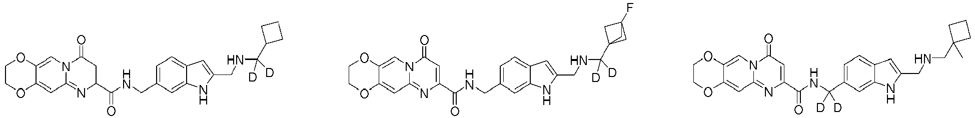

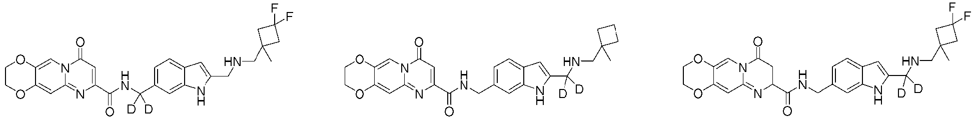

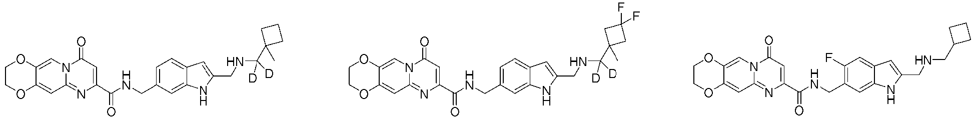

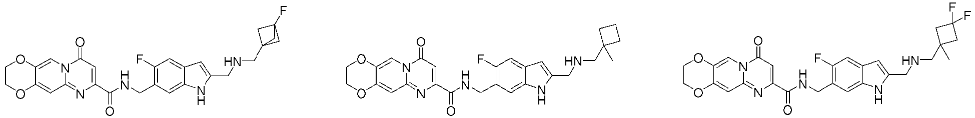

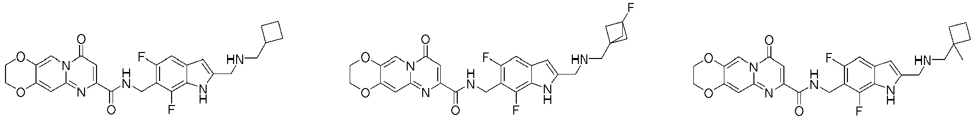

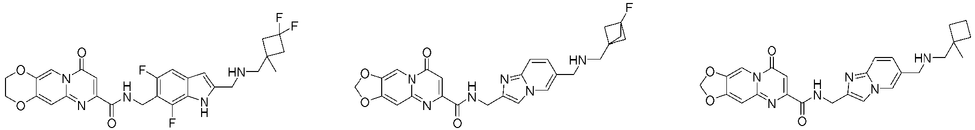

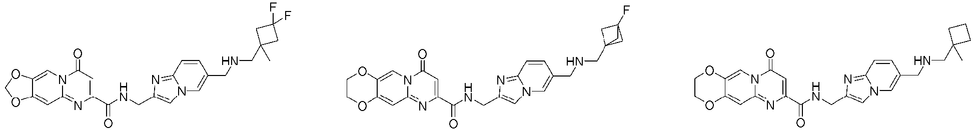

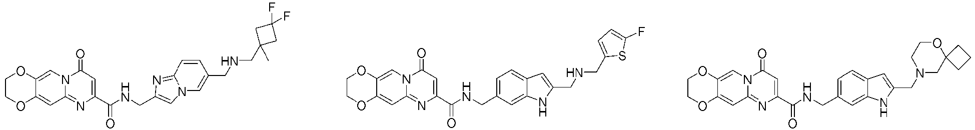

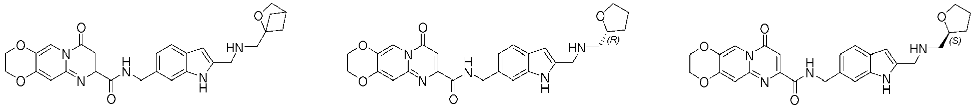

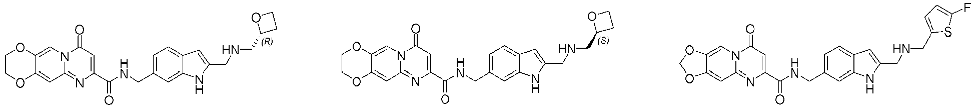

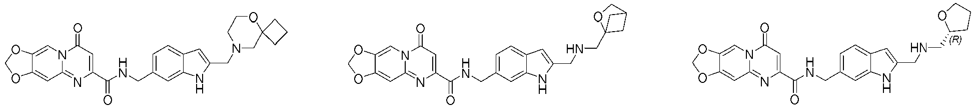

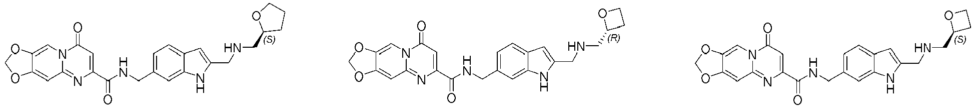

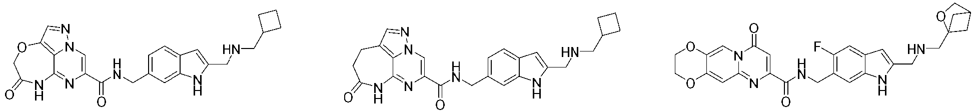

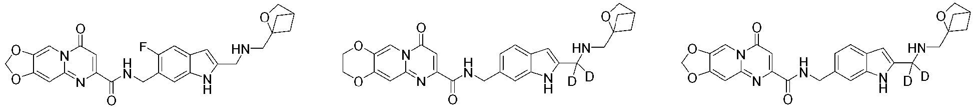

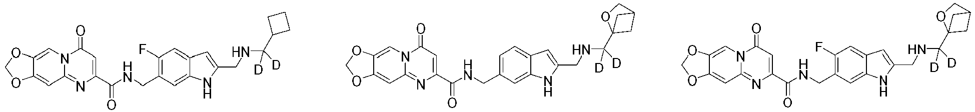

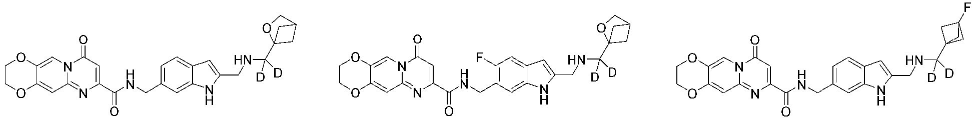

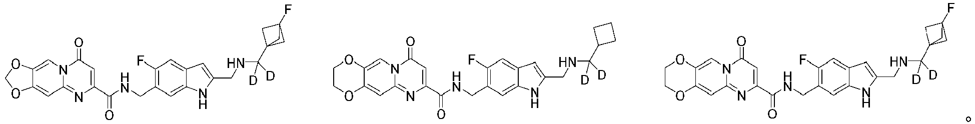

In another aspect, the present invention provides a pharmaceutical composition comprising the compound, or the deuteride, the stereo isomer, or the pharmaceutically acceptable salt, or the pharmaceutically acceptable solvate thereof according to any one of the above, and a pharmaceutically acceptable carrier thereof.

In another aspect, the present invention provides the use of the compound, or the deuteride, the stereo isomer, or the pharmaceutically acceptable salt, or the pharmaceutically acceptable solvate thereof according to any one of the above, or the above pharmaceutical composition in the preparation of a medicament for treating and/or preventing METTL3-related diseases.

Preferably, the METTL3-related diseases are selected from autoimmune diseases, neurological diseases, infectious diseases, and tumors.

Preferably, the METTL3-related diseases are selected from AML, myeloid leukemia, solid tumors such as hepatocellular carcinoma, colorectal cancer, and prostate cancer.

The compound provided by the present invention has strong efficacy, good pharmacokinetic properties and low toxic side effects, and is an ideal METTL3 inhibitor. The compound provided by the present invention has better METTL3 inhibitory activity. The compound of the present invention is an ideal high-activity METTL3 inhibitor, which can be used for the treatment and/or prevention of diseases, including AML, myeloid leukemia, solid tumors such as hepatocellular carcinoma, colorectal cancer, and prostate cancer.

Compared with the control drug, the tricyclic compound provided by the present invention has stronger METTL3 inhibitory activity(including kinase and cell), better pharmacokinetic properties (higher AUC and lower clearance rate), and lower toxic side effects.

### Detailed Description

The present invention will be further described in detail below with reference to specific examples, but the present invention is not limited to the following examples.

### Definitions

As used herein, "optional" or "optionally" means that any optional item may be selected or not selected. For example , "group A is optionally substituted with 1-3 groups B" includes four cases: group A is not substituted with group B, group A is substituted with 1 group B, group A is substituted with 2 groups B, and group A is substituted with 3 groups B.

As used herein, "substituted" or "substituted with" means that any one or more hydrogen atoms on any atom in a group or fragment are replaced by substituents, which may include deuterium and variants of hydrogen, as long as the valence state of the specific atom is normal and the substituted compound is stable. When the substituent is "oxo" (i.e., =O), it means that two hydrogen atoms are replaced by oxygen, and when the oxygen-substituted atom is a carbon atom, a carbonyl group (C=O) is formed. Oxygen substitution does not occur on aromatic groups.

As used herein, "optionally substituted" means that it may be substituted or not substituted. Unless otherw ise specified, the type and number of substituents may be arbitrary on the bas is of chemical feasibility. For example , "optionally substituted5- to 6-membered heterocyclyl" refers to a5- to 6-membered heterocyclylsubstituted or unsubstituted with any group.

When any variable (such as R) appears more than once in the composition or structure of a compound, its definition in each case is independent. Thus, for example , if a group is substituted with 0-2 R, the group may optionally be substituted with up to two R, and R in each case has independent options. In addition, combinations of substituents and/or their variants are only permitted if such combinations result in stable compounds.

When the number of a linking group is 0, such as -(CRₐR^{b})₀-, it means that the linking group is a single bond/chemical bond.

When one of the variables is selected from a chemical bond/single bond, it means that the two groups it connects are directly connected. For example , when L₁in A-L₁-R₁represents a single bond, it means that the structure is actually A-R₁.

As used herein, "chemical bond" generally refers to a covalent bond formed by sharing a pair of electrons between two atoms in a compound molecule.

When a substituent is vacant, it means that the substituent does not ex ist. For example , when X in A-X is vacant, it means that the structure is actually A. When a 1 isted substituent does not specify through which atom it is attached to the substituted group, such substituent can be bonded through any of its atoms. For example , pyridyl as a substituent can be attached to the substituted group through any carbon atom on the pyridine ring.

When the listed linking group does not specify its connection direction, its connection direction is arbitrary. For example , in ring A-L₁-R₁, the linking group L₁ is -M-W-, at this time -M-W- can connect ring A and R₁in the same direction as the left-to-right reading order to form ring A-M-W-R₁, or connect ring A and R₁ in the opposite direction to the left-to-right reading order to form ring A-W-M-R₁. Combinations of the linking groups, substituents and/or their variants are only permitted if such combinations result in stable compounds.

Unless otherw ise specified, when a group has one or more connectable sites, any one or more sites of the group can be connected to other groups through chemical bonds. When the connection mode of the chemical bond is not positioned and there are H atoms at the connectable sites, the number of H atoms at the site will decrease correspondingly with the number of connected chemical bonds to become a group with the corresponding valence when connecting the chemical bond. The chemical bond connecting the site to other groups can be represented by a straight solid bond " 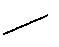 " and a dashed bond " 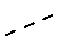 ". For example , the straight solid bond in -OCH₃indicates that it is connected to other groups through the oxygen atom in the group; the straight dashed bond in 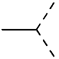 indicates that it is connected to other groups through both ends of the carbon atom in the group; the dashed line in 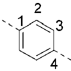 indicates that it is connected to other groups through the 1st and 4th carbon atoms in the phenyl group; 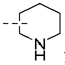 indicates that any connectable site on the piperidinyl group can be connected to other groups through one chemical bond, including at least the 4 connection modes: 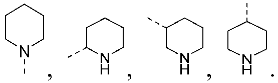 Even though an H atom is drawn on -N-, 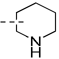 still includes the group with the connection mode of 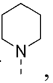 but when connecting one chemical bond, the H at the site will be reduced by 1 to become the corresponding monovalent piperidinyl group.

Unless otherw ise specified, in a fused ring group, the connection site of the group or fragment is located on the ring connected by the dashed line. For example , 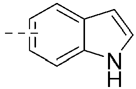 indicates that any connection site on the benzene ring of the group can be connected to other groups through one chemical bond, including at least the 4 connection modes:

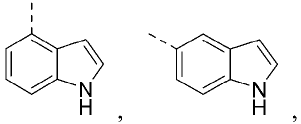

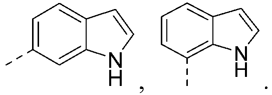

In general, in aromatic or heteroaromatic rings, double bonds Single bonds are not limiting. For example , 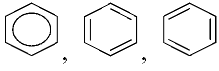 all refer to benzene ring or phenyl group, where the double bond or 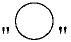 specifically refers to the delocalized large π bond on the benzene ring plane; in 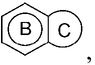 when ring C is defined as a 5-membered aryl or heteroaryl group, ring B and ring C form a conjugated fused ring aryl or fused ring heteroaryl group, and non-limiting examples thereof include 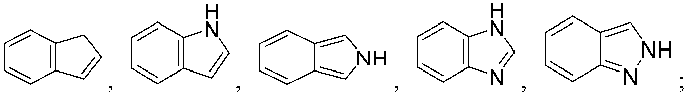 even though the left ring in 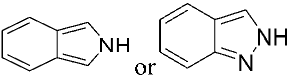 contains only two double bonds, 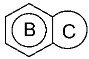 still includes groups such as 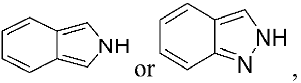 where the double bond represents the delocalized large π bond on the fused aromatic ring.

The numerical ranges used herein include the endpoint values and any numerical values between the endpoint values. For example , "0-3" may include 0, 1, 2 or 3, and "1-3" may include 1, 2 or 3. As used herein, "C₁₋ₙ" includes C₁₋₂, C₁₋₃, ..., C₁₋ₙ. For example , a "C₁₋₆" group means that this part has 1-6 carbon atoms, i.e., the group contains 1 carbon atom, 2 carbon atoms, 3 carbon atoms, 4 carbon atoms, 5 carbon atoms or 6 carbon atoms. Thus, for example , "C₁₋₄alkyl" refers to an alkyl group containing 1-4 carbon atoms, i.e., the alkyl group is selected from methyl, ethyl, propyl, isopropyl, n-butyl, isobutyl, sec-butyl and tert-butyl. Numerical ranges herein, such as "1-6", refer to each integer in the given range.
ring atoms refer to non-hydrogen atoms used for ring formation in cyclic groups. For example , the ring atoms in 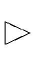 are 3 carbon atoms; the ring atoms in 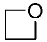 are three carbon atoms and 1 oxygen atom; the ring atoms in 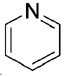 are 1 N atom and 5 carbon atoms; the ring atoms in 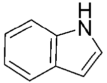 are 8 carbon atoms and 1 nitrogen atom.

As used herein, "n-m-membered" refers to the number of ring atoms in a cyclic group. For example , a "3- to 8-membered" group means that this part has 3-8 ring atoms, i.e., the group contains 3 ring atoms, 4 ring atoms, 5 ring atoms, 6 ring atoms, 7 ring atoms, or 8 ring atoms. Therefore, for example , "3- to 8-membered cycloalkyl" refers to a saturated cyclic alkyl group containing 3-8 carbon atoms, i.e., the alkyl group is selected from cyclopropyl, cyclobutyl, cyclopentyl, cyclohexyl, cycloheptyl, and cyclooctyl.

The term "alkyl" as used herein, alone or in combination, refers to an optionally substituted straight-chain or optionally substituted branched saturated aliphatic hydrocarbon. The "alkyl" herein preferably has 1-6 carbon atoms, for example , 1-5 carbon atoms, or 1-4 carbon atoms, or 1-3 carbon atoms. Non-limiting examples of alkyl groups include methyl, ethyl, n-propyl, isopropyl, 2-methyl-1-propyl, 2-methyl-2-propyl, 2-methyl-1-butyl, 3-methyl-1-butyl, 2-methyl-3-butyl, 2, 2-dimethyl-1-propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2, 2-dimethyl-1-butyl, 3, 3-dimethyl-1-butyl, 2-ethyl-1-butyl, n-butyl, isobutyl, sec-butyl, tert-butyl, n-pentyl, isopentyl, neopentyl, tert-pentyl, hexyl, etc. In the groups defined herein, when a numerical range appears for "alkyl", for example , "C₁-₆alkyl" refers to an alkyl group that can be composed of 1 carbon atom, 2 carbon atoms, 3 carbon atoms, 4 carbon atoms, 5 carbon atoms, or 6 carbon atoms, and the alkyl group herein also includes cases where the numerical range is not specified.Alkyl groups may be optionally substituted or unsubstituted.

The "alkyl" used in combination herein refers to an alkyl group connected to other groups, for example , the alkyl group in alkoxy, which has the same definition as when used alone.

The term "alkoxy" or "-O-alkyl" as used herein, alone or in combination, is represented as "alkyl-O-". Non-limiting examples of alkoxy groups include methoxy, ethoxy, n-propoxy, isopropoxy, n-butoxy, isobutoxy, sec-butoxy, tert-butoxy, etc.Alkoxy groups may be optionally substituted or unsubstituted.

The term "cycloalkyl" as used herein, alone or in combination, refers to saturated monocyclic, bicyclic, fused, bridged, spiro, and other carbocyclic rings. Herein, it is preferably a 3- to 12-membered cycloalkyl, more preferably a 3- to 10-membered cycloalkyl, and most preferably a 3-to 8-membered cycloalkyl. Non-limiting examples of monocyclic cycloalkyl groups include, but are not limited to, cyclopropyl, cyclobutyl, cyclopentyl, cyclohexyl, cycloheptyl, cyclooctyl and other cycloalkyl groups, which may be optionally substituted or unsubstituted.

The term "aryl" as used herein, alone or in combination, refers to an aromatic hydrocarbon ring group. The term "aryl" includes monocyclic aromatic hydrocarbons and polycyclic fused (condensed) ring aromatic hydrocarbons, where all fused ring systems (excluding any ring system that is part of an optional substituent or formed by an optional substituent) are aromatic. examples of aryl groups/moieties include phenyl, naphthyl, anthracenyl, and phenanthrenyl. Unless otherw ise specified, the term "aryl" does not include "heteroaryl".

The term "heterocyclyl" as used herein, alone or in combination, includes aliphatic heterocyclyl and heteroaryl, where one or more (such as one, two, three, or four) ring atoms are heteroatoms, such as oxyge N,Nitrogen, sulfur atoms, etc., including monocyclic, fused, bridged, Spiro rings. examples of heterocyclyl groups include heterocycloalkyl, heterocycloalkenyl, and heteroaryl as d iscussed below.Herein, a 3- to 10-membered monocyclic, bicyclic, or tricyclic heterocyclyl is preferred, which may contain 1, 2, or 3 ring atoms selected from nitrogen, oxygen, and/or sulfur. Non-limiting examples of "heterocyclyl" include azethenyl, azetidinyl, oxetanyl, thietanyl, pyrrolidinyl, 2-oxo-pyrrolidinyl, tetrahydrofuranyl, tetrahydrothienyl, pyrazolidinyl, imidazolidinyl, dioxolanyl, oxathiolanyl, piperidinyl, 2-oxo-piperidinyl, tetrahydropyranyl, thialkyl, piperazinyl, piperazin-2-one, dioxanyl, morpholinyl, thiomorpholinyl, 1, 1-dioxothiomorpholinyl, etc.Heterocyclyl groups may be optionally substituted or unsubstituted.

"Fused ring" in the definition of "heterocyclyl" refers to a heteroatom-containing polycyclic ring group containing two or more cyclic structures that sh are a pair of atoms with each other. Among them, one or more rings may contain several unsaturated bonds, or one ring may have an aromatic system with fully conjugated π electrons, where the ring atoms are heteroatoms selected from nitrogen, oxygen, or S(O)ₙ (where n is selected from 0, 1, or 2), and the remaining ring atoms are carbon. Herein, 6- to 12-membered is preferred, more preferably 8- to 9-membered. Depending on the number of constituent rings, it can be divided into bicyclic, tricyclic, tetracyclic, or polycyclic fused ring groups, preferably bicyclic or tricyclic, more preferably 5-membered/5-membered or 5-membered/6-membered bicyclic fused ring groups.

The term "heterocycloalkyl" as used herein, alone or in combination, refers to a saturated monocyclic, bicyclic, or polycyclic saturated heterocyclyl in which one or more (such as one, two, three, or four) ring atoms are heteroatoms, which may be spiro or bridged rings. Herein, it is preferably a 3- to 12-membered heterocycloalkyl, more preferably a 3- to 10-membered heterocycloalkyl, and most preferably a 3- to 8-membered heterocycloalkyl. Non-limiting examples of monocyclic heterocycloalkyl groups include, but are not limited to, propylene oxide, thiirane, aziridine, azetidine, oxetane, thietane, tetrahydrofuran, tetrahydrothiophene, tetrahydropyrrole, oxazolidine, thiazolidine, imidazolidine, tetrahydropyran, piperidine, dioxane, and azepane.

The term "heteroaryl" as used herein, alone or in combination, refers to a 5- to 12-membered (preferably 5- to 10-membered, more preferably 5- to 6-membered) monocyclic, bicyclic, or tricyclic system, where at least one ring is aromatic, and at least one ring contains one or more heteroatoms selected from nitrogen, oxygen, Sulfur, and the heteroaryl has one or more attachment points connected to the rest of the molecule. Non-limiting examples of "heteroaryl" includefuryl, imidazolyl, isoxazolyl, oxazolyl, pyrrolyl, pyrazolyl, pyridyl, pyrimidinyl, pyridazinyl, pyrazinyl, thienyl, thiazolyl, etc.; also include the following bicyclic rings, but not limited to these bicyclic rings: benzimidazolyl, benzofuryl, benzothienyl, indolyl, oxindolyl, indolinyl, imidazopyridyl, pyrazolopyridyl, pyrazolopyrimidinyl, quinolinyl, isoquinolinyl, quinazolinyl, indazole, 1, 8-naphthyridine, benzo[d] isoxazole, benzo[d]thiazole, pyrrolo[3, 2-b]pyridine, furo[3, 2-b]pyridine, pyrrolo[1, 2-b]pyridazine, imidazo[1, 2-b]pyridazine, pyrazolo[1, 5-a]pyrimidine, thiazolo[4, 5-c]pyridine, thieno[3, 2-b]pyridine, pyrrolo[1, 2-b]pyridazine, 2, 3-dihydrobenzofuran, benzo[c][1, 2, 5]oxadiazole, 1, 3-dihydro-2H-benzo[d]imidazol-2-one, benzo[d]oxazol-2(3H)-one, etc. Heteroaryl groups may be optionally substituted or unsubstituted.

The term "halogen" as used herein, alone or in combination, refers to fluorine, chlorine, bromine, or iodine.

The term "hydroxyl" as used herein, alone or in combination, refers to -OH.

The term "cyano" as used herein, alone or in combination, refers to -CN.

The term "(substituted)" or "substituted with" as used herein means that one or more hydrogens on a specific atom are replaced by a specified group (such as halogen, alkyl, etc.), provided that the normal valence of the specified atom is not exceeded under ex isting conditions, and the substitution results in a stable compound.

The term "pharmaceutically acceptable salt" as used herein is well known to those skilled in the art. The term"pharmaceutically acceptable" as used herein refers to a substance (such as a carrier or diluent) that does not affect the biological activity or properties of the compound of the present invention and is relatively non-toxic, i.e., the substance can be admin istered to an individual without causing adverse biological reactions or interacting in an adverse manner with any component contained in the composition.

The term "pharmaceutical composition" as used herein refers to a biologically active compound optionally mixed with at least one pharmaceutically acceptable chemical component, including but not limited to carriers, stabilizers, diluents, d ispersants, suspending agents, thickeners, and/or excipients.

The term "carrier" as used herein refers to a relatively non-toxic chemical compound or reagent that helps introduce the compound into cells or t issues.

The term "stereo isomer" as used herein includes, but is not limited to, enantiomers, cis-trans isomers, and the like.

The term "enantiomer" as used herein refers to the phenomenon of isomer ism in compounds with the same molecular formula caused by different spatial configurations of atoms or atomic groups ( groups). Two compounds that are enantiomers of each other are mirror images and cannot overlap. The term "cis-trans isomer" as used herein generally refers to the stereo isomer ism phenomenon of diastereomer ism in compound molecules where the arrangement of groups in space is different due to the restriction of free rotation. Organic molecules containing such isomer ism, such as alkenes, azo compounds, alicyclic hydrocarbons, etc., are regarded as cis-trans isomers. In this application, cis-trans isomer ism is mainly embodied in the form of alicyclic hydrocarbons. For example , in cyclohexane, when cyclohexane is substituted by two substituents, cis-trans isomer ism occurs: when the two substituents are substituted on the same side of the ring, it is a "c is" isomer; when on different sides, it is a "trans" isomer.

The compounds of the present invention may contain asymmetric centers or chiral centers, thus ex isting in different stereo isomeric forms. It is expected that all stereo isomeric forms of the compounds of the present invention, including but not limited to diastereomers, enantiomers, atrop isomers, and geometric (conformational) isomers, and mixtures thereof, such as racemic mixtures, are within the scope of the present invention.

Unless otherw ise specified, the structures described in the present invention also include all isomers of the structure (e.g., diastereomers, enantiomers, cis-trans isomers, atrop isomers, geometric (conformational) isomeric forms), for example , R and S configurations at each asymmetric center, (Z) and (E) double bond isomers, cis-trans isomer ism of aliphatic cyclic hydrocarbons, atrop isomers of biphenyl structures (see "Basic Organic Chem istry" (2nd Edition), Volume 1, Xing Qiyi et al., p104-105); PAC, 1996, 68, 2193. (Basic terminology of stereochem istry (IUPAC Recommendations 1996, on page 2201 )), (Z) and (E) conformational isomers. Therefore, individual stereo isomers of the compounds of the present invention, as well as mixtures of enantiomers, diastereomers, atrop isomers, and geometric (conformational) isomers, are within the scope of the present invention.

In some preferred embodiments, the compound,or the deuteride, the stereo isomer, or the pharmaceutically acceptable salt, or the pharmaceutically acceptable solvate thereof, has the structure of formula (II): 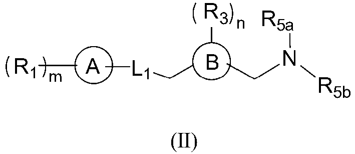 wherein each group is defined as previously described (in the claims or the summary of the invention); when L₁ is 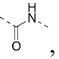 the compound has the structure of general formula (IV):

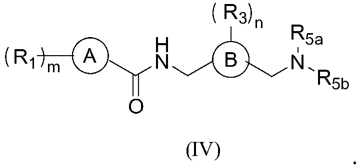

For compounds of general formula (IV), the following method can generally be used for synthesis:

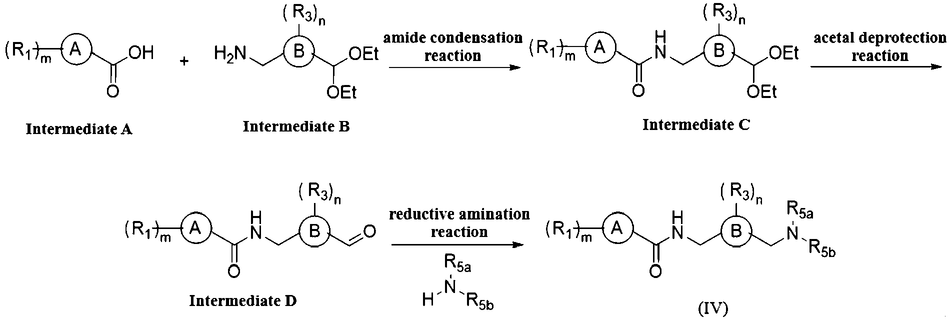

Intermediate A and Intermediate B undergo an amide condensation reaction in the presence of a condensing reagent to obtain Intermediate C; Intermediate C undergoes an acetal deprotection reaction under acidic conditions to obtain Intermediate D; Intermediate D and an amine raw material undergo a reductive amination reaction under reducing conditions to obtain the compound of general formula (IV).

### Step 1: Synthes is of Intermediate C.

Intermediate A and Intermediate B undergo an amide condensation reaction in the presence of a condensing reagent to obtain Intermediate C. The specific reaction conditions are as follows:

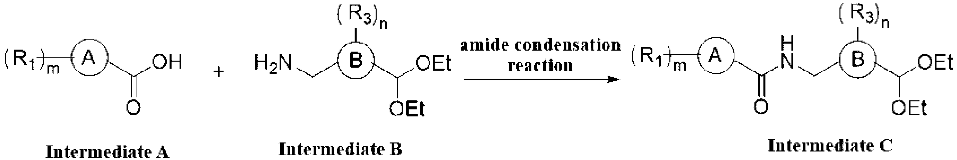

Dissolve Intermediate A and Intermediate B in a solvent, add a condensing reagent and a base, react at room temperature or above room temperature, and monitor the reaction by TLC, LCMS, or other methods until completion to obtain Intermediate C. The solvent, condensing reagent, and base can be selected from common amide condensation reaction systems. In some specific embodiments, the solvent can be selected from N,N-dimethylformamide, dichloromethane, dichloroethane, tetrahydrofuran, or combinations thereof. In some specific embodiments, the condensing reagent can be HATU, HBTU, T3P, T4P, CDI, EDCI, PyBOP, or phosphorus oxychloride. In some specific embodiments, the base can be N,N-diisopropylethylamine, triethylamine, potassium carbonate, or pyridine. When the reaction temperature is above room temperature, the reflux temperature of the solvent or a temperature slightly lower than the reflux temperature can also be selected, specifically any temperature in the range of 50-80°C.

### Step 2: Synthes is of Intermediate D.

Intermediate C undergoes an acetal deprotection reaction under acidic conditions to obtain Intermediate D. The specific reaction conditions are as follows:

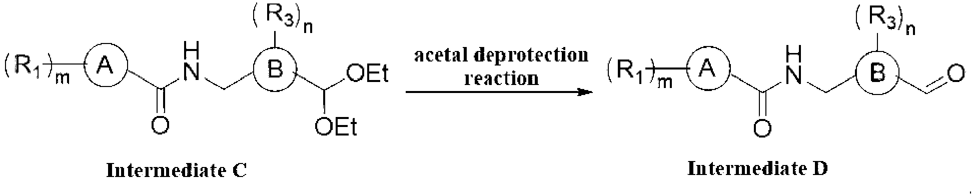

Dissolve Intermediate C in a solvent, add water and an acid reagent, react at room temperature or above room temperature, and monitor the reaction by TLC, LCMS, or other methods until completion to obtain Intermediate D. The solvent and acid reagent can be selected from common acetal deprotection reaction systems. In some specific embodiments, the solvent can be tetrahydrofuran, water, or a combination thereof. In some specific embodiments, the acid reagent can be acetic acid, p-toluenesulfonic acid, or hydrochloric acid. When the reaction temperature is above room temperature, the reflux temperature of the solvent or a temperature slightly lower than the reflux temperature can also be selected, specifically any temperature in the range of 40-80°C. Step 3: Synthes is of the compound represented by general formula (IV).

Intermediate D and an amine raw material undergo a reductive amination reaction under reducing conditions to obtain the compound of general formula (IV). The specific reaction conditions are as follows:

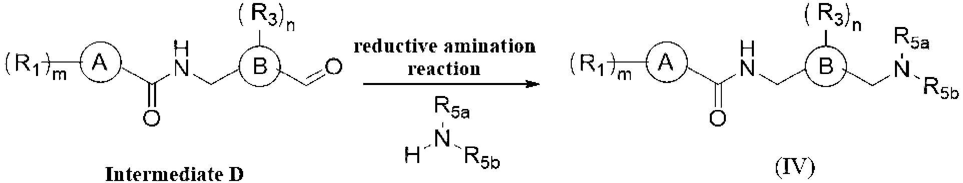

Dissolve Intermediate D and the amine raw material in a solvent, add an acid catalyst, then add a reducing agent, react at room temperature or above room temperature, and monitor the reaction by TLC, LCMS, or other methods until completion to obtain the compound of general formula (IV). The solvent, acid catalyst, and reducing agent can be selected from common reductive amination reaction systems. In some specific embodiments, the solvent can be methanol, *N,N-*dimethylformamide, or a combination thereof. In some specific embodiments, the acid catalyst can be acetic acid. In some specific embodiments, the reducing agent can be sodium cyanoborohydride, sodium triacetoxyborohydride, or sodium borohydride. When the reaction temperature is above room temperature, the reflux temperature of the solvent or a temperature slightly lower than the reflux temperature can also be selected, specifically any temperature in the range of 40-80°C. The present invention will be further described in detail below with reference to specific examples, but the present invention is not limited to the following examples.

Some preparation conditions used in the examples are as follows:
Preparation Prep-HPLC conditions: Instrument: GILSON-GX281; Wavelength: 220 nm & 254 nm; Column model: Waters X-bridge (30×100 mm, 10 µm) or Luna C18 (30×75 mm, 3 µm) or Luna C18 (30×75 mm, 3 µm); Mobile phase: A: 10 mM ammonium bicarbonate or H₂O (0.1% formic acid) or H₂O (0.1% trifluoroacetic acid), B: acetonitrile; Run time: 15 min; Flow rate: 25 mL/min. Reverse-phase column purification uses a C18 reverse-phase silica gel column (Spherical C18, 40-60 µm, 40 g-120 g) with water/acetonitrile (95/5~30/70) as the mobile phase.

The synthetic steps of some intermediates in the present invention are as follows:

### Intermediate 1: tert-Butyl 6-(aminomethyl)-2-(((tert-butoxycarbonyl)(cyclobutylmethyl)amin o)methyl)-1H-indole-1-carboxylate

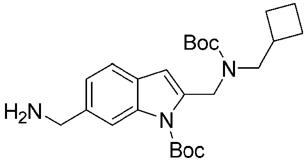

### (1) 3-Amino-4-iodobenzonitrile

4-Iodo-3-nitrobenzonitrile (10 g, 36.5 mmol), iron powder (6.1 g, 109.5 mmol), and ammonium chloride (5.9 g, 109.5 mmol) were dissolved in a mixed solvent of ethanol/water (80 mL/20 mL), and the reaction was stirred under reflux at 80°C for 16 hours. The reaction solution was filtered, the filtrate was concentrated, water (50 mL) was added, and the aqueous phase was extracted with ethyl acetate (30 mL x 3). The organic phases were combined, washed with saturated sodium chloride solution (50 mL), dried over anhydrous sodium sulfate, and concentrated. The residue was purified by silica gel column chromatography (petroleum ether/ethyl acetate = 90/10) to obtain the title compound (4.3 g, pale yellow solid), yield: 48%. MS (ESI): m/z 244.9 [M+H]⁺.

### (2) 3-Amino-4-(3,3-diethoxyprop-1-yn-1-yl)benzonitrile

3-Amino-4-iodobenzonitrile (4.2 g, 17 mmol), bis(triphenylphosphine)palladium dichloride (119 mg, 0.17 mmol), triphenylphosphine (89 mg, 0.34 mmol), cuprous iodide (65 mg, 0.34 mmol), and triethylamine (5.2 g, 51 mmol) were sequentially added to tetrahydrofuran (40 mL). The mixture was stirred at room temperature under nitrogen protection for 20 minutes. Then a solution of 3,3-diethoxyprop-1-yne (2.6 g, 20 mmol) in tetrahydrofuran (20 mL) was slowly added, and the reaction was continued to stir at room temperature under nitrogen protection for 16 hours. The reaction solution was concentrated, and the residue was purified by silica gel column chromatography (petroleum ether/ethyl acetate = 90/10) to obtain the title compound (3.8 g, yellow oil), yield: 90%. MS (ESI): m/z 199.0 [M+H-EtOH]⁺.

### (3) 2-(Diethoxymethyl)-1H-indole-6-carbonitrile

3-Amino-4-(3,3-diethoxyprop-1-yn-1-yl)benzonitrile (3.3 g, 13.5 mmol)was dissolved in tetrahydrofuran (30 mL), and 1 M lithium bis(trimethylsilyl)amide (27 mL, 27 mmol) was slowly added dropwise at 0°C. After the dropwise addition, the reaction solution was stirred under reflux at 75°C for 4 hours. Water (60 mL) was added to the reaction solution, and the aqueous phase was extracted with ethyl acetate (50 mL x 3). The organic phases were combined, washed with saturated sodium chloride solution (50 mL), dried over anhydrous sodium sulfate, and concentrated. The residue was purified by silica gel column chromatography (petroleum ether/ethyl acetate = 93/7) to obtain the title compound (1.6 g, white solid), yield: 58%. MS (ESI): m/z 245.0 [M+H]⁺. ¹H NMR (400 MHz, DMSO-*d*₆) δ 11.68 (s, 1H), 7.80 (s, 1H), 7.69 (d, *J* = 8.0 Hz, 1H), 7.32 (d, *J* = 8.4 Hz, 1H), 6.57 (s, 1H), 5.77 (s, 1H), 3.61-3.58 (m, 4H), 1.21-1.17 (m, 6H).

### (4) 2-Formyl-1H-indole-6-carbonitrile

2-(Diethoxymethyl)-1H-indole-6-carbonitrile (200 mg, 0.8 mmol) and acetic acid (0.5 mL) were added to a mixed solvent of tetrahydrofuran/water (5 mL/0.5 mL), and the reaction was stirred at room temperature for 2 hours. The reaction solution was concentrated, water (10 mL) was added, and the aqueous phase was extracted with ethyl acetate (10 mL x 3). The organic phases were combined, washed with saturated sodium chloride solution (10 mL), dried over anhydrous sodium sulfate, and concentrated to obtain the title compound (130 mg, yellow solid), yield: 94%. MS (ESI): m/z 171.0 [M+H]⁺.

### (5) 2-(((Cyclobutylmethyl)amino)methyl)-1H-indole-6-carbonitrile

2-Formyl-1H-indole-6-carbonitrile (250 mg, 1.5 mmol) and cyclobutylmethylamine (383 mg, 4.5 mmol) were dissolved in dichloroethane (5 mL), and the mixture was heated to 65°C stirred for 1 hour. Then sodium cyanoborohydride (331 mg, 5.25 mmol) was added, stirring was continued at 65°C for 2 hours. The reaction solution was concentrated, saturated sodium bicarbonate solution (20 mL) was added, and the aqueous phase was extracted with ethyl acetate (20 mL x 3). The organic phases were combined, washed with saturated sodium chloride solution (20 mL x 3), dried over anhydrous sodium sulfate, and concentrated. The residue was purified by silica gel column chromatography (petroleum ether/ethyl acetate = 95/5) to obtain the title compound (200 mg, green oil), yield: 57%. MS (ESI): m/z 240.1 [M+H]⁺.

### (6) tert-Butyl 2-(((tert-butoxycarbonyl)(cyclobutylmethyl)amino)methyl)-6-cyano-1H-indole-1-carboxylate

2-(((Cyclobutylmethyl)amino)methyl)-1H-indole-6-carbonitrile (200 mg, 0.84 mmol) and sodium hydride (224 mg, 3.36 mmol, 60%) were dissolved in tetrahydrofuran (10 mL) and stirred at room temperature for 5 minutes. Then di-tert-butyl dicarbonate (732 mg, 3.36 mmol) was added, and the reaction was continued to stir at room temperature for 4 hours. Water (20 mL) was added to the reaction solution, and the aqueous phase was extracted with ethyl acetate (20 mL x 3). The organic phases were combined, washed with saturated sodium chloride solution (20 mL), dried over anhydrous sodium sulfate, and concentrated. The residue was purified by silica gel column chromatography (petroleum ether/ethyl acetate = 95/5) to obtain the title compound (300 mg, colorless oil), yield: 82%. MS (ESI): m/z 340.1 [M+H-100]⁺.

### (7) tert-Butyl 6-(aminomethyl)-2-(((tert-butoxycarbonyl)(cyclobutylmethyl)amino)methyl)-1H-indole-1-carboxylate

2-(((tert-Butoxycarbonyl)(cyclobutylmethyl)amino)methyl)-6-cyano-1H-indole-1-carboxylate (300 mg, 0.68 mmol) and Raney nickel (30 mg, 10%) were added to a methanol solution of ammonia (10 mL). Hydrogen was replaced 3 times, and the reaction was stirred at room temperature under a hydrogen atmosphere for 16 hours. Raney nickel was removed by filtration, and the filtrate was concentrated to obtain the title compound (300 mg, green oil, containing approximately 10% Intermediate 2), yield: 99%. MS (ESI): m/z 444.1 [M+H]⁺.

### Intermediate 2: tert-Butyl ((6-(aminomethyl)-1H-indol-2-yl)methyl)(cyclobutylmethyl)carbamate

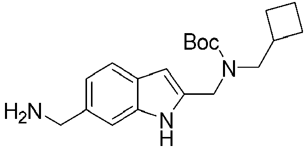

2-(((tert-Butoxycarbonyl)(cyclobutylmethyl)amino)methyl)-6-cyano-1H-indole-1-carboxylate (2.5 g, 5.7 mmol) and Raney nickel (250 mg, 10%) were added to a methanol solution of ammonia (50 mL). Hydrogen was replaced 3 times, and the reaction was stirred at room temperature under a hydrogen atmosphere for 16 hours. Raney nickel was removed by filtration, and the filtrate was concentrated to obtain the title compound (2 g, green oil, containing approximately 20% Intermediate 1), yield: 99%. MS (ESI): m/z 344.1 [M+H]⁺.

### Intermediate 3: 4-Oxo-4H-pyrido[1,2-a]pyrimidine-2-carboxylic acid

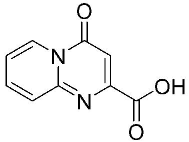

Methyl 4-oxo-4H-pyrido[1,2-a]pyrimidine-2-carboxylate (2 g, 10 mmol) was dissolved in a mixed solvent of methanol/water (20 mL/4 mL), sodium hydroxide (1.2 g, 30 mmol) was added, and the mixture was stirred at room temperature for 2 hours. The reaction solution was concentrated, diluted with water, adjusted to acidic pH with dilute hydrochloric acid, allowed standing and for 1 hour to precipitate the solid, filtered, and the solid was dried to obtain the title compound (1.6 g, white solid), yield: 86%. MS (ESI): m/z 191.0 [M+H]⁺.

### Intermediate 4: 9-Oxo-2,3-dihydro-9H-[1,4]dioxino[2',3':4,5]pyrido[1,2-a]pyrimidine-7-carboxylic acid

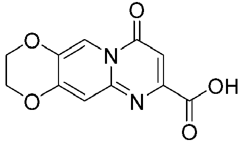

### (1) 2-((6-Bromo-4-iodopyridin-3-yl)oxy)ethan-1-ol

At 0°C, 2-bromo-5-fluoro-4-iodopyridine (3.7 g, 12.26 mmol), ethylene glycol (3.8 g, 61.28 mmol), and sodium tert-butoxide (1.51 g, 13.48 mmol) were dissolved in N,Ndimethylformamide (30 mL), and the mixture was heated to 80°C and stirred for 1 hour. The reaction solution was added to water (100 mL), and the aqueous phase was extracted with ethyl acetate (100 mL x 2). The organic phases were combined, washed with saturated sodium chloride solution (50 mL x 3), dried over anhydrous sodium sulfate, and concentrated. The residue was purified by silica gel column chromatography (petroleum ether/ethyl acetate = 60/40) to obtain the title compound (1.0 g, pink solid), yield: 24%. MS (ESI): m/z 343.8 [M+H]⁺.

### (2) 7-Bromo-2,3-dihydro-[1,4]dioxino[2,3-c]pyridine

2-((6-Bromo-4-iodopyridin-3-yl)oxy)ethan-1-ol (1.0 g, 2.91 mmol) was dissolved in isopropanol (20 mL), and potassium tert-butoxide (490 mg, 4.36 mmol), 3,4,7,8-tetramethyl-1,10-phenanthroline (69 mg, 0.29 mmol), and cuprous iodide (34 mg, 0.17 mmol) were added. The mixture was heated to 80°C and stirred for 1 hour. The reaction solution was added to water (20 mL), and saturated ammonium chloride solution (10 mL) was added. The aqueous phase was extracted with ethyl acetate (40 mL x 2). The organic phases were combined, washed with saturated sodium chloride solution (30 mL x 3), dried over anhydrous sodium sulfate, and concentrated. The residue was purified by silica gel column chromatography (petroleum ether/ethyl acetate = 85/15) to obtain the title compound (400 mg, white solid), yield: 64%. MS (ESI): m/z 215.9 [M+H]⁺.

### (3) tert-Butyl (2,3-dihydro-[1,4]dioxino[2,3-c]pyridin-7-yl)carbamate

7-Bromo-2,3-dihydro-[1,4]dioxino[2,3-c]pyridine (350 mg, 1.62 mmol) and tert-butyl carbamate (574 mg, 4.86 mmol) were dissolved in 1,4-dioxane (20 mL), and 2-dicyclohexylphosphino-2',4',6'-triisopropylbiphenyl (161 mg, 0.32 mmol), tris(dibenzylideneacetone)dipalladium (154 mg, 0.16 mmol), and cesium carbonate (1.32 g, 4.05 mmol) were added. The mixture washeated to 90°C under nitrogen protection and stirred for 16 hours. The reaction solution was concentrated, and the residue was purified by silica gel column chromatography (petroleum ether/ethyl acetate = 80/20) to obtain the title compound (310 mg, yellow solid), yield: 76%. MS (ESI): m/z 197.0 [M+H-56]⁺.

### (4) 2,3-Dihydro-[1,4]dioxino[2,3-c]pyridin-7-amine

tert-Butyl (2,3-dihydro-[1,4]dioxino[2,3-c]pyridin-7-yl)carbamate (600 mg, 2.38 mmol) was dissolved in dichloromethane (5 mL), and trifluoroacetic acid (5 mL) was added, followed by stirring at room temperature for 12 hours. The reaction solution was concentrated, and the residue was purified by silica gel column chromatography (100% ethyl acetate) to obtain the title compound (140 mg, yellow solid), yield: 39%. MS (ESI): m/z 153.0 [M+H]⁺.

### (5) Methyl 9-oxo-2,3-dihydro-9H-[1,4]dioxino[2',3':4,5]pyrido[1,2-a]pyrimidine-7-carboxylate

2,3-Dihydro-[1,4]dioxino[2,3-c]pyridin-7-amine (100 mg, 0.66 mmol) and dimethyl but-2-ynedioate (160 mg, 0.72 mmol) were dissolved in water (15 mL) and stirred at room temperature for 12 hours. Ethyl acetate (30 mL) was added to the reaction solution. The organic phase was extracted and concentrated. The residue was purified by silica gel column chromatography (petroleum ether/ethyl acetate = 90/10) to obtain the title compound (100 mg, yellow solid), yield: 58%. MS (ESI): m/z 263.0 [M+H]⁺.

### (6) 9-Oxo-2,3-dihydro-9H-[1,4]dioxino[2',3':4,5]pyrido[1,2-a]pyrimidine-7-carboxylic acid

Methyl 9-oxo-2,3-dihydro-9H-[1,4]dioxino[2',3':4,5]pyrido[1,2-a]pyrimidine-7-carboxylate (100 mg, 0.38 mmol) was dissolved in methanol/water (5 mL/5 mL), sodium hydroxide (76 mg, 1.91 mmol) was added, and the mixture was heated to 80°C and stirred for 4 hours. The reaction solution was concentrated, 1 M dilute hydrochloric acid was added to adjust the pH to 5, and the aqueous phase was extracted with dichloromethane (30 mL x 3). The organic phases were combined and concentrated to obtain the title compound (60 mg, yellow solid), yield: 63%. MS (ESI): m/z 249.0 [M+H]⁺.

### Intermediate 5: 8-Oxo-8H-[1,3]dioxolo[4',5':4,5]pyrido[1,2-a]pyrimidine-6-carboxylic acid

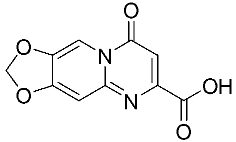

### (1) 2-Bromo-5-(methoxymethoxy)pyridine

6-Bromopyridin-3-ol (4.46 g, 25.8 mmol) was dissolved in anhydrous tetrahydrofuran (50 mL), and 1 M solution of potassium bis(trimethylsilyl)amide in tetrahydrofuran (25.8 mL, 25.8 mmol) was added dropwise at 0°C. After 10 minutes, bromo(methoxy)methane (3.87 g, 31 mmol) was added, and the reaction was stirred at room temperature overnight. Saturated aqueous ammonium chloride solution (80 mL) was added to the reaction solution, and the aqueous phase was extracted with ethyl acetate (2 x 50 mL). The organic phases were combined, washed with saturated sodium chloride solution (75 mL), dried over anhydrous sodium sulfate, filtered, and concentrated. The residue was purified by silica gel column chromatography (petroleum ether/ethyl acetate = 80/20) to obtain the title compound (3.93 g, pale yellow oil), yield: 70%. MS (ESI): m/z 217.6 [M+H]⁺.

### (2) 2-Bromo-5-(methoxymethoxy)pyridin-4-ol

Diisopropylamine (2.67 g, 26.4 mmol) was dissolved in anhydrous tetrahydrofuran (40 mL), cooled to -70°C, and a 2.5 M solution of n-butyllithium in n-hexane (10.6 mL, 26.5 mmol) was slowly added dropwise. The mixture was stirred at -70°C for 30 minutes. Then a solution of 2-bromo-5-(methoxymethoxy)pyridine (3.85 g, 17.6 mmol) in anhydrous tetrahydrofuran (15 mL) was slowly added dropwise, and after continued stirring for 1 hour, a solution of triisopropyl borate (3.31 g, 17.6 mmol) in anhydrous tetrahydrofuran (15 mL) was added dropwise. The reaction solution was stirred at -70°C for 2 hours, then 30% hydrogen peroxide solution (4 g, 35.2 mmol) was slowly added, and the mixture was slowly warmed to room temperature and stirred overnight. Water (50 mL) was added to the reaction solution, adjusted to pH < 4 with 1 M dilute hydrochloric acid, and the aqueous phase was extracted with ethyl acetate (2 x 75 mL). The organic phases were combined, washed with saturated brine (75 mL), dried over anhydrous sodium sulfate, filtered, and concentrated. The residue was purified by silica gel column chromatography (petroleum ether/ethyl acetate = 40/60) to obtain the title compound (2.73 g, white solid), yield: 66%. MS (ESI): m/z 233.9 [M+H]⁺.

### (3) 6-Bromopyridine-3,4-diol

2-Bromo-5-(methoxymethoxy)pyridin-4-ol (2.73 g, 11.7 mmol) was dissolved in 1,4-dioxane (20 mL), water (2 mL) and 4 M hydrogen chloride in dioxane solution (20 mL) were added, and the mixture was heated to 60°C for 2 hours. The reaction solution was concentrated to obtain the title compound (3 g, white solid), yield: 100%. MS (ESI): m/z 189.9 [M+H]⁺.

### (4) 6-Bromo-[1,3]dioxolo[4,5-c]pyridine

6-Bromopyridine-3,4-diol (1.63 g, 8.58 mmol) was dissolved in N,N-dimethylformamide (30 mL), cesium carbonate (7.0 g, 21.5 mmol) and chloriodomethane (3.03 g, 17.2 mmol) were added, and the mixture was heated to 60°C for 3 hours. The reaction solution was poured into water (70 mL), and the aqueous phase was extracted with ethyl acetate (2 x 35 mL). The organic phases were combined, washed with saturated sodium chloride solution (40 mL), dried over anhydrous sodium sulfate, filtered, and concentrated. The residue was purified by silica gel column chromatography (petroleum ether/ethyl acetate = 70/30) to obtain the title compound (630 mg, white solid), yield: 36.4%. MS (ESI): m/z 201.8 [M+H]⁺.

### (5) tert-Butyl [1,3]dioxolo[4,5-c]pyridin-6-ylcarbamate

6-Bromo-[1,3]dioxolo[4,5-c]pyridine (633 mg, 3.1 mmol) was dissolved in 1,4-dioxane (15 mL), and tris(dibenzylideneacetone)dipalladium (275 mg, 0.3 mmol), tert-butyl carbamate (1.09 g, 9.3 mmol), 4,5-bisdiphenylphosphino-9,9-dimethylxanthene (247 mg, 0.6 mmol) and cesium carbonate (2.02 g, 6.2 mmol) were added. The mixture was heated to 100°C under nitrogen protection and stirred overnight. The reaction solution was concentrated, and the residue was purified by silica gel column chromatography (petroleum ether/ethyl acetate = 70/30) to obtain the title compound (210 mg, white solid), yield: 28.5%. MS (ESI): m/z 183.0 [M+H-56]⁺.

### (6) [1,3]Dioxolo[4,5-c]pyridin-6-amine

tert-Butyl [1,3]dioxolo[4,5-c]pyridin-6-ylcarbamate (210 mg, 0.88 mmol) was dissolved in dichloromethane (4 mL), trifluoroacetic acid (4 mL) was added, and the mixture was stirred at room temperature for 2 hours. The reaction solution was concentrated, saturated sodium bicarbonate solution (30 mL) was added, and the aqueous phase was extracted with ethyl acetate (2 x 20 mL). The organic phases were combined, washed with saturated brine (20 mL), dried over anhydrous sodium sulfate, filtered, and concentrated to obtain the title compound (105 mg, pale yellow solid), yield: 87%. MS (ESI): m/z 139.0 [M+H]⁺.

### (7) Methyl 8-oxo-8H-[1,3]dioxolo[4',5':4,5]pyrido[1,2-a]pyrimidine-6-carboxylate

[1,3]Dioxolo[4,5-c]pyridin-6-amine (80 mg, 0.58 mmol), water (8 mL) and dimethyl but-2-ynedioate (82 mg, 0.58 mmol) were added to a 25 mL single-necked flask and stirred at room temperature overnight. The reaction solution was diluted with water (10 mL), and the aqueous phase was extracted with ethyl acetate (2 x 20 mL). The organic phases were combined, washed with saturated brine (20 mL), dried over anhydrous sodium sulfate, filtered, and concentrated. The residue was purified by silica gel column chromatography (petroleum ether/ethyl acetate = 20/80) to obtain the title compound (100 mg, pink solid), yield: 69.4%. MS (ESI): m/z 248.9 [M+H]⁺.

### (8) 8-Oxo-8H-[1,3]dioxolo[4',5':4,5]pyrido[1,2-a]pyrimidine-6-carboxylic acid

Methyl 8-oxo-8H-[1,3]dioxolo[4',5':4,5]pyrido[1,2-a]pyrimidine-6-carboxylate (100 mg, 0.4 mmol) was dissolved in methanol (5 mL), sodium hydroxide (48 mg, 1.0 mmol) and water (5 mL) were added, and the reaction was stirred at room temperature overnight. The reaction solution was concentrated to remove methanol, adjusted to pH ~4 with 1 M dilute hydrochloric acid, and the solid precipitated after standing, which was filtered and the filter cake was dried to obtain the title compound (40 mg, pale yellow solid), yield: 42.7%. MS (ESI): m/z 235.0 [M+H]⁺.

### Intermediate 6: tert-Butyl 6-(aminomethyl)-2-(((tert-butoxycarbonyl)((3,3-difluorocyclobutyl)methyl)amino)methyl)-1H-indole-1-carboxylate

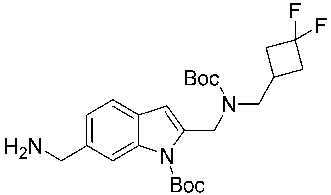

### (1) 2-((((3,3-Difluorocyclobutyl)methyl)amino)methyl)-1H-indole-6-carbonitrile

2-Formyl-1H-indole-6-carbonitrile (100 mg, 0.6 mmol, Intermediate 1, Step 4) and (3,3-difluorocyclobutyl)methanamine hydrochloride (142 mg, 0.9 mmol) were dissolved in dichloroethane (5 mL), and the mixture was heated to 65°C and stirred for 1 hour. sodium cyanoborohydride (132 mg, 2.1 mmol) was then added, and stirring was continued at 65°C for 2 hours. The reaction solution was poured into saturated sodium bicarbonate solution (20 mL), and the aqueous phase was extracted with ethyl acetate (15 mL x 3). The organic phases were combined, washed with saturated sodium chloride solution (30 mL), dried over anhydrous sodium sulfate, and concentrated. The residue was purified by silica gel column chromatography (petroleum ether/ethyl acetate = 40/60) to afford the title compound (65 mg, pale yellow oil), yield: 40%. MS (ESI): m/z 276.0 [M+H]⁺.

### (2) tert-Butyl 2-(((tert-butoxycarbonyl)((3,3-difluorocyclobutyl)methyl)amino)methyl)-6-cyano-1H-indole-1-carboxylate

2-((((3,3-Difluorocyclobutyl)methyl)amino)methyl)-1H-indole-6-carbonitrile (65 mg, 0.24 mmol) was dissolved in tetrahydrofuran solution (10 mL), sodium hydride (38 mg, 0.96 mmol, 60%) was added, and the mixture was stirred at room temperature for 10 minutes. Then di-tert-butyl dicarbonate (209 mg, 0.96 mmol) was added, stirring was continued at room temperature for 1 hour. The reaction solution was quenched with methanol (10 mL), concentrated, and the residue was purified by silica gel column chromatography (petroleum ether/ethyl acetate = 90/10) to obtain the title compound (87 mg, pale yellow oil), yield: 78%. MS (ESI): m/z 498.0 [M+Na]⁺.

### (3) tert-Butyl 6-(aminomethyl)-2-(((tert-butoxycarbonyl)((3,3-difluorocyclobutyl)methyl)amino)methyl)-1H-indole-1-carboxylate

tert-Butyl 2-(((tert-butoxycarbonyl)((3,3-difluorocyclobutyl)methyl)amino)methyl)-6-cyano-1H-indole-1-carboxylate (40 mg, 0.08 mmol) was dissolved in 7M ammonia methanol solution (10 mL), Raney nickel (4 mg, 10%) was added, hydrogen was replaced 3 times, and the mixture was heated to 50°C and stirred for 2 hours. The reaction solution was filtered to remove Raney nickel, and the filtrate was concentrated to obtain the title compound (40 mg, green oil), yield: 100%. MS (ESI): m/z 480.1 [M+H]⁺.

### Intermediate 7: N-((2-Formyl-1H-indol-6-yl)methyl)-8-oxo-8H-[1,3]dioxolo[4',5':4,5]pyrido[1,2-a]pyrimidine-6-carboxamide

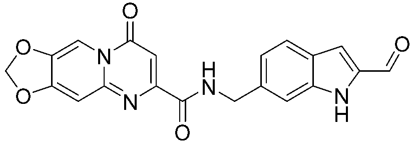

### (1) (2-(Diethoxymethyl)-1H-indol-6-yl)methanamine

2-(Diethoxymethyl)-1H-indole-6-carbonitrile (1.25 g, 5.1 mmol, Step 3 of Intermediate 1) was dissolved in 7M ammonia methanol solution (10 mL), Raney nickel (125 mg, 10%) was added, hydrogen was replaced three times, and the reaction was stirred at room temperature under hydrogen atmosphere for 13 hours. The reaction solution was filtered to remove Raney nickel, and the filtrate was concentrated to obtain the title compound (1.27 g, green oil), yield: 100%. MS (ESI): m/z 158.0 [M+H-91]⁺.

### (2) N-((2-(Diethoxymethyl)-1H-indol-6-yl)methyl)-8-oxo-8H-[1,3]dioxolo[4',5':4,5]pyrido[1,2-a]pyrimidine-6-carboxamide

Intermediate 5 (130 mg, 0.55 mmol), N,N-diisopropylethylamine (359 mg, 2.78 mmol) and 1-propylphosphonic anhydride (530 mg, 0.83 mmol, 50% ethyl acetate solution) were dissolved in N,N-dimethylformamide (5 mL), (2-(Diethoxymethyl)-1H-indol-6-yl)methanamine (179 mg, 0.72 mmol) was added, and the reaction was stirred at room temperature for 3 hours. The reaction solution was poured into saturated aqueous sodium bicarbonate solution (30 mL) and extracted with ethyl acetate (2 x 20 mL). The organic phases were combined, washed with saturated sodium chloride solution (30 mL), dried over anhydrous sodium sulfate, filtered, and concentrated. The residue was purified by silica gel column chromatography (petroleum ether/ethyl acetate = 40/60) to obtain the title compound (180 mg, white solid), yield: 69.8%. MS (ESI): m/z 465.0 [M+H]⁺.

### (3) N-((2-Formyl-1H-indol-6-yl)methyl)-8-oxo-8H-[1,3]dioxolo[4',5':4,5]pyrido[1,2-a]pyrimidine-6-carboxamide

N-((2-(Diethoxymethyl)-1H-indol-6-yl)methyl)-8-oxo-8H-[1,3]dioxolo[4',5':4,5]pyrido[1,2-a]pyrimidine-6-carboxamide (100 mg, 0.22 mmol) was dissolved in tetrahydrofuran (10 mL), water (1 mL) and glacial acetic acid (1 mL) were added, and the reaction was stirred at room temperature for 2 hours. A solid precipitated from the reaction solution. After concentrating the reaction solution, water (10 mL) was added for dilution, and the precipitated solid was filtered and dried to obtain the title compound (80 mg, white solid), yield: 95%. MS (ESI): m/z 390.9 [M+H]⁺.

### Intermediate 8: N-((2-Formyl-1H-indol-6-yl)methyl)-9-oxo-2,3-dihydro-9H-[1,4]dioxino[2',3':4,5]pyrido[1,2-a]pyrimidine-7-carboxamide

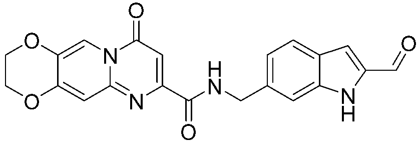

Referring to the synthetic method of Intermediate 7, Intermediate 4 was used to replace Intermediate 5 in Step 2, and Intermediate 8 (155 mg, white solid) was obtained through similar steps. MS (ESI): m/z 405.1 [M+H]⁺.

### Intermediate 9: tert-Butyl 6-(1-aminocyclopropyl)-2-(diethoxymethyl)-1H-indole-1-carboxylate

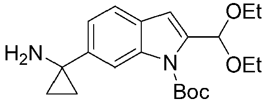

### (1) tert-Butyl 6-cyano-2-(diethoxymethyl)-1H-indole-1-carboxylate

2-(Diethoxymethyl)-1H-indole-6-carbonitrile (200 mg, 0.82 mmol, Step 3 of Intermediate 1) was dissolved in tetrahydrofuran (15 mL), sodium hydride (98 mg, 2.4 mmol, 60%) was added, and the mixture was stirred at room temperature for half an hour. Di-tert-butyl dicarbonate (268 mg, 1.2 mmol) was added, and the mixture was stirred at room temperature overnight. Water (20 mL) was added to the reaction solution, and the mixture was extracted with ethyl acetate (20 mL x 3). The organic phases were combined, washed with saturated sodium chloride solution (20 mL x 3), dried over anhydrous sodium sulfate, filtered, and concentrated. The residue was purified by silica gel column chromatography (petroleum ether/ethyl acetate = 10/1) to obtain the title compound (280 mg, colorless oil), yield: 99%. MS (ESI): m/z 366.9 [M+Na]⁺.

### (2) tert-Butyl 6-(1-aminocyclopropyl)-2-(diethoxymethyl)-1H-indole-1-carboxylate

tert-Butyl 6-cyano-2-(diethoxymethyl)-1H-indole-1-carboxylate (280 mg, 0.8 mmol) was dissolved in diethyl ether (3 mL), nitrogen was purged, and tetraisopropyl titanate (1.1 g, 4.0 mmol) was added at -70°C, followed by stirring for 10 minutes. Then a 2M solution of ethylmagnesium bromide in tetrahydrofuran (2 mL, 4.0 mmol) was slowly added dropwise, and the mixture was stirred at -70°C for 10 minutes, then warmed to room temperature and stirred for another 1 hour. The reaction solution was quenched by adding water (30 mL), extracted with ethyl acetate (20 mL x 3), washed with saturated sodium chloride solution (30 mL), dried over anhydrous sodium sulfate, filtered, and concentrated. The residue was purified by silica gel column chromatography (dichloromethane/methanol = 92/8) to obtain the title compound (40 mg, yellow solid), yield: 13%. MS (ESI): m/z 396.9 [M+Na]⁺.

### Intermediate 10: 7,8-Dihydro-6H-9-oxa-2,2a,5,6-tetraazabenzo[cd]azulene-4-carboxylic acid

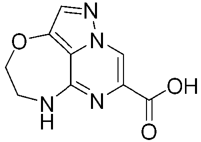

### (1) 4,6-Dichloro-3-iodopyrazolo[1,5-a]pyrazine

4,6-Dichloropyrazolo[1,5-a]pyrazine (2.0 g, 10.64 mmol) and N-iodosuccinimide (2.63 g, 11.70 mmol) were dissolved in *N,N-*dimethylformamide (50 mL) and heated to 50°C with stirring for 12 hours. The reaction solution was added to water (100 mL) and extracted with ethyl acetate (100 mL x 2). The organic phases were combined, washed with saturated sodium chloride solution (50 mL x 3), dried over anhydrous sodium sulfate, filtered, and concentrated. The residue was purified by silica gel column chromatography (petroleum ether/ethyl acetate = 95/5) to obtain the title compound (3.1 g, pale yellow solid), yield: 93%. MS (ESI): m/z 313.7 [M+H]⁺.

### (2) 4,6-Dichloro-3-vinylpyrazolo[1,5-a]pyrazine

4,6-Dichloro-3-iodopyrazolo[1,5-a]pyrazine (1.0 g, 3.19 mmol), vinylboronic acid pinacol ester (736 mg, 4.78 mmol), potassium carbonate (1.32 g, 9.56 mmol) and [1,1'-bis(diphenylphosphino)ferrocene]palladium dichloride (258 mg, 0.32 mmol) were dissolved in 1,4-dioxane (19 mL) and water (1 mL), and heated to 90°C under nitrogen protection with stirring for 12 hours. The reaction solution was added to water (50 mL) and extracted with ethyl acetate (40 mL x 2). The organic phases were combined, washed with saturated sodium chloride solution (30 mL x 3), dried over anhydrous sodium sulfate, filtered, and concentrated. The residue was purified by silica gel column chromatography (petroleum ether/ethyl acetate = 90/10) to obtain the title compound (445 mg, yellow solid), yield: 65%. MS (ESI): m/z 213.9 [M+H]⁺.

### (3) 4,6-Dichloropyrazolo[1,5-a]pyrazine-3-carbaldehyde

4,6-Dichloro-3-vinylpyrazolo[1,5-a]pyrazine (560 mg, 2.6 mmol) was dissolved in tetrahydrofuran/water (15 mL/5 mL). Potassium osmate dihydrate (95 mg, 0.26 mmol) was added at 0°C, the mixture was warmed to room temperature and stirred for half an hour, then sodium periodate (1.7 g, 7.8 mmol) was added, stirring was continued at room temperature overnight. Water (20 mL) was added to the reaction solution, and the mixture was extracted with ethyl acetate (20 mL x 3). The organic phases were combined, washed with saturated sodium chloride solution (20 mL x 3), dried over anhydrous sodium sulfate, filtered, and concentrated. The residue was purified by silica gel column chromatography (petroleum ether/ethyl acetate = 10/1) to obtain the title compound (300 mg, white solid), yield: 53%. MS (ESI): m/z 215.8 [M+H]⁺.

### (4) 4,6-Dichloropyrazolo[1,5-a]pyrazin-3-ol

4,6-Dichloropyrazolo[1,5-a]pyrazine-3-carbaldehyde (300 mg, 1.4 mmol) and meta-chloroperoxybenzoic acid (366 mg, 2.1 mmol) were dissolved in dichloromethane (10 mL). Trifluoroacetic acid (16 mg, 0.14 mmol) was added at 0°C, and the mixture was warmed to room temperature and stirred overnight. Aqueous sodium bicarbonate solution (20 mL) was added to the reaction solution, and the mixture was extracted with dichloromethane (20 mL x 3). The organic phases were combined and concentrated. The residue was dissolved in methanol/water (10 mL/2 mL), potassium carbonate (386 mg, 2.8 mmol) was added, and the mixture was stirred at room temperature for half an hour. The reaction solution was adjusted to neutral pH with 1 M dilute hydrochloric acid and extracted with dichloromethane (20 mL x 3). The combined organic phases were dried over anhydrous sodium sulfate, filtered, and concentrated. The residue was purified by silica gel column chromatography (petroleum ether/ethyl acetate = 50/50) to obtain the title compound (250 mg, yellow solid), yield: 88%. MS (ESI): m/z 203.8 [M+H]⁺.

### (5) tert-Butyl (2-((4,6-dichloropyrazolo[1,5-a]pyrazin-3-yl)oxy)ethyl)carbamate

4,6-Dichloropyrazolo[1,5-a]pyrazin-3-ol (80 mg, 0.39 mmol), tert-butyl (2-hydroxyethyl)carbamate (95 mg, 0.59 mmol) and triphenylphosphine (204 mg, 0.78 mmol) were dissolved in tetrahydrofuran (5 mL). Diisopropyl azodicarboxylate (158 mg, 0.78 mmol) was added dropwise, and the mixture was heated to 50°C and stirred overnight. The reaction solution was concentrated, and the residue was purified by silica gel column chromatography (petroleum ether/ethyl acetate = 50/50) to obtain the title compound (120 mg, yellow solid), yield: 88%. MS (ESI): m/z 291.1 [M+H-56]⁺.

### (6) 2-((4,6-Dichloropyrazolo[1,5-a]pyrazin-3-yl)oxy)ethan-1-amine

tert-Butyl (2-((4,6-dichloropyrazolo[1,5-a]pyrazin-3-yl)oxy)ethyl)carbamate (120 mg, 0.35 mmol) was dissolved in dichloromethane (5 mL), trifluoroacetic acid (2 mL) was added, and the mixture was stirred at room temperature for 2 hours. The reaction solution was concentrated to obtain the title compound (85 mg, colorless oil), yield: 97%. MS (ESI): m/z 246.8 [M+H]⁺.

### (7) 4-Chloro-7,8-dihydro-6H-9-oxa-2,2a,5,6-tetraazabenzo[cd]azulene

2-((4,6-Dichloropyrazolo[1,5-a]pyrazin-3-yl)oxy)ethan-1-amine (85 mg, 0.34 mmol) and *N,N-*diisopropylethylamine (134 mg, 1.04 mmol) were dissolved in dimethyl sulfoxide (5 mL), heated to 50°C and stirred overnight. The reaction solution was added to water (20 mL), and extracted with ethyl acetate (20 mL x 3). The organic phases were combined, washed with saturated sodium chloride solution (20 mL x 3), dried over anhydrous sodium sulfate, filtered, and concentrated. The residue was purified by silica gel column chromatography (petroleum ether/ethyl acetate = 75/25) to obtain the title compound (65 mg, yellow solid), yield: 90%. MS (ESI): m/z 210.9 [M+H]⁺.

### (8) Methyl 7,8-dihydro-6H-9-oxa-2,2a,5,6-tetraazabenzo[cd]azulene-4-carboxylate

4-Chloro-7,8-dihydro-6H-9-oxa-2,2a,5,6-tetraazabenzo[cd]azulene (65 mg, 0.3 mmol), palladium acetate (6.8 mg, 0.03 mmol) and 1,3-bis(diphenylphosphino)propane (18 mg, 0.045 mmol) were dissolved in methanol/acetonitrile (10 mL/5 mL). Triethylamine (60 mg, 0.6 mmol) was added, and the mixture was heated to 120°C in an autoclave (5 Mpa, carbon monoxide atmosphere) and stirred overnight. The reaction solution was concentrated, and the residue was purified by silica gel column chromatography (100% ethyl acetate) to obtain the title compound (30 mg, yellow solid), yield: 42%. MS (ESI): m/z 234.9 [M+H]⁺.

### (9) 7,8-Dihydro-6H-9-oxa-2,2a,5,6-tetraazabenzo[cd]azulene-4-carboxylic acid

Methyl 7,8-dihydro-6H-9-oxa-2,2a,5,6-tetraazabenzo[cd]azulene-4-carboxylate (30 mg, 0.13 mmol) and sodium hydroxide (8.0 mg, 0.20 mmol) were dissolved in methanol/water (10 mL/10 mL) and stirred at room temperature for half an hour. The reaction solution was concentrated to remove methanol, 1 M dilute hydrochloric acid was slowly added dropwise to the reaction solution, and the solid was filtered to obtain the title compound (20 mg, white solid), yield: 70%. MS (ESI): m/z 221.0 [M+H]⁺.

### Intermediate 11: 6,7,8,9-Tetrahydro-2,2a,5,6-tetraazabenzo[cd]azulene-4-carboxylic acid

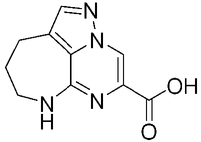

### (1) N-Allyl-6-chloro-3-vinylpyrazolo[1,5-a]pyrazin-4-amine

4,6-Dichloro-3-vinylpyrazolo[1,5-a]pyrazine (500 mg, 2.34 mmol, Step 2 of Intermediate 10), cesium carbonate (1.52 g, 4.67 mmol) and allylamine hydrochloride (262 mg, 2.80 mmol) were dissolved in acetonitrile (20 mL). The mixture was heated to 70°C and stirred for 2 hours under nitrogen protection. The reaction solution was concentrated, and the residue was purified by silica gel column chromatography (petroleum ether/ethyl acetate = 90/10) to obtain the title compound (140 mg, yellow solid), yield: 76%. MS (ESI): m/z 234.9 [M+H]⁺.

### (2) tert-Butyl allyl(6-chloro-3-vinylpyrazolo[1,5-a]pyrazin-4-yl)carbamate

N-Allyl-6-chloro-3-vinylpyrazolo[1,5-a]pyrazin-4-amine (300 mg, 1.28 mmol) was dissolved in dichloromethane (10 mL), and triethylamine (260 mg, 2.56 mmol), di-tert-butyl dicarbonate (1.39 g, 6.39 mmol) and 4-dimethylaminopyridine (15.6 mg, 0.13 mmol) were added, and the mixture was stirred at room temperature for 1 hour. The reaction solution was concentrated, and the residue was purified by silica gel column chromatography (petroleum ether/ethyl acetate = 90/10) to obtain the title compound (250 mg, green solid), yield: 58%. MS (ESI): m/z 278.9 [M+H-56]⁺.

### (3) tert-Butyl 4-chloro-2,2a,5,6-tetraazabenzo[cd]azulene-6(7H)-carboxylate

tert-Butyl allyl(6-chloro-3-vinylpyrazolo[1,5-a]pyrazin-4-yl)carbamate (250 mg, 0.75 mmol) and Grubbs Generation 2 catalyst (63.4mg, 0.07 mmol) were dissolved in dichloromethane (10 mL) and stirred at 25°C for 12 hours. The reaction solution was concentrated, and the residue was purified by silica gel column chromatography (petroleum ether/ethyl acetate = 85/15) to obtain the title compound (100 mg, yellow solid), yield: 44%. MS (ESI): m/z 328.9 [M+Na]⁺.

### (4) Methyl 6,7-dihydro-2,2a,5,6-tetraazabenzo[cd]azulene-4-carboxylate

tert-Butyl 4-chloro-2,2a,5,6-tetraazabenzo[cd]azulene-6(7H)-carboxylate (100 mg, 0.33 mmol), palladium acetate (14.6 mg, 0.07 mmol) and 1,3-bis(diphenylphosphino)propane (21.2mg, 0.07 mmol) were dissolved in methanol/acetonitrile (10 mL/10 mL). Triethylamine (66 mg, 0.65 mmol) was added, and the mixture was heated to 140°C and stirred for 72 hours in an autoclave (5 Mpa, carbon monoxide atmosphere). The reaction solution was concentrated, and the residue was purified by silica gel column chromatography (petroleum ether/ethyl acetate = 40/60) to obtain the title compound (36 mg, yellow solid), yield: 48%. MS (ESI): m/z 230.8 [M+H]⁺.

### (5) Methyl 6,7,8,9-tetrahydro-2,2a,5,6-tetraazabenzo[cd]azulene-4-carboxylate

Methyl 6,7-dihydro-2,2a,5,6-tetraazabenzo[cd]azulene-4-carboxylate (36 mg, 0.16 mmol) was dissolved in ethyl acetate (5 mL), 10% palladium on carbon (3.6 mg) was added, and the reaction was stirred at room temperature under hydrogen atmosphere for 1 hour. The reaction solution was filtered through celite, and the filtrate was concentrated to obtain the title compound (34 mg, yellow solid), yield: 94%. MS (ESI): m/z 232.9 [M+H]⁺.

### (6) 6,7,8,9-Tetrahydro-2,2a,5,6-tetraazabenzo[cd]azulene-4-carboxylic acid

Methyl 6,7,8,9-tetrahydro-2,2a,5,6-tetraazabenzo[cd]azulene-4-carboxylate (34 mg, 0.15 mmol) and sodium hydroxide (8.8mg, 0.22 mmol) were dissolved in methanol/water (10 mL/10 mL) and stirred at room temperature for half an hour. The reaction solution was concentrated to remove methanol, 1 M dilute hydrochloric acid was slowly added dropwise to the reaction solution, and the solid was filtered to obtain the title compound (25 mg, white solid), yield: 78%. MS (ESI): m/z 218.9 [M+H]⁺.

### Example 1

### N-((2-(((Cyclobutylmethyl)amino)methyl)-1H-indol-6-yl)methyl)imidazo[1,2-b]pyridazine-2-carboxamide

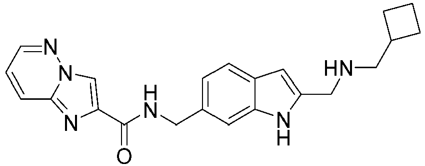

### (1) tert-Butyl 2-(((tert-butoxycarbonyl)(cyclobutylmethyl)amino)methyl)-6-((imidazo[1,2-b]pyridazine-2-carboxamido)methyl)-1H-indole-1-carboxylate

Imidazo[1,2-b]pyridazine-2-carboxylic acid (16 mg, 0.10 mmol), *N,N*-diisopropylethylamine (71 mg, 0.55 mmol) and HATU (84 mg, 0.22 mmol) were dissolved in *N,N-*dimethylformamide (2 mL) and stirred at room temperature for 5 minutes. Then a solution of Intermediate 1 (50 mg, 0.11 mmol) in *N,N-*dimethylformamide (1 mL) was added, and the reaction was continued to stir at room temperature for 10 minutes. Saturated sodium bicarbonate solution (20 mL) was added to the reaction solution, and the aqueous phase was extracted with ethyl acetate (20 mL x 3). The organic phases were combined, washed with saturated sodium chloride solution (20 mL x 3), dried over anhydrous sodium sulfate, and concentrated. The residue was purified by silica gel column chromatography (petroleum ether/ethyl acetate = 50/50) to obtain the title compound (60 mg, yellow oil), yield: 69%. MS (ESI): m/z 589.1 [M+H]⁺.

### (2) N-((2-(((Cyclobutylmethyl)amino)methyl)-1H-indol-6-yl)methyl)imidazo[1,2-b]pyridazine-2-carboxamide

tert-Butyl 2-(((tert-butoxycarbonyl)(cyclobutylmethyl)amino)methyl)-6-((imidazo[1,2-b]pyridazine-2-carboxamido)methyl)-1H-indole-1-carboxylate (60 mg, 0.1 mmol) was dissolved in dichloromethane (2 mL), trifluoroacetic acid (0.5 mL) was added, and the mixture was stirred at room temperature for 4 hours. The reaction solution was concentrated, and the crude product was diluted with *N,N-*dimethylformamide and purified by Prep-HPLC to obtain the title compound (9.3 mg, white solid), yield: 23%. MS (ESI): m/z 777.3 [2M+H]⁺. ¹H NMR (400 MHz, DMSO-*d*₆) δ 10.83 (s, 1H), 8.90 (t, *J* = 6.0 Hz, 1H), 8.65 (s, 1H), 8.60 (dd, *J* = 4.4, 1.6 Hz, 1H), 8.15 (dd, *J* = 9.6, 0.8 Hz, 1H), 7.35 (d, *J* = 6.8 Hz, 1H), 7.32 (t, *J* = 4.8 Hz, 1H), 7.29 (s, 1H), 6.96 (dd, *J* = 8.0, 1.2 Hz, 1H), 6.19 (s, 1H), 4.55 (d, *J* = 6.4 Hz, 2H), 3.77 (s, 2H), 2.51-2.50 (m, 2H), 2.41-2.37 (m, 1H), 1.98-1.94 (m, 2H), 1.82-1.76 (m, 2H), 1.63-1.59 (m, 2H).

### Example 2

### N-((2-(((Cyclobutylmethyl)amino)methyl)-1H-indol-6-yl)methyl)pyrazolo[1,5-a]pyrimidine-2-carboxamide

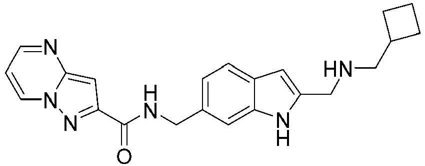

### (1) Pyrazolo[1,5-a]pyrimidine-2-carboxylic acid

Methyl pyrazolo[1,5-a]pyrimidine-2-carboxylate (150 mg, 0.85 mmol) was dissolved in methanol/water (10 mL/2 mL), sodium hydroxide (104 mg, 2.6 mmol) was added, and the mixture was stirred at room temperature for 2 hours. The reaction solution was adjusted to acidic with 1M dilute hydrochloric acid, and the solid product precipitated out, which was filtered to obtain the title compound (100 mg, white solid), yield: 72%. MS (ESI): m/z 164.0 [M+H]⁺.

### (2) tert-Butyl 2-(((tert-butoxycarbonyl)(cyclobutylmethyl)amino)methyl)-6-((pyrazolo[1,5-a]pyrimidine-2-carboxamido)methyl)-1H-indole-1-carboxylate

Pyrazolo[1,5-a]pyrimidine-2-carboxylic acid (29 mg, 0.18 mmol) and Intermediate 1 (80 mg, 0.18 mmol) were dissolved in *N,N*-dimethylformamide (5 mL), 1-propylphosphonic anhydride (229 mg, 0.36 mmol, 50% ethyl acetate solution) and triethylamine (54 mg, 0.54 mmol) were added, and the mixture was stirred at room temperature for 2 hours. The reaction solution was poured into water (20 mL), and the aqueous phase was extracted with ethyl acetate (2 x 20 mL). The organic phases were combined, washed with saturated sodium chloride solution (20 mL), dried over anhydrous sodium sulfate, filtered, and concentrated. The residue was purified by silica gel column chromatography (petroleum ether/ethyl acetate = 50/50) to obtain the title compound (40 mg, yellow oil), yield: 38%. MS (ESI): m/z 589.1 [M+H]⁺.

### (3)N-((2-(((Cyclobutylmethyl)amino)methyl)-1H-indol-6-yl)methyl)pyrazolo[1,5-a]pyrimidine-2-carboxamide

tert-Butyl 2-(((tert-butoxycarbonyl)(cyclobutylmethyl)amino)methyl)-6-((pyrazolo[1,5-a]pyrimidine-2-carboxamido)methyl)-1H-indole-1-carboxylate (40 mg, 0.07 mmol) was dissolved in dichloromethane (5 mL), trifluoroacetic acid (3 mL) was added, and the reaction was stirred at room temperature for 2 hours. The reaction solution was concentrated, and the residue was purified by Prep-HPLC to obtain the title compound (6.4 mg, white solid), yield: 25%. MS (ESI): m/z 777.2 [2M+H]⁺. ¹H NMR (400 MHz, DMSO-*d*₆) δ 10.84 (s, 1H), 9.11 (d, *J* = 6.8 Hz, 1H), 9.00 (t, *J* = 6.0 Hz, 1H), 8.63 (dd, *J* = 4.0, 1.6 Hz, 1H), 7.36 (d, *J* = 8.0 Hz, 1H), 7.30 (s, 1H), 7.17 (dd, *J* = 7.2, 4.0 Hz, 1H), 7.10 (d, *J* = 0.8 Hz, 1H), 6.96 (dd, *J* = 8.0, 1.2 Hz, 1H), 6.20 (s, 1H), 4.57 (d, *J* = 6.4 Hz, 2H), 3.77 (s, 2H), 2.51-2.50 (m, 2H), 2.43-2.35 (m, 1H), 2.00-1.92 (m, 2H), 1.85-1.74 (m, 2H), 1.66-1.59 (m, 2H).

### Example 3

### N-((2-(((Cyclobutylmethyl)amino)methyl)-1H-indol-6-yl)methyl)pyrazolo[1,5-a]pyrimidine-3-carboxamide

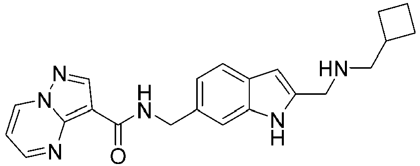

Following the synthetic method of **Example 1,** using pyrazolo[1,5-a]pyrimidine-3-carboxylic acid and Intermediate 1 as starting materials, the title compound (12 mg, white solid) was obtained through similar steps. MS (ESI): m/z 777.3 [2M+H]⁺. ¹H NMR (400 MHz, DMSO-*d*₆) δ 10.89 (s, 1H), 9.30 (dd, *J* = 6.8, 1.6 Hz, 1H), 8.76 (dd, *J* = 4.0, 1.6 Hz, 1H), 8.62 (s, 1H), 8.30 (t, *J* = 6.0 Hz, 1H), 7.40 (d, *J* = 8.0 Hz, 1H), 7.34 (s, 1H), 7.25 (dd, *J* = 6.8, 4.0 Hz, 1H), 6.98 (dd, *J* = 8.0, 1.2 Hz, 1H), 6.27 (s, 1H), 4.65 (d, *J =* 5.6 Hz, 2H), 3.85 (s, 2H), 2.60-2.58 (m, 2H), 2.45-2.41 (m, 1H), 2.01-1.96 (m, 2H), 1.83-1.77 (m, 2H), 1.66-1.61 (m, 2H).

### Example 4

### N-((2-(((Cyclobutylmethyl)amino)methyl)-1H-indol-6-yl)methyl)imidazo[1,2-b]pyridazine-7-carboxamide

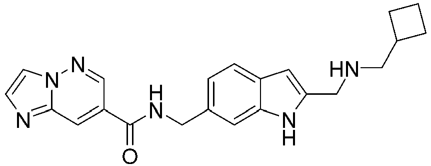

### (1) Methyl 6-((tert-butoxycarbonyl)amino)pyridazine-4-carboxylate

Methyl 6-chloropyridazine-4-carboxylate (900 mg, 5.2 mmol) was dissolved in 1,4-dioxane (10 mL), and tert-butyl carbamate (1.2 g, 10.4 mmol), tris(dibenzylideneacetone)dipalladium (476 mg, 0.52 mmol), 4,5-bisdiphenylphosphino-9,9-dimethylxanthene (601 mg, 1.04 mmol) and cesium carbonate (3.4 g, 10.4 mmol) were added. The mixture was heated to 100°C and stirred overnight under nitrogen protection. The reaction solution was concentrated, and the residue was purified by silica gel column chromatography (petroleum ether/ethyl acetate = 4/1) to obtain the title compound (400 mg, yellow solid), yield: 31%. MS (ESI): m/z 197.9 [M+H-56]⁺.

### (2) Methyl 6-aminopyridazine-4-carboxylate

Methyl 6-((tert-butoxycarbonyl)amino)pyridazine-4-carboxylate (400 mg, 1.6 mmol) was dissolved in dichloromethane (10 mL), trifluoroacetic acid (5 mL) was added, and the mixture was stirred at room temperature for 1 hour. The reaction solution was concentrated, saturated sodium bicarbonate solution (20 mL) was added, and the aqueous phase was extracted with dichloromethane (10 mL x 3). The organic phases were combined and concentrated to obtain the title compound (242 mg, yellow oil). MS (ESI): m/z 154.0 [M+H]⁺.

### (3) Methyl imidazo[1,2-b]pyridazine-7-carboxylate

Methyl 6-aminopyridazine-4-carboxylate (242 mg, 1.6 mmol) was dissolved in ethanol/water (10 mL/10 mL), 2-bromo-1,1-diethoxyethane (630 mg, 3.2 mmol) was added, a few drops of 1 M dilute hydrochloric acid were added to acidify the solution, and the reaction was stirred at 80°C overnight. The reaction solution was concentrated and extracted with ethyl acetate (2 x 20 mL). The organic phases were combined, washed sequentially with saturated sodium bicarbonate solution (20 mL) Saturated sodium chloride solution (20 mL), dried over anhydrous sodium sulfate, filtered, and concentrated to obtain the title compound (200 mg, yellow solid), yield: 71%. MS (ESI): m/z 178.0 [M+H]⁺.

### (4) Imidazo[1,2-b]pyridazine-7-carboxylic acid

Methyl imidazo[1,2-b]pyridazine-7-carboxylate (100 mg, 0.56 mmol) was dissolved in methanol/water (10 mL/2 mL), sodium hydroxide (67.2 mg, 1.68 mmol) was added, and the reaction was stirred at room temperature overnight. The solution was adjusted to acidic with 1M dilute hydrochloric acid, the reaction solution was concentrated, and the residue was purified by reverse-phase column to obtain the title compound (30 mg, white solid), yield: 33%. MS (ESI): m/z 164.0 [M+H]⁺.

### (5) tert-Butyl 2-(((tert-butoxycarbonyl)(cyclobutylmethyl)amino)methyl)-6-((imidazo[1,2-b]pyridazine-7-carboxamido)methyl)-1H-indole-1-carboxylate

Following the synthetic method of Step 2 in **Example 2,** using imidazo[1,2-b]pyridazine-7-carboxylic acid (30 mg, 0.18 mmol) and Intermediate 1 (80 mg, 0.18 mmol) as raw materials, the title compound (35 mg, white solid) was obtained, yield: 39%. MS (ESI): m/z 489.1 [M+H-100]⁺.

### (6) N-((2-(((Cyclobutylmethyl)amino)methyl)-1H-indol-6-yl)methyl)imidazo[1,2-b]pyridazine-7-carboxamide

Following the synthetic method of Step 3 in **Example 2,** using tert-butyl 2-(((tert-butoxycarbonyl)(cyclobutylmethyl)amino)methyl)-6-((imidazo[1,2-b]pyridazine-7-carboxamido)methyl)-1H-indole-1-carboxylate (30 mg, 0.06 mmol) as the raw material, the title compound (6.2 mg, white solid) was obtained, yield: 31%. MS (ESI): m/z 389.1 [M+H]⁺. ¹H NMR (400 MHz, DMSO-*d*₆) δ 10.85 (s, 1H), 9.33 (t, *J* = 5.2 Hz, 1H), 8.92 (d, *J* = 2.4 Hz, 1H), 8.63 (d, *J* = 1.6 Hz, 1H), 8.44 (s, 1H), 7.94 (d, *J* = 1.2 Hz, 1H), 7.38 (d, *J* = 8.0 Hz, 1H), 7.31 (s, 1H), 6.96 (dd, *J =* 8.0, 1.2 Hz, 1H), 6.21 (s, 1H), 4.58 (d, *J* = 5.6 Hz, 2H), 3.78 (s, 2H), 2.52-2.51 (m, 2H), 2.45-2.36 (m, 1H), 2.01-1.93 (m, 2H), 1.85-1.74 (m, 2H), 1.66-1.59 (m, 2H).

### Example 5

### N-((2-(((Cyclobutylmethyl)amino)methyl)-1H-indol-6-yl)methyl)-[1,2,4]triazolo[1,5-a]pyrazine-6-carboxamide

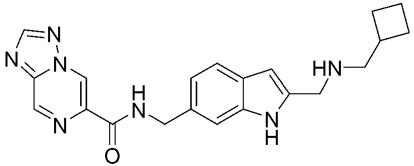

### (1) Ethyl [1,2,4]triazolo[1,5-a]pyrazine-6-carboxylate

6-Bromo-[1,2,4]triazolo[1,5-a]pyrazine (299 mg, 1.5 mmol) was dissolved in ethanol (10 mL), and [1,1'-bis(diphenylphosphino)ferrocene]palladium dichloride (110 mg, 0.15 mmol) and triethylamine (455 mg, 4.5 mmol) were added. After replacing carbon monoxide gas 3 times, the mixture was heated to 80°C under the protection of a carbon monoxide balloon and stirred for 16 hours. The reaction solution was concentrated, and the residue was purified by silica gel column chromatography (petroleum ether/ethyl acetate = 40/60) to obtain the title compound (96 mg, pale yellow solid), yield: 33%. MS (ESI): m/z 192.8 [M+H]⁺.

### (2) [1,2,4]triazolo[1,5-a]pyrazine-6-carboxylic acid

Ethyl [1,2,4]triazolo[1,5-a]pyrazine-6-carboxylate (96 mg, 0.5 mmol) was dissolved in ethanol (10 mL), and water (4 mL) and sodium hydroxide (40 mg, 1 mmol) were added, and the reaction was stirred at room temperature for 16 hours. The reaction solution was concentrated, adjusted to pH=3 with 1M dilute hydrochloric acid, with the solid precipitating after standing for 5 minutes. The solid was filtered, and the filter cake was dried to obtain the title compound (57 mg, brown solid), yield: 69.5%. MS (ESI): m/z 187.0 [M+Na]⁺.

### (3) tert-Butyl ((6-(([1,2,4]triazolo[1,5-a]pyrazine-6-carboxamido)methyl)-1H-indol-2-yl)methyl)(cyclobutylmethyl)carbamate

[1,2,4]Triazolo[1,5-a]pyrazine-6-carboxylic acid (16.4 mg, 0.1 mmol) was dissolved in *N,N-*dimethylformamide (2 mL), and Intermediate 2 (34 mg, 0.1 mmol), 1-propylphosphonic anhydride (127 mg, 0.2 mmol, 50% ethyl acetate solution) and triethylamine (50 mg, 0.5 mmol) were added, and the mixture was stirred at room temperature for 16 hours. The reaction solution was poured into water (30 mL), and the aqueous phase was extracted with ethyl acetate (2 x 20 mL). The organic phases were combined, washed with saturated brine (30 mL), dried over anhydrous sodium sulfate, filtered, and concentrated. The residue was purified by silica gel column chromatography (petroleum ether/ethyl acetate = 50/50) to obtain the title compound (25 mg, pale yellow oil), yield: 51%. MS (ESI): m/z 512.0 [M+Na]⁺.

### (4) N-((2-(((Cyclobutylmethyl)amino)methyl)-1H-indol-6-yl)methyl)-[1,2,4]triazolo[1,5-a]pyrazine-6-carboxamide

tert-Butyl ((6-(([1,2,4]triazolo[1,5-a]pyrazine-6-carboxamido)methyl)-1H-indol-2-yl)methyl)(cyclobutylmethyl)carbamate (25 mg, 0.05 mmol) was dissolved in dichloromethane (3 mL), and trifluoroacetic acid (3 mL) was added, and the reaction was stirred at room temperature for 3 hours. The reaction solution was concentrated, and the residue was dissolved in methanol (1 mL) and purified by Prep-HPLC to obtain the title compound (4 mg, white solid), yield: 20%. MS (ESI): m/z 779.2 [2M+H]⁺. ¹H NMR (400 MHz, DMSO-*d*₆) δ 10.96 (s, 1H), 9.46 (s, 2H), 9.41 (t, *J= 6.0* Hz, 1H), 8.88 (s, 1H), 7.43 (d, *J* = 8.0 Hz, 1H), 7.38 (s, 1H), 7.03 (d, *J* = 8.0 Hz, 1H), 6.38 (s, 1H), 4.61 (d, *J* = 6.4 Hz, 2H), 4.01 (s, 2H), 2.75 (d, *J* = 6.8 Hz, 2H), 2.61-2.60 (m, 1H), 2.04-1.98 (m, 2H), 1.85-1.66 (m, 4H).

### Example 6

### N-((2-(((Cyclobutylmethyl)amino)methyl)-1H-indol-6-yl)methyl)-[1,2,4]triazolo[1,5-a]pyridine-6-carboxamide

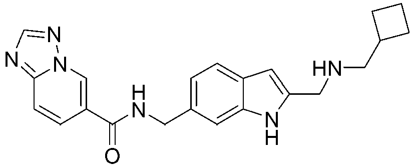

Following the synthetic method of **Example 1,** using [1,2,4]triazolo[1,5-a]pyridine-6-carboxylic acid and Intermediate 1 as starting materials, the title compound (7.2 mg, white solid) was obtained through similar steps. MS (ESI): m/z 389.1 [M+H]⁺. ¹H NMR (400 MHz, DMSO-*d*₆) δ 10.84 (s, 1H), 9.48 (s, 1H), 9.25 (t, *J* = 6.0 Hz, 1H), 8.62 (s, 1H), 8.11 (dd, *J* = 9.6 Hz, 2.0 Hz, 1H), 7.92 (dd, *J* = 9.2 Hz, 0.4 Hz, 1H), 7.38 (d, *J* = 8.0 Hz, 1H), 7.30 (s, 1H), 6.96 (dd, *J* = 8.0 Hz, 1.2 Hz, 1H), 6.20 (s, 1H), 4.58 (d, *J* = 6.0 Hz, 2H), 3.77 (s, 2H), 2.62-2.51 (m, 2H), 2.45-2.35 (m, 1H), 2.02-1.91 (m, 2H), 1.85-1.72 (m, 2H), 1.67-1.56 (m, 2H).

### Example 7

### N-((2-(((Cyclobutylmethyl)amino)methyl)-1H-indol-6-yl)methyl)benzo[b]thiophene-3-carboxamide

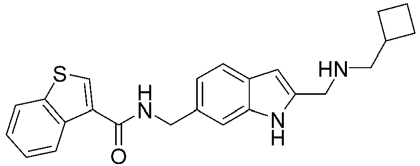

Following the synthetic method of **Example 1,** using benzo[b]thiophene-3-carboxylic acid and Intermediate 1 as starting materials, the title compound (5.9 mg, white solid) was obtained through similar steps. MS (ESI): m/z 807.2 [2M+H]⁺. ¹H NMR (400 MHz, DMSO-*d*₆) δ 11.01 (s, 1H), 8.99 (t, *J* = 5.6 Hz, 1H), 8.49 (d, *J* = 7.2 Hz, 1H), 8.40 (s, 1H), 8.04 (d, *J* = 7.2 Hz, 1H), 7.47-7.40 (m, 3H), 7.33 (s, 1H), 6.99 (d, *J* = 8.4 Hz, 1H), 6.28 (s, 1H), 4.57 (d, *J* = 6.0 Hz, 2H), 3.88 (s, 2H), 2.61 (d, *J* = 7.2 Hz, 2H), 2.47-2.43 (m, 1H), 2.01-1.94 (m, 2H), 1.86-1.75 (m, 2H), 1.69-1.62 (m, 2H).

### Example 8

### N-((2-(((Cyclobutylmethyl)amino)methyl)-1H-indol-6-yl)methyl)-8-morpholinoimidazo[1,2-b]pyridazine-2-carboxamide

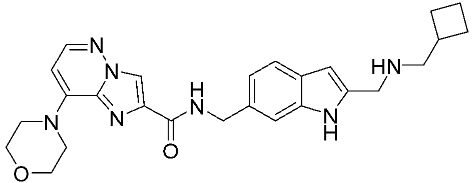

### (1) Methyl 8-bromo-6-chloroimidazo[1,2-b]pyridazine-2-carboxylate

4-Bromo-6-chloropyridazin-3-amine (2.08 g, 10 mmol) was dissolved in N,N-dimethylformamide (40 mL), methyl 3-bromo-2-oxopropanoate (3.62 g, 20 mmol) was added, and the mixture was heated to 70°C and stirred overnight. The reaction solution was poured into water (300 mL), and the aqueous phase was extracted with ethyl acetate (100 mL x 2). The organic phases were combined, washed with saturated sodium chloride solution (70 mL x 2), dried over anhydrous sodium sulfate, and concentrated. The residue was purified by silica gel column chromatography (petroleum ether/ethyl acetate = 60/40) to obtain the title compound (1.6 g, pale yellow solid), yield: 55%. MS (ESI): m/z 289.9 [M+H]⁺.

### (2) Methyl 6-chloro-8-morpholinoimidazo[1,2-b]pyridazine-2-carboxylate

Methyl 8-bromo-6-chloroimidazo[1,2-b]pyridazine-2-carboxylate (1.3 g, 4.5 mmol) was dissolved in 1,4-dioxane (25 mL), and palladium acetate (102 mg, 0.45 mmol), 4,5-bis(diphenylphosphino)-9,9-dimethylxanthene (260 mg, 0.45 mmol), morpholine (587 mg, 6.75 mmol) and cesium carbonate (2.93 g, 9.0 mmol) were added. The mixture was heated to 90°C under nitrogen protection and stirred for 16 hours. The reaction solution was concentrated, and the residue was purified by silica gel column chromatography (petroleum ether/ethyl acetate = 60/40) to obtain the title compound (550 mg, white solid), yield: 41.2%. MS (ESI): m/z 297.0 [M+H]⁺.

### (3) Methyl 8-morpholinoimidazo[1,2-b]pyridazine-2-carboxylate

Methyl 6-chloro-8-morpholinoimidazo[1,2-b]pyridazine-2-carboxylate (490 mg, 1.65 mmol) was dissolved in methanol (75 mL) and ethyl acetate (75 mL), 10% palladium on carbon (200 mg) was added, and after replacing hydrogen 3 times, the reaction was carried out at room temperature under hydrogen atmosphere for 16 hours. The reaction solution was filtered through celite, the filter cake was washed with a small amount of methanol, and the filtrate was concentrated to obtain the title compound (400 mg, white solid), yield: 92.5%. MS (ESI): m/z 263.0 [M+H]⁺.

### (4) 8-morpholinoimidazo[1,2-b]pyridazine-2-carboxylic acid

Methyl 8-morpholinoimidazo[1,2-b]pyridazine-2-carboxylate (100 mg, 0.38 mmol) was dissolved in methanol (5 mL), sodium hydroxide (40 mg, 1.0 mmol) and water (5 mL) were added, and the mixture was heated to 60°C for 3 hours. The reaction solution was concentrated, adjusted to pH about 4 with 1M dilute hydrochloric acid, allowed to stand and to precipitate a white solid, filtered, and the solid was dried to obtain the title compound (74 mg, white solid), yield: 78.5%. MS (ESI): m/z 248.8 [M+H]⁺.

### (5) tert-Butyl 2-(((tert-butoxycarbonyl)(cyclobutylmethyl)amino)methyl)-6-((8-morpholinoimidazo[1,2-b]pyridazine-2-carboxamido)methyl)-1H-indole-1-carboxylate

8-Morpholinoimidazo[1,2-b]pyridazine-2-carboxylic acid (25 mg, 0.1 mmol), *N,N-*diisopropylethylamine (65 mg, 0.5 mmol), HATU (76 mg, 0.2 mmol) were dissolved in *N,N-*dimethylformamide (3 mL) and stirred at room temperature for 5 minutes. Then Intermediate 1 (44 mg, 0.1 mmol) was added, stirring was continued at room temperature for 1 hour. The reaction solution was purified by reverse phase column to obtain the title compound (30 mg, yellow oil), yield: 44.6%. MS (ESI): m/z 574.1 [M+H-100]⁺.

### (6) N-((2-(((Cyclobutylmethyl)amino)methyl)-1H-indol-6-yl)methyl)-8-morpholinoimidazo[1,2-b]pyridazine-2-carboxamide

tert-Butyl 2-(((tert-butoxycarbonyl)(cyclobutylmethyl)amino)methyl)-6-((8-morpholinoimidazo[1,2-b]pyridazine-2-carboxamido)methyl)-1H-indole-1-carboxylate (30 mg, 0.045 mmol) was dissolved in trifluoroacetic acid (3 mL) and stirred at room temperature for 1 hour. The reaction solution was concentrated, and the residue was diluted with methanol (1 mL) and purified by Prep-HPLC to obtain the title compound (7.9 mg, pale yellow solid), yield: 37.5%. MS (ESI): m/z 474.2 [M+H]⁺. ¹H NMR (400 MHz, DMSO-*d*₆) δ 11.03 (s, 1H), 8.92 (t, *J=* 6.4 Hz, 1H), 8.41 (s, 1H), 8.16 (d, *J* = 5.6 Hz, 1H), 7.48 (d*, J =* 8.0 Hz, 1H), 7.38 (s, 1H), 7.04 (dd, *J =* 8.4, 1.2 Hz, 1H), 6.42 (s, 1H), 6.36 (d, *J =* 6.0 Hz, 1H), 4.59 (d, *J =* 6.8 Hz, 2H), 4.20 (s, 2H), 4.01-3.99 (m, 4H), 3.79-3.77 (m, 4H), 2.94 (d, *J =* 7.6 Hz, 2H), 2.61-2.58 (m, 1H), 2.07-2.00 (m, 2H), 1.87-1.71 (m, 4H).

### Example 9

### 2-Amino-N-((2-(((cyclobutylmethyl)amino)methyl)-1H-indol-6-yl)methyl)quinoline-6-carboxamide

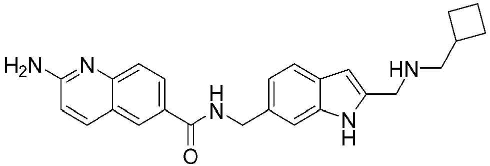

Following the synthetic method of **Example 1,** using 2-aminoquinoline-6-carboxylic acid and Intermediate 1 as starting materials, the title compound (30 mg, white solid) was obtained through similar steps. MS (ESI): m/z 414.1 [M+H]⁺. ¹H NMR (400 MHz, DMSO-*d*₆) δ 10.82 (s, 1H), 8.95 (t, *J* = 6.0 Hz, 1H), 8.22 (d, *J =* 2.0 Hz, 1H), 7.97 (dd, *J =* 8.8, 2.0 Hz, 1H), 7.94 (d, *J =* 8.8 Hz, 1H), 7.45 (d, *J =* 8.8 Hz, 1H), 7.37 (d, *J* = 8.0 Hz, 1H), 7.29 (s, 1H), 6.96 (dd, *J =* 8.0, 1.2 Hz, 1H), 6.80 (d, *J* = 9.2 Hz, 1H), 6.67 (s, 2H), 6.20 (s, 1H), 4.56 (d, *J =* 6.0 Hz, 2H), 3.77 (s, 2H), 2.52-2.51 (m, 2H), 2.41-2.38 (m, 1H), 1.99-1.95 (m, 2H), 1.81-1.76 (m, 2H), 1.64-1.59 (m, 2H).

### Example 10

### 4-(Aminomethyl)-N-((2-(((cyclobutylmethyl)amino)methyl)-1H-indol-6-yl)methyl)-1-oxo-1,2-dihydrophthalazine-6-carboxamide

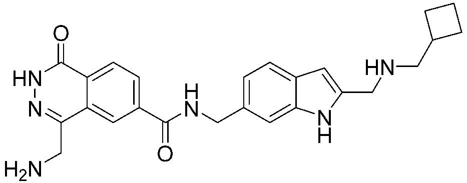

### (1) Methyl 4-(((tert-butoxycarbonyl)amino)methyl)-1-oxo-1,2-dihydrophthalazine-6-carboxylate

tert-Butyl ((7-bromo-4-oxo-3,4-dihydrophthalazin-1-yl)methyl)carbamate (354 mg, 1.0 mmol) was dissolved in methanol (50 mL), and [1,1'-bis(diphenylphosphino)ferrocene]palladium dichloride (73 mg, 0.1 mmol) and triethylamine (303 mg, 3 mmol) were added. After replacing carbon monoxide gas 3 times, the reaction solution was heated to 60°C under the protection of a carbon monoxide balloon and reacted for 16 hours. The reaction solution was concentrated, and the residue was purified by silica gel column chromatography (petroleum ether/ethyl acetate = 50/50) to obtain the title compound (300 mg, light reddish brown solid), yield: 90%. MS (ESI): m/z 334.0 [M+H]⁺.

### (2) 4-(((tert-Butoxycarbonyl)amino)methyl)-1-oxo-1,2-dihydrophthalazine-6-carboxylic acid

Methyl 4-(((tert-butoxycarbonyl)amino)methyl)-1-oxo-1,2-dihydrophthalazine-6-carboxylate (347 mg, 1.04 mmol) was dissolved in methanol (10 mL), and water (10 mL) and sodium hydroxide (83 mg, 2.08 mmol) were added, and the mixture was heated to 60°C and stirred for 16 hours. The reaction solution was concentrated, adjusted to pH=4 with 1M dilute hydrochloric acid, allowed to stand and for 5 minutes to precipitate a solid, filtered, and the solid was dried to obtain the title compound (230 mg, brown solid), yield: 69%. MS (ESI): m/z 320.0 [M+H]⁺.

### (3) tert-Butyl ((6-((4-(((tert-butoxycarbonyl)amino)methyl)-1-oxo-1,2-dihydrophthalazine-6-carboxamido)methyl)-1H-indol-2-yl)methyl)(cyclobutylmethyl)carbamate

Following the synthetic method of Step 3 in **Example 5,** using 4-(((tert-butoxycarbonyl)amino)methyl)-1-oxo-1,2-dihydrophthalazine-6-carboxylic acid (96 mg, 0.3 mmol) and Intermediate 2 (133 mg, 0.3 mmol) as raw materials, the title compound (46 mg, yellow solid) was obtained, yield: 23.8%. MS (ESI): m/z 667.2 [M+Na]⁺.

### (4) 4-(Aminomethyl)-N-((2-(((cyclobutylmethyl)amino)methyl)-1H-indol-6-yl)methyl)-1-oxo-1,2-dihydrophthalazine-6-carboxamide

Following the synthetic method of Step 4 in **Example 5,** using tert-butyl ((6-((4-(((tert-butoxycarbonyl)amino)methyl)-1-oxo-1,2-dihydrophthalazine-6-carboxamido)methyl)-1H-indol-2-yl)methyl)(cyclobutylmethyl)carbamate (46 mg, 0.07 mmol) as the raw material, the title compound (4.6 mg, pink solid) was obtained, yield: 14.8%. MS (ESI): m/z 445.1 [M+H]⁺. ¹H NMR (400 MHz, DMSO-*d*₆) δ 12.88 (brs, 1H), 11.53 (s, 1H), 9.45 (t, *J =* 6.0 Hz, 1H), 8.45 (s, 1H), 8.38-8.32 (m, 2H), 8.30 (s, 2H), 7.44 (d, *J =* 8.0 Hz, 1H), 7.35 (s, 1H), 7.02 (d, *J =* 8.4 Hz, 1H), 6.36 (s, 1H), 4.63 (d, *J =* 6.0 Hz, 2H), 4.37 (s, 2H), 4.00 (s, 2H), 2.69 (d, *J =* 7.6 Hz, 2H), 2.49-2.46 (m, 1H), 2.03-1.96 (m, 2H), 1.86-1.64 (m, 4H).

### Example 11

### 1-Benzyl-N-((2-(((cyclobutylmethyl)amino)methyl)-1H-indol-6-yl)methyl)-2-oxo-1,2-dihydropyridine-4-carboxamide

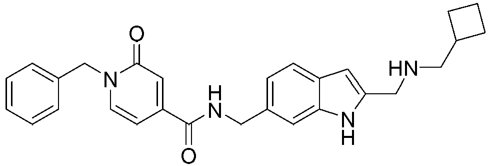

### (1) Methyl 1-benzyl-2-oxo-1,2-dihydropyridine-4-carboxylate

Methyl 2-oxo-1,2-dihydropyridine-4-carboxylate (500 mg, 3.25 mmol), (bromomethyl)benzene (840 mg, 4.90 mmol) and potassium carbonate (1.35 g, 9.80 mmol) were dissolved in *N,N-*dimethylformamide (30 mL) and stirred at room temperature for 12 hours. The reaction solution was added to water (50 mL), and the aqueous phase was extracted with ethyl acetate (50 mL x 3). The organic phases were combined, washed with saturated sodium chloride solution (50 mL x 3), dried over anhydrous sodium sulfate, and concentrated. The residue was purified by silica gel column chromatography (petroleum ether/ethyl acetate = 90/10) to obtain the title compound (750 mg, pale yellow solid), yield: 94%. MS (ESI): m/z 243.8 [M+H]⁺.

### (2) 1-Benzyl-2-oxo-1,2-dihydropyridine-4-carboxylic acid

Methyl 1-benzyl-2-oxo-1,2-dihydropyridine-4-carboxylate (800 mg, 3.29 mmol) and lithium hydroxide (236 mg, 9.87 mmol) were dissolved in tetrahydrofuran/methanol/water (10 mL/10 mL/30 mL) and stirred at room temperature for 12 hours. The reaction solution was concentrated, water (100 mL) was added, the pH was adjusted to 1 with concentrated hydrochloric acid, and the solid was collected by filtration to obtain the title compound (510 mg, white solid), yield: 68%. MS (ESI): m/z 229.6 [M+H]⁺.

### (3) tert-Butyl 6-((1-benzyl-2-oxo-1,2-dihydropyridine-4-carboxamido)methyl)-2-(((tert-butoxycarbonyl)(cyclobutylmethyl)amino)methyl)-1H-indole-1-carboxylate

Following the synthetic method of Step 1 in **Example 1,** using 1-benzyl-2-oxo-1,2-dihydropyridine-4-carboxylic acid (50 mg, 0.22 mmol) and Intermediate 1 (145 mg, 0.33 mmol) as raw materials, the title compound (46 mg, pale yellow oil) was obtained, yield: 70%. MS (ESI): m/z 655.0 [M+H]⁺.

### (4) 1-Benzyl-N-((2-(((cyclobutylmethyl)amino)methyl)-1H-indol-6-yl)methyl)-2-oxo-1,2-dihydropyridine-4-carboxamide

Following the synthetic method of Step 2 in **Example 1,** using tert-butyl 6-((1-benzyl-2-oxo-1,2-dihydropyridine-4-carboxamido)methyl)-2-(((tert-butoxycarbonyl)(cyclobutylmethyl)amino)methyl)-1H-indole-1-carboxylate (46 mg, 0.07 mmol) as the raw material, the title compound (12.5 mg, white solid) was obtained, yield: 39%. MS (ESI): m/z 455.1 [M+H]⁺. ¹H NMR (400 MHz, DMSO-*d*₆) δ 10.82 (s, 1H), 9.13 (t, *J* = 6.4 Hz, 1H), 7.89 (d, *J* = 6.8 Hz, 1H), 7.37-7.23 (m, 7H), 6.91-6.87 (m, 2H), 6.58 (dd, *J =* 8.8 Hz, 2.0 Hz, 1H), 6.19 (s, 1H), 5.12 (s, 2H), 4.48 (d, *J =* 6.4 Hz, 2H), 3.77 (s, 2H), 2.45-2.36 (m, 3H), 2.02-1.91 (m, 2H), 1.86-1.74 (m, 2H), 1.66-1.58 (m, 2H).

### Example 12

### 1-((1H-Pyrazol-5-yl)methyl)-N-((2-(((cyclobutylmethyl)amino)methyl)-1H-indol-6-yl)methyl)-6-oxo-1,6-dihydropyridazine-4-carboxamide

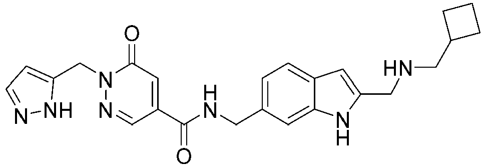

Following the synthetic method of **Example 11,** using tert-butyl 5-(bromomethyl)-1H-pyrazole-1-carboxylate and methyl 6-oxo-1,6-dihydropyridazine-4-carboxylate as starting materials, the title compound (6.2 mg, pale yellow solid) was obtained through similar steps. MS (ESI): m/z 446.1 [M+H]⁺. ¹H NMR (400 MHz, DMSO-*d*₆) δ 12.70 (s, 1H), 10.84 (s, 1H), 9.34 (t, *J =* 6.0 Hz, 1H), 8.19 (d, *J* = 2.0 Hz, 1H), 7.63 (s, 1H), 7.37 (d, *J =* 8.4 Hz, 1H), 7.32 (d, *J =* 2.0 Hz, 1H), 7.25 (s, 1H), 6.91 (dd, *J* = 8.0, 1.6 Hz, 1H), 6.20 (s, 1H), 6.12 (d, *J =* 2.0 Hz, 1H), 5.25 (s, 2H), 4.51 (d, *J* = 5.6 Hz, 2H), 3.78 (s, 2H), 2.52-2.51 (m, 2H), 2.43-2.36 (m, 1H), 2.01-1.93 (m, 2H), 1.85-1.74 (m, 2H), 1.66-1.59 (m, 2H).

### Example 13

### N-((2-(((Cyclobutylmethyl)amino)methyl)-1H-indol-6-yl)methyl)-4-(methylamino)pyrazolo[1,5-a]pyrazine-6-carboxamide

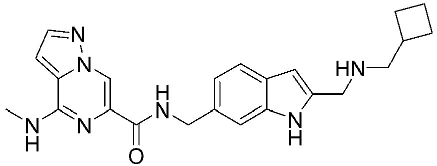

### (1) 6-Chloro-N-methylpyrazolo[1,5-a]pyrazin-4-amine

4,6-Dichloropyrazolo[1,5-a]pyrazine (500 mg, 2.66 mmol) was dissolved in dimethyl sulfoxide (10 mL), and methylamine hydrochloride (359 mg, 5.32 mmol) and N,N-diisopropylethylamine (1.03 g, 8.0 mmol) were added, and the mixture was heated to 60°C to react overnight. The reaction solution was poured into water (80 mL), and the aqueous phase was extracted with ethyl acetate (30 mL x 2). The organic phases were combined, washed with saturated sodium chloride solution (30 mL x 2), dried over anhydrous sodium sulfate, and concentrated. The residue was purified by silica gel column chromatography (petroleum ether/ethyl acetate = 65/35) to obtain the title compound (400 mg, pale yellow solid), yield: 82.6%. MS (ESI): m/z 183.0 [M+H]⁺.

### (2) Methyl 4-(methylamino)pyrazolo[1,5-a]pyrazine-6-carboxylate

6-Chloro-*N*-methylpyrazolo[1,5-a]pyrazin-4-amine (182 mg, 1.0 mmol) was dissolved in a mixed solvent of methanol/acetonitrile (5 mL/5 mL), and palladium acetate (23 mg, 0.1 mmol), diphenyl-1-styrylphosphine (54 mg, 0.13 mmol) and triethylamine (202 mg, 2 mmol) were added. The mixture was heated to 120°C in a high-pressure reactor (23 atmospheres, carbon monoxide atmosphere) and stirred for 12 hours. The reaction solution was concentrated, and the residue was purified by silica gel column chromatography (petroleum ether/ethyl acetate = 50/50) to obtain the title compound (140 mg, pale yellow solid), yield: 68%. MS (ESI): m/z 207.0 [M+H]⁺.

### (3) 4-(Methylamino)pyrazolo[1,5-a]pyrazine-6-carboxylic acid

Methyl 4-(methylamino)pyrazolo[1,5-a]pyrazine-6-carboxylate (103 mg, 0.5 mmol) was dissolved in methanol (8 mL), and sodium hydroxide (40 mg, 1 mmol) and water (4 mL) were added, and the reaction was carried out at room temperature for 16 hours. The reaction solution was concentrated, adjusted to pH about 6 with 1 M dilute hydrochloric acid, and a white solid precipitated after standing, which was filtered and dried to obtain the title compound (70 mg, white solid), yield: 73%. MS (ESI): m/z 192.8 [M+H]⁺.

### (4) tert-Butyl 2-(((tert-butoxycarbonyl)(cyclobutylmethyl)amino)methyl)-6-((4-(methylamino)pyrazolo[1,5-a]pyrazine-6-carboxamido)methyl)-1H-indole-1-carboxylate

4-(Methylamino)pyrazolo[1,5-a]pyrazine-6-carboxylic acid (30 mg, 0.16 mmol) was dissolved in *N,N*-dimethylformamide (2 mL), and *N*,*N*-diisopropylethylamine (148 mg, 1.15 mmol) and HATU (175 mg, 0.46 mmol) were added, and the reaction was stirred at room temperature for 5 minutes. Then a solution of Intermediate 1 (102 mg, 0.23 mmol) in *N,N*-dimethylformamide (1 mL) was added, and the reaction was continued to stir at room temperature for 30 minutes. Saturated sodium bicarbonate solution (10 mL) was added to the reaction solution, and the aqueous phase was extracted with ethyl acetate (10 mL x 3). The organic phases were combined, washed with saturated sodium chloride solution (10 mL x 3), dried over anhydrous sodium sulfate, and concentrated. The residue was purified by silica gel column chromatography (petroleum ether/ethyl acetate = 50/50) to obtain the title compound (75 mg, pale yellow oil), yield: 77%. MS (ESI): m/z 618.1 [M+H]⁺.

### (5)N-((2-(((Cyclobutylmethyl)amino)methyl)-1H-indol-6-yl)methyl)-4-(methylamino)pyrazolo[1,5-a]pyrazine-6-carboxamide

tert-Butyl 2-(((tert-butoxycarbonyl)(cyclobutylmethyl)amino)methyl)-6-((4-(methylamino)pyrazolo[1,5-a]pyrazine-6-carboxamido)methyl)-1H-indole-1-carboxylate (75 mg, 0.12 mmol) was dissolved in dichloromethane (3 mL), and trifluoroacetic acid (1 mL) was added, and the mixture was stirred at room temperature for 6 hours. The reaction solution was concentrated, and the residue was diluted with *N*,*N*-dimethylformamide and purified by Prep-HPLC to obtain the title compound (10.4 mg, white solid), yield: 20%. MS (ESI): m/z 418.1 [M+H]⁺. ¹H NMR (400 MHz, DMSO-*d*₆) δ 10.83 (s, 1H), 8.85 (t, *J* = 6.4 Hz, 1H), 8.36 (d, *J* = 0.4 Hz, 1H), 8.02 (d, *J =* 2.4 Hz, 1H), 7.89 (q, *J =* 4.4 Hz, 1H), 7.37 (d, *J =* 8.0 Hz, 1H), 7.30 (s, 1H), 7.01 (dd, *J =* 2.0, 0.8 Hz, 1H), 6.96 (dd, *J =* 8.0, 0.8 Hz, 1H), 6.20 (s, 1H), 4.59 (d, *J =* 6.4 Hz, 2H), 3.77 (s, 2H), 3.05 (d, *J* = 4.4 Hz, 3H), 2.52-2.51 (m, 2H), 2.41-2.35 (m, 1H), 1.98-1.93 (m, 2H), 1.82-1.76 (m, 2H), 1.64-1.59 (m, 2H).

### Example 14

### N-((2-(((Cyclobutylmethyl)amino)methyl)-1H-indol-6-yl)methyl)-4-((4-methoxybenzyl)amino)pyrazolo[1,5-a]pyrazine-6-carboxamide

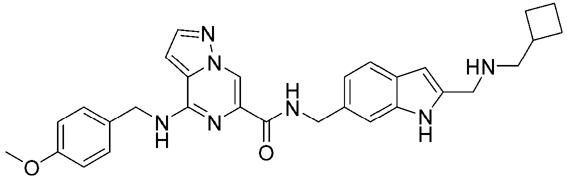

Following the synthetic method of **Example 13,** using 4,6-dichloropyrazolo[1,5-a]pyrazine and (4-methoxyphenyl)methanamine as starting materials, the title compound (12.9 mg, white solid) was obtained through similar steps. MS (ESI): m/z 524.1 [M+H]⁺. ¹H NMR (400 MHz, DMSO-*d*₆) δ 11.86 (s, 1H), 8.77 (t, *J =* 6.0 Hz, 1H), 8.38-8.35 (m, 2H), 8.02 (d, *J* = 2.0 Hz, 1H), 7.39 (d, *J* = 8.4 Hz, 1H), 7.31-7.27 (m, 3H), 7.09 (dd, *J =* 2.0, 0.4 Hz, 1H), 6.97 (dd, *J =* 8.4, 1.2 Hz, 1H), 6.73-6.71 (m, 2H), 6.21 (s, 1H), 4.69 (d, *J =* 5.6 Hz, 2H), 4.57 (d, *J =* 6.4 Hz, 2H), 3.77 (s, 2H), 3.63 (s, 3H), 2.56-2.53 (m, 2H), 2.43-2.35 (m, 1H), 2.00-1.92 (m, 2H), 1.84-1.58 (m, 4H).

### Example 15

### N-((2-(((Cyclobutylmethyl)amino)methyl)-1H-indol-6-yl)methyl)-4-(pyrrolidin-1-yl)pyrazolo[1,5-a]pyrazine-6-carboxamide

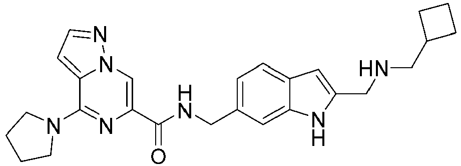

Following the synthetic method of **Example 13,** using 4,6-dichloropyrazolo[1,5-a]pyrazine and pyrrolidine as starting materials, the title compound (4.1 mg, white solid) was obtained through similar steps. MS (ESI): m/z 458.2 [M+H]⁺. ¹H NMR (400 MHz, DMSO-*d*₆) δ 10.83 (s, 1H), 8.76 (t, *J* = 6.4 Hz, 1H), 8.34 (s, 1H), 8.05 (d, *J =* 2.4 Hz, 1H), 7.36 (d, *J =* 8.0 Hz, 1H), 7.28 (s, 1H), 7.12 (d, *J =* 2.0 Hz, 1H), 6.95 (dd, *J =* 8.0, 0.8 Hz, 1H), 6.19 (s, 1H), 4.57 (d, *J =* 6.4 Hz, 2H), 3.89-3.79 (m, 4H), 3.77 (s, 2H), 2.62-2.76 (m, 2H), 2.43-2.35 (m, 1H), 2.01-1.93 (m, 6H), 1.83-1.59 (m, 4H).

### Example 16

### N-((2-(((Cyclobutylmethyl)amino)methyl)-1H-indol-6-yl)methyl)-4-morpholinopyrazolo[1,5-a]pyrazine-6-carboxamide

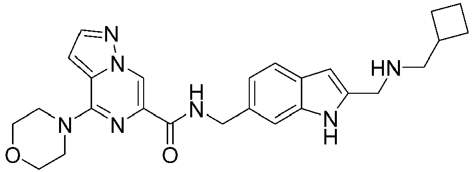

Following the synthetic method of **Example 13,** using 4,6-dichloropyrazolo[1,5-a]pyrazine and morpholine as starting materials, the title compound (11.5 mg, white solid) was obtained through similar steps. MS (ESI): m/z 474.2 [M+H]⁺. ¹H NMR (400 MHz, DMSO-*d*₆) δ 10.88 (s, 1H), 8.95 (t, *J =* 6.4 Hz, 1H), 8.53 (d, *J =* 0.4 Hz, 1H), 8.14 (d, *J* = 2.4 Hz, 1H), 7.39 (d, *J =* 8.4 Hz, 1H), 7.30 (s, 1H), 7.17 (dd, *J =* 2.4, 0.8 Hz, 1H), 6.97 (dd, *J =* 8.0, 1.2 Hz, 1H), 6.28 (s, 1H), 4.59 (d, *J* = 6.4 Hz, 2H), 3.88 (s, 2H), 3.87-3.84 (m, 4H), 3.77-3.75 (m, 4H), 2.62 (d, *J =* 7.2 Hz, 2H), 2.48-2.42 (m, 1H), 2.01-1.94 (m, 2H), 1.84-1.62 (m, 4H).

### Example 17

### N-((2-(2-(Cyclobutylamino)ethyl)-2H-indazol-6-yl)methyl)-4-oxo-4H-pyrido[1,2-a]pyrimidine-2-carboxamide

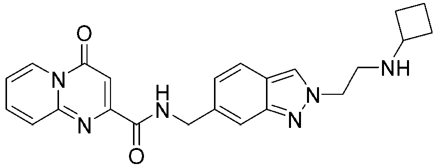

### (1) 2-(2-Hydroxyethyl)-2H-indazole-6-carbonitrile

1H-Indazole-6-carbonitrile (2.0 g, 14.0 mmol) was dissolved in *N,N*-dimethylformamide (30 mL), 2-bromo-1-ol (2.6 g, 21.0 mmol) and potassium carbonate (3.8 g, 28.0 mmol) were added, and the mixture was heated to 80°C to react overnight. The reaction solution was poured into water (20 mL) and extracted with ethyl acetate (2 x 20 mL). The organic phases were combined, washed with saturated sodium chloride solution (20 mL), dried over anhydrous sodium sulfate, filtered, and concentrated. The residue was purified by silica gel column chromatography (petroleum ether/ethyl acetate = 50/50) to obtain the title compound (900 mg, yellow solid), yield: 35%. MS (ESI): m/z 188.0 [M+H]⁺. ¹H NMR (400 MHz, DMSO-*d*₆) δ 8.55 (s, 1H), 8.31 (s, 1H), 7.93 (d, *J =* 8.8 Hz, 1H), 7.28 (d, *J =* 8.4 Hz, 1H), 5.01 (t, *J =* 5.6 Hz, 1H), 4.54 (t, *J =* 5.2 Hz, 2H), 3.90 (t, *J =* 5.6 Hz, 1H).

### (2) 2-(6-(Aminomethyl)-2H-indazol-2-yl)ethan-1-ol

2-(2-Hydroxyethyl)-2H-indazole-6-carbonitrile (300 mg, 1.6 mmol) was dissolved in ammonia methanol solution (7 M, 10 mL), Raney nickel (30 mg) was added, and the mixture was heated to 50°C under hydrogen atmosphere for 3 hours. The reaction solution was filtered, and the filtrate was concentrated to obtain the title compound (306 mg, colorless oil). MS (ESI): m/z 192.0 [M+H]⁺.

### (3) N-((2-(2-Hydroxyethyl)-2H-indazol-6-yl)methyl)-4-oxo-4H-pyrido[1,2-a]pyrimidine-2-carboxamide

2-(6-(Aminomethyl)-2H-indazol-2-yl)ethan-1-ol (200 mg, 1.0 mmol) and Intermediate 3 (200 mg, 1.0 mmol) were dissolved in *N,N*-dimethylformamide (5 mL), 1-propylphosphonic anhydride (1.3 g, 2 mmol, 50% ethyl acetate solution) and triethylamine (303 mg, 3 mmol) were added, and the mixture was stirred at room temperature overnight. The reaction solution was purified by reverse phase column to obtain the title compound (270 mg, white solid), yield: 71%. MS (ESI): m/z 364.0 [M+H]⁺.

### (4) 4-Oxo-N-((2-(2-oxoethyl)-2H-indazol-6-yl)methyl)-4H-pyrido[1,2-a]pyrimidine-2-carboxamide

*N*-((2-(2-Hydroxyethyl)-2H-indazol-6-yl)methyl)-4-oxo-4H-pyrido[1,2-a]pyrimidine-2-carboxamide (50 mg, 0.14 mmol) was dissolved in dichloromethane (5 mL), Dess-Martin periodinane (89 mg, 0.21 mmol) was added, and the reaction was carried out at room temperature for 2 hours. The reaction solution was filtered and concentrated to obtain the title compound (50 mg, yellow oil). MS (ESI): m/z 362.0 [M+H]⁺.

### (5) N-((2-(2-(Cyclobutylamino)ethyl)-2H-indazol-6-yl)methyl)-4-oxo-4H-pyrido[1,2-a]pyrimidine-2-carboxamide

4-Oxo-*N*-((2-(2-oxoethyl)-2H-indazol-6-yl)methyl)-4H-pyrido[1,2-a]pyrimidine-2-carboxamide (50 mg, 0.14 mmol) and cyclobutylamine (30 mg, 0.42 mmol) were dissolved in methanol (5 mL), stirred at room temperature for half an hour, then sodium cyanoborohydride (18 mg, 0.28 mmol) was added, and the mixture was stirred at room temperature overnight. The reaction solution was concentrated, and the residue was purified by silica gel column chromatography (dichloromethane/methanol = 30/1) to obtain a crude product, which was further purified by Prep-HPLC to obtain the title compound (3.7 mg, colorless oil), yield: 6%. MS (ESI): m/z 417.1 [M+H]⁺. ¹H NMR (400 MHz, DMSO-*d*₆) δ 9.35 (t, *J =* 6.0 Hz, 1H), 9.02 (d, *J =* 6.8 Hz, 1H), 8.30 (s, 1H), 8.09-8.05 (m, 1H), 7.79 (d, *J =* 8.8 Hz, 1H), 7.64 (d, *J =* 8.4 Hz, 1H), 7.49 (s, 1H), 7.45 (td, *J* = 6.8, 1.2 Hz, 1H), 7.04 (dd, *J =* 8.4, 1.2 Hz, 1H), 6.90 (s, 1H), 4.58 (d, *J =* 6.0 Hz, 2H), 4.39 (t, *J =* 6.4 Hz, 2H), 3.16-3.10 (m, 1H), 2.93 (t, *J =* 6.4 Hz, 2H), 2.08-2.02 (m, 2H), 1.62-1.53 (m, 4H).

### Example 18

### N-((2-(((Cyclobutylmethyl)amino)methyl)quinazolin-7-yl)methyl)-4-oxo-4H-pyrido[1,2-a]pyrimidine-2-carboxamide

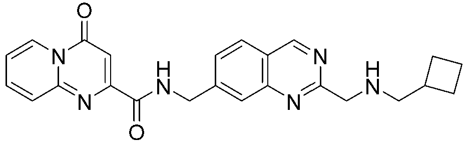

### (1) N-(5-Bromo-2-formylphenyl)-2-chloroacetamide

2-Amino-4-bromobenzaldehyde (1.8 g, 9.0 mmol) and triethylamine (1.8 g, 18.0 mmol) were dissolved in dichloromethane (20 mL), and chloroacetyl chloride (1.5 g, 13.5 mmol) was added dropwise at 0°C, and the reaction was carried out at room temperature for 3 hours under nitrogen protection. The reaction solution was poured into water (20 mL) and extracted with dichloromethane (2 x 20 mL). The organic phases were combined, washed with saturated sodium chloride solution (20 mL), dried over anhydrous sodium sulfate, filtered, and concentrated. The residue was purified by silica gel column chromatography (petroleum ether/ethyl acetate = 20/1) to obtain the title compound (1.9 g, yellow solid), yield: 77%. MS (ESI): m/z 275.8 [M+H]⁺.

### (2) 7-Bromo-2-(chloromethyl)quinazoline

N-(5-Bromo-2-formylphenyl)-2-chloroacetamide (1.5 g, 5.0 mmol) and ammonium acetate (2.3 g, 30.0 mmol) were dissolved in acetic acid (20 mL), and the mixture was heated to 80°C and stirred for 1 hour. The reaction solution was concentrated to remove acetic acid, saturated sodium bicarbonate solution (20 mL) was added, and the aqueous phase was extracted with ethyl acetate (2 x 20 mL). The organic phases were combined, washed with saturated sodium chloride solution (20 mL), dried over anhydrous sodium sulfate, filtered, and concentrated to obtain the title compound (1.3 g, yellow solid), yield: 93%. MS (ESI): m/z 256.8 [M+H]⁺.

### (3) 1-(7-Bromoquinazolin-2-yl)-N-(cyclobutylmethyl)methanamine

7-Bromo-2-(chloromethyl)quinazoline (800 mg, 3.1 mmol) and cyclobutylmethanamine (527 mg, 6.2 mmol) were dissolved in acetonitrile (20 mL), potassium carbonate (856 mg, 6.2 mmol) was added, and the reaction was carried out at room temperature overnight. The reaction solution was concentrated, poured into water (20 mL), and the aqueous phase was extracted with dichloromethane (2 x 20 mL). The organic phases were combined, washed with saturated sodium chloride solution (20 mL), dried over anhydrous sodium sulfate, filtered, and concentrated. The residue was purified by silica gel column chromatography (dichloromethane/methanol = 10/1) to obtain the title compound (480 mg, yellow oil), yield: 50%. MS (ESI): m/z 305.9 [M+H]⁺.

### (4) (9H-Fluoren-9-yl)methyl ((7-bromoquinazolin-2-yl)methyl)(cyclobutylmethyl)carbamate

1-(7-Bromoquinazolin-2-yl)-*N*-(cyclobutylmethyl)methanamine (100 mg, 0.33 mmol) and 9-fluorenylmethyl chloroformate (128 mg, 0.49 mmol) were dissolved in acetonitrile/water (20 mL/2 mL), sodium carbonate (70 mg, 0.66 mmol) was added, and the reaction was carried out at room temperature overnight. The reaction solution was concentrated, water (20 mL) was added, and the aqueous phase was extracted with ethyl acetate (2 x 20 mL). The organic phases were combined, washed with saturated sodium chloride solution (20 mL), dried over anhydrous sodium sulfate, filtered, and concentrated. The residue was purified by silica gel column chromatography (petroleum ether/ethyl acetate = 10/1) to obtain the title compound (130 mg, yellow oil), yield: 75%. MS (ESI): m/z 528.0 [M+H]⁺.

### (5) (9H-Fluoren-9-yl)methyl ((7-(((tert-butoxycarbonyl)amino)methyl)quinazolin-2-yl)methyl)(cyclobutylmethyl)carbamate

(9H-Fluoren-9-yl)methyl ((7-bromoquinazolin-2-yl)methyl)(cyclobutylmethyl)carbamate (130 mg, 0.25 mmol) and potassium ((trifluoro-4-bornyl)methyl)carbamate tert-butyl ester (118 mg, 0.5 mmol) were dissolved in 1,4-dioxane/water (10 mL/2 mL), Pd(dppf)Cl₂ (18 mg, 0.025 mmol) and potassium carbonate (69 mg, 0.5 mmol) were added, and the reaction was stirred overnight at 100°C under nitrogen protection. The reaction solution was poured into water (20 mL) and extracted with ethyl acetate (2 x 20 mL). The organic phases were combined, washed with saturated sodium chloride solution (20 mL), dried over anhydrous sodium sulfate, filtered, and concentrated. The residue was purified by silica gel column chromatography (petroleum ether/ethyl acetate = 50/50) to obtain the title compound (70 mg, yellow oil), yield: 49%. MS (ESI): m/z 579.0 [M+H]⁺.

### (6) (9H-Fluoren-9-yl)methyl ((7-(aminomethyl)quinazolin-2-yl)methyl)(cyclobutylmethyl)carbamate

(9H-Fluoren-9-yl)methyl ((7-(((tert-butoxycarbonyl)amino)methyl)quinazolin-2-yl)methyl)(cyclobutylmethyl)carbamate (30 mg, 0.05 mmol) was dissolved in dichloromethane (10 mL), trifluoroacetic acid (5 mL) was added, and the reaction was stirred at room temperature for 1 hour. The reaction solution was concentrated to obtain the title compound (25 mg, yellow oil). MS (ESI): m/z 479.2 [M+H]⁺.

### (7) N-((2-(((Cyclobutylmethyl)amino)methyl)quinazolin-7-yl)methyl)-4-oxo-4H-pyrido[1,2-a]pyrimidine-2-carboxamide

(9H-Fluoren-9-yl)methyl ((7-(aminomethyl)quinazolin-2-yl)methyl)(cyclobutylmethyl)carbamate (25 mg, 0.05 mmol) and Intermediate 3 (10 mg, 0.05 mmol) were dissolved in *N,N-*dimethylformamide (5 mL), 1-propylphosphonic anhydride (64 mg, 0.1 mmol, 50% ethyl acetate solution) and triethylamine (10 mg, 0.1 mmol) were added, and the mixture was stirred at room temperature overnight. The reaction solution was poured into water (20 mL) and extracted with ethyl acetate (2 x 20 mL). The organic phases were combined, washed with saturated sodium chloride solution (20 mL), dried over anhydrous sodium sulfate, filtered, and concentrated. The residue was purified by Prep-HPLC to obtain the title compound (1.6 mg, white solid), yield: 7%. MS (ESI): m/z 429.1 [M+H]⁺. ¹H NMR (400 MHz, DMSO-*d*₆) δ 9.66-9.62 (m, 1H), 9.53 (s, 1H), 9.03 (d, *J =* 6.4 Hz, 1H), 8.12-8.07 (m, 2H), 7.83-7.80 (m, 2H), 7.70 (d, *J =* 8.8 Hz, 1H), 7.49-7.46 (m, 1H), 6.91 (s, 1H), 4.76 (d, *J* = 6.4 Hz, 2H), 4.00 (s, 2H), 2.61 (d, *J* = 7.2 Hz, 2H), 2.43-2.38 (m, 1H), 2.01-1.97 (m, 2H), 1.83-1.78 (m, 2H), 1.68-1.60 (m, 2H).

### Example 19

### N-((3-(((Cyclobutylmethyl)amino)methyl)-1H-indol-6-yl)methyl)-4-oxo-4H-pyrido[1,2-a]pyrimidine-2-carboxamide

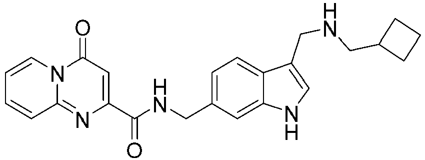

### (1) 3-(((Cyclobutylmethyl)amino)methyl)-1H-indole-6-carbonitrile

3-Formyl-1H-indole-6-carbonitrile (400 mg, 2.3 mmol) and cyclobutylmethylamine (391 mg, 4.6 mmol) were dissolved in methanol (5 mL) and stirred at room temperature for 1 hour. Then sodium cyanoborohydride (290 mg, 4.6 mmol) was added, and the reaction was stirred at 40°C overnight. The reaction solution was concentrated, saturated sodium bicarbonate solution (20 mL) was added, and the aqueous phase was extracted with ethyl acetate (20 mL x 3). The organic phases were combined, washed with saturated sodium chloride solution (20 mL x 3), dried over anhydrous sodium sulfate, and concentrated. The residue was purified by silica gel column chromatography (100% ethyl acetate) to obtain the title compound (500 mg, yellow oil), yield: 89%. MS (ESI): m/z 239.8 [M+H]⁺.

### (2) tert-Butyl 3-(((tert-butoxycarbonyl)(cyclobutylmethyl)amino)methyl)-6-cyano-1H-indole-1-carboxylate

3-(((Cyclobutylmethyl)amino)methyl)-1H-indole-6-carbonitrile (200 mg, 0.84 mmol) and sodium hydride (100 mg, 2.52 mmol, 60%) were dissolved in tetrahydrofuran (10 mL) and stirred at room temperature for 5 minutes. Then di-tert-butyl dicarbonate (545 mg, 2.5 mmol) was added, and the mixture was stirred at room temperature for 3 hours. Water (20 mL) was added to the reaction solution, and the aqueous phase was extracted with ethyl acetate (20 mL x 3). The organic phases were combined, washed with saturated sodium chloride solution (20 mL), dried over anhydrous sodium sulfate, and concentrated. The residue was purified by silica gel column chromatography (petroleum ether/ethyl acetate = 10/1) to obtain the title compound (100 mg, yellow solid), yield: 27%. MS (ESI): m/z 440.0 [M+H]⁺.

### (3) tert-Butyl 6-(aminomethyl)-3-(((tert-butoxycarbonyl)(cyclobutylmethyl)amino)methyl)-1H-indole-1-carboxylate

tert-Butyl 3-(((tert-butoxycarbonyl)(cyclobutylmethyl)amino)methyl)-6-cyano-1H-indole-1-carboxylate (100 mg, 0.23 mmol) was dissolved in ammonia methanol solution (10 mL, 7M), Raney nickel (10 mg) was added, and the reaction was stirred overnight at 50°C under hydrogen atmosphere. The reaction solution was filtered, and the filtrate was concentrated to obtain the title compound (100 mg, yellow oil). MS (ESI): m/z 466.0 [M+Na]⁺.

### (4) tert-Butyl 3-(((tert-butoxycarbonyl)(cyclobutylmethyl)amino)methyl)-6-((4-oxo-4H-pyrido[1,2-a]pyrimidine-2-carboxamido)methyl)-1H-indole-1-carboxylate

Intermediate 3 (17 mg, 0.09 mmol) was dissolved in *N,N*-dimethylformamide (5 mL), *N,N-*diisopropylethylamine (23 mg, 0.18 mmol) and HATU (51 mg, 0.14 mmol) were added, and the mixture was stirred at room temperature for 5 minutes. Then tert-Butyl 6-(aminomethyl)-3-(((tertbutoxycarbonyl)(cyclobutylmethyl)amino)methyl)-1H-indole-1-carboxylate (40 mg, 0.09 mmol) was added, stirring was continued at room temperature for 1 hour. Water (20 mL) was added to the reaction solution, and the aqueous phase was extracted with ethyl acetate (20 mL x 3). The organic phases were combined, washed with saturated sodium chloride solution (20 mL x 3), dried over anhydrous sodium sulfate, and concentrated. The residue was purified by silica gel column chromatography (petroleum ether/ethyl acetate = 50/50) to obtain the title compound (20 mg, colorless oil), yield: 36%. MS (ESI): m/z 638.2 [M+Na]⁺.

### (5) N-((3-(((Cyclobutylmethyl)amino)methyl)-1H-indol-6-yl)methyl)-4-oxo-4H-pyrido[1,2-a]pyrimidine-2-carboxamide

tert-Butyl 3-(((tert-butoxycarbonyl)(cyclobutylmethyl)amino)methyl)-6-((4-oxo-4H-pyrido[1,2-a]pyrimidine-2-carboxamido)methyl)-1H-indole-1-carboxylate (20 mg, 0.03 mmol) was dissolved in dichloromethane (5 mL), trifluoroacetic acid (2 mL) was added, and the mixture was stirred at room temperature for 1 hour. The reaction solution was concentrated, and the residue was purified by Prep-HPLC to obtain the title compound (2.0 mg, white solid), yield: 15%. MS (ESI): m/z 416.1 [M+H]⁺. ¹H NMR (400 MHz, DMSO-*d*₆) δ 10.80 (s, 1H), 9.24 (t, *J =* 6.4 Hz, 1H), 9.01 (d, *J* = 6.4 Hz, 1H), 8.08-8.04 (m, 1H), 7.78 (d, *J =* 8.8 Hz, 1H), 7.55 (d, *J =* 8.0 Hz, 1H), 7.45 (td, *J* = 7.2*,* 1.6 Hz, 1H), 7.33 (s, 1H), 7.19 (s, 1H), 7.00 (d, *J =* 9.2 Hz, 1H), 6.90 (s, 1H), 4.58 (d, *J =* 6.0 Hz, 2H), 3.82 (s, 2H), 2.57 (d, *J =* 7.2 Hz, 2H), 2.45-2.37 (m, 1H), 2.03-1.94 (m, 2H), 1.82-1.76 (m, 2H), 1.65-1.59 (m, 2H).

### Example 20

### N-((2-(((Cyclobutylmethyl)amino)methyl)-1H-indol-6-yl)methyl)-9-oxo-2,3-dihydro-9H-[1,4]dioxolo[2',3':4,5]pyrido[1,2-a]pyrimidine-7-carboxamide

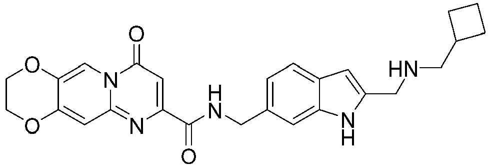

### (1) 2-((6-Bromo-4-iodopyridin-3-yl)oxy)ethan-1-ol

At 0°C, 2-bromo-5-fluoro-4-iodopyridine (3.7 g, 12.26 mmol), ethylene glycol (3.8 g, 61.28 mmol) and sodium tert-butoxide (1.51 g, 13.48 mmol) were dissolved in *N,N*-dimethylformamide (30 mL), and the mixture was heated to 80°C and stirred for 1 hour. The reaction solution was added to water (100 mL), and the aqueous phase was extracted with ethyl acetate (100 mL x 2). The organic phases were combined, washed with saturated sodium chloride solution (50 mL x 3), dried over anhydrous sodium sulfate, and concentrated. The residue was purified by silica gel column chromatography (petroleum ether/ethyl acetate = 60/40) to obtain the title compound (1.0 g, pink solid), yield: 24%. MS (ESI): m/z 343.8 [M+H]⁺.

### (2) 7-Bromo-2,3-dihydro-[1,4]dioxino[2,3-c]pyridine

2-((6-Bromo-4-iodopyridin-3-yl)oxy)ethan-1-ol (1.0 g, 2.91 mmol) was dissolved in isopropanol (20 mL), and potassium tert-butoxide (490 mg, 4.36 mmol), 3,4,7,8-tetramethyl-1,10-phenanthroline (69 mg, 0.29 mmol) and copper iodide (34 mg, 0.17 mmol) were added. The mixture was heated to 80°C and stirred for 1 hour. The reaction solution was added to water (20 mL), then saturated ammonium chloride solution (10 mL) was added, and the aqueous phase was extracted with ethyl acetate (40 mL x 2). The organic phases were combined, washed with saturated sodium chloride solution (30 mL x 3), dried over anhydrous sodium sulfate, and concentrated. The residue was purified by silica gel column chromatography (petroleum ether/ethyl acetate = 85/15) to obtain the title compound (400 mg, white solid), yield: 22%. MS (ESI): m/z 215.9 [M+H]⁺.

### (3) tert-Butyl (2,3-dihydro-[1,4]dioxino[2,3-c]pyridin-7-yl)carbamate

7-Bromo-2,3-dihydro-[1,4]dioxino[2,3-c]pyridine (350 mg, 1.62 mmol) and tert-butyl carbamate (574 mg, 4.86 mmol) were dissolved in 1,4-dioxane (20 mL), and 2-dicyclohexylphosphino-2',4',6'-triisopropylbiphenyl (161 mg, 0.32 mmol), tris(dibenzylideneacetone)dipalladium (154 mg, 0.16 mmol) and cesium carbonate (1.32 g, 4.05 mmol) were added. The mixture was heated to 90°C under nitrogen protection and stirred for 16 hours. The reaction solution was concentrated, and the residue was purified by silica gel column chromatography (petroleum ether/ethyl acetate = 80/20) to obtain the title compound (620 mg, yellow solid), yield: 76%. MS (ESI): m/z 197.0 [M+H-56]⁺.

### (4) 2,3-Dihydro-[1,4]dioxino[2,3-c]pyridin-7-amine

tert-Butyl (2,3-dihydro-[1,4]dioxino[2,3-c]pyridin-7-yl)carbamate (600 mg, 2.38 mmol) was dissolved in dichloromethane (5 mL), trifluoroacetic acid (5 mL) was added, and the mixture was stirred at room temperature for 12 hours. The reaction solution was concentrated, and the residue was purified by silica gel column chromatography (100% ethyl acetate) to obtain the title compound (140 mg, yellow solid), yield: 39%. MS (ESI): m/z 153.0 [M+H]⁺.

### (5) Methyl 9-oxo-2,3-dihydro-9H-[1,4]dioxolo[2',3':4,5]pyrido[1,2-a]pyrimidine-7-carboxylate

2,3-Dihydro-[1,4]dioxino[2,3-c]pyridin-7-amine (100 mg, 0.66 mmol) and dimethyl but-2-ynedioate (160 mg, 0.72 mmol) were dissolved in water (15 mL) and stirred at room temperature for 12 hours. The reaction solution was extracted with ethyl acetate (30 mL), and the organic phase was concentrated. The residue was purified by silica gel column chromatography (petroleum ether/ethyl acetate = 90/10) to obtain the title compound (100 mg, yellow solid), yield: 58%. MS (ESI): m/z 263.0 [M+H]⁺.

### (6) 9-Oxo-2,3-dihydro-9H-[1,4]dioxolo[2',3':4,5]pyrido[1,2-a]pyrimidine-7-carboxylic acid

Methyl 9-oxo-2,3-dihydro-9H-[1,4]dioxolo[2',3':4,5]pyrido[1,2-a]pyrimidine-7-carboxylate (100 mg, 0.38 mmol) was dissolved in methanol/water (5 mL/5 mL), sodium hydroxide (76 mg, 1.91 mmol) was added, and the mixture was heated to 80°C and stirred for 4 hours. The reaction solution was concentrated, 1 M dilute hydrochloric acid was added to adjust the pH to 5, and the aqueous phase was extracted with dichloromethane (30 mL x 3). The organic phases were combined and concentrated to obtain the title compound (60 mg, yellow solid), yield: 63%. MS (ESI): m/z 249.0 [M+H]⁺.

### (7) tert-Butyl 2-(((tert-butoxycarbonyl)(cyclobutylmethyl)amino)methyl)-6-((9-oxo-2,3-dihydro-9H-[1,4]dioxolo[2',3':4,5]pyrido[1,2-a]pyrimidine-7-carboxamido)methyl)-1H-indole-1-carboxylate

9-Oxo-2,3-dihydro-9H-[1,4]dioxolo[2',3':4,5]pyrido[1,2-a]pyrimidine-7-carboxylic acid (60 mg, 0.24 mmol) was dissolved in *N,N-*dimethylformamide (10 mL), *N,N*-diisopropylethylamine (94 mg, 0.72 mmol) and HATU (185 mg, 0.48 mmol) were added, and the mixture was stirred at room temperature for 5 minutes. Then a solution of Intermediate 1 (321 mg, 0.73 mmol) in *N,N-*dimethylformamide (5 mL) was added, stirring was continued at room temperature for 12 hours. The reaction solution was added to saturated sodium bicarbonate solution (10 mL), and the aqueous phase was extracted with ethyl acetate (20 mL x 3). The organic phases were combined, washed with saturated sodium chloride solution (20 mL x 3), dried over anhydrous sodium sulfate, and concentrated. The residue was purified by silica gel column chromatography (petroleum ether/ethyl acetate = 50/50) to obtain the title compound (90 mg, yellow oil), yield: 14%. MS (ESI): m/z 574.1 [M+H-100]⁺.

### (8) N-((2-(((Cyclobutylmethyl)amino)methyl)-1H-indol-6-yl)methyl)-9-oxo-2,3-dihydro-9H-[1,4]dioxolo[2',3':4,5]pyrido[1,2-a]pyrimidine-7-carboxamide

tert-Butyl 2-(((tert-butoxycarbonyl)(cyclobutylmethyl)amino)methyl)-6-((9-oxo-2,3-dihydro-9H-[1,4]dioxolo[2',3':4,5]pyrido[1,2-a]pyrimidine-7-carboxamido)methyl)-1H-indole-1-carboxylate (90 mg, 0.04 mmol) was dissolved in dichloromethane (5 mL), trifluoroacetic acid (5 mL) was added, and the mixture was stirred at room temperature for 2 hours. The reaction solution was added to saturated sodium bicarbonate solution (10 mL), and the aqueous phase was extracted with dichloromethane (10 mL x 3). The organic phases were combined, washed with saturated sodium chloride solution (10 mL x 3), dried over anhydrous sodium sulfate, and concentrated. The residue was diluted with *N,N*-dimethylformamide and purified by Prep-HPLC to obtain the title compound (4.0 mg, white solid), yield: 21%. MS (ESI): m/z 474.1 [M+H]⁺. ¹H NMR (400 MHz, DMSO-*d*₆) δ 10.86 (s, 1H), 9.05 (t, *J =* 6.0 Hz, 1H), 8.62 (s, 1H), 7.36 (d, *J =* 8.0 Hz, 1H), 7.29 (s, 1H), 7.19 (s, 1H), 6.94 (d, *J =* 8.0 Hz, 1H), 6.67 (s, 1H), 6.19 (s, 1H), 4.59-4.52 (m, 4H), 4.49 (d, *J =* 6.0 Hz, 2H), 3.76 (s, 2H), 2.59-2.52 (m, 2H), 2.41-2.38 (m, 1H), 2.01-1.95 (m, 2H), 1.83-1.75 (m, 2H), 1.66-1.59 (m, 2H).

### Example 21

### N-((6-(((Cyclobutylmethyl)amino)methyl)-1H-indol-2-yl)methyl)-4-oxo-4H-pyrido[1,2-a]pyrimidine-2-carboxamide

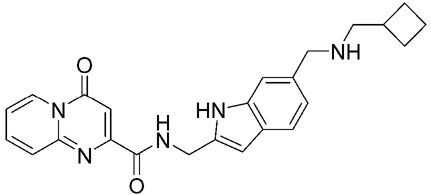

### (1) 6-Formyl-1H-indole-2-carbonitrile

6-Bromo-1H-indole-2-carbonitrile (600 mg, 2.7 mmol) was dissolved in tetrahydrofuran (5 mL), sodium hydride (432 mg, 10.8 mmol, 60%) was added at 0°C, and the mixture was stirred under nitrogen protection for 15 minutes. The reaction solution was cooled to -78°C, n-butyllithium (2.7 mL, 6.75 mmol, 2.5 M) was slowly added dropwise, and the reaction was continued to stir for 20 minutes. Then *N,N*-dimethylformamide (1.18 g, 16.2 mmol) was added, and the mixture was continued to stir at -78°C for 2 hours. The reaction solution was warmed to room temperature, saturated ammonium chloride solution (40 mL) was added to quench the reaction, and extracted with ethyl acetate (30 mL x 3). The organic phases were combined, washed with saturated sodium chloride solution (50 mL), dried over anhydrous sodium sulfate, and concentrated. The residue was purified by silica gel column chromatography (petroleum ether/ethyl acetate = 80/20) to obtain the title compound (270 mg, yellow solid), yield: 58%. MS (ESI): m/z 171.0 [M+H]⁺. ¹H NMR (300 MHz, DMSO-*d*₆) δ 12.89 (s, 1H), 10.11 (s, 1H), 8.11 (s, 1H), 7.88 (d, *J =* 8.4 Hz, 1H), 7.68 (dd, *J* = 8.4, 1.2 Hz, 1H), 7.52 (s, 1H).

### (2) 6-(((Cyclobutylmethyl)amino)methyl)-1H-indole-2-carbonitrile

6-Formyl-1H-indole-2-carbonitrile (270 mg, 1.6 mmol) and cyclobutylmethylamine (272 mg, 3.2 mmol) were dissolved in dichloroethane (5 mL), and the mixture was stirred at 65°C for 1 hour. Then sodium cyanoborohydride (302 mg, 4.8 mmol) was added, and the reaction was continued to stir at 65°C for 2 hours. The reaction solution was added to saturated sodium bicarbonate solution (30 mL), and the aqueous phase was extracted with ethyl acetate (15 mL x 3). The organic phases were combined, washed with saturated sodium chloride solution (20 mL x 2), dried over anhydrous sodium sulfate, and concentrated. The residue was purified by silica gel column chromatography (dichloromethane/methanol = 97/3) to obtain the title compound (150 mg, red oil), yield: 39%. MS (ESI): m/z 240.1 [M+H]⁺.

### (3) tert-Butyl 6-(((tert-butoxycarbonyl)(cyclobutylmethyl)amino)methyl)-2-cyano-1H-indole-1-carboxylate

6-(((Cyclobutylmethyl)amino)methyl)-1H-indole-2-carbonitrile (150 mg, 0.63 mmol) and sodium hydride (101 mg, 2.52 mmol, 60%) were dissolved in tetrahydrofuran (3 mL) and stirred at room temperature for 5 minutes. Then di-tert-butyl dicarbonate (549 mg, 2.52 mmol) was added, stirring was continued at room temperature for 17 hours. The reaction solution was added to water (10 mL), and the aqueous phase was extracted with ethyl acetate (10 mL x 3). The organic phases were combined, washed with saturated sodium chloride solution (20 mL), dried over anhydrous sodium sulfate, and concentrated. The residue was purified by silica gel column chromatography (petroleum ether/ethyl acetate = 95/5) to obtain the title compound (100 mg, colorless oil), yield: 36%. MS (ESI): m/z 462.1 [M+Na]⁺.

### (4) tert-Butyl 2-(aminomethyl)-6-(((tert-butoxycarbonyl)(cyclobutylmethyl)amino)methyl)-1H-indole-1-carboxylate

tert-Butyl 6-(((tert-butoxycarbonyl)(cyclobutylmethyl)amino)methyl)-2-cyano-1H-indole-1-carboxylate (40 mg, 0.09 mmol) was dissolved in 7 M ammonia methanol solution (10 mL), Raney nickel (10 mg) was added, and the mixture was stirred overnight at room temperature under hydrogen atmosphere. The reaction solution was filtered to remove Raney nickel, the filtrate was concentrated, and the residue was purified by silica gel column chromatography (dichloromethane/methanol = 10/1) to obtain the title compound (20 mg, colorless oil), yield: 36%. MS (ESI): m/z 444.3 [M+H]⁺.

### (5) tert-Butyl 6-(((tert-butoxycarbonyl)(cyclobutylmethyl)amino)methyl)-2-((4-oxo-4H-pyrido[1,2-a]pyrimidine-2-carboxamido)methyl)-1H-indole-1-carboxylate

tert-Butyl 2-(aminomethyl)-6-(((tert-butoxycarbonyl)(cyclobutylmethyl)amino)methyl)-1H-indole-1-carboxylate (15 mg, 0.03 mmol) was dissolved in N,N-dimethylformamide (5 mL), and Intermediate 3 (5.7 mg, 0.03 mmol), N,N-diisopropylethylamine (7.7 mg, 0.06 mmol) and HATU (22.8 mg, 0.06 mmol) were added, and the mixture was stirred at room temperature for 2 hours. The reaction solution was added to water (20 mL), and the aqueous phase was extracted with ethyl acetate (20 mL x 3). The organic phases were combined, washed with saturated sodium chloride solution (20 mL x 3), dried over anhydrous sodium sulfate, and concentrated. The residue was purified by silica gel column chromatography (petroleum ether/ethyl acetate = 50/50) to obtain the title compound (10 mg, colorless oil), yield: 48%. MS (ESI): m/z 616.3 [M+H]⁺.

### (6)N-((6-(((Cyclobutylmethyl)amino)methyl)-1H-indol-2-yl)methyl)-4-oxo-4H-pyrido[1,2-a]pyrimidine-2-carboxamide

tert-Butyl 6-(((tert-butoxycarbonyl)(cyclobutylmethyl)amino)methyl)-2-((4-oxo-4H-pyrido[1,2-a]pyrimidine-2-carboxamido)methyl)-1H-indole-1-carboxylate (10 mg, 0.02 mmol) was dissolved in dichloromethane (5 mL), trifluoroacetic acid (2 mL) was added, and the mixture was stirred at room temperature for 1 hour. The reaction solution was added to sodium bicarbonate solution (20 mL), and the aqueous phase was extracted with dichloromethane/methanol mixed solvent. The organic phases were combined, concentrated, and the residue was purified by Prep-HPLC to obtain the title compound (1.7 mg, white solid), yield: 25%. MS (ESI): m/z 416.1 [M+H]⁺. ¹H NMR (400 MHz, DMSO-*d*₆) δ 10.89 (s, 1H), 9.19 (t, *J =* 6.0 Hz, 1H), 9.02 (d, *J =* 6.4 Hz, 1H), 8.10-8.05 (m, 1H), 7.80 (d, *J* = 8.4 Hz, 1H), 7.46 (td, *J* = 7.2, 1.2 Hz, 1H), 7.37 (d, *J =* 8.0 Hz, 1H), 7.30 (s, 1H), 6.94-6.92 (m, 2H), 6.27 (s, 1H), 4.65 (d, *J =* 6.4 Hz, 2H), 3.75 (s, 2H), 2.54-2.51 (m, 2H), 2.44-2.39 (m, 1H), 2.00-1.95 (m, 2H), 1.82-1.75 (m, 2H), 1.66-1.59 (m, 2H).

### Example 22

### N-((2-(((Cyclobutylmethyl)amino)methyl)-1H-indol-6-yl)methyl)pyrazolo[1,5-a]pyrido[2,3-e]pyrazine-8-carboxamide

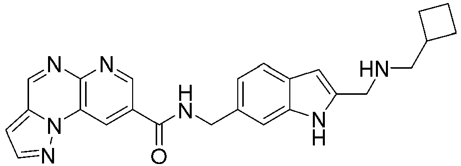

### (1) Methyl 6-(((1H-pyrazol-3-yl)methyl)amino)-5-bromonicotinate

Methyl 5-bromo-6-chloronicotinate (900 mg, 3.6 mmol) and (1H-pyrazol-3-yl)methanamine (349 mg, 3.6 mmol) were dissolved in N,N-dimethylformamide (10 mL), triethylamine (727 mg, 7.2 mmol) was added, and the mixture was heated to 90°C to react overnight. The reaction solution was purified by reverse-phase column to obtain the title compound (790 mg, yellow solid), yield: 71%. MS (ESI): m/z 310.9 [M+H]⁺.

### (2) Methyl pyrazolo[1,5-a]pyrido[2,3-e]pyrazine-8-carboxylate

Methyl 6-(((1H-pyrazol-3-yl)methyl)amino)-5-bromonicotinate (600 mg, 1.9 mmol), L-proline (44 mg, 0.38 mmol) and cuprous iodide (36 mg, 0.19 mmol) were dissolved in *N*,*N-*dimethylformamide (10 mL), cesium carbonate (1.2 g, 3.8 mmol) was added, and the mixture was heated to 100°C to react overnight. The reaction solution was added to water (30 mL), and the aqueous phase was extracted with ethyl acetate (2 x 20 mL). The organic phases were combined, washed with saturated sodium chloride solution (20 mL), dried over anhydrous sodium sulfate, filtered, and concentrated. The residue was purified by silica gel column chromatography (petroleum ether/ethyl acetate = 50/50) to obtain the title compound (100 mg, yellow solid), yield: 23%. MS (ESI): m/z 228.8 [M+H]⁺.

### (3) Pyrazolo[1,5-a]pyrido[2,3-e]pyrazine-8-carboxylic acid

Methyl pyrazolo[1,5-a]pyrido[2,3-e]pyrazine-8-carboxylate (100 mg, 0.44 mmol) was dissolved in methanol/water (10 mL/2 mL), sodium hydroxide (88 mg, 2.2 mmol) was added, and the mixture was stirred at room temperature overnight. The reaction solution was adjusted to acidic with 1 M dilute hydrochloric acid, and the solid was filtered to obtain the title compound (70 mg, yellow solid), yield: 75%. MS (ESI): m/z 214.8 [M+H]⁺.

### (4) tert-Butyl 2-(((tert-butoxycarbonyl)(cyclobutylmethyl)amino)methyl)-6-((pyrazolo[1,5-a]pyrido[2,3-e]pyrazine-8-carboxamido)methyl)-1H-indole-1-carboxylate

Pyrazolo[1,5-a]pyrido[2,3-e]pyrazine-8-carboxylic acid (20 mg, 0.09 mmol) and Intermediate 2 (40 mg, 0.09 mmol) were dissolved in *N,N*-dimethylformamide (5 mL), 1-propylphosphonic anhydride (114 mg, 0.18 mmol, 50% ethyl acetate solution) and triethylamine (18 mg, 0.18 mmol) were added, and the mixture was stirred at room temperature overnight. The reaction solution was poured into water (20 mL), and the aqueous phase was extracted with ethyl acetate (2 x 20 mL). The organic phases were combined, washed with saturated sodium chloride solution (20 mL), dried over anhydrous sodium sulfate, filtered, and concentrated. The residue was purified by silica gel column chromatography (petroleum ether/ethyl acetate = 50/50) to obtain the title compound (20 mg, yellow oil), yield: 35%. MS (ESI): m/z 662.2 [M+Na]⁺.

### (5) N-((2-(((Cyclobutylmethyl)amino)methyl)-1H-indol-6-yl)methyl)pyrazolo[1,5-a]pyrido[2,3-e]pyrazine-8-carboxamide

tert-Butyl 2-(((tert-butoxycarbonyl)(cyclobutylmethyl)amino)methyl)-6-((pyrazolo[1,5-a]pyrido[2,3-e]pyrazine-8-carboxamido)methyl)-1H-indole-1-carboxylate (20 mg, 0.03 mmol) was dissolved in dichloromethane (5 mL), trifluoroacetic acid (3 mL) was added, and the reaction was carried out at room temperature for 2 hours. The reaction solution was poured into sodium bicarbonate solution (20 mL), and the aqueous phase was extracted with dichloromethane/methanol mixed solvent. The organic phases were combined, concentrated, and the residue was purified by Prep-HPLC to obtain the title compound (0.8 mg, yellow solid), yield: 6%. MS (ESI): m/z 440.1 [M+H]⁺. ¹H NMR (400 MHz, DMSO-*d*₆) δ 10.86 (s, 1H), 9.63 (t, *J =* 5.6 Hz, 1H), 9.53 (s, 1H), 9.34-9.32 (m, 2H), 8.38 (d, *J =* 2.0 Hz, 1H), 7.40 (d, *J =* 8.0 Hz, 1H), 7.34 (s, 1H), 7.27 (d, *J* = 2.0 Hz, 1H), 7.00 (d, *J =* 9.2 Hz, 1H), 6.21 (s, 1H), 4.63 (d, *J =* 6.0 Hz, 2H), 3.78 (s, 2H), 2.59-2.57 (m, 2H), 2.43-2.37 (m, 1H), 2.03-1.99 (m, 2H), 1.82-1.75 (m, 2H), 1.64-1.59 (m, 2H).

### Example 23

### N-((2-(((Cyclobutylmethyl)amino)methyl)-1H-indol-6-yl)methyl)-3-cyclopropyl-[1,2,4]triazolo[4,3-a]pyrimidine-6-carboxamide

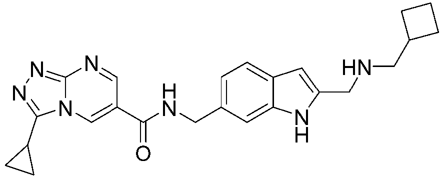

### (1) 6-Bromo-3-cyclopropyl-[1,2,4]triazolo[4,3-a]pyrimidine

5-Bromo-2-hydrazinopyrimidine (360 mg, 1.9 mmol) was dissolved in dichloromethane (20 mL), cyclopropyl formaldehyde (133 mg, 1.9 mmol) was added, then two drops of glacial acetic acid were added, and the mixture was stirred at room temperature for 2 hours. After the system gradually became clear, iodobenzene diacetate (676 mg, 2.1 mmol) was added, and the reaction was continued for 2 hours. The reaction solution was concentrated, and the residue was purified by silica gel column chromatography (dichloromethane/methanol = 94/6) to obtain the title compound (370 mg, white solid), yield: 81.5%. MS (ESI): m/z 238.9 [M+H]⁺.

### (2) Methyl 3-cyclopropyl-[1,2,4]triazolo[4,3-a]pyrimidine-6-carboxylate

6-Bromo-3-cyclopropyl-[1,2,4]triazolo[4,3-a]pyrimidine (700 mg, 2.9 mmol) was dissolved in methanol/acetonitrile (10 mL/10 mL), 1,3-bis(diphenylphosphino)propane (157 mg, 0.38 mmol), palladium acetate (65 mg, 0.29 mmol) and triethylamine (737 mg, 7.3 mmol) were added, and the mixture was heated to 100°C in a high-pressure reactor (3 MPa, carbon monoxide atmosphere) to react overnight. The reaction solution was filtered, concentrated, and the residue was purified by silica gel column chromatography (petroleum ether/ethyl acetate = 50/50) to obtain the title compound (130 mg, yellow solid), yield: 20%. MS (ESI): m/z 218.8 [M+H]⁺.

### (3) 3-Cyclopropyl-[1,2,4]triazolo[4,3-a]pyrimidine-6-carboxylic acid

Methyl 3-cyclopropyl-[1,2,4]triazolo[4,3-a]pyrimidine-6-carboxylate (130 mg, 0.6 mmol) was dissolved in methanol/water (10 mL/2 mL), sodium hydroxide (120 mg, 3 mmol) was added, and the reaction was carried out at room temperature overnight. The reaction solution was adjusted to acidic with 1M dilute hydrochloric acid, concentrated, and the residue was purified by reverse-phase column to obtain the title compound (20 mg, white solid), yield: 16%. MS (ESI): m/z 204.8 [M+H]⁺.

### (4) tert-Butyl 2-(((tert-butoxycarbonyl)(cyclobutylmethyl)amino)methyl)-6-((3-cyclopropyl-[1,2,4]triazolo[4,3-a]pyrimidine-6-carboxamido)methyl)-1H-indole-1-carboxylate

3-Cyclopropyl-[1,2,4]triazolo[4,3-a]pyrimidine-6-carboxylic acid (20 mg, 0.1 mmol) and Intermediate 1 (44 mg, 0.1 mmol) were dissolved in N,N-dimethylformamide (5 mL), 1-propylphosphonic anhydride (190 mg, 0.3 mmol, 50% ethyl acetate solution) and triethylamine (20 mg, 0.2 mmol) were added, and the mixture was heated to 50°C and stirred overnight. The reaction solution was poured into water (20 mL), and the aqueous phase was extracted with ethyl acetate (2 x 20 mL). The organic phases were combined, washed with saturated sodium chloride solution (20 mL), dried over anhydrous sodium sulfate, filtered, and concentrated. The residue was purified by silica gel column chromatography (petroleum ether/ethyl acetate = 50/50) to obtain the title compound (10 mg, yellow oil), yield: 16%. MS (ESI): m/z 630.2 [M+H]⁺.

### (5) N-((2-(((Cyclobutylmethyl)amino)methyl)-1H-indol-6-yl)methyl)-3-cyclopropyl-[1,2,4]triazolo[4,3-a]pyrimidine-6-carboxamide

tert-Butyl 2-(((tert-butoxycarbonyl)(cyclobutylmethyl)amino)methyl)-6-((3-cyclopropyl-[1,2,4]triazolo[4,3-a]pyrimidine-6-carboxamido)methyl)-1H-indole-1-carboxylate (10 mg, 0.01 mmol) was dissolved in dichloromethane (5 mL), trifluoroacetic acid (3 mL) was added, and the reaction was carried out at room temperature for 2 hours. Saturated aqueous sodium bicarbonate solution (20 mL) was added to the reaction solution, extracted with dichloromethane/methanol (10 mL/1 mL), the organic phase was concentrated, and the residue was purified by Prep-HPLC to obtain the title compound (1.0 mg, white solid), yield: 15%. MS (ESI): m/z 430.1 [M+H]⁺. ¹H NMR (400 MHz, DMSO-*d*₆) δ 10.86 (s, 1H), 9.64 (d, *J =* 2.4 Hz, 1H), 9.27 (t, *J =* 4.8 Hz, 1H), 9.17 (d, *J* = 2.4 Hz, 1H), 7.38 (d, *J =* 8.4 Hz, 1H), 7.31 (s, 1H), 6.97 (d, *J =* 8.0 Hz, 1H), 6.21 (s, 1H), 4.58 (d, *J =* 5.2 Hz, 2H), 3.78 (s, 2H), 2.59-2.56 (m, 2H), 2.41-2.37 (m, 1H), 2.22-2.18 (m, 1H), 2.03-1.96 (m, 2H), 1.83-1.75 (m, 2H), 1.66-1.59 (m, 2H), 1.14-1.10 (m, 2H), 1.05-1.03 (m, 2H).

### Example 24

### N-((2-((((3,3-Difluorocyclobutyl)methyl)amino)methyl)-1H-indol-6-yl)methyl)-9-oxo-2,3-dihydro-9H-[1,4]dioxino[2',3':4,5]pyrido[1,2-a]pyrimidine-7-carboxamide

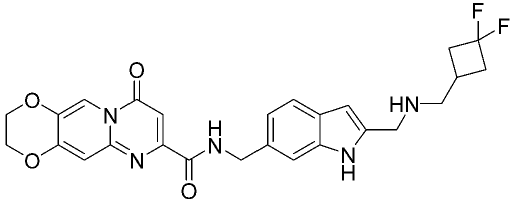

### (1) tert-Butyl 2-(((tert-butoxycarbonyl)((3,3-difluorocyclobutyl)methyl)amino)methyl)-6-((9-oxo-2,3-dihydro-9H-[1,4]dioxino[2',3':4,5]pyrido[1,2-a]pyrimidine-7-carboxamido)methyl)-1H-indole-1-carboxylate

Intermediate 4 (15 mg, 0.06 mmol) was dissolved in N,N-dimethylformamide (2 mL), *N,N-*diisopropylethylamine (39 mg, 0.30 mmol) and HATU (46 mg, 0.12 mmol) were added, and the reaction was stirred at room temperature for 5 minutes. Then a solution of Intermediate 6 (40 mg, 0.08 mmol) in N,N-dimethylformamide (2 mL) was added, and the reaction was continued to stir at room temperature for 1 hour. The reaction solution was added to saturated sodium bicarbonate solution (20 mL), and the aqueous phase was extracted with ethyl acetate (20 mL x 3). The organic phases were combined, washed with saturated sodium chloride solution (20 mL x 3), dried over anhydrous sodium sulfate, and concentrated. The residue was purified by silica gel column chromatography (petroleum ether/ethyl acetate = 40/60) to obtain the title compound (17 mg, yellow oil), yield: 40%. MS (ESI): m/z 710.0 [M+H]⁺.

### (2) N-((2-((((3,3-Difluorocyclobutyl)methyl)amino)methyl)-1H-indol-6-yl)methyl)-9-oxo-2,3-dihydro-9H-[1,4]dioxino[2',3':4,5]pyrido[1,2-a]pyrimidine-7-carboxamide

tert-Butyl 2-(((tert-butoxycarbonyl)((3,3-difluorocyclobutyl)methyl)amino)methyl)-6-((9-oxo-2,3-dihydro-9H-[1,4]dioxino[2',3':4,5]pyrido[1,2-a]pyrimidine-7-carboxamido)methyl)-1H-indole-1-carboxylate (17 mg, 0.024mmol) was dissolved in dichloromethane (3 mL), trifluoroacetic acid (1 mL) was added, and the mixture was stirred at room temperature for 2 hours. The reaction solution was added to saturated sodium bicarbonate solution (30 mL), extracted with dichloromethane (15 mL x 3), the organic phases were combined and concentrated. The residue was diluted with methanol and purified by Prep-HPLC to obtain the title compound (3.4 mg, white solid), yield: 28%. MS (ESI): m/z 510.1 [M+H]⁺. ¹H NMR (400 MHz, DMSO-*d*₆) δ 10.86 (s, 1H), 9.05 (t, *J =* 6.0 Hz, 1H), 8.62 (s, 1H), 7.37 (d, *J =* 8.0 Hz, 1H), 7.29 (s, 1H), 7.19 (s, 1H), 6.95 (dd, *J* = 8.0, 1.2 Hz, 1H), 6.67 (s, 1H), 6.21 (s, 1H), 4.57-4.53 (m, 4H), 4.47-4.45 (m, 2H), 3.80 (s, 2H), 2.58-2.57 (m, 2H), 2.54-2.53 (m, 1H), 2.32-2.18 (m, 4H).

### Example 25

### N-((2-(((Cyclobutylmethyl)amino)methyl)-1H-indol-6-yl)methyl)-8-oxo-8H-[1,3]dioxolo[4',5':4,5]pyrido[1,2-a]pyrimidine-6-carboxamide

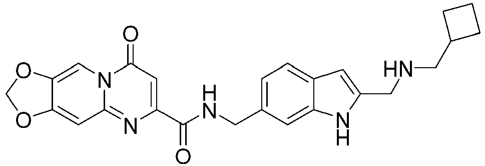

### (1) tert-Butyl 2-(((tert-butoxycarbonyl)(cyclobutylmethyl)amino)methyl)-6-((8-oxo-8H-[1,3]dioxolo[4',5':4,5]pyrido[1,2-a]pyrimidine-6-carboxamido)methyl)-1H-indole-1-carboxylate

Intermediate 5 (23 mg, 0.1 mmol) was dissolved in *N,N-*dimethylformamide (3 mL), *N,N-*diisopropylethylamine (65 mg, 0.5 mmol) and HATU (76 mg, 0.2 mmol) were added, and the reaction was stirred at room temperature for 5 minutes, then Intermediate 1 (44 mg, 0.1 mmol) was added, and the reaction was continued to stir at room temperature for 1 hour. The reaction solution was purified by reverse-phase column to obtain the title compound (11 mg, pale yellow oil), yield: 16.7%. MS (ESI): m/z 660.4 [M+H]⁺.

### (2) N-((2-(((Cyclobutylmethyl)amino)methyl)-1H-indol-6-yl)methyl)-8-oxo-8H-[1,3]dioxolo[4',5':4,5]pyrido[1,2-a]pyrimidine-6-carboxamide

tert-Butyl 2-(((tert-butoxycarbonyl)(cyclobutylmethyl)amino)methyl)-6-((8-oxo-8H-[1,3]dioxolo[4',5':4,5]pyrido[1,2-a]pyrimidine-6-carboxamido)methyl)-1H-indole-1-carboxylate (11 mg, 0.017 mmol) was dissolved in dichloromethane (3 mL), trifluoroacetic acid (3 mL) was added, and the mixture was stirred at room temperature for 3 hours. The reaction solution was concentrated, and the residue was diluted with methanol (1 mL) and purified by Prep-HPLC to obtain the title compound (3.3 mg, white solid), yield: 43%. MS (ESI): m/z 460.1 [M+H]⁺. ¹H NMR (400 MHz, DMSO-*d*₆) δ 10.86 (s, 1H), 9.02 (t, *J =* 6.0 Hz, 1H), 8.59 (s, 1H), 7.36 (d, *J =* 8.0 Hz, 1H), 7.29 (s, 1H), 7.14 (s, 1H), 6.94 (dd, *J =* 8.0, 1.2 Hz, 1H), 6.73 (s, 1H), 6.37 (s, 2H), 6.20 (s, 1H), 4.54 (d, *J =* 6.4 Hz, 2H), 3.77 (s, 2H), 2.54-2.52 (m, 2H), 2.43-2.37 (m, 1H), 2.01-1.93 (m, 2H), 1.85-1.72 (m, 2H), 1.66-1.59 (m, 2H).

### Example 26

### N-((2-((((3,3-Difluorocyclobutyl)methyl)amino)methyl)-1H-indol-6-yl)methyl)-8-oxo-8H-[1,3]dioxolo[4',5':4,5]pyrido[1,2-a]pyrimidine-6-carboxamide

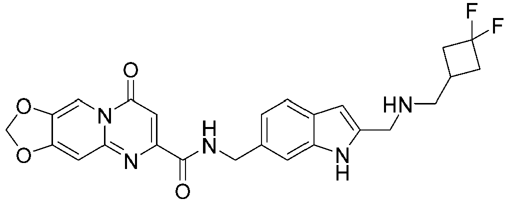

### (1) tert-Butyl 2-(((tert-butoxycarbonyl)((3,3-difluorocyclobutyl)methyl)amino)methyl)-6-((8-oxo-8H-[1,3]dioxolo[4',5':4,5]pyrido[1,2-a]pyrimidine-6-carboxamido)methyl)-1H-indole-1-carboxylate

Intermediate 5 (19 mg, 0.08 mmol) was dissolved in *N,N*-dimethylformamide (2 mL), *N,N-*diisopropylethylamine (52 mg, 0.40 mmol) and HATU (61 mg, 0.16 mmol) were added, and the reaction was stirred at room temperature for 5 minutes. Then a solution of Intermediate 6 (37 mg, 0.08 mmol) in *N,N*-dimethylformamide (1 mL) was added, stirring was continued at room temperature for 1 hour. The reaction solution was added to saturated sodium bicarbonate solution (20 mL), and the aqueous phase was extracted with ethyl acetate (20 mL x 3). The organic phases were combined, washed with saturated sodium chloride solution (20 mL x 3), dried over anhydrous sodium sulfate, and concentrated. The residue was purified by silica gel column chromatography (petroleum ether/ethyl acetate = 30/70) to obtain the title compound (25 mg, yellow oil), yield: 46%. MS (ESI): m/z 696.1 [M+H]⁺.

### (2) N-((2-((((3,3-Difluorocyclobutyl)methyl)amino)methyl)-1H-indol-6-yl)methyl)-8-oxo-8H-[1,3]dioxolo[4',5':4,5]pyrido[1,2-a]pyrimidine-6-carboxamide

tert-Butyl 2-(((tert-butoxycarbonyl)((3,3-difluorocyclobutyl)methyl)amino)methyl)-6-((8-oxo-8H-[1,3]dioxolo[4',5':4,5]pyrido[1,2-a]pyrimidine-6-carboxamido)methyl)-1H-indole-1-carboxylate (25 mg, 0.036mmol) was dissolved in dichloromethane (3 mL), trifluoroacetic acid (1 mL) was added, and the mixture was stirred at room temperature for 2 hours. The reaction solution was added to saturated sodium bicarbonate solution (30 mL), and the aqueous phase was extracted with dichloromethane (15 mL x 3). The organic phases were combined, concentrated, and the residue was diluted with methanol (2mL) and purified by Prep-HPLC to obtain the title compound (1.0 mg, pale yellow solid), yield: 6%. MS (ESI): m/z 496.1 [M+H]⁺. ¹H NMR (400 MHz, DMSO-*d*₆) δ 10.87 (s, 1H), 9.04 (t, *J =* 6.0 Hz, 1H), 8.59 (s, 1H), 7.37 (d, *J =* 8.0 Hz, 1H), 7.29 (s, 1H), 7.14 (s, 1H), 6.94 (dd, *J =* 8.4, 1.6 Hz, 1H), 6.73 (s, 1H), 6.37 (s, 2H), 6.21 (d, *J* =1.6 Hz, 1H), 4.54 (d, *J* = 6.4 Hz, 2H), 3.79 (s, 2H), 2.58-2.56 (m, 3H), 2.27-2.20 (m, 4H).

### Example 27

### N-((2-((4,4-Difluoropiperidin-1-yl)methyl)-1H-indol-6-yl)methyl)-9-oxo-2,3-dihydro-9H-[1,4]dioxolo[2',3':4,5]pyrido[1,2-a]pyrimidine-7-carboxamide

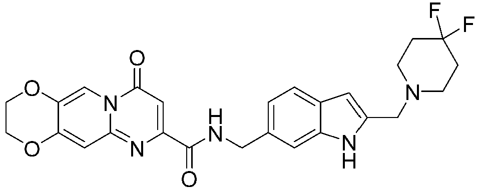

### (1) 2-((4,4-Difluoropiperidin-1-yl)methyl)-1H-indole-6-carbonitrile

2-Formyl-1H-indole-6-carbonitrile (250 mg, 1.50 mmol, Step 4 of Intermediate 1) and 4,4-difluoropiperidine hydrochloride (591 mg, 3.75 mmol) were dissolved in dichloroethane (5 mL) and stirred at 65°C for 1 hour. Then sodium cyanoborohydride (331 mg, 5.25 mmol) was added, and the reaction was continued to stir at 65°C for 2 hours. The reaction solution was added to saturated sodium bicarbonate solution (30 mL), and the aqueous phase was extracted with ethyl acetate (20 mL x 3). The organic phases were combined, washed with saturated sodium chloride solution (50 mL), dried over anhydrous sodium sulfate, and concentrated. The residue was purified by silica gel column chromatography (petroleum ether/ethyl acetate = 40/60) to obtain the title compound (260 mg, pale yellow solid), yield: 64%. MS (ESI): m/z 276.0 [M+H]⁺.

### (2) tert-Butyl ((2-((4,4-difluoropiperidin-1-yl)methyl)-1H-indol-6-yl)methyl)carbamate

2-((4,4-Difluoropiperidin-1-yl)methyl)-1H-indole-6-carbonitrile (130 mg, 0.47mmol) was dissolved in methanol (5 mL), nickel chloride hexahydrate (12 mg, 0.05 mmol) and sodium borohydride (133 mg, 3.5 mmol) were added, followed by di-tert-butyl dicarbonate (218 mg, 1.0 mmol), and the mixture was stirred at room temperature for 1 hour. The reaction solution was concentrated to remove methanol, then water (30 mL) was added, and the aqueous phase was extracted with ethyl acetate (20 mL x 3). The organic phases were combined, washed with saturated sodium chloride solution (30 mL), dried over anhydrous sodium sulfate, and concentrated. The residue was purified by silica gel column chromatography (petroleum ether/ethyl acetate = 50/50) to obtain the title compound (100 mg, pale yellow oil), yield: 56%. MS (ESI): m/z 402.0 [M+Na]⁺.

### (3) (2-((4,4-Difluoropiperidin-1-yl)methyl)-1H-indol-6-yl)methanamine

tert-Butyl ((2-((4,4-difluoropiperidin-1-yl)methyl)-1H-indol-6-yl)methyl)carbamate (50 mg, 0.13 mmol) was dissolved in dichloromethane (2 mL), trifluoroacetic acid (1 mL) was added, and the mixture was stirred at room temperature for 1 hour. The reaction solution was concentrated to obtain the title compound (37 mg, brown oil), yield: 100%. MS (ESI): m/z 263.0 [M+H-NH₃]⁺.

### (4) N-((2-((4,4-Difluoropiperidin-1-yl)methyl)-1H-indol-6-yl)methyl)-9-oxo-2,3-dihydro-9H-[1,4]dioxolo[2',3':4,5]pyrido[1,2-a]pyrimidine-7-carboxamide

Intermediate 4 (10 mg, 0.04 mmol) and (2-((4,4-difluoropiperidin-1-yl)methyl)-1H-indol-6-yl)methanamine (11 mg, 0.04 mmol) were dissolved in *N,N-*dimethylformamide (5 mL), then *N,N-*diisopropylethylamine (10 mg, 0.08 mmol) and HATU (23 mg, 0.06 mmol) were added, and the reaction was stirred at room temperature overnight. The reaction solution was added to water (20 mL), and the aqueous phase was extracted with ethyl acetate (20 mL x 3). The organic phases were combined, washed with saturated sodium chloride solution (20 mL x 3), dried over anhydrous sodium sulfate, and concentrated. The residue was purified by Prep-HPLC to obtain the title compound (7.2 mg, white solid), yield: 35%. MS (ESI): m/z 510.1 [M+H]⁺. ¹H NMR (400 MHz, DMSO-*d*₆) δ 10.97 (s, 1H), 9.07 (t, *J =* 6.0 Hz, 1H), 8.62 (s, 1H), 7.39 (d, *J =* 8.4 Hz, 1H), 7.30 (s, 1H), 7.19 (s, 1H), 6.96 (dd, *J =* 8.4, 1.2 Hz, 1H), 6.66 (s, 1H), 6.25 (d, *J =* 1.2 Hz, 1H), 4.57-4.53 (m, 4H), 4.47-4.45 (m, 2H), 3.67 (s, 2H), 2.54-2.51 (m, 4H), 1.98-1.91 (m, 4H).

### Example 28

### N-((2-((((3-(Difluoromethylene)cyclobutyl)methyl)amino)methyl)-1H-indol-6-yl)methyl)-9-oxo-2,3-dihydro-9H-[1,4]dioxino[2',3':4,5]pyrido[1,2-a]pyrimidine-7-carboxamide

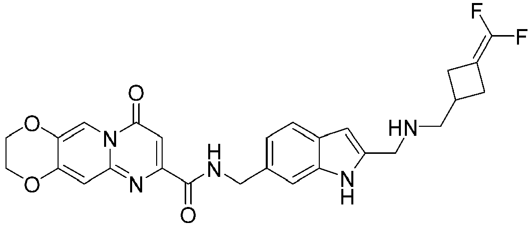

### (1) 3-(Aminomethyl)-1-(difluoro(pyridin-2-ylsulfonyl)methyl)cyclobutanol

Potassium tert-butoxide (336 mg, 3.0 mmol) was dissolved in tetrahydrofuran (10 mL), cooled to -50°C. tert-Butyl ((3-oxocyclobutyl)methyl)carbamate (500 mg, 2.5 mmol) and 2-((difluoromethyl)sulfonyl)pyridine (483 mg, 2.5 mmol) were dissolved in tetrahydrofuran (10 mL), added dropwise to the reaction system, and the mixture was stirred at -50°C for 1 hour. Saturated ammonium chloride (10 mL) and concentrated hydrochloric acid (10 mL) were added to the reaction solution, warmed to room temperature and stirred overnight. Saturated sodium bicarbonate solution was added to the reaction solution for neutralization, the aqueous phase was extracted with ethyl acetate (20 mL x 3), the aqueous phase was concentrated under reduced pressure, and the residue was purified by reverse-phase column to obtain the title compound (250 mg, yellow solid), yield: 34%. MS (ESI): m/z 293.0 [M+H]⁺.

### (2) 2-((((3-(Difluoro(pyridin-2-ylsulfonyl)methyl)-3-hydroxycyclobutyl)methyl)amino)methyl)-1H-indole-6-carbonitrile

2-Formyl-1H-indole-6-carbonitrile (150 mg, 0.88 mmol, Step 4 of Intermediate 1) and3-(Aminomethyl)-1-(difluoro(pyridin-2-ylsulfonyl)methyl)cyclobutanol (230 mg, 0.79 mmol)were dissolved in methanol (5 mL) and stirred at room temperature for 1 hour. Then sodium cyanoborohydride (164 mg, 2.6 mmol) was added and stirred at room temperature overnight. The reaction solution was concentrated, and the residue was purified by reverse-phase column to obtain the title compound (150 mg, colorless oil), yield: 38%. MS (ESI): m/z 447.0 [M+H]⁺.

### (3) tert-Butyl 2-(((tert-butoxycarbonyl)((3-((tert-butoxycarbonyl)oxy)-3-(difluoro(pyridin-2-ylsulfonyl)methyl)cyclobutyl)methyl)amino)methyl)-6-cyano-1H-indole-1-carboxylate

2-((((3-(Difluoro(pyridin-2-ylsulfonyl)methyl)-3-hydroxycyclobutyl)methyl)amino)methyl)-1H-indole-6-carbonitrile (150 mg, 0.34 mmol) was dissolved in tetrahydrofuran (10 mL), sodium hydride (40 mg, 1.0 mmol, 60%) was added and stirred at room temperature for 5 minutes. Then di-tert-butyl dicarbonate (218 mg, 1.0 mmol) was added, and the mixture was stirred at room temperature overnight. Water (20 mL) was added to the reaction solution, and the aqueous phase was extracted with ethyl acetate (20 mL x 3). The organic phases were combined, washed with saturated sodium chloride solution (20 mL), dried over anhydrous sodium sulfate, and concentrated. The residue was purified by silica gel column chromatography (petroleum ether/ethyl acetate = 2/1) to obtain the title compound (140 mg, yellow oil), yield: 56%. MS (ESI): m/z 747.0 [M+H]⁺.

### (4) tert-Butyl 6-(aminomethyl)-2-(((tert-butoxycarbonyl)((3-((tert-butoxycarbonyl)oxy)-3-(difluoro(pyridin-2-ylsulfonyl)methyl)cyclobutyl)methyl)amino)methyl)-1H-indole-1-carboxylate

tert-Butyl 2-(((tert-butoxycarbonyl)((3-((tert-butoxycarbonyl)oxy)-3-(difluoro(pyridin-2-ylsulfonyl)methyl)cyclobutyl)methyl)amino)methyl)-6-cyano-1H-indole-1-carboxylate (70 mg, 0.1 mmol) was dissolved in 7M ammonia methanol solution (10 mL), Raney nickel (10 mg) was added, and the reaction was stirred at room temperature under a hydrogenatmosphere for 2 hours. The reaction solution was filtered to remove Raney nickel, and the filtrate was concentrated to obtain the title compound (70 mg, yellow oil). MS (ESI): m/z 751.1 [M+H]⁺.

### (5) tert-Butyl 2-(((tert-butoxycarbonyl)((3-((tert-butoxycarbonyl)oxy)-3-(difluoro(pyridin-2-ylsulfonyl)methyl)cyclobutyl)methyl)amino)methyl)-6-((9-oxo-2,3-dihydro-9H-[1,4]dioxino[2',3':4,5]pyrido[1,2-a]pyrimidine-7-carboxamido)methyl)-1H-indole-1-carboxylate

Intermediate 4 (20 mg, 0.08 mmol) and tert-Butyl 6-(aminomethyl)-2-(((tert-butoxycarbonyl)((3-((tert-butoxycarbonyl)oxy)-3-(difluoro(pyridin-2-ylsulfonyl)methyl)cyclobutyl)methyl)amino)methyl)-1H-indole-1-carboxylate (60 mg, 0.08 mmol) were dissolved in *N,N-*dimethylformamide (5 mL), then *N,N*-diisopropylethylamine (20 mg, 0.16 mmol) and HATU (60 mg, 0.16 mmol) were added, and the reaction was continued to stir at room temperature overnight. Water (20 mL) was added to the reaction solution, and the aqueous phase was extracted with ethyl acetate (20 mL x 3). The organic phases were combined, washed with saturated sodium chloride solution (20 mL x 3), dried over anhydrous sodium sulfate, and concentrated. The residue was purified by silica gel column chromatography (petroleum ether/ethyl acetate = 2/1) to obtain the title compound (30 mg, yellow solid), yield: 38%. MS (ESI): m/z 981.1 [M+H]⁺.

### (6) N-((2-((((3-(Difluoromethylene)cyclobutyl)methyl)amino)methyl)-1H-indol-6-yl)methyl)-9-oxo-2,3-dihydro-9H-[1,4]dioxino[2',3':4,5]pyrido[1,2-a]pyrimidine-7-carboxamide

tert-Butyl 2-(((tert-butoxycarbonyl)((3-((tert-butoxycarbonyl)oxy)-3-(difluoro(pyridin-2-ylsulfonyl)methyl)cyclobutyl)methyl)amino)methyl)-6-((9-oxo-2,3-dihydro-9H-[1,4]dioxino[2',3':4,5]pyrido[1,2-a]pyrimidine-7-carboxamido)methyl)-1H-indole-1-carboxylate (10 mg, 0.01 mmol) was dissolved in *N,N*-dimethylformamide (5 mL), concentrated hydrochloric acid/water (2 mL/2 mL) Saturated ammonium chloride aqueous solution (2 mL) were added, and the mixture was heated to 80°C and stirred overnight. The reaction solution was concentrated, and the residue was purified by Prep-HPLC to obtain the title compound (1.1 mg, white solid), yield: 21%. MS (ESI): m/z 522.1 [M+H]⁺. ¹H NMR (400 MHz, DMSO-*d*₆) δ 10.86 (s, 1H), 9.05 (t, *J =* 5.6 Hz, 1H), 8.62 (s, 1H), 7.37 (d, *J =* 8.4 Hz, 1H), 7.29 (s, 1H), 7.19 (s, 1H), 6.95 (dd, *J =* 8.0, 1.2 Hz, 1H), 6.67 (s, 1H), 6.21 (s, 1H), 4.57-4.53 (m, 4H), 4.47-4.45 (m, 2H), 3.79 (s, 2H), 2.71-2.65 (m, 2H), 2.57-2.51 (m, 3H), 2.36-2.30 (m, 2H).

### Example 29

### N-((2-((4,4-Difluoropiperidin-1-yl)methyl)-1H-indol-6-yl)methyl)-8-oxo-8H-[1,3]dioxolo[4',5':4,5]pyrido[1,2-a]pyrimidine-6-carboxamide

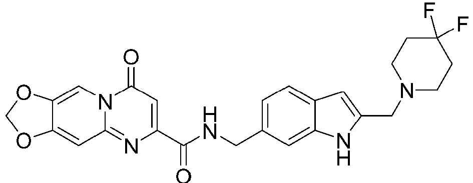

Intermediate 5 (20 mg, 0.085 mmol) was dissolved in *N,N-*dimethylformamide (3 mL), *N,N-*diisopropylethylamine (55 mg, 0.43 mmol) and HATU (65 mg, 0.17 mmol) were added and stirred at room temperature for 5 minutes. Then (2-((4,4-Difluoropiperidin-1-yl)methyl)-1H-indol-6-yl)methanamine (24 mg, 0.085 mmol, Step 3 of **Example 27**) was added, and the reaction was continued to stir at room temperature for 2 hours. Water (20 mL) was added to the reaction solution, and the aqueous phase was extracted with ethyl acetate (20 mL x 3). The organic phases were combined, washed with saturated sodium chloride solution (20 mL x 3), dried over anhydrous sodium sulfate, and concentrated. The residue was purified by Prep-HPLC to obtain the title compound (10.1 mg, white solid), yield: 24%. MS (ESI): m/z 496.1 [M+H]⁺. ¹H NMR (400 MHz, DMSO-*d*₆) δ 10.96 (s, 1H), 9.04 (t, *J =* 6.0 Hz, 1H), 8.59 (s, 1H), 7.39 (d, *J =* 8.0 Hz, 1H), 7.31 (s, 1H), 7.14 (s, 1H), 6.96 (dd, *J =* 8.0, 1.2 Hz, 1H), 6.73 (s, 1H), 6.37 (s, 2H), 6.25 (d, *J =* 0.8 Hz, 1H), 4.54 (d, *J =* 6.0 Hz, 2H), 3.67 (s, 2H), 2.63-2.50 (m, 4H), 2.00-1.90 (m, 4H).

### Example 30

### N-((2-(((Cyclobutylmethyl)amino)methyl)-1H-indol-6-yl)methyl)-2,2-difluoro-8-oxo-8H-[1,3]dioxolo[4',5':4,5]pyrido[1,2-a]pyrimidine-6-carboxamide

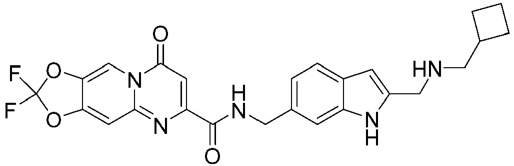

### (1) 6-Bromo-[1,3]dioxolo[4,5-c]pyridine-2-thione

6-Bromopyridine-3,4-diol (1.69 g, 8.9 mmol, Step 3 of Intermediate 5) was dissolved in dichloromethane (50 mL), and 4-dimethylaminopyridine (2.71 g, 22.2 mmol) and thiophosgene (1.53 g, 13.4 mmol) were added, and reacted at room temperature for 2 hours. The reaction solution was concentrated, and the residue was purified by silica gel column chromatography (petroleum ether/ethyl acetate = 60/40) to obtain the title compound (1.1 g, pale yellow solid), yield: 53.3%. MS (ESI): m/z 231.6 [M+H]⁺.

### (2) 6-Bromo-2,2-difluoro-[1,3]dioxolo[4,5-c]pyridine

6-Bromo-[1,3]dioxolo[4,5-c]pyridine-2-thione (1.1 g, 4.74 mmol) was dissolved in dichloromethane (40 mL), and 70% hydrogen fluoride pyridine solution (18 mL) and dibromohydantoin (4.07 g, 14.2 mmol) were addedat -70°C. The reaction solution was slowly warmed to room temperature and continued to react for 2 hours. 2M sodium hydroxide solution was added to the reaction solution until the pH value was greater than 10, a large amount of solid precipitated, filtered, and the filter cake was washed with dichloromethane (20 mL). After separating the organic phase from the filtrate, the aqueous phase was extracted with dichloromethane (30 mLx 3). The organic phases were combined, washed with saturated brine (50 mL), dried over anhydrous sodium sulfate, filtered, and concentrated. The residue was purified by silica gel column chromatography (100% petroleum ether) to obtain the title compound (466 mg, pale yellow liquid), yield: 41.3%. MS (ESI): m/z 237.6 [M+H]⁺.

### (3) tert-Butyl (2,2-difluoro-[1,3]dioxolo[4,5-c]pyridin-6-yl)carbamate

6-Bromo-2,2-difluoro-[1,3]dioxolo[4,5-c]pyridine (466 mg, 1.96 mmol) was dissolved in 1,4-dioxane (15 mL), and tris(dibenzylideneacetone)dipalladium (183 mg, 0.2 mmol), tert-butyl carbamate (688 mg, 5.88 mmol), 4,5-bisdiphenylphosphino-9,9-dimethylxanthene (231 mg, 0.4 mmol) and cesium carbonate (1.28 g, 3.92 mmol) were added. The mixture was heated to 100°Cunder nitrogen protection and stirred overnight. The reaction solution was concentrated, and the residue was purified by silica gel column chromatography (petroleum ether/ethyl acetate = 85/15) to obtain the title compound (301 mg, white solid), yield: 56%. MS (ESI): m/z 218.6 [M+H-56]⁺.

### (4) 2,2-Difluoro-[1,3]dioxolo[4,5-c]pyridin-6-amine

tert-Butyl (2,2-difluoro-[1,3]dioxolo[4,5-c]pyridin-6-yl)carbamate (310 mg, 1.1 mmol) was dissolved in dichloromethane (4 mL), and trifluoroacetic acid (4 mL) was added, and reacted at room temperature for 2 hours. The reaction solution was spin-dried, saturated sodium bicarbonate solution (30 mL) was added, and extracted with ethyl acetate (2 x 20 mL). The organic phases were combined, washed with saturated brine (20 mL), dried over anhydrous sodium sulfate, filtered, and concentrated to obtain the title compound (165 mg, pale yellow solid), yield: 86.2%. MS (ESI): m/z 174.6 [M+H]⁺.

### (5) Methyl 2,2-difluoro-8-oxo-8H-[1,3]dioxolo[4',5':4,5]pyrido[1,2-a]pyrimidine-6-carboxylate

2,2-Difluoro-[1,3]dioxolo[4,5-c]pyridin-6-amine (52 mg, 0.3 mmol), water (8 mL) and dimethyl but-2-ynedioate (51 mg, 0.36 mmol) were added to a 25 mL single-necked flask.The mixture was heated to 70°C and stirred overnight. The reaction solution was diluted with water (10 mL), and extracted with ethyl acetate (2 x 20 mL). The organic phases were combined, washed with saturated brine (20 mL), dried over anhydrous sodium sulfate, filtered, and concentrated. The residue was purified by silica gel column chromatography (petroleum ether/ethyl acetate = 70/30) to obtain the title compound (50 mg, pink solid), yield: 58.8%. MS (ESI): m/z 284.6 [M+H]⁺.

### (6) 2,2-Difluoro-8-oxo-8H-[1,3]dioxolo[4',5':4,5]pyrido[1,2-a]pyrimidine-6-carboxylic acid

Methyl 2,2-difluoro-8-oxo-8H-[1,3]dioxolo[4',5':4,5]pyrido[1,2-a]pyrimidine-6-carboxylate (23 mg, 0.08 mmol) was dissolved in 1,2-dichloroethane (2 mL), and trimethyltin hydroxide (44 mg, 0.24 mmol) was added. The mixture was heated to 80°C and stirred overnight. The reaction solution was concentrated to obtain the title compound (30 mg, pale yellow solid), yield: 100%. The crude product was directly used in the next reaction. MS (ESI): m/z 270.6 [M+H]⁺.

### (7) tert-Butyl 2-(((tert-butoxycarbonyl)(cyclobutylmethyl)amino)methyl)-6-((2,2-difluoro-8-oxo-8H-[1,3]dioxolo[4',5':4,5]pyrido[1,2-a]pyrimidine-6-carboxamido)methyl)-1H-indole-1-carboxylate

2,2-Difluoro-8-oxo-8H-[1,3]dioxolo[4',5':4,5]pyrido[1,2-a]pyrimidine-6-carboxylic acid (21.6 mg, 0.08 mmol) was dissolved in *N,N*-dimethylformamide (2 mL), and *N,N*-diisopropylethylamine (52 mg, 0.4 mmol) and HATU (46 mg, 0.12 mmol) were added, and stirred at room temperature for 5 minutes. Then Intermediate 1 (35.5mg, 0.08 mmol) was added, stirring was continued at room temperature for 2 hours. The reaction solution was poured into saturated aqueous sodium bicarbonate solution (20 mL), and extracted with ethyl acetate (2 x 15 mL). The organic phases were combined, washed with saturated brine (20 mL), dried over anhydrous sodium sulfate, filtered, and concentrated. The residue was purified by silica gel column chromatography (petroleum ether/ethyl acetate = 70/30) to obtain the title compound (12 mg, yellow oil), yield: 25.2%. MS (ESI): m/z 696.1 [M+H]⁺.

### (8) N-((2-(((Cyclobutylmethyl)amino)methyl)-1H-indol-6-yl)methyl)-2,2-difluoro-8-oxo-8H-[1,3]dioxolo[4',5':4,5]pyrido[1,2-a]pyrimidine-6-carboxamide

tert-Butyl 2-(((tert-butoxycarbonyl)(cyclobutylmethyl)amino)methyl)-6-((2,2-difluoro-8-oxo-8H-[1,3]dioxolo[4',5':4,5]pyrido[1,2-a]pyrimidine-6-carboxamido)methyl)-1H-indole-1-carboxylate (12 mg, 0.02 mmol) was dissolved in dichloromethane (3 mL), and trifluoroacetic acid (3 mL) was added, and stirred at room temperature for 3 hours. The reaction solution was concentrated, and the residue was diluted with methanol (1 mL) and purified by Prep-HPLC to obtain the title compound (1.7 mg, white solid), yield: 17%. MS (ESI): m/z 991.1 [2M+H]⁺. ¹H NMR (400 MHz, DMSO-*d*₆) δ 10.86 (s, 1H), 9.20 (s, 1H), 9.01 (t, *J =* 6.4 Hz, 1H), 7.70 (s, 1H), 7.37 (d, *J =* 8.0 Hz, 1H), 7.30 (s, 1H), 6.95 (d, *J =* 8.0 Hz, 1H), 6.88 (s, 1H), 6.20 (s, 1H), 4.56 (d, *J =* 6.4 Hz, 2H), 3.77 (s, 2H), 2.63-2.58 (m, 2H), 2.41-2.35 (m, 1H), 2.02-1.93 (m, 2H), 1.83-1.75 (m, 2H), 1.67-1.57 (m, 2H).

### Example 31

### N-((5-(((cyclobutylmethyl)amino)methyl)-4H-thieno[3,2-b]pyrrol-2-yl)methyl)-9-oxo-2,3-dihydro-9H-[1,4]dioxino[2',3':4,5]pyrido[1,2-a]pyrimidine-7-carboxamide

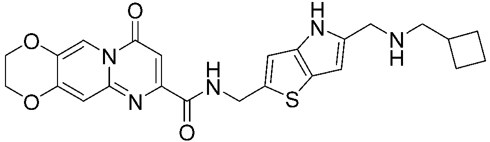

### (1)N-(Cyclobutylmethyl)-2-methyl-4-thieno[3,2-b]pyrrole-5-carboxamide

2-Methyl-4H-thieno[3,2-b]pyrrole-5-carboxylic acid (800 mg, 4.4 mmol) and *N,N-*carbonyldiimidazole (858 mg, 5.3 mmol) were dissolved in *N,N-*dimethylformamide (15 mL) and stirred at room temperature for half an hour. Cyclobutylmethylamine (450 mg, 5.3 mmol) was added and stirred at room temperature overnight. Water (20 mL) was added to precipitate the solid product, which was filtered and dried to obtain the title compound (1 g, yellow solid), yield: 92%. MS (ESI): m/z 249.0 [M+H]⁺.

### (2) 1-Cyclobutyl-N-((2-methyl-4H-thieno[3,2-b]pyrrol-5-yl)methyl)methanamine

*N*-(Cyclobutylmethyl)-2-methyl-4-thieno[3,2-b]pyrrole-5-carboxamide (1 g, 4.0 mmol) was dissolved in 1,4-dioxane (15 mL), and lithium aluminum hydride (460 mg, 12.0 mmol) was added, and the reaction was stirred at 100°C for 3 hours. Water was added (0.5 mL), followed by 10% aqueous sodium hydroxide solution (0.5 mL).The reaction solution was filtered through celite, and the filtrate was concentrated to obtain the title compound (943 mg, colorless oil), yield: 100%. MS (ESI): m/z 150.0 [M+H-85]⁺.

### (3) tert-Butyl 5-(((tert-butoxycarbonyl)(cyclobutylmethyl)amino)methyl)-2-methyl-4H-thieno[3,2-b]pyrrole-4-carboxylate

1-Cyclobutyl-*N*-((2-methyl-4H-thieno[3,2-b]pyrrol-5-yl)methyl)methanamine (1.6 g, 6.8 mmol) and triethylamine (2 g, 20.1 mmol) were dissolved in acetonitrile (20 mL), and 4-dimethylaminopyridine (83 mg, 0.68 mmol) and di-tert-butyl dicarbonate (2.9 g, 13.6 mmol) were added, and stirred at room temperature overnight. The reaction solution was concentrated, and the residue was purified by silica gel column chromatography (petroleum ether/ethyl acetate = 20/1) to obtain the title compound (1.6 g, yellow oil), yield: 55%. MS (ESI): m/z 457.0 [M+Na]⁺.

### (4) tert-Butyl 5-(((tert-butoxycarbonyl)(cyclobutylmethyl)amino)methyl)-2-formyl-4H-thieno[3,2-b]pyrrole-4-carboxylate

tert-Butyl 5-(((tert-butoxycarbonyl)(cyclobutylmethyl)amino)methyl)-2-methyl-4H-thieno[3,2-b]pyrrole-4-carboxylate (1.6 g, 3.6 mmol) and 2,3-dichloro-5,6-dicyano-1,4-benzoquinone (DDQ) (1.1 g, 4.7 mmol) were dissolved in methanol/water (20 mL/5 mL) and stirred at room temperature overnight. To the reaction solution was added aqueous sodium bicarbonate solution (20 mL), and extracted with ethyl acetate (20 mL x 3). The organic phases were combined, washed with saturated sodium chloride solution (20 mL x 3), dried over anhydrous sodium sulfate, and concentrated. The residue was purified by silica gel column chromatography (petroleum ether/ethyl acetate = 10/1) to obtain the title compound (370 mg, yellow oil), yield: 23%. MS (ESI): m/z 470.9 [M+Na]⁺.

### (5) (E)-tert-Butyl 5-(((tert-butoxycarbonyl)(cyclobutylmethyl)amino)methyl)-2-((hydroxyimino)methyl)-4H-thieno[3,2-b]pyrrole-4-carboxylate

tert-Butyl 5-(((tert-butoxycarbonyl)(cyclobutylmethyl)amino)methyl)-2-formyl-4H-thieno[3,2-b]pyrrole-4-carboxylate (370 mg, 0.8 mmol) and hydroxylamine hydrochloride (166 mg, 2.4 mmol) were dissolved in tetrahydrofuran/ethanol/water (10 mL/2 mL/1 mL) and stirred at room temperature overnight. To the reaction solution was added water (20 mL), and extracted with ethyl acetate (20 mL x 3). The organic phases were combined, washed with saturated sodium chloride solution (20 mL x 3), dried over anhydrous sodium sulfate, filtered, and concentrated to obtain the title compound (350 mg, yellow solid), yield: 92%. MS (ESI): m/z 464.4 [M+H]⁺.

### (6) tert-Butyl 2-(Aminomethyl)-5-(((tert-butoxycarbonyl)(cyclobutylmethyl)amino)methyl)-4H-thieno[3,2-b]pyrrole-4-carboxylate

(E)-tert-Butyl 5-(((tert-butoxycarbonyl)(cyclobutylmethyl)amino)methyl)-2-((hydroxyimino)methyl)-4H-thieno[3,2-b]pyrrole-4-carboxylate (350 mg, 0.76 mmol) and zinc powder (35 mg) were added to acetic acid (10 mL), and the reaction was stirred at room temperature overnight. The reaction solution was filtered through Celite, the filtrate was concentrated, sodium bicarbonate aqueous solution (20 mL) was added, and extracted with dichloromethane/methanol (20 mL x 3). The organic phases were combined, concentrated, and the residue was purified by silica gel column chromatography (dichloromethane/methanol = 20/1) to obtain the title compound (140 mg, yellow oil), yield: 41%. MS (ESI): m/z 471.9 [M+Na]⁺.

### (7) tert-Butyl 5-(((tert-Butoxycarbonyl)(cyclobutylmethyl)amino)methyl)-2-((9-oxo-2,3-dihydro-9H-[1,4]dioxino[2',3':4,5]pyrido[1,2-a]pyrimidine-7-carboxamido)methyl)-4H-thieno[3,2-b]pyrrole-4-carboxylate

tert-Butyl 2-(Aminomethyl)-5-(((tert-butoxycarbonyl)(cyclobutylmethyl)amino)methyl)-4H-thieno[3,2-b]pyrrole-4-carboxylate (20 mg, 0.04 mmol) and Intermediate 4 (10 mg, 0.04 mmol) were dissolved in N,N-dimethylformamide (5 mL), then N,N-diisopropylethylamine (10 mg, 0.08 mmol) and HATU (30 mg, 0.08 mmol) were added, and stirred at room temperature overnight. Water (20 mL) was added to the reaction solution, and extracted with ethyl acetate (20 mL x 3). The organic phases were combined, washed with saturated sodium chloride solution (20 mL x 3), dried over anhydrous sodium sulfate, filtered, and concentrated. The residue was purified by silica gel column chromatography (petroleum ether/ethyl acetate = 50/50) to obtain the title compound (20 mg, yellow solid), yield: 67%. MS (ESI): m/z 680.3 [M+H]⁺.

### (8) N-((5-(((Cyclobutylmethyl)amino)methyl)-4H-thieno[3,2-b]pyrrol-2-yl)methyl)-9-oxo-2,3-dihydro-9H-[1,4]dioxino[2',3':4,5]pyrido[1,2-a]pyrimidine-7-carboxamide

tert-Butyl 5-(((tert-Butoxycarbonyl)(cyclobutylmethyl)amino)methyl)-2-((9-oxo-2,3-dihydro-9H-[1,4]dioxino[2',3':4,5]pyrido[1,2-a]pyrimidine-7-carboxamido)methyl)-4H-thieno[3,2-b]pyrrole-4-carboxylate (20 mg, 0.03 mmol) was dissolved in formic acid (5 mL), and the reaction was stirred at 40°C overnight. The reaction solution was concentrated, and the residue was purified by Prep-HPLC to obtain the title compound (1.2 mg, yellow solid), yield: 8.6%. MS (ESI): m/z 395.0 [M+H-85]⁺. ¹H NMR (400 MHz, DMSO-*d*₆) δ 10.92 (s, 1H), 9.06 (t, *J =* 6.4 Hz, 1H), 8.61 (s, 1H), 7.18 (s, 1H), 6.92 (s, 1H), 6.65 (s, 1H), 6.12 (s, 1H), 4.61-4.55 (m, 4H), 4.47-4.45 (m, 2H), 3.69 (s, 2H), 2.54-2.52 (m, 2H), 2.42-2.36 (m, 1H), 2.03-1.94 (m, 2H), 1.80-1.76 (m, 2H), 1.65-1.59 (m, 2H).

### Example 32

### N-((5-(((Cyclobutylmethyl)amino)methyl)-4H-thieno[3,2-b]pyrrol-2-yl)methyl)-8-oxo-8H-[1,3]dioxolo[4',5':4,5]pyrido[1,2-a]pyrimidine-6-carboxamide

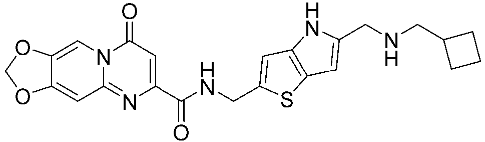

### (1) tert-Butyl 5-(((tert-Butoxycarbonyl)(cyclobutylmethyl)amino)methyl)-2-((8-oxo-8H-[1,3]dioxolo[4',5':4,5]pyrido[1,2-a]pyrimidine-6-carboxamido)methyl)-4H-thieno[3,2-b]pyrrole-4-carboxylate

tert-Butyl 2-(Aminomethyl)-5-(((tert-butoxycarbonyl)(cyclobutylmethyl)amino)methyl)-4H-thieno[3,2-b]pyrrole-4-carboxylate (60 mg, 0.13 mmol, Step 6 of **Example 31**) and Intermediate 5 (30 mg, 0.13 mmol) were dissolved in *N,N*-dimethylformamide (5 mL), then *N,N-*diisopropylethylamine (34 mg, 0.26 mmol) and HATU (60 mg, 0.16 mmol) were added, and the reaction was stirred at room temperature overnight. Water (20 mL) was added to the reaction solution, and extracted with ethyl acetate (20 mL x 3). The organic phases were combined, washed with saturated sodium chloride solution (20 mL x 3), dried over anhydrous sodium sulfate, filtered, and concentrated. The residue was purified by silica gel column chromatography (petroleum ether/ethyl acetate = 50/50) to obtain the title compound (60 mg, yellow solid), yield: 68%. MS (ESI): m/z 665.9 [M+H]⁺.

### (2) N-((5-(((Cyclobutylmethyl)amino)methyl)-4H-thieno[3,2-b]pyrrol-2-yl)methyl)-8-oxo-8H-[1,3]dioxolo[4',5':4,5]pyrido[1,2-a]pyrimidine-6-carboxamide

tert-Butyl 5-(((tert-Butoxycarbonyl)(cyclobutylmethyl)amino)methyl)-2-((8-oxo-8H-[1,3]dioxolo[4',5':4,5]pyrido[1,2-a]pyrimidine-6-carboxamido)methyl)-4H-thieno[3,2-b]pyrrole-4-carboxylate (60 mg, 0.09 mmol) was dissolved in formic acid (5 mL), and the reaction was stirred at 40°C overnight. The reaction solution was concentrated, and the residue was purified by Prep-HPLC to obtain the title compound (2.6 mg, yellow solid), yield: 6%. MS (ESI): m/z 381.0 [M+H-85]⁺. ¹H NMR (400 MHz, DMSO-*d*₆) δ 10.98 (s, 1H), 9.06 (t, *J =* 6.4 Hz, 1H), 8.59 (s, 1H), 7.14 (s, 1H), 6.92 (s, 1H), 6.72 (s, 1H), 6.38 (s, 2H), 6.15 (s, 1H), 4.61 (d, *J =* 6.0 Hz, 2H), 3.73 (s, 2H), 2.54-2.52 (m, 2H), 2.42-2.36 (m, 1H), 2.03-1.95 (m, 2H), 1.83-1.76 (m, 2H), 1.66-1.59 (m, 2H).

### Example 33

### N-((2-(((Cyclobutylmethyl)amino)methyl)-1H-indol-6-yl)methyl)-4-oxo-6,11-dihydro-4H-pyrimido[2,1-b]quinazoline-2-carboxamide

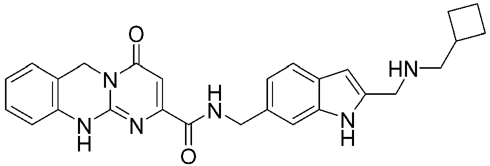

### (1) 2-((4-Methoxybenzyl)amino)benzonitrile

2-Aminobenzonitrile (30.0 g, 0.25 mol), triethylamine (51.7 g, 0.51 mol) and 4-methoxybenzyl chloride (47.7 g, 0.30 mol) were dissolved in acetonitrile (300 mL), heated to 120°C and stirred for 24 hours. Water (300 mL) was added to the reaction solution, and extracted with ethyl acetate (500 mL x 2). The organic phases were combined, washed with saturated sodium chloride solution (500 mL x 3), dried over anhydrous sodium sulfate, filtered, and concentrated. The residue was purified by silica gel column chromatography (petroleum ether/ethyl acetate = 93/7) to obtain the title compound (4.4 g, white solid), yield: 7%. MS (ESI): m/z 261.0 [M+Na]⁺.

### (2) 2-(Aminomethyl)-N-(4-methoxybenzyl)aniline

At 0°C, 2-((4-methoxybenzyl)amino)benzonitrile (4.4 g, 18.46 mmol) and lithium aluminum hydride (2.10 g, 55.39 mmol) were dissolved in tetrahydrofuran (100 mL), heated to 75°C and stirred for 16 hours. The reaction solution was cooled to 0°C, water (100 mL) was slowly added dropwise to the reaction solution, then ethyl acetate (200 mL) was added, and stirred at room temperature for 30 minutes. The reaction solution was filtered through Celite, and the filtrate was extracted with ethyl acetate (100 mL x 2). The organic phases were combined, washed with saturated sodium chloride solution (100 mL x 2), dried over anhydrous sodium sulfate, filtered, and concentrated to obtain the title compound (4.0 g, yellow solid), yield: 89%. MS (ESI): m/z 226.0 [M+H-17]⁺.

### (3) 1-(4-Methoxybenzyl)-1,4-dihydroquinazolin-2-amine

2-(Aminomethyl)-*N*-(4-methoxybenzyl)aniline (4.0 g, 16.51 mmol) and cyanogen bromide (2.62 g, 24.76 mmol) were dissolved in acetonitrile (50 mL), and stirred at room temperature under nitrogen protection for 4 hours. The reaction solution was concentrated, and the residue was purified by silica gel column chromatography (dichloromethane/methanol = 95/5) to obtain the title compound (2.07 g, yellow oil), yield: 47%. MS (ESI): m/z 268.0 [M+H]⁺.

### (4) Methyl 11-(4-methoxybenzyl)-4-oxo-6,11-dihydro-4H-pyrimido[2,1-b]quinazoline-2-carboxylate

1-(4-Methoxybenzyl)-1,4-dihydroquinazolin-2-amine (2.07 g, 7.74 mmol), dimethyl but-2-ynedioate (1.32 g, 9.29 mmol) and triethylamine (1.18 g, 11.61 mmol) were dissolved in methanol (50 mL), and stirred at 60°C for 4 hours. The reaction solution was concentrated, and the residue was purified by silica gel column chromatography (petroleum ether/ethyl acetate = 20/80) to obtain the title compound (600 mg, yellow solid), yield: 21%. MS (ESI): m/z 377.9 [M+H]⁺. ¹H NMR (400 MHz, DMSO-*d*₆) δ 7.34-7.26 (m, 4H), 7.20-7.15 (m, 2H), 6.89 (d, *J =* 8.4 Hz, 2H), 5.91 (s, 1H), 5.33 (s, 2H), 4.78 (s, 2H), 3.72 (s, 3H), 3.71 (s, 3H).

### (5) Methyl 4-oxo-6,11-dihydro-4H-pyrimido[2,1-b]quinazoline-2-carboxylate

Methyl 11-(4-methoxybenzyl)-4-oxo-6,11-dihydro-4H-pyrimido[2,1-b]quinazoline-2-carboxylate (350 mg, 0.93 mmol) was dissolved in trifluoroacetic acid (5 mL) and stirred at 60°C for 1 hour. The reaction solution was concentrated, and the residue was purified by silica gel column chromatography (dichloromethane/methanol = 92/8) to obtain the title compound (50 mg, yellow solid), yield: 21%. MS (ESI): m/z 257.9 [M+H]⁺.

### (6) 4-Oxo-6,11-dihydro-4H-pyrimido[2,1-b]quinazoline-2-carboxylic acid

Methyl 4-oxo-6,11-dihydro-4H-pyrimido[2,1-b]quinazoline-2-carboxylate (50 mg, 0.19 mmol) was dissolved in concentrated hydrochloric acid (1 mL) and stirred at 80°C for 1 hour. The reaction solution was concentrated to obtain the title compound (40 mg, yellow solid), yield: 85%. MS (ESI): m/z 243.9 [M+H]⁺.

### (7) tert-Butyl 2-(((tert-butoxycarbonyl)(cyclobutylmethyl)amino)methyl)-6-((4-oxo-6,11-dihydro-4H-pyrimido[2,1-b]quinazoline-2-carboxamido)methyl)-1H-indole-1-carboxylate

4-Oxo-6,11-dihydro-4H-pyrimido[2,1-b]quinazoline-2-carboxylic acid (40 mg, 0.24 mmol), 1-propylphosphonic anhydride (157 mg, 0.49 mmol), Intermediate 1 (88 mg, 0.20 mmol) and *N,N-*diisopropylethylamine (64 mg, 0.49 mmol) were dissolved in *N,N*-dimethylformamide (10 mL) and stirred at room temperature for 12 hours. Water (20 mL) was added to the reaction solution, and extracted with dichloromethane (10 mL x 3). The organic phases were combined, washed with saturated sodium chloride solution (10 mL x 3), dried over anhydrous sodium sulfate, filtered, and concentrated. The residue was purified by silica gel column chromatography (petroleum ether/ethyl acetate = 50/50) to obtain the title compound (5 mg, yellow solid), yield: 5%. MS (ESI): m/z 569.0 [M+H-100]⁺.

### (8) N-((2-(((Cyclobutylmethyl)amino)methyl)-1H-indol-6-yl)methyl)-4-oxo-6,11-dihydro-4H-pyrimido[2,1-b]quinazoline-2-carboxamide

tert-Butyl 2-(((tert-butoxycarbonyl)(cyclobutylmethyl)amino)methyl)-6-((4-oxo-6,11-dihydro-4H-pyrimido[2,1-b]quinazoline-2-carboxamido)methyl)-1H-indole-1-carboxylate (5 mg, 0.007 mmol) was dissolved in dichloromethane (0.5 mL), trifluoroacetic acid (0.5 mL) was added, and stirred at room temperature for 30 minutes. The reaction solution was concentrated, and the residue was diluted with N,N-dimethylformamide (2 mL) and purified by Prep-HPLC to obtain the title compound (1.2 mg, pale yellow solid), yield: 34%. MS (ESI): m/z 469.1 [M+H]⁺. ¹H NMR (400 MHz, DMSO-*d*₆) δ 11.18 (s, 1H), 10.89 (s, 1H), 7.40-7.37 (m, 1H), 7.27-7.24 (m, 3H), 7.19 (s, 1H), 7.11-7.05 (m, 2H), 6.91 (d, *J =* 8.0 Hz, 1H), 6.64 (s, 1H), 6.23 (s, 1H), 5.21 (s, 0.5H), 4.69 (s, 1.5H), 4.49-4.43 (m, 2H), 3.81-3.79 (m, 2H), 2.60-2.58 (m, 2H), 2.43-2.41 (m, 1H), 2.04-2.01 (m, 2H), 1.84-1.76 (m, 2H), 1.68-1.61 (m, 2H).

### Example 34

### N-((2-((3,4-dihydroisoquinolin-2(1H)-yl)methyl)-1H-indol-6-yl)methyl)-8-oxo-8H-[1,3]dioxolo[4',5':4,5]pyrido[1,2-a]pyrimidine-6-carboxamide

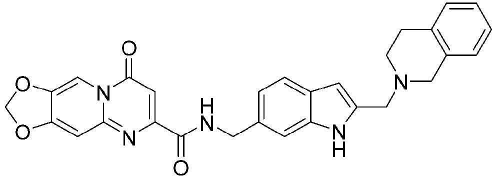

Intermediate 7 (30 mg, 0.064 mmol) and 1,2,3,4-tetrahydroisoquinoline (26 mg, 0.2 mmol) were dissolved in methanol (4 mL), glacial acetic acid (0.2 mL) was added, and the reaction mixture was heated to 50°C and stirred for 10 minutes. Then sodium cyanoborohydride (13 mg, 0.2 mmol) was added, and the reaction was continued to stir at 50°C overnight. The reaction mixture was filtered, the filtrate was concentrated, and the residue was purified by Prep-HPLC to obtain the title compound (5.7 mg, white solid), yield: 14.6%. MS (ESI): m/z 508.1 [M+H]⁺. ¹H NMR (400 MHz, DMSO-*d*₆) δ 11.01 (s, 1H), 9.03 (t, *J =* 6.0 Hz, 1H), 8.58 (s, 1H), 7.40 (d, *J =* 8.0 Hz, 1H), 7.30 (s, 1H), 7.14 (s, 1H), 7.09-7.05 (m, 3H), 7.00-6.96 (m, 2H), 6.73 (s, 1H), 6.37 (s, 2H), 6.30 (s, 1H), 4.54 (d, *J =* 6.4 Hz, 2H), 3.77 (s, 2H), 3.57 (s, 2H), 2.83 (t, *J =* 5.6 Hz, 2H), 2.71 (t, *J =* 5.6 Hz, 2H).

### Example 35

### N-((2-((5,6-Dihydroimidazo[1,2-a]pyrazin-7(8H)-yl)methyl)-1H-indol-6-yl)methyl)-8-oxo-8H-[1,3]dioxolo[4',5':4,5]pyrido[1,2-a]pyrimidine-6-carboxamide

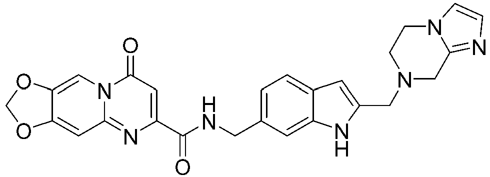

Referring to the synthetic method of **Example 34,** using Intermediate 7 and 5,6, 7,8-tetrahydroimidazo[1,2-a]pyrazine as starting materials, the title compound (4.9 mg, white solid) was obtained through similar steps. MS (ESI): m/z 498.1 [M+H]⁺. ¹H NMR (400 MHz, DMSO-*d*₆) δ 11.05 (s, 1H), 9.04 (t, *J =* 6.0 Hz, 1H), 8.58 (s, 1H), 7.42 (d, *J =* 8.0 Hz, 1H), 7.31 (s, 1H), 7.14 (s, 1H), 7.03 (d, *J =* 0.8 Hz, 1H), 6.97 (dd, *J =* 8.0, 0.8 Hz, 1H), 6.81 (d, *J =* 0.8 Hz, 1H), 6.73 (s, 1H), 6.37 (s, 2H), 6.32 (s, 1H), 4.55 (d, *J =* 6.4 Hz, 2H), 3.95 (t*, J =* 5.2 Hz, 2H), 3.82 (s, 2H), 3.60 (s, 2H), 2.84 (t, *J =* 5.6 Hz, 2H).

### Example 36

### N-((2-((((3-Fluorobicyclo[1.1.1]pentan-1-yl)methyl)amino)methyl)-1H-indol-6-y1)methyl)-8-oxo-8H-[1,3]dioxolo[4',5':4,5]pyrido[1,2-a]pyrimidine-6-carboxamide

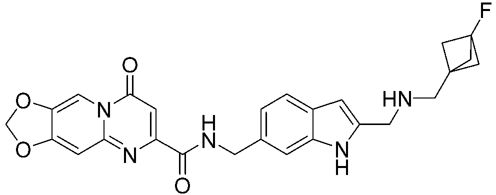

Intermediate 7 (20 mg, 0.05 mmol) and (3-fluorobicyclo[1.1.1]pentan-1-yl)methanamine hydrochloride (14 mg, 0.1 mmol) were dissolved in methanol (4 mL), triethylamine (0.1 mL) and glacial acetic acid (0.2 mL) were added. The reaction solution was heated to 50°C and stirred for 10 minutes, then sodium cyanoborohydride (13 mg, 0.2 mmol) was added, and the reaction was continued to stir at 50 °C overnight. The reaction solution became clear after overnight, filtered, the filtrate was concentrated, and the residue was purified by Prep-HPLC to obtain the title compound (6.3 mg, white solid), yield: 25%. MS (ESI): m/z 490.1 [M+H]⁺. ¹H NMR (400 MHz, DMSO-*d*₆) δ 10.86 (s, 1H), 9.02 (t, *J =* 6.0 Hz, 1H), 8.59 (s, 1H), 7.37 (d, *J =* 8.0 Hz, 1H), 7.29 (s, 1H), 7.14 (s, 1H), 6.95 (d, *J =* 8.4 Hz, 1H), 6.73 (s, 1H), 6.38 (s, 2H), 6.21 (s, 1H), 4.54 (d, *J* = 6.0 Hz, 2H), 3.80 (s, 2H), 2.75 (s, 2H), 1.92 (d, *J =* 2.8 Hz, 6H).

### Example 37

### N-((2-(((4,4-Difluorocyclohexyl)amino)methyl)-1H-indol-6-yl)methyl)-8-oxo-8H-[1,3]dioxolo[4',5':4,5]pyrido[1,2-a]pyrimidine-6-carboxamide

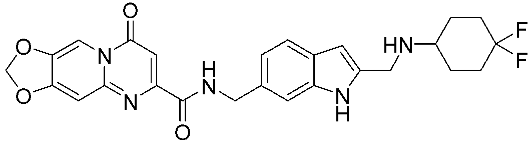

Referring to the synthetic method of **Example 34,** using Intermediate 7 and 4,4-difluorocyclohexan-1-amine as starting materials, the title compound (1.1 mg, white solid) was obtained through similar steps. MS (ESI): m/z 510.1 [M+H]⁺. ¹H NMR (400 MHz, DMSO-*d*₆) δ 10.85 (s, 1H), 9.02 (t, *J =* 6.0 Hz, 1H), 8.59 (s, 1H), 7.36 (d, *J =* 8.0 Hz, 1H), 7.30 (s, 1H), 7.14 (s, 1H), 6.94 (d, *J =* 8.4 Hz, 1H), 6.73 (s, 1H), 6.37(s, 2H), 6.22(s, 1H), 4.54 (d, *J =* 6.0 Hz, 2H), 3.82 (s, 2H), 2.58-2.55 (m, 1H), 2.03-1.98 (m, 2H), 1.80-1.70 (m, 4H), 1.46-1.41 (m, 2H).

### Example 38

### N-((2-(((3,3-Difluorocyclopentyl)amino)methyl)-1H-indol-6-yl)methyl)-8-oxo-8H-[1,3]dioxolo[4',5':4,5]pyrido[1,2-a]pyrimidine-6-carboxamide

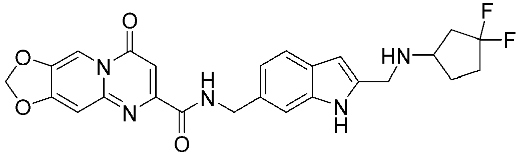

Referring to the synthetic method of **Example 36,** using Intermediate 7 and 3,3-difluorocyclopentan-1-amine hydrochloride as starting materials, the title compound (13.3 mg, white solid) was obtained through similar steps. MS (ESI): m/z 496.0 [M+H]⁺. ¹H NMR (400 MHz, DMSO-*d*₆) δ 10.87 (s, 1H), 9.02 (t, *J =* 6.0 Hz, 1H), 8.59 (s, 1H), 7.37 (d, *J =* 8.0 Hz, 1H), 7.29 (s, 1H), 7.14 (s, 1H), 6.95 (d, *J =* 8.4 Hz, 1H), 6.73 (s, 1H), 6.37 (s, 2H), 6.23 (s, 1H), 4.54 (d, *J =* 6.0 Hz, 2H), 3.78 (s, 2H), 3.20-3.13 (m, 1H), 2.41-2.14 (m, 2H), 1.99-1.84 (m, 3H), 1.59-1.51 (m, 1H).

### Example 39

### N-((2-(((3,3-Difluorocyclobutyl)amino)methyl)-1H-indol-6-yl)methyl)-8-oxo-8H-[1,3]dioxolo[4',5':4,5]pyrido[1,2-a]pyrimidine-6-carboxamide

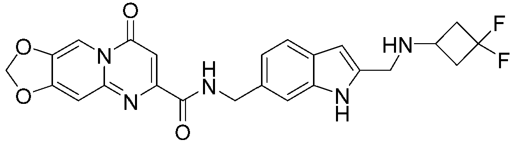

Referring to the synthetic method of **Example 34,** using Intermediate 7 and 3,3-difluorocyclobutan-1-amine as starting materials, the title compound (15.2 mg, white solid) was obtained through similar steps. MS (ESI): m/z 482.1 [M+H]⁺. ¹H NMR (400 MHz, DMSO-*d*₆) δ 10.89 (s, 1H), 9.02 (t, *J =* 6.0 Hz, 1H), 8.59 (s, 1H), 7.37 (d, *J =* 8.0 Hz, 1H), 7.29 (s, 1H), 7.14 (s, 1H), 6.95 (d, *J* = 8.0 Hz, 1H), 6.73 (s, 1H), 6.37 (s, 2H), 6.22 (s, 1H), 4.54 (d, *J =* 6.4 Hz, 2H), 3.74 (s, 2H), 3.15-3.07 (m, 1H), 2.72-2.63 (m, 2H), 2.38-2.26 (m, 2H).

### Example 40

### 8-Oxo-N-((2-((((1-(trifluoromethyl)cyclopropyl)methyl)amino)methyl)-1H-indol-6-yl)methyl)-8H-[1,3]dioxolo[4',5':4,5]pyrido[1,2-a]pyrimidine-6-carboxamide

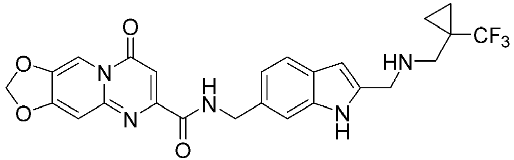

Referring to the synthetic method of **Example 36,** using Intermediate 7 and (1-(trifluoromethyl)cyclopropyl)methanamine hydrochloride as starting materials, the title compound (12.8 mg, white solid) was obtained through similar steps. MS (ESI): m/z 514.0 [M+H]⁺. ¹H NMR (400 MHz, DMSO-*d*₆) δ 10.84 (s, 1H), 9.03 (t, *J =* 6.0 Hz, 1H), 8.59 (s, 1H), 7.37 (d, *J =* 8.4Hz, 1H), 7.30 (s, 1H), 7.14 (s, 1H), 6.95 (d, *J =* 8.0 Hz, 1H), 6.73 (s, 1H), 6.37 (s, 2H), 6.22 (s, 1H), 4.54 (d, *J =* 6.4 Hz, 2H), 3.80 (s, 2H), 2.74 (s, 2H), 0.86-0.78 (m, 4H).

### Example 41

### N-((6-(((cyclobutylmethyl)amino)methyl)imidazo[1,2-a]pyridin-2-yl)methyl)-8-oxo-8H-[1,3]dioxolo[4',5':4,5]pyrido[1,2-a]pyrimidine-6-carboxamide

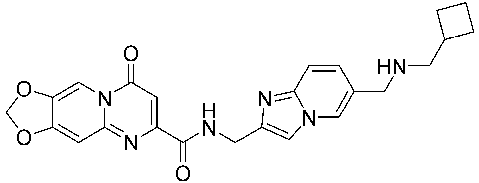

### (1) 2-(Chloromethyl)imidazo[1,2-a]pyridine-6-carbonitrile

6-Aminonicotinonitrile (1.19 g, 10 mmol) was dissolved in ethanol (20 mL), 1,3-dichloropropan-2-one (1.52 g, 12 mmol) was added, and the mixture was heated to 80°C and stirred overnight. The reaction solution was concentrated, and the residue was purified by silica gel column chromatography (petroleum ether/ethyl acetate=40/60) to obtain the title compound (380 mg, white solid), yield: 20%. MS (ESI): m/z 192.0 [M+H]⁺.

### (2) 2-(Aminomethyl)imidazo[1,2-a]pyridine-6-carbonitrile

2-(Chloromethyl)imidazo[1,2-a]pyridine-6-carbonitrile (19 mg, 0.1 mmol) and concentrated ammonia water (2 mL) were added to a 10 mL sealed tube, and heated to 80°C and stirred for 2 hours. The reaction solution was concentrated to obtain the title compound (17 mg, gray solid), yield: 100%. MS (ESI): m/z 173.1 [M+H]⁺.

### (3) N-((6-Cyanoimidazo[1,2-a]pyridin-2-yl)methyl)-8-oxo-8H-[1,3]dioxolo[4',5':4,5]pyrido[1,2-a]pyrimidine-6-carboxamide

Intermediate 5 (23 mg, 0.1 mmol) and 2-(Aminomethyl)imidazo[1,2-a]pyridine-6-carbonitrile (17 mg, 0.1 mmol) were dissolved in *N*,*N-*dimethylformamide (2 mL), *N,N*-diisopropylethylamine (64 mg, 0.5 mmol) and1-propylphosphonic anhydride (63 mg, 0.2 mmol, 50% ethyl acetate solution) were added, and stirred at room temperature for 3 hours. The reaction solution was purified by reverse-phase column (acetonitrile/water=50/50) to obtain the title compound (10 mg, white solid), yield: 26%. MS (ESI): m/z 389.0 [M+H]⁺.

### (4) N-((6-Formylimidazo[1,2-a]pyridin-2-yl)methyl)-8-oxo-8H-[1,3]dioxolo[4',5^{,}:4,5]pyrido[1,2-a]pyrimidine-6-carboxamide

N-((6-Cyanoimidazo[1,2-a]pyridin-2-yl)methyl)-8-oxo-8H-[1,3]dioxolo[4',5':4,5]pyrido[1,2-a]pyrimidine-6-carboxamide (10 mg, 0.026 mmol), sodium hypophosphite monohydrate (22 mg, 0.21 mmol), pyridine (1 mL), glacial acetic acid (0.5 mL), water (0.5 mL) and Raney nickel (10 mg) were placed in a 10 mL sealed tube, heated to 100°C and stirred for 2 hours. The reaction solution was filtered, the filtrate was concentrated, a small amount of water was added and then filtered, and the filter cake was dried to obtain the title compound (5 mg, light brown solid), yield: 49.6%. MS (ESI): m/z 392.0 [M+H]⁺.

### (5) N-((6-(((Cyclobutylmethyl)amino)methyl)imidazo[1,2-a]pyridin-2-yl)methyl)-8-oxo-8H-[1,3]dioxolo[4',5':4,5]pyrido[1,2-a]pyrimidine-6-carboxamide

N-((6-Formylimidazo[1,2-a]pyridin-2-yl)methyl)-8-oxo-8H-[1,3]dioxolo[4',5':4,5]pyrido[1,2-a]pyrimidine-6-carboxamide (5 mg, 0.013 mmol) and cyclobutylmethanamine (3 mg, 0.04 mmol) were dissolved in methanol (4 mL), glacial acetic acid (0.2 mL) was added, heated to 50°C and stirred for 10 minutes, then sodium cyanoborohydride (4 mg, 0.05 mmol) was added, stirring was continued at 50°C overnight. The reaction solution was filtered, and the filtrate was concentrated. The residue was purified by Prep-HPLC to obtain the title compound (1.7 mg, white solid), yield: 29%. MS (ESI): m/z 461.1 [M+H]⁺. ¹H NMR (400 MHz, DMSO-*d*₆) δ 8.97 (t, *J =* 6.0 Hz, 1H), 8.60 (s, 1H), 8.37 (s, 1H), 7.77 (s, 1H), 7.44 (d, *J* = 9.6 Hz, 1H), 7.22 (d, *J* = 9.6 Hz, 1H), 7.19 (s, 1H), 6.73 (s, 1H), 6.38 (s, 2H), 4.59 (d, *J =* 6.0 Hz, 2H), 3.64 (s, 2H), 2.64-2.61 (m, 2H), 2.42-2.37 (m, 1H), 2.01-1.93 (m, 2H), 1.82-1.75 (m, 2H), 1.65-1.57 (m, 2H).

### Example 42

### N-(1-(2-(((Cyclobutylmethyl)amino)methyl)-1H-indol-6-yl)cyclopropyl)-8-oxo-8H-[1,3]dioxolo[4',5':4,5]pyrido[1,2-a]pyrimidine-6-carboxamide

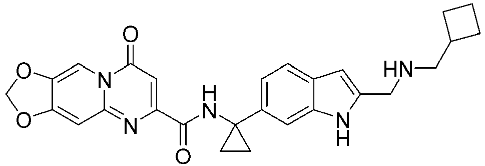

### (1) tert-Butyl 2-(diethoxymethyl)-6-(1-(8-oxo-8H-[1,3]dioxolo[4',5':4,5]pyrido[1,2-a]pyrimidine-6-carboxamido)cyclopropyl)-1H-indole-1-carboxylate

Intermediate 9 (40 mg, 0.11 mmol) and Intermediate 5 (19 mg, 0.08 mmol) were dissolved in *N,N-*dimethylformamide (5 mL), then 1-propylphosphonic anhydride (140 mg, 0.22 mmol, 50% ethyl acetate solution) and triethylamine (56 mg, 0.55 mmol) were added, and the mixture was stirred at room temperature for 1 hour. The reaction solution was poured into water (50 mL) and extracted with ethyl acetate (20 mL x 3). The combined organic phases were washed with saturated sodium chloride solution (15 mL x 3), dried over anhydrous sodium sulfate, filtered, and concentrated. The residue was purified by silica gel column chromatography (petroleum ether/ethyl acetate = 50/50) to afford the title compound (40 mg, pale yellow oil), yield: 91%. MS (ESI): m/z 591.0 [M+H]⁺.

### (2) tert-Butyl 2-formyl-6-(1-(8-oxo-8H-[1,3]dioxolo[4',5':4,5]pyrido[1,2-a]pyrimidine-6-carboxamido)cyclopropyl)-1H-indole-1-carboxylate

tert-Butyl 2-(diethoxymethyl)-6-(1-(8-oxo-8H-[1,3]dioxolo[4',5':4,5]pyrido[1,2-a]pyrimidine-6-carboxamido)cyclopropyl)-1H-indole-1-carboxylate (40 mg, 0.07 mmol) and acetic acid (1.5 mL) were dissolved in tetrahydrofuran/water mixture (10 mL/1 mL) and stirred at room temperature for 4 hours. The reaction solution was concentrated to remove tetrahydrofuran, water (30 mL) was added, and the mixture was extracted with ethyl acetate (20 mL x 3). The combined organic phases were washed with saturated sodium chloride solution (30 mL), dried over anhydrous sodium sulfate, filtered, and concentrated to obtain the title compound (30 mg, yellow solid), yield: 86%. MS (ESI): m/z 517.0 [M+H]⁺.

### (3) tert-Butyl 2-(((cyclobutylmethyl)amino)methyl)-6-(1-(8-oxo-8H-[1,3]dioxolo[4',5':4,5]pyrido[1,2-a]pyrimidine-6-carboxamido)cyclopropyl)-1H-indole-1-carboxylate

tert-Butyl 2-formyl-6-(1-(8-oxo-8H-[1,3]dioxolo[4',5':4,5]pyrido[1,2-a]pyrimidine-6-carboxamido)cyclopropyl)-1H-indole-1-carboxylate (30 mg, 0.06 mmol) and cyclobutylmethanamine (15 mg, 0.17 mmol) were dissolved in dichloroethane (5 mL), heated to 65°C and stirred for 1 hour. Then sodium cyanoborohydride (13 mg, 0.20 mmol) was added, stirring was continued at 65°C for 2 hours. The reaction solution was poured into water (30 mL) and extracted with ethyl acetate (20 mL x 3). The combined organic phases were washed with saturated sodium chloride solution (30 mL), dried over anhydrous sodium sulfate, filtered, and concentrated to obtain the title compound (34 mg, yellow solid), yield: 100%. MS (ESI): m/z 585.9 [M+H]⁺.

### (4) N-(1-(2-(((Cyclobutylmethyl)amino)methyl)-1H-indol-6-yl)cyclopropyl)-8-oxo-8H-[1,3]dioxolo[4',5':4,5]pyrido[1,2-a]pyrimidine-6-carboxamide

tert-Butyl 2-(((cyclobutylmethyl)amino)methyl)-6-(1-(8-oxo-8H-[1,3]dioxolo[4',5':4,5]pyrido[1,2-a]pyrimidine-6-carboxamido)cyclopropyl)-1H-indole-1-carboxylate (34 mg, 0.06 mmol) was dissolved in dichloromethane (2 mL), trifluoroacetic acid (1 mL) was added, and the mixture was stirred at room temperature for 4 hours. The reaction solution was concentrated, and purified by Prep-HPLC to obtain the title compound (3.3 mg, white solid), yield: 12%. MS (ESI): m/z 971.2 [2M+H]⁺. ¹H NMR (400 MHz, DMSO-*d*₆) δ 10.77 (s, 1H), 9.27 (s, 1H), 8.59 (s, 1H), 7.33-7.31 (m, 2H), 7.19 (s, 1H), 6.90 (d, *J =* 8.8 Hz, 1H), 6.67 (s, 1H), 6.39 (s, 2H), 6.17 (s, 1H), 3.76 (s, 2H), 2.55-2.50 (m, 2H), 2.42-2.33 (m, 1H), 1.96-1.92 (m, 2H), 1.82-1.75 (m, 2H), 1.65-1.58 (m, 2H), 1.30-1.21 (m, 4H).

### Example 43

### N-(1-(2-(((Cyclobutylmethyl)amino)methyl)-1H-indol-6-yl)cyclopropyl)-9-oxo-2,3-dihydro-9H-[1,4]dioxino[2',3':4,5]pyrido[1,2-a]pyrimidine-7-carboxamide

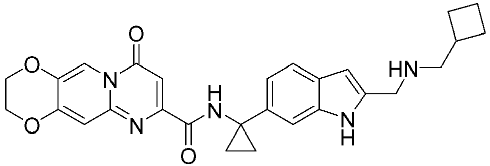

Referring to the synthetic method of **Example 42,** using Intermediate 9 and Intermediate 4 as starting materials, the title compound (4 mg, white solid) was obtained through similar steps. MS (ESI): m/z 500.1 [M+H]⁺. ¹H NMR (400 MHz, DMSO-*d*₆) δ 10.77 (s, 1H), 9.27 (s, 1H), 8.61 (s, 1H), 7.32-7.30 (m, 2H), 7.24 (s, 1H), 6.90 (dd, *J =* 8.4, 1.2 Hz, 1H), 6.60 (s, 1H), 6.16 (s, 1H), 4.58-4.56 (m, 2H), 4.48-4.47 (m, 2H), 3.75 (s, 2H), 2.50-2.48 (m, 2H), 2.40-2.36 (m, 1H), 1.98-1.93 (m, 2H), 1.82-1.74 (m, 2H), 1.65-1.58 (m, 2H), 1.28-1.22 (m, 4H).

### Example 44

### N-((2-(((Cyclobutylmethyl)amino)methyl)-1H-indol-6-yl)methyl)-7,8-dihydro-6H-9-oxa-2,2a,5,6-tetraazabenzo[cd]azulene-4-carboxamide

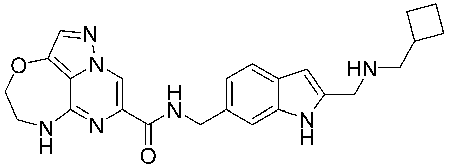

### (1) tert-Butyl (cyclobutylmethyl)((6-((7,8-dihydro-6H-9-oxa-2,2a,5,6-tetraazabenzo[cd]azulene-4-carboxamido)methyl)-1H-indol-2-yl)methyl)carbamate

Intermediate 10 (20 mg, 0.09 mmol) was dissolved in *N,N*-dimethylformamide (2 mL), and *N,N-*diisopropylethylamine (58 mg, 0.45 mmol) and HATU (51 mg, 0.13 mmol) were added, and the reaction was stirred at room temperature for 5 minutes. Then a solution of Intermediate 2 (46 mg, 0.13 mmol) in *N*,*N*-dimethylformamide (1 mL) was added, and the reaction was continued to stir at room temperature for 30 minutes. Saturated sodium bicarbonate solution (10 mL) was added to the reaction solution, and the aqueous phase was extracted with ethyl acetate (10 mL x 3). The combined organic phases were washed with saturated sodium chloride solution (10 mL x 3), dried over anhydrous sodium sulfate, and concentrated. The residue was purified by silica gel column chromatography (petroleum ether/ethyl acetate =50/50) to obtain the title compound (25 mg, pale yellow oil),, yield: 51%. MS (ESI): m/z 546.1 [M+H]⁺.

### (5) N-((2-(((Cyclobutylmethyl)amino)methyl)-1H-indol-6-yl)methyl)-7,8-dihydro-6H-9-oxa-2,2a,5,6-tetraazabenzo[cd]azulene-4-carboxamide

tert-Butyl (cyclobutylmethyl)((6-((7,8-dihydro-6H-9-oxa-2,2a,5,6-tetraazabenzo[cd]azulene-4-carboxamido)methyl)-1H-indol-2-yl)methyl)carbamate (25 mg, 0.046 mmol) was dissolved in dichloromethane (3 mL), and trifluoroacetic acid (1 mL) was added, followed by stirring at room temperature for 2 hours. The reaction solution was concentrated, and the residue was purified by Prep-HPLC to obtain the title compound (5.5 mg, white solid),, yield: 27%. MS (ESI): m/z 446.1 [M+H]⁺. ¹H NMR (400 MHz, DMSO-*d*₆) δ 10.89 (s, 1H), 8.28 (t, *J =* 6.4 Hz, 1H), 8.20 (s, 1H), 7.89 (t, *J =* 3.6 Hz, 1H), 7.81 (s, 1H), 7.40 (d, *J =* 8.0 Hz, 1H), 7.28 (s, 1H), 6.93 (dd, *J =* 8.0, 1.2 Hz, 1H), 6.24 (s, 1H), 4.55 (d, *J =* 6.0 Hz, 2H), 4.32-4.31 (m, 2H), 3.82 (s, 2H), 3.60-3.57 (m, 2H), 2.56-2.55 (m, 2H), 2.45-2.41 (m, 1H), 2.03-1.94 (m, 2H), 1.84-1.75 (m, 2H), 1.67-1.60 (m, 2H).

### Example 45

### N-((2-(((Cyclobutylmethyl)amino)methyl)-1H-indol-6-yl)methyl)-6,7,8,9-tetrahydro-2,2a,5,6-tetraazabenzo[cd]azulene-4-carboxamide

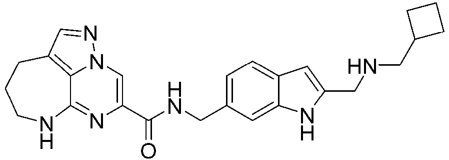

Referring to the synthetic method of **Example 44,** using Intermediate 11 and Intermediate 2 as starting materials, the title compound (7.6 mg, white solid) was obtained through similar steps. MS (ESI): m/z 444.1 [M+H]⁺. ¹H NMR (400 MHz, DMSO-*d*₆) δ 10.89 (s, 1H), 8.32 (s, 1H), 8.29 (t, *J* = 6.0 Hz, 1H), 7.93 (s, 1H), 7.76 (t, *J =* 4.0 Hz, 1H), 7.39 (d, *J =* 8.4 Hz, 1H), 7.28 (s, 1H), 6.93 (dd, *J =* 8.0 Hz, 1.2Hz, 1H), 6.22 (s, 1H), 4.55 (d, *J =* 6.0 Hz, 2H), 3.79 (s, 2H), 3.38-3.35 (m, 2H), 2.88 (t, *J =* 6.0 Hz, 2H), 2.53-2.52 (m, 2H), 2.44-2.40 (m, 1H), 2.03-1.93 (m, 4H), 1.84-1.78 (m, 2H), 1.66-1.58 (m, 2H).

### Example 46

### N-((2-((((3-Fluorobicyclo[1.1.1]pentan-1-yl)methyl)amino)methyl)-1H-indol-6-yl)methyl)-9-oxo-2,3-dihydro-9H-[1,4]dioxolo[2',3':4,5]pyrido[1,2-a]pyrimidine-7-carboxamide

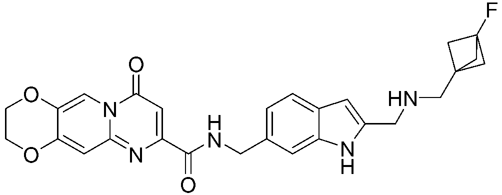

Referring to the synthetic method of **Example 36,** using Intermediate 8 and (3-fluorobicyclo[1.1.1]pentan-1-yl)methanamine hydrochloride as starting materials, the title compound (10.5 mg, white solid) was obtained through similar steps. MS (ESI): m/z 504.1 [M+H]⁺. ¹H NMR (400 MHz, DMSO-*d*₆) δ 10.85 (s, 1H), 9.04 (t, *J =* 6.4 Hz, 1H), 8.62 (s, 1H), 7.36 (d, *J =* 8.0 Hz, 1H), 7.30 (s, 1H), 7.19 (s, 1H), 6.94 (dd, *J =* 8.4, 1.2 Hz, 1H), 6.66 (s, 1H), 6.20 (s, 1H), 4.59-4.53 (m, 4H), 4.47-4.45 (m, 2H), 3.80 (s, 2H), 2.75 (s, 2H), 1.92 (d, *J =* 2.8 Hz, 6H).

### Example 47

### N-((2-(((4,4-Difluorocyclohexyl)amino)methyl)-1H-indol-6-yl)methyl)-9-oxo-2,3-dihydro-9H-[1,4]dioxolo[2',3':4,5]pyrido[1,2-a]pyrimidine-7-carboxamide

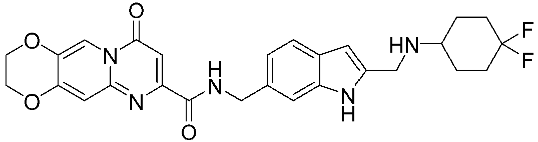

Referring to the synthetic method of **Example 34,** using Intermediate 8 and 4,4-difluorocyclohexan-1-amine as starting materials, the title compound (9.8 mg, white solid) was obtained through similar steps. MS (ESI): m/z 524.1 [M+H]⁺. ¹H NMR (400 MHz, DMSO-*d*₆) δ 10.85 (s, 1H), 9.04 (t, *J =* 6.4 Hz, 1H), 8.62 (s, 1H), 7.36 (d, *J =* 8.0 Hz, 1H), 7.30 (s, 1H), 7.18 (s, 1H), 6.94 (dd, *J =* 8.0, 1.6 Hz, 1H), 6.67 (s, 1H), 6.22 (s, 1H), 4.57-4.53 (m, 4H), 4.47-4.45 (m, 2H), 3.82 (s, 2H), 2.63-2.56 (m, 1H), 2.05-1.96 (m, 2H), 1.84-1.70 (m, 4H), 1.46-1.36 (m, 2H).

### Example 48

### N-((2-(((3,3-Difluorocyclopentyl)amino)methyl)-1H-indol-6-yl)methyl)-9-oxo-2,3-dihydro-9H-[1,4]dioxino[2',3':4,5]pyrido[1,2-a]pyrimidine-7-carboxamide

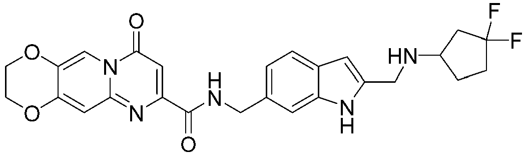

Following the synthetic method of **Example 36,** using Intermediate 8 and 3,3-difluorocyclopentan-1-amine hydrochloride as starting materials, the title compound was obtained through similar procedures (11.1 mg, white solid). MS (ESI): m/z 510.1 [M+H]⁺. ¹H NMR (400 MHz, DMSO-*d*₆) δ 10.87 (s, 1H), 9.04 (t, *J =* 6.4 Hz, 1H), 8.62 (s, 1H), 7.37 (d, *J =* 8.4 Hz, 1H), 7.30 (s, 1H), 7.18 (s, 1H), 6.95 (dd, *J* = 8.0, 1.2 Hz, 1H), 6.67 (s, 1H), 6.23 (s, 1H), 4.57-4.53 (m, 4H), 4.47-4.45 (m, 2H), 3.79 (s, 2H), 3.22-3.12 (m, 1H), 2.35-2.14 (m, 2H), 2.09-1.81 (m, 4H).

### Example 49

### N-((2-(((3,3-Difluorocyclobutyl)amino)methyl)-1H-indol-6-yl)methyl)-9-oxo-2,3-dihydro-9H-[1,4]dioxolo[2',3':4,5]pyrido[1,2-a]pyrimidine-7-carboxamide

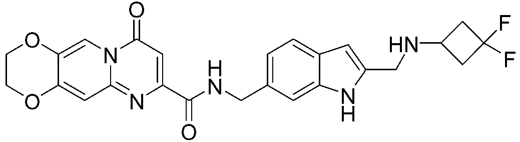

Following the synthetic method of **Example 34,** using Intermediate 8 and 3,3-difluorocyclobutan-1-amine as starting materials, the title compound was obtained through similar procedures (15.5 mg, white solid). MS (ESI): m/z 496.1 [M+H]⁺. ¹H NMR (400 MHz, DMSO-*d*₆) δ 10.87 (s, 1H), 9.04 (t, *J =* 6.4 Hz, 1H), 8.62 (s, 1H), 7.37 (d, *J =* 8.4 Hz, 1H), 7.30 (s, 1H), 7.18 (s, 1H), 6.94 (dd, *J* = 8.0, 1.2 Hz, 1H), 6.68 (s, 1H), 6.22 (s, 1H), 4.57-4.52 (m, 4H), 4.48-4.44 (m, 2H), 3.78 (s, 2H), 3.15-3.07 (m, 1H), 2.72-2.63 (m, 2H), 2.38-2.26 (m, 2H).

### Example 50

### 9-Oxo-N-((2-((((1-(trifluoromethyl)cyclopropyl)methyl)amino)methyl)-1H-indol-6-yl)methyl)-2,3-dihydro-9H-[1,4]dioxino[2',3':4,5]pyrido[1,2-a]pyrimidine-7-carboxamide

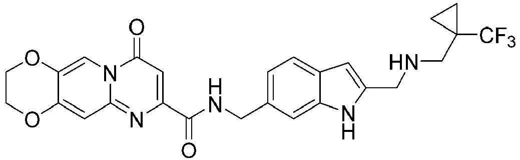

Following the synthetic method of **Example 36,** using Intermediate 8 and (1-(trifluoromethyl)cyclopropyl)methanamine hydrochloride as starting materials, the title compound was obtained through similar procedures (9.7 mg, white solid). MS (ESI): m/z 528.1 [M+H]⁺. ¹H NMR (400 MHz, DMSO-*d*₆) δ 10.84 (s, 1H), 9.04 (t, *J =* 6.4 Hz, 1H), 8.62 (s, 1H), 7.37 (d, *J* = 8.0 Hz, 1H), 7.30 (s, 1H), 7.19 (s, 1H), 6.95 (dd, *J =* 8.0, 1.2 Hz, 1H), 6.67 (s, 1H), 6.22 (s, 1H), 4.57-4.52 (m, 4H), 4.47-4.45 (m, 2H), 3.80 (s, 2H), 2.74 (s, 2H), 0.85-0.80 (m, 4H).

### Example 51

### N-((2-((((1-Methylcyclobutyl)methyl)amino)methyl)-1H-indol-6-yl)methyl)-8-oxo-8H-[1,3]dioxolo[4',5':4,5]pyrido[1,2-a]pyrimidine-6-carboxamide

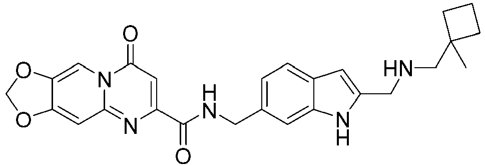

Following the synthetic method of **Example 36,** using Intermediate 7 and (1-methylcyclobutyl)methanamine hydrochloride as starting materials, the title compound was obtained through similar procedures (6.8 mg, white solid). MS (ESI): m/z 474.1 [M+H]⁺. ¹H NMR (400 MHz, DMSO-*d*₆) δ 10.85 (s, 1H), 9.02 (t, *J =* 6.4 Hz, 1H), 8.59 (s, 1H), 7.37 (d, *J =* 8.0 Hz, 1H), 7.30 (s, 1H), 7.14 (s, 1H), 6.95 (dd, *J =* 8.0, 1.6 Hz, 1H), 6.73 (s, 1H), 6.37 (s, 2H), 6.21 (s, 1H), 4.54 (d, *J =* 6.4 Hz, 2H), 3.81 (s, 2H), 2.41 (s, 2H), 1.86-1.79 (m, 3H), 1.75-1.68 (m, 1H), 1.58-1.52 (m, 2H), 1.08 (s, 3H).

### Example 52

### N-((2-((((1-Methylcyclobutyl)methyl)amino)methyl)-1H-indol-6-yl)methyl)-9-oxo-2,3-dihydro-9H-[1,4]dioxino[2',3':4,5]pyrido[1,2-a]pyrimidine-7-carboxamide

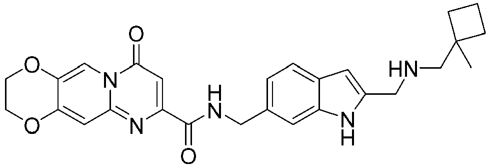

Following the synthetic method of **Example 36,** using Intermediate 8 and (1-methylcyclobutyl)methanamine hydrochloride as starting materials, the title compound was obtained through similar procedures (7.4 mg, white solid). MS (ESI): m/z 488.1 [M+H]⁺. ¹H NMR (400 MHz, DMSO-*d*₆) δ 10.85 (s, 1H), 9.03 (t, *J =* 6.4 Hz, 1H), 8.62 (s, 1H), 7.36 (d, *J =* 8.0 Hz, 1H), 7.30 (s, 1H), 7.19 (s, 1H), 6.94 (dd, *J* = 8.0, 1.2 Hz, 1H), 6.67 (s, 1H), 6.21 (s, 1H), 4.57-4.53 (m, 4H), 4.47-4.45 (m, 2H), 3.81 (s, 2H), 2.41 (s, 2H), 1.88-1.68 (m, 4H), 1.60-1.50 (m, 2H), 1.08 (s, 3H).

### Example 53

### N-((2-((((3,3-Difluoro-1-methylcyclobutyl)methyl)amino)methyl)-1H-indol-6-yl)methyl)-8-oxo-8H-[1,3]dioxolo[4',5':4,5]pyrido[1,2-a]pyrimidine-6-carboxamide

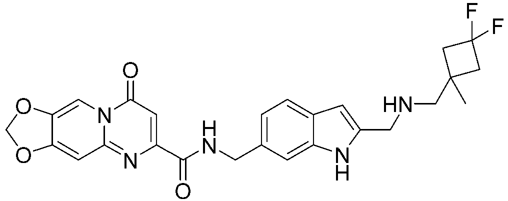

Following the synthetic method of **Example 36,** using Intermediate 7 and (3,3-difluoro-1-methylcyclobutyl)methanamine hydrochloride as starting materials, the title compound (5.7 mg, white solid) was obtained through similar procedures. MS (ESI): m/z 510.1 [M+H]⁺. ¹H NMR (400 MHz, DMSO-*d*₆) δ 10.85 (s, 1H), 9.02 (t, *J =* 6.4 Hz, 1H), 8.59 (s, 1H), 7.37 (d, *J =* 8.0 Hz, 1H), 7.30 (s, 1H), 7.14 (s, 1H), 6.95 (dd, *J =* 8.0, 1.6 Hz, 1H), 6.73 (s, 1H), 6.37 (s, 2H), 6.22 (s, 1H), 4.55 (d, *J =* 6.4 Hz, 2H), 3.83 (s, 2H), 2.54-2.52 (m, 1H), 2.47 (s, 2H), 2.45-2.42 (m, 1H), 2.20-2.10 (m, 2H), 1.16 (s, 3H).

### Example 54

### N-((2-((((3,3-Difluoro-1-methylcyclobutyl)methyl)amino)methyl)-1H-indol-6-yl)methyl)-9-oxo-2,3-dihydro-9H-[1,4]dioxino[2',3':4,5]pyrido[1,2-a]pyrimidine-7-carboxamide

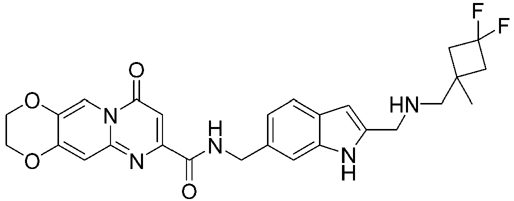

Following the synthetic method of **Example 36,** using Intermediate 8 and (3,3-difluoro-1-methylcyclobutyl)methanamine hydrochloride as starting materials, the title compound (6.5 mg, white solid) was obtained through similar procedures. MS (ESI): m/z 524.1 [M+H]⁺. ¹H NMR (400 MHz, DMSO-*d*₆) δ 10.84 (s, 1H), 9.04 (t, *J =* 6.4 Hz, 1H), 8.62 (s, 1H), 7.36 (d, *J =* 8.0 Hz, 1H), 7.30 (s, 1H), 7.19 (s, 1H), 6.94 (dd, *J =* 8.0, 1.2 Hz, 1H), 6.67 (s, 1H), 6.22 (s, 1H), 4.57-4.53 (m, 4H), 4.47-4.45 (m, 2H), 3.82 (s, 2H), 2.47-2.45 (m, 4H), 2.20-2.10 (m, 2H), 1.16 (s, 3H).

### Example 55

### N-((6-(((Cyclobutylmethyl)amino)methyl)imidazo[1,2-a]pyridin-2-yl)methyl)-9-oxo-2,3-dihydro-9H-[1,4]dioxino[2',3':4,5]pyrido[1,2-a]pyrimidine-7-carboxamide

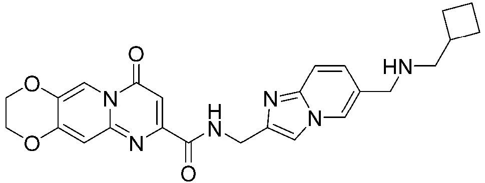

Following the synthetic method of **Example 41,** replacing Intermediate 5 with Intermediate 4 in Step 3, the title compound (5.1 mg, white solid) was obtained through similar procedures. MS (ESI): m/z 475.1 [M+H]⁺. ¹H NMR (400 MHz, DMSO-*d*₆) δ 9.01 (t, *J =* 4.8 Hz, 1H), 8.63 (s, 1H), 8.42 (s, 1H), 7.80 (s, 1H), 7.47 (d, *J =* 10.0 Hz, 1H), 7.24 (d, *J* = 9.6 Hz, 1H), 7.22 (s, 1H), 6.66 (s, 1H), 4.60-4.56 (m, 4H), 4.48-4.46 (m, 2H), 3.75 (brs, 1H), 3.30 (s, 2H), 2.52-2.51 (m, 2H), 2.38-2.35 (m, 1H), 2.02-1.95 (m, 2H), 1.87-1.73 (m, 2H), 1.69-1.62 (m, 2H).

### Example 56

### 8-Oxo-N-((2-((((1-(trifluoromethyl)cyclobutyl)methyl)amino)methyl)-1H-indol-6-yl)methyl)-8H-[1,3]dioxolo[4',5':4,5]pyrido[1,2-a]pyrimidine-6-carboxamide

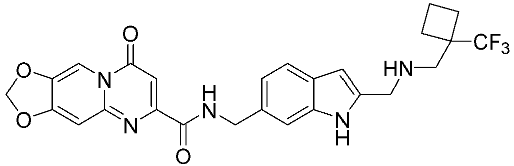

Following the synthetic method of **Example 34,** using Intermediate 7 and [1-(trifluoromethyl)cyclobutyl]methanamine as starting materials, the title compound (3.2 mg, white solid) was obtained through similar procedures. MS (ESI): m/z 528.1 [M+H]⁺. ¹H NMR (400 MHz, DMSO-*d*₆) δ 10.88 (s, 1H), 9.03 (t, *J =* 6.0 Hz, 1H), 8.59 (s, 1H), 7.39 (d, *J =* 8.0Hz, 1H), 7.31 (s, 1H), 7.14 (s, 1H), 6.95 (dd, *J* = 8.0 , 1.2 Hz, 1H), 6.73 (s, 1H), 6.37 (s, 2H), 6.25 (s, 1H), 4.55 (d, *J* = 6.0Hz, 2H), 3.87 (s, 2H), 2.68 (s, 2H), 2.31-2.25 (m, 1H), 2.11-2.06 (m, 4H), 1.89-1.81 (m, 2H).

### Example 57

### 9-Oxo-N-((2-((((1-(trifluoromethyl)cyclobutyl)methyl)amino)methyl)-1H-indol-6-yl)methyl)-2,3-dihydro-9H-[1,4]dioxino[2',3':4,5]pyrido[1,2-a]pyrimidine-7-carboxamide

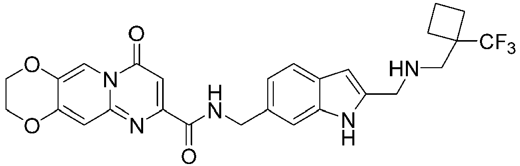

Following the synthetic method of **Example 34,** using Intermediate 8 and [1-(trifluoromethyl)cyclobutyl]methanamine as starting materials, the title compound (2.5 mg, white solid) was obtained through similar procedures. MS (ESI): m/z 542.1 [M+H]⁺. ¹H NMR (400 MHz, DMSO-*d*₆) δ 10.89 (s, 1H), 9.05 (t, *J =* 6.0 Hz, 1H), 8.62 (s, 1H), 7.39 (d, *J =* 7.6 Hz, 1H), 7.31 (s, 1H), 7.19 (s, 1H), 6.96 (d, *J =* 8.0 Hz, 1H), 6.67 (s, 1H), 6.25 (s, 1H), 4.57-4.54 (m, 4H), 4.47-4.45 (m, 2H), 3.87 (s, 2H), 2.70-2.67 (m, 2H), 2.11-2.07 (m, 4H), 1.90-1.83 (m, 2H).

### Example 58

### 8-Oxo-N-((2-(((spiro[3.3]heptan-2-ylmethyl)amino)methyl)-1H-indol-6-yl)methyl)-8H-[1,3]dioxolo[4',5':4,5]pyrido[1,2-a]pyrimidine-6-carboxamide

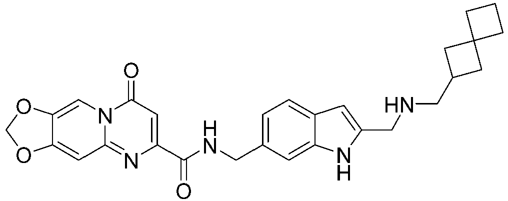

### (1) Spiro[3.3]heptane-2-carboxamide

Spiro[3.3]heptane-2-carboxylic acid (300 mg, 2.14 mmol) was dissolved in dichloromethane (10 mL), and HATU (977 mg, 2.57 mmol), N,N-diisopropylethylamine (1.38 g, 10.7 mmol) and ammonium chloride (344 mg, 6.4 mmol) were added, and the reaction was stirred at room temperature overnight. The reaction mixture was poured into saturated aqueous sodium bicarbonate solution (40 mL) and extracted with ethyl acetate (2 x 30 mL). The organic phases were combined, washed with saturated brine (40 mL), dried over anhydrous sodium sulfate, filtered, and concentrated to give the title compound (300 mg, yellow oil), yield: 100%. MS (ESI): m/z 139.8 [M+H]⁺.

### (2) Spiro[3.3]heptan-2-ylmethanamine

Spiro[3.3]heptane-2-carboxamide (100 mg, 0.72 mmol) was dissolved in tetrahydrofuran (5 mL), and lithium aluminum hydride (85 mg, 2.16 mmol) was added. The reaction mixture was heated to 70°C and stirred for 2 hours. The reaction mixture was cooled to room temperature, diluted with ethyl acetate (15 mL), then two drops of water were added. After thorough stirring, 5 g of anhydrous sodium sulfate was added, and the mixture was stirred for 5 minutes, then filtered. The filtrate was concentrated to approximately 0.5 mL and directly used in the next reaction, yield: 100%. MS (ESI): m/z 126.2 [M+H]⁺.

### (3) 8-Oxo-N-((2-(((spiro[3.3]heptan-2-ylmethyl)amino)methyl)-1H-indol-6-yl)methyl)-8H-[1,3]dioxolo[4',5':4,5]pyrido[1,2-a]pyrimidine-6-carboxamide

The crude product obtained in the previous step was diluted with methanol (4 mL), and Intermediate 7 (19.5 mg, 0.05 mmol), glacial acetic acid (0.2 mL) and triethylamine (0.2 mL) were added. Insoluble matter was present in the reaction mixture. After heating to 50°C stirring for 10 minutes, sodium cyanoborohydride (10 mg, 0.15 mmol) was added, and the reaction was continued at 50°C overnight. The reaction mixture was filtered, concentrated, and the residue was purified by Prep-HPLC to give the title compound (5.2 mg, white solid), yield: 20%. MS (ESI): m/z 500.1 [M+H]⁺. ¹H NMR (400 MHz, DMSO-*d*₆) δ 10.84 (s, 1H), 9.01 (t, *J =* 6.0 Hz, 1H), 8.59 (s, 1H), 7.36 (d, *J =* 8.0 Hz, 1H), 7.28 (s, 1H), 7.14 (s, 1H), 6.94 (d, *J =* 8.4 Hz, 1H), 6.73 (s, 1H), 6.37 (s, 2H), 6.19 (s, 1H), 4.54 (d, *J =* 6.0 Hz, 2H), 3.75 (s, 2H), 2.46-4.45 (m, 2H), 2.22-2.18 (m, 1H), 2.03-1.98 (m, 2H), 1.96-1.92 (m, 2H), 1.83-1.79 (m, 2H), 1.76-1.70 (m, 2H), 1.62-1.57 (m, 2H).

### Example 59

### N-((2-(((Cyclobutylmethyl)amino)methyl)-1H-indol-6-yl)methyl)-6-methyl-7,8-dihydro-6H-9-oxa-2,2a,5,6-tetraazabenzo[cd]azulene-4-carboxamide

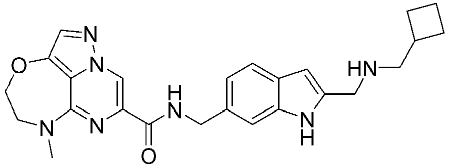

### (1) 4-Chloro-6-methyl-7,8-dihydro-6H-9-oxa-2,2a,5,6-tetraazabenzo[cd]azulene

4-Chloro-7,8-dihydro-6H-9-oxa-2,2a,5,6-tetraazabenzo[cd]azulene (150 mg, 0.71 mmol, Step 7 of Intermediate 10) was dissolved in tetrahydrofuran (5 mL), sodium hydride (56 mg, 1.4 mmol) was added, followed by iodomethane (203 mg, 1.4 mmol), and the reaction was stirred at room temperature overnight. The reaction mixture was poured into water (20 mL) and extracted with ethyl acetate (20 mL x 3). The combined organic phases were washed with saturated sodium chloride solution (20 mL x 3), dried over anhydrous sodium sulfate, filtered, and concentrated. The residue was purified by silica gel column chromatography (petroleum ether/ethyl acetate = 5/1) to afford the title compound (40 mg, yellow solid), yield: 25%. MS (ESI): m/z 224.9 [M+H]⁺.

### (2) Methyl 6-methyl-7,8-dihydro-6H-9-oxa-2,2a,5,6-tetraazabenzo[cd]azulene-4-carboxylate

4-Chloro-6-methyl-7,8-dihydro-6H-9-oxa-2,2a,5,6-tetraazabenzo[cd]azulene (40 mg, 0.18 mmol), palladium acetate (4 mg, 0.018 mmol) and 1,3-bis(diphenylphosphino)propane (11 mg, 0.027 mmol) were dissolved in methanol/acetonitrile (10 mL/5 mL), triethylamine (66 mg, 0.36 mmol) was added, and the mixture was heated to 120°C in an autoclave (5 Mpa, carbon monoxide atmosphere) and stirred overnight. The reaction mixture was concentrated, and the residue was purified by silica gel column chromatography (100% ethyl acetate) to afford the title compound (20 mg, yellow solid), yield: 45%. MS (ESI): m/z 249.1 [M+H]⁺.

### (3) 6-Methyl-7,8-dihydro-6H-9-oxa-2,2a,5,6-tetraazabenzo[cd]azulene-4-carboxylic acid

Methyl 6-methyl-7,8-dihydro-6H-9-oxa-2,2a,5,6-tetraazabenzo[cd]azulene-4-carboxylate (20 mg, 0.08 mmol) was dissolved in methanol/water (5 mL/1 mL), sodium hydroxide (6 mg, 0.16 mmol) was added, and the reaction was stirred at room temperature for 3 hours. The mixture was adjusted to acidic with 1M dilute hydrochloric acid, and the solid precipitated, which was filtered and dried to afford the title compound (15 mg, white solid), yield: 83%. MS (ESI): m/z 234.9 [M+H]⁺.

### (4) tert-Butyl (cyclobutylmethyl)((6-((6-methyl-7,8-dihydro-6H-9-oxa-2,2a,5,6-tetraazabenzo[cd]azulene-4-carboxamido)methyl)-1H-indol-2-yl)methyl)carbamate

6-Methyl-7,8-dihydro-6H-9-oxa-2,2a,5,6-tetraazabenzo[cd]azulene-4-carboxylic acid (15 mg, 0.06 mmol) and Intermediate 2 (21 mg, 0.06 mmol) were dissolved in *N,N*-dimethylformamide (5 mL), then *N,N*-diisopropylethylamine (15 mg, 0.12 mmol) and HATU (27 mg, 0.072 mmol) were added, and the reaction was stirred at room temperature overnight. The reaction mixture was poured into water (20 mL) and extracted with ethyl acetate (20 mL x 3). The combined organic phases were washed with saturated sodium chloride solution (20 mL x 3), dried over anhydrous sodium sulfate, filtered, and concentrated. The residue was purified by silica gel column chromatography (petroleum ether/ethyl acetate = 50/50) to afford the title compound (20 mg, yellow solid), yield: 56%. MS (ESI): m/z 560.4 [M+H]⁺.

### (5) N-((2-(((Cyclobutylmethyl)amino)methyl)-1H-indol-6-yl)methyl)-6-methyl-7,8-dihydro-6H-9-oxa-2,2a,5,6-tetraazabenzo[cd]azulene-4-carboxamide

tert-Butyl (cyclobutylmethyl)((6-((6-methyl-7,8-dihydro-6H-9-oxa-2,2a,5,6-tetraazabenzo[cd]azulene-4-carboxamido)methyl)-1H-indol-2-yl)methyl)carbamate (20 mg, 0.04 mmol) was dissolved in dichloromethane (5 mL), trifluoroacetic acid (3 mL) was added, and the reaction was stirred at room temperature for 2 hours. The reaction mixture was concentrated, and the residue was purified by Prep-HPLC to afford the title compound (7.9 mg, white solid), yield: 49%. MS (ESI): m/z 460.2 [M+H]⁺. ¹H NMR (400 MHz, DMSO-*d*₆) δ 10.84 (s, 1H), 8.84 (t, *J =* 6.4 Hz, 1H), 8.19 (s, 1H), 7.81 (s, 1H), 7.36 (d, *J =* 8.0 Hz, 1H), 7.27 (s, 1H), 6.94 (dd, *J* = 8.0, 1.2 Hz, 1H), 6.19 (s, 1H), 4.57 (d, *J =* 6.4 Hz, 2H), 4.39-4.385 (m, 2H), 3.82-3.81 (m, 2H), 3.76 (s, 2H), 3.27 (s, 3H), 2.56-2.51 (m, 2H), 2.42-2.37 (m, 1H), 2.03-1.92 (m, 2H), 1.82-1.75 (m, 2H), 1.65-1.59 (m, 2H).

### Example 60

### N-((2-(((Bicyclo[3.1.0]hexan-1-ylmethyl)amino)methyl)-1H-indol-6-yl)methyl)-8-oxo-8H-[1,3]dioxolo[4',5':4,5]pyrido[1,2-a]pyrimidine-6-carboxamide

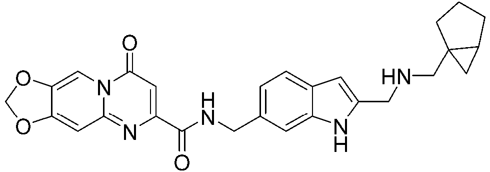

Following the synthetic method of **Example 58,** using bicyclo[3.1.0]hexane-1-carboxylic acid as the starting material in the first step, the title compound was obtained through similar procedures (15.1 mg, white solid). MS (ESI): m/z 486.1 [M+H]⁺. ¹H NMR (400 MHz, DMSO-*d*₆) δ 10.85 (s, 1H), 9.02 (t, *J =* 6.4 Hz, 1H), 8.59 (s, 1H), 7.36(d, *J* = 8.0 Hz, 1H), 7.29 (s, 1H), 7.14 (s, 1H), 6.95 (dd, *J* = 8.0, 1.2 Hz, 1H), 6.73 (s, 1H), 6.37 (s, 2H), 6.19 (s, 1H), 4.54 (d, *J =* 6.4 Hz, 2H), 3.79 (s, 2H), 2.58 (s, 2H), 1.69-1.62 (m, 3H), 1.61-1.51 (m, 2H), 1.19-1.11 (m, 1H), 1.02-0.98 (m, 1H), 0.32-0.26 (m, 2H).

### Example 61

### N-((2-(((Cyclobutylmethyl)amino)methyl)-1H-indol-6-yl)methyl)-7,8-dihydro-6,9-dioxa-2,2a,5-triazabenzo[cd]azulene-4-carboxamide

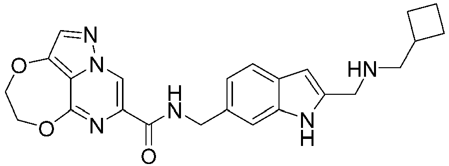

### (1) 6-Chloro-4-(2-((tetrahydro-2H-pyran-2-yl)oxy)ethoxy)pyrazolo[1,5-a]pyrazin-3-ol

4,6-Dichloropyrazolo[1,5-a]pyrazin-3-ol (300 mg, 1.48 mmol, Step 4 of Intermediate 10) and sodium hydride (118 mg, 2.96 mmol) were dissolved in tetrahydrofuran (5 mL), 2-((tetrahydro-2H-pyran-2-yl)oxy)ethan-1-ol (432 mg, 2.96 mmol) was added, and the reaction was stirred at room temperature overnight. The reaction mixture was poured into water (20 mL), adjusted to acidic pH with dilute hydrochloric acid, and extracted with ethyl acetate (20 mL x 3). The combined organic phases were washed with saturated sodium chloride solution (20 mL x 3), dried over anhydrous sodium sulfate, and concentrated. The residue was purified by silica gel column chromatography (petroleum ether/ethyl acetate = 2/1) to afford the title compound (380 mg, yellow oil), yield: 82%. MS (ESI): m/z 314.2 [M+H]⁺.

### (2) 6-Chloro-4-(2-hydroxyethoxy)pyrazolo[1,5-a]pyrazin-3-ol

6-Chloro-4-(2-((tetrahydro-2H-pyran-2-yl)oxy)ethoxy)pyrazolo[1,5-a]pyrazin-3-ol (380 mg, 1.2 mmol) was dissolved in methanol (10 mL), p-toluenesulfonic acid monohydrate (22.8 mg, 0.12 mmol) was added, and the reaction was stirred at room temperature overnight. The reaction mixture was concentrated, and the residue was purified by silica gel column chromatography (petroleum ether/ethyl acetate = 1/1) to afford the title compound (200 mg, white solid), yield: 72%. MS (ESI): m/z 230.1 [M+H]⁺.

### (3) 4-Chloro-7,8-dihydro-6,9-dioxa-2,2a,5-triazabenzo[cd]azulene

6-Chloro-4-(2-hydroxyethoxy)pyrazolo[1,5-a]pyrazin-3-ol (200 mg, 0.87 mmol) and triphenylphosphine (681 mg, 2.6 mmol) were dissolved in tetrahydrofuran (10 mL), diisopropyl azodicarboxylate (525 mg, 2.6 mmol) was added dropwise, and the mixture was heated to 50°C and stirred overnight. The reaction mixture was concentrated, and the residue was purified by silica gel column chromatography (petroleum ether/ethyl acetate = 5/1) to afford the title compound (70 mg, white solid), yield: 38%. MS (ESI): m/z 212.0 [M+H]⁺.

### (4) Methyl 7,8-dihydro-6,9-dioxa-2,2a,5-triazabenzo[cd]azulene-4-carboxylate

4-Chloro-7,8-dihydro-6,9-dioxa-2,2a,5-triazabenzo[cd]azulene (70 mg, 0.33 mmol), palladium acetate (7.5 mg, 0.033 mmol) and 1,3-bis(diphenylphosphino)propane (21 mg, 0.05 mmol) were dissolved in methanol/acetonitrile (10 mL/5 mL), triethylamine (67 mg, 0.66 mmol) was added, and the mixture was heated to 120°C in an autoclave (5 Mpa, carbon monoxide atmosphere) and stirred overnight. The reaction mixture was concentrated, and the residue was purified by silica gel column chromatography (petroleum ether/ethyl acetate = 1/1) to afford the title compound (40 mg, white solid), yield: 51%. MS (ESI): m/z 236.0 [M+H]⁺.

### (5) 7,8-Dihydro-6,9-dioxa-2,2a,5-triazabenzo[cd]azulene-4-carboxylic acid

Methyl 7,8-dihydro-6,9-dioxa-2,2a,5-triazabenzo[cd]azulene-4-carboxylate (40 mg, 0.17 mmol) was dissolved in methanol/water (5 mL/1 mL), sodium hydroxide (13.6 mg, 0.34 mmol) was added, and the reaction was stirred at room temperature for 3 hours. The pH was adjusted to acidic with 1M dilute hydrochloric acid, and the solid was filtered and dried to afford the title compound (19 mg, white solid), yield: 50%. MS (ESI): m/z 222.1 [M+H]⁺.

### (6) Tert-Butyl (cyclobutylmethyl)((6-((7,8-dihydro-6,9-dioxa-2,2a,5-triazabenzo[cd]azulene-4-carboxamido)methyl)-1H-indol-2-yl)methyl)carbamate

7,8-Dihydro-6,9-dioxa-2,2a,5-triazabenzo[cd]azulene-4-carboxylic acid (19 mg, 0.08 mmol) and Intermediate 2 (27 mg, 0.08 mmol) were dissolved in N,N-dimethylformamide (5 mL), followed by N,N-diisopropylethylamine (21 mg, 0.16 mmol) and HATU (38 mg, 0.1 mmol), and the reaction was stirred at room temperature overnight. The reaction mixture was poured into water (20 mL) and extracted with ethyl acetate (20 mL x 3). The combined organic phases were washed with saturated sodium chloride solution (20 mL x 3), dried over anhydrous sodium sulfate, filtered, and concentrated. The residue was purified by silica gel column chromatography (petroleum ether/ethyl acetate = 1/1) to afford the title compound (20 mg, colorless oil), yield: 43%. MS (ESI): m/z 547.4 [M+H]⁺.

### (7) N-((2-(((Cyclobutylmethyl)amino)methyl)-1H-indol-6-yl)methyl)-7,8-dihydro-6,9-dioxa-2,2a,5-triazabenzo[cd]azulene-4-carboxamide

Tert-Butyl (cyclobutylmethyl)((6-((7,8-dihydro-6,9-dioxa-2,2a,5-triazabenzo[cd]azulene-4-carboxamido)methyl)-1H-indol-2-yl)methyl)carbamate (20 mg, 0.04 mmol) was dissolved in dichloromethane (5 mL), trifluoroacetic acid (3 mL) was added, and the reaction was stirred at room temperature for 2 hours. The reaction mixture was concentrated, and the residue was purified by Prep-HPLC to afford the title compound (4.3 mg, white solid), yield: 27%. MS (ESI): m/z 893.2 [2M+H]⁺. ¹H NMR (400 MHz, DMSO-*d*₆) δ 10.85 (s, 1H), 8.86 (t, *J =* 6.0Hz, 1H), 8.60 (s, 1H), 8.01 (s, 1H), 7.37 (d, *J =* 8.4 Hz, 1H), 7.28 (s, 1H), 6.95 (d, *J =* 8.0 Hz, 1H), 6.22 (s, 1H), 4.78-4.77 (m, 2H), 4.59-4.58 (m, 2H), 4.53 (d, *J =* 6.0 Hz, 2H), 3.79 (s, 2H), 2.62-2.59 (m, 2H), 2.44-2.36 (m, 1H), 1.98-1.96 (m, 2H), 1.83-1.75 (m, 2H), 1.66-1.59 (m, 2H).

### Example 62

### N-((2-(((cyclobutylmethyl)amino)methyl-d₂)-1H-indol-6-yl)methyl)-8-oxo-8H-[1,3]dioxolo[4',5':4,5]pyrido[1,2-a]pyrimidine-6-carboxamide

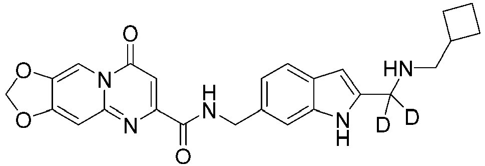

### (1) 6-Cyano-1H-indole-2-carboxylic acid

Methyl 6-cyano-1H-indole-2-carboxylate (2 g, 10 mmol) was dissolved in methanol/water (20 mL/5 mL), sodium hydroxide (800 mg, 20 mmol) was added, and the reaction mixture was heated to 40°C and stirred for 3 hours. The pH was adjusted to acidic with 1M dilute hydrochloric acid, and the solid precipitated, filtered and dried to afford the title compound (1.8 g, white solid), yield: 97%. MS (ESI): m/z 186.9 [M+H]⁺.

### (2) 6-Cyano-N-(cyclobutylmethyl)-1H-indole-2-carboxamide

6-Cyano-1H-indole-2-carboxylic acid (1.8 g, 10 mmol) and *N,N*-carbonyldiimidazole (1.9 g, 12 mmol) were dissolved in *N,N*-dimethylformamide (30 mL), cyclobutylmethanamine (1.02 g, 12 mmol) was added, and the reaction was stirred at room temperature overnight. Ethyl acetate (10 mL) was added to the reaction mixture with the solid precipitating, and the solid was filtered. Ethyl acetate (20 mL) and water (20 mL) were added to the filtrate, a white solid precipitated, filtered and dried to afford the title compound (1.5 g, white solid), yield: 60%. MS (ESI): m/z 254.2 [M+H]⁺.

### (3) 6-(Aminomethyl)-N-(cyclobutylmethyl)-1H-indole-2-carboxamide

6-Cyano-*N*-(cyclobutylmethyl)-1H-indole-2-carboxamide (800 mg, 3.2 mmol) was dissolved in 7 M ammonia in methanol (10 mL), Raney nickel (80 mg) was added, and the mixture was heated to 40°C under a hydrogen atmosphere and stirred for 3 hours. The reaction mixture was filtered, and the filtrate was concentrated to afford the title compound (700 mg, white solid). MS (ESI): m/z 241.2 [M+H-NH₃]⁺.

### (4) 1-(6-(Aminomethyl)-1H-indol-2-yl)-N-(cyclobutylmethyl)methane-d₂-amine

6-(Aminomethyl)-*N*-(cyclobutylmethyl)-1H-indole-2-carboxamide (100 mg, 3.2 mmol) was dissolved in 1,4-dioxane (10 mL), lithium aluminum deuteride (50 mg, 1.2 mmol) was added, and the mixture was heated to 110°C and stirred overnight. The reaction mixture was quenched with methanol, concentrated, and the residue was purified by silica gel column chromatography (dichloromethane/methanol = 10/1) to afford the title compound (30 mg, yellow oil), yield: 32%. MS (ESI): m/z 229.2 [M+H-NH₃]⁺.

### (5) N-((2-(((Cyclobutylmethyl)amino)methyl-d₂)-1H-indol-6-yl)methyl)-8-oxo-8H-[1,3]dioxolo[4',5':4,5]pyrido[1,2-a]pyrimidine-6-carboxamide

1-(6-(Aminomethyl)-1H-indol-2-yl)-*N*-(cyclobutylmethyl)methane-*d*₂-amine (30 mg, 0.12 mmol) and Intermediate 5 (14 mg, 0.06 mmol) were dissolved in *N,N*-dimethylformamide (5 mL), followed by *N,N*-diisopropylethylamine (30 mg, 0.24 mmol) and HATU (27 mg, 0.07 mmol), and the reaction was stirred at room temperature overnight. Water (20 mL) was added to the reaction mixture, and the mixture was extracted with ethyl acetate (20 mL x 3). The combined organic phases were washed with saturated sodium chloride solution (20 mL x 3), dried over anhydrous sodium sulfate, filtered, and concentrated. The residue was purified by silica gel column chromatography (dichloromethane/methanol = 10/1) to obtain a crude product, which was further purified by Prep-HPLC to afford the title compound (3.3 mg, white solid), yield: 12%. MS (ESI): m/z 462.1 [M+H]⁺. ¹H NMR (400 MHz, DMSO-*d*₆) δ 10.87 (s, 1H), 9.04 (t, *J =* 6.4 Hz, 1H), 8.59 (s, 1H), 7.37 (d, *J =* 8.0 Hz, 1H), 7.29 (s, 1H), 7.14 (s, 1H), 6.94 (d, *J =* 8.0 Hz, 1H), 6.73 (s, 1H), 6.37 (s, 2H), 6.20 (s, 1H), 4.54(d, *J =* 6.4 Hz, 2H), 2.54-2.52 (m, 2H), 2.42-2.35 (m, 1H), 2.01-1.93 (m, 2H), 1.83-1.75 (m, 2H), 1.65-1.58 (m, 2H).

### Example 63

### N-((2-(((Bicyclo[3.1.0]hexan-1-ylmethyl)amino)methyl)-1H-indol-6-yl)methyl)-9-oxo-2,3-dihydro-9H-[1,4]dioxino[2',3':4,5]pyrido[1,2-a]pyrimidine-7-carboxamide

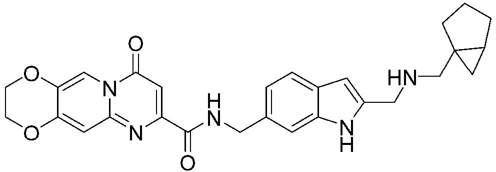

Following the synthetic method of **Example 58,** using bicyclo[3.1.0]hexane-1-carboxylic acid as the starting material in the first step and replacing Intermediate 7 with Intermediate 8 in the third step, the title compound (5.5 mg, white solid) was obtained through similar procedures. MS (ESI): m/z 500.1 [M+H]⁺. ¹H NMR (400 MHz, DMSO-*d*₆) δ 10.89 (s, 1H), 9.06 (t, *J =* 4.4 Hz, 1H), 8.62 (s, 1H), 7.37 (d, *J =* 7.6 Hz, 1H), 7.30 (s, 1H), 7.19 (s, 1H), 6.95 (d, *J =* 8.0 Hz, 1H), 6.66 (s, 1H), 6.24 (s, 1H), 4.58-4.52 (m, 4H), 4.47-4.44 (m, 2H), 3.85 (s, 2H), 2.75-2.64 (m, 2H), 1.71-1.57 (m, 5H), 1.23-1.17 (m, 1H), 1.07-0.98 (m, 1H), 0.36-0.27 (m, 2H).

### Examples 64~Example 70

The preparation of **Examples 64~Example 70** refers to the method of **Example 62,** using corresponding raw materials and intermediates, and synthesized through similar steps.

| Example | Compound Structure | Raw Materials and Intermediates | MS (ESI)Value m/z [M+H]⁺ |
|---|---|---|---|
| **64** | 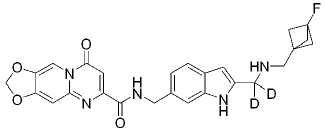 | (3-Fluorobicyclo[ 1.1.1]pentan-1-yl)methanamine hydrochloride and Intermediate 5 | 492.1 |
| **65** | 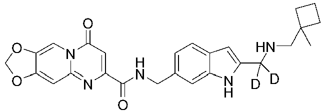 | (1-Methylcyclobutyl)methanamine hydrochloride and Intermediate 5 | 476.1 |
| **66** | 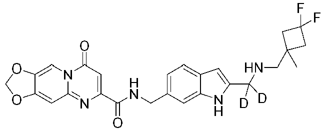 | (3,3-Difluoro-1-methylcyclobut yl)methanamine hydrochloride and Intermediate 5 | 512.1 |
| **67** | 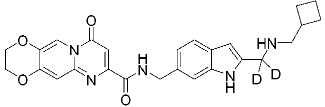 | Cyclobutylmet hanamine and Intermediate 4 | 476.1 |
| **68** | 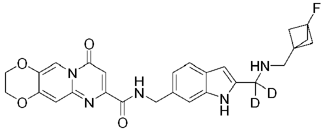 | (3-Fluorobicyclo[ 1.1.1]pentan-1-yl)methanamine hydrochloride and Intermediate 4 | 506.1 |
| **69** | 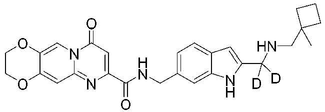 | (1-Methylcyclobut yl)methanamine hydrochloride and Intermediate 4 | 490.1 |
| **70** | 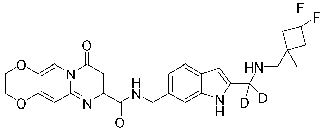 | (3,3-Difluoro-1-methylcyclobut yl)methanamine hydrochloride and Intermediate 4 | 526.1 |

### Example 71

### N-((2-(((Cyclobutylmethyl-d₂)amino)methyl)-1H-indol-6-yl)methyl)-8-oxo-8H-[1,3]dioxolo[4',5':4,5]pyrido[1,2-a]pyrimidine-6-carboxamide

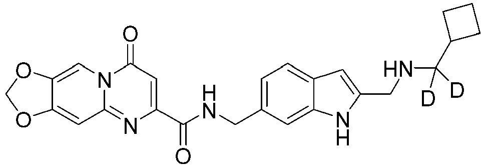

### (1) Cyclobutylmethane-d₂-amine hydrochloride

Cyclobutanamide (50 mg, 0.5 mmol) was dissolved in tetrahydrofuran (5 mL), lithium aluminum deuteride (63 mg, 1.5 mmol) was added, and the mixture was heated to 70°C and stirred for 2 hours. The reaction solution was cooled to room temperature, diluted with ethyl acetate (5 mL), then two drops of water were added. After thorough stirring, 5 g of anhydrous sodium sulfate was added, and the mixture was stirred for 5 minutes and filtered. 4M hydrochloric acid/dioxane solution (1 mL) was added to the filtrate, and concentrated to obtain the title compound (50 mg, white solid), yield: 80%. MS (ESI): m/z 88.2 [M+H]⁺.

### (2) N-((2-(((Cyclobutylmethyl-d₂)amino)methyl)-1H-indol-6-yl)methyl)-8-oxo-8H-[1,3]dioxolo[4',5':4,5]pyrido[1,2-a]pyrimidine-6-carboxamide

Cyclobutylmethane-*d*₂-amine hydrochloride (35 mg, 0.28 mmol) was dissolved in methanol (2 mL), Intermediate 7 (11 mg, 0.028 mmol), glacial acetic acid (0.2 mL) and triethylamine (0.2 mL) were added. The reaction solution was heated to 50°C and stirred for 10 minutes, then sodium cyanoborohydride (10 mg, 0.15 mmol) was added, stirring was continued at 50°C overnight. The reaction solution was filtered, concentrated, and the residue was purified by Prep-HPLC to obtain the title compound (8.5 mg, white solid), yield: 65%. MS (ESI): m/z 462.1 [M+H]⁺. ¹H NMR (400 MHz, DMSO-*d*₆) δ 10.86(s, 1H), 9.03 (t, *J* = 6.0 Hz, 1H), 8.59 (s, 1H), 7.37 (d, *J* = 8.0 Hz, 1H), 7.29 (s, 1H), 7.14 (s, 1H), 6.94 (d, *J* = 8.0Hz, 1H), 6.73 (s, 1H), 6.38 (s, 2H), 6.20 (s, 1H), 4.54 (d, *J* = 6.0 Hz, 2H), 3.76 (s, 2H), 2.42-2.36 (m, 1H), 2.00-1.92 (m, 2H), 1.85-1.75 (m, 2H), 1.65-1.59 (m, 2H).

### Example 72

### N-((2-((((3-Fluorobicyclo[1.1.1]pentan-1-yl)methyl-d₂)amino)methyl)-1H-indol-6-yl)methyl)-8-oxo-8H-[1,3]dioxolo[4',5':4,5]pyrido[1,2-a]pyrimidine-6-carboxamide

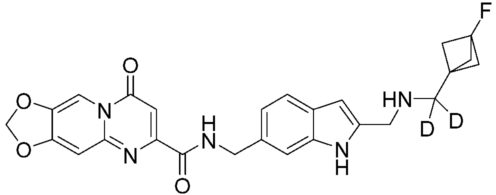

Following the synthetic method of **Example 71,** using 3-fluorobicyclo[1.1.1]pentane-1-carboxamide as the starting material in the first step, the title compound (4.3 mg, white solid) was obtained through similar procedures. MS (ESI): m/z 492.1 [M+H]⁺. ¹H NMR (400 MHz, DMSO-*d*₆) δ 10.91 (s, 1H), 9.06 (t, *J* = 6.0 Hz, 1H), 8.60 (s, 1H), 7.40 (d, *J* = 7.6 Hz, 1H), 7.31 (s, 1H), 7.14 (s, 1H), 6.97 (d, *J =* 7.6 Hz, 1H), 6.73 (s, 1H), 6.38 (s, 2H), 6.26 (s, 1H), 4.55 (d, *J* = 6.0 Hz, 2H), 3.89 (s, 2H), 1.95 (d, *J* = 2.8 Hz, 6H).

### Example 73

### N-((2-(((Cyclobutylmethyl)amino)methyl)-5-fluoro-1H-indol-6-yl)methyl)-8-oxo-8H-[1,3]dioxolo[4',5':4,5]pyrido[1,2-a]pyrimidine-6-carboxamide

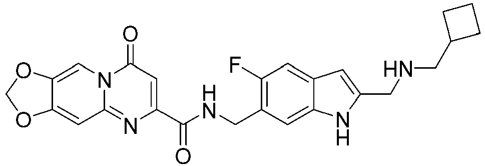

### (1) 5-Amino-4-(3,3-diethoxyprop-1-yn-1-yl)-2-fluorobenzonitrile

5-Amino-4-bromo-2-fluorobenzonitrile (2.0 g, 9.30 mmol) and 3,3-diethoxyprop-1-yne (2.98 g, 23.25 mmol) were dissolved in triethylamine (25 mL), and bis(triphenylphosphine)palladium dichloride (330 mg, 0.47 mmol), triphenylphosphine (240 mg, 0.93 mmol) and cuprous iodide (180 mg, 0.93 mmol) were added sequentially. The mixture was heated to 80°C under nitrogen protection and stirred for 16 hours. The reaction solution was concentrated, and the residue was purified by silica gel column chromatography (petroleum ether/ethyl acetate = 90/10) to obtain the title compound (2.0 g, brown oil), yield: 82%. MS (ESI): m/z 217.0 [M+H-EtOH]⁺.

### (2) 2-(Diethoxymethyl)-5-fluoro-1H-indole-6-carbonitrile

5-Amino-4-(3,3-diethoxyprop-1-yn-1-yl)-2-fluorobenzonitrile (2.0 g, 7.6 mmol) was dissolved in tetrahydrofuran (20 mL), and a 1M solution of lithium bis(trimethylsilyl)amide in tetrahydrofuran (15.2 mL, 15.2 mmol) was slowly added dropwise at 25°C. After the dropwise addition, the mixture was heated to 75°C and stirred for 4 hours. The reaction solution was added with water (100 mL) and extracted with ethyl acetate (50 mL x 3). The combined organic phases were washed with saturated sodium chloride solution (100 mL), dried over anhydrous sodium sulfate, filtered and concentrated. The residue was purified by silica gel column chromatography (petroleum ether/ethyl acetate = 90/10) to obtain the title compound (700 mg, yellow solid), yield: 36%. MS (ESI): m/z 263.0 [M+H]⁺. ¹H NMR (300 MHz, DMSO-*d*₆) δ 11.82 (s, 1H), 7.83 (d, *J =* 5.4 Hz, 1H), 7.60 (d, *J* = 10.5 Hz, 1H), 6.58 (d, *J =* 0.9 Hz, 1H), 5.79 (s, 1H), 3.65-3.58 (m, 4H), 1.21 (t, *J* = 7.2 Hz, 6H).

### (3) (2-(Diethoxymethyl)-5-fluoro-1H-indol-6-yl)methanamine

2-(Diethoxymethyl)-5-fluoro-1H-indole-6-carbonitrile (300 mg, 1.14 mmol) was dissolved in 7M ammonia in methanol (20 mL), 10% Raney nickel (30 mg) was added, hydrogen was replaced 3 times, and the mixture was heated to 50°C and stirred for 16 hours. The reaction solution was filtered to remove Raney nickel, and the filtrate was concentrated to obtain the title compound (290 mg, green oil), yield: 95%. MS (ESI): m/z 175.9 [M+H-91]⁺.

### (4) N-((2-(Diethoxymethyl)-5-fluoro-1H-indol-6-yl)methyl)-8-oxo-8H-[1,3]dioxolo[4',5':4,5]pyrido[1,2-a]pyrimidine-6-carboxamide

8-Oxo-8H-[1,3]dioxolo[4',5':4,5]pyrido[1,2-a]pyrimidine-6-carboxylic acid (40 mg, 0.17 mmol) and (2-(diethoxymethyl)-5-fluoro-1H-indol-6-yl)methanamine (68 mg, 0.26 mmol) were dissolved in *N,N-*dimethylformamide (5 mL), and a solution of 1-propylphosphonic anhydride in ethyl acetate (218 mg, 0.34 mmol, 50%) and *N,N*-diisopropylethylamine (110 mg, 0.85 mmol) were added, and the mixture was stirred at room temperature for 1 hour. The reaction solution was added to saturated sodium bicarbonate solution (30 mL) and extracted with ethyl acetate (20 mL x 3). The combined organic phases were washed with saturated sodium chloride solution (30 mL x 2), dried over anhydrous sodium sulfate, filtered and concentrated. The residue was purified by silica gel column chromatography (petroleum ether/ethyl acetate =30/70) to obtain the title compound (25 mg, pale yellow oil), yield: 61%. MS (ESI): m/z 483.0 [M+H]⁺.

### (5) N-((5-Fluoro-2-formyl-1H-indol-6-yl)methyl)-8-oxo-8H-[1,3]dioxolo[4',5':4,5]pyrido[1,2-a]pyrimidine-6-carboxamide

*N*-((2-(diethoxymethyl)-5-fluoro-1H-indol-6-yl)methyl)-8-oxo-8H-[1,3]dioxolo[4',5':4,5]pyrido[1,2-a]pyrimidine-6-carboxamide (50 mg, 0.10 mmol) and acetic acid (1 mL) were dissolved in tetrahydrofuran/water mixture (5 mL/0.5 mL), and the mixture was heated to 50°C and stirred for 3 hours. The reaction solution was concentrated, diluted with ethyl acetate, filtered and dried to obtain the title compound (32 mg, white solid), yield: 76%. MS (ESI): m/z 408.8 [M+H]⁺.

### (6) N-((2-(((Cyclobutylmethyl)amino)methyl)-5-fluoro-1H-indol-6-yl)methyl)-8-oxo-8H-[1,3]dioxolo[4',5':4,5]pyrido[1,2-a]pyrimidine-6-carboxamide

*N*-((5-fluoro-2-formyl-1H-indol-6-yl)methyl)-8-oxo-8H-[1,3]dioxolo[4',5':4,5]pyrido[1,2-a]pyrimidine-6-carboxamide (16 mg, 0.04 mmol) and cyclobutylmethanamine (10 mg, 0.12 mmol) were dissolved in a mixed solvent of methanol/acetic acid/ N,N-dimethylformamide (3 mL/0.3 mL/1 mL), and the mixture was heated to 50°C and stirred for 1 hour. Then sodium cyanoborohydride (12 mg, 0.20 mmol) was added, stirring was continued at 50°C for 48 hours. The reaction solution was concentrated, and the residue was purified by Prep-HPLC to obtain the title compound (6.5 mg, white solid), yield: 35%. MS (ESI): m/z 478.1 [M+H]⁺. ¹H NMR (400 MHz, DMSO-*d*₆) δ 10.96 (s, 1H), 8.99 (t, *J =* 6.0 Hz, 1H), 8.60 (s, 1H), 7.29 (d, *J* = 6.4 Hz, 1H), 7.20-7.17 (m, 2H), 6.73 (s, 1H), 6.38 (s, 2H), 6.22 (s, 1H), 4.59 (d, *J* = 6.0 Hz, 2H), 3.77 (s, 2H), 2.55-2.50 (m, 2H), 2.43-2.33 (m, 1H), 1.98-1.92 (m, 2H), 1.82-1.76 (m, 2H) , 1.66-1.59 (m, 2H).

### Example 74

### N-((5-fluoro-2-((((3-fluorobicyclo[1.1.1]pentan-1-yl)methyl)amino)methyl)-1H-indol-6-yl)methyl)-8-oxo-8H-[1,3]dioxolo[4',5':4,5]pyrido[1,2-a]pyrimidine-6-carboxamide

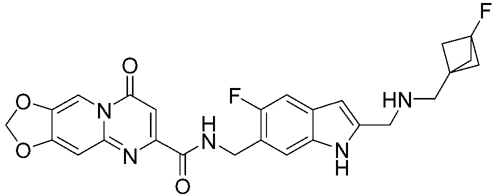

Following the synthetic method of Step 6 in **Example 73,** replacing cyclobutylmethanamine with (3-fluorobicyclo[1.1.1]pentan-1-yl)methanamine hydrochloride, the title compound (5.0 mg, white solid) was obtained through similar procedures. MS (ESI): m/z 508.1 [M+H]⁺. ¹H NMR (400 MHz, DMSO-*d*₆) δ 10.95 (s, 1H), 9.00 (t, *J =* 6.0 Hz, 1H), 8.60 (s, 1H), 7.28 (d, *J* = 6.8 Hz, 1H), 7.21-7.17 (m, 2H), 6.73 (s, 1H), 6.38 (s, 2H), 6.22 (s, 1H), 4.59 (d, *J =* 5.6 Hz, 2H), 3.79 (s, 2H), 2.74 (s, 2H), 1.92 (s, 6H).

### Example 75

### N-((2-(((Cyclobutylmethyl)amino)methyl)-5,7-difluoro-1H-indol-6-yl)methyl)-8-oxo-8H-[1,3]dioxolo[4',5':4,5]pyrido[1,2-a]pyrimidine-6-carboxamide

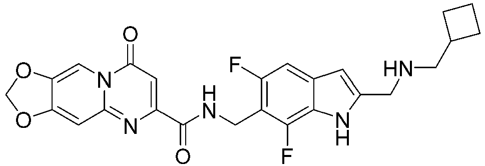

Following the synthetic method of **Example 73,** using 3-amino-4-bromo-2,6-difluorobenzonitrile as the starting material in the first step, the title compound (8.2 mg, white solid) was obtained through similar procedures. MS (ESI): m/z 496.1 [M+H]⁺. ¹H NMR (400 MHz, DMSO-*d*₆) δ 11.49 (s, 1H), 8.74 (t, *J* = 5.2 Hz, 1H), 8.58 (s, 1H), 7.17 (s, 1H), 7.13 (d, *J* = 10.8 Hz, 1H), 6.69 (s, 1H), 6.37-6.35 (m, 3H), 4.66 (d, *J* = 5.6 Hz, 2H), 3.82 (s, 2H), 2.54-2.51 (m, 2H), 2.42-2.40 (m, 1H), 2.01-1.97 (m, 2H), 1.81-1.79 (m, 2H) , 1.65-1.60 (m, 2H).

The synthetic method of 3-amino-4-bromo-2,6-difluorobenzonitrile is as follows: 3-amino-2,6-difluorobenzonitrile (1 g, 6.5 mmol) was dissolved in dichloromethane (10 mL), *N-*bromosuccinimide (1.39 mg, 7.8 mmol) was added, and the mixture was stirred at room temperature for 16 hours. The reaction solution was concentrated, and the residue was purified by silica gel column chromatography (petroleum ether/ethyl acetate = 97/3) to obtain 3-amino-4-bromo-2,6-difluorobenzonitrile (1 g, white solid), yield: 66%. MS (ESI): m/z 233.0 [M+H]⁺. ¹H NMR (400 MHz, DMSO-*d*₆) δ 7.66 (dd, *J =* 8.8, 2.0 Hz, 1H), 5.79 (brs, 2H).

### Example 76

### N-((2-((((5-fluorothiophen-2-yl)methyl)amino)methyl)-1H-indol-6-yl)methyl)-9-oxo-2,3-dihydro-9H-[1,4]dioxino[2',3':4,5]pyrido[1,2-a]pyrimidin-7-carboxamide

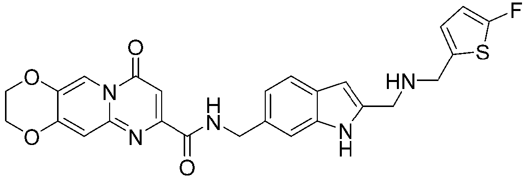

Intermediate 8 (15 mg, 0.04 mmol) and (5-fluorothiophen-2-yl)methanamine (7.30 mg, 0.06 mmol) were dissolved in methanol (5 mL) and stirred at 50 °Cfor 1 hour. Then sodium cyanoborohydride (6.99 mg, 0.11 mmol) was added, and the reaction was continued to stir at 50 °C for 12 hours. The reaction solution was concentrated, the residue was diluted with anhydrous methanol, and purified by Prep-HPLC to obtain the title compound (7.7 mg, white solid), yield: 40%. MS (ESI): m/z 520.0 [M+H]⁺. ¹H NMR (400 MHz, DMSO-*d*₆) δ 10.88 (s, 1H), 9.06 (t, *J =* 6.4 Hz, 1H), 8.62 (s, 1H), 7.38 (d, *J* = 8.0 Hz, 1H), 7.30 (s, 1H), 7.19 (s, 1H), 6.95 (d, *J =* 7.6 Hz, 1H), 6.67 (s, 1H), 6.61 (t, *J* = 3.6 Hz, 1H), 6.51-6.49 (m, 1H), 6.24 (s, 1H), 4.56-4.53 (m, 4H), 4.47-4.45 (m, 2H), 3.80 (s, 2H), 3.74-3.73 (m, 2H).

### Example 77

### N-((2-((((2-oxabicyclo[2.1.1]hexan-1-yl)methyl)amino)methyl)-1H-indol-6-yl)methyl)-9-oxo-2,3-dihydro-9H-[1,4]dioxino[2',3':4,5]pyrido[1,2-a]pyrimidin-7-carboxamide

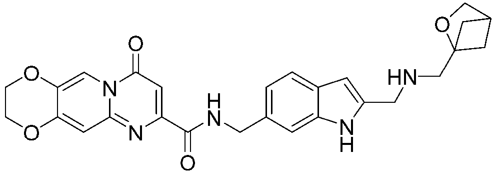

Intermediate 8 (15 mg, 0.04 mmol) and (2-oxabicyclo[2.1.1]hexan-1-yl)methanamine hydrochloride (8.94 mg, 0.06 mmol) were dissolved in methanol (5 mL) and stirred at 50 °Cfor 1 hour. Then sodium cyanoborohydride (6.99 mg, 0.11 mmol) was added, and the reaction was continued to stir at 50 °C for 12 hours. The reaction solution was concentrated, diluted with anhydrous methanol, and purified by Prep-HPLC to obtain the title compound (5.0 mg, white solid), yield: 27%. MS (ESI): m/z 502.1 [M+H]⁺. ¹H NMR (400 MHz, DMSO-*d*₆) δ 10.89 (s, 1H), 9.04 (t, *J =* 6.4 Hz, 1H), 8.62 (s, 1H), 7.37 (d, *J =* 8.0 Hz, 1H), 7.29 (s, 1H), 7.19 (s, 1H), 6.94 (d, *J =* 8.0 Hz, 1H), 6.67 (s, 1H), 6.21 (s, 1H), 4.57-4.53 (m, 4H), 4.47-4.45 (m, 2H), 3.83 (s, 2H), 3.61 (s, 2H), 2.84-2.83 (m, 1H), 2.77 (s, 2H), 1.76-1.69 (m, 2H), 1.32-1.30 (m, 2H).

### Example 78

### (R)-9-Oxo-N-((2-((((tetrahydrofuran-2-yl)methyl)amino)methyl)-1H-indol-6-yl)methyl)-2,3-dihydro-9H-[1,4]dioxino[2',3':4,5]pyrido[1,2-a]pyrimidine-7-carboxamide

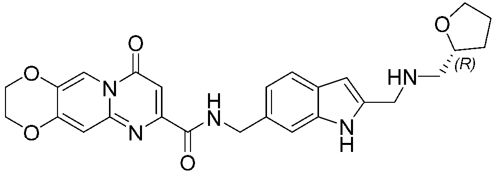

Following the synthetic method of **Example 76,** using Intermediate 8 and (R)-(tetrahydrofuran-2-yl)methanamine as starting materials, the title compound (9.0 mg, white solid) was obtained through similar procedures. MS (ESI): m/z 490.1 [M+H]⁺. ¹H NMR (400 MHz, DMSO-*d*₆) δ 10.91 (s, 1H), 9.06 (t, *J =* 6.4 Hz, 1H), 8.62 (s, 1H), 7.38 (d, *J =* 8.0 Hz, 1H), 7.30 (s, 1H), 7.19 (s, 1H), 6.95 (d, *J* = 9.2 Hz, 1H), 6.66 (s, 1H), 6.24 (s, 1H), 4.59-4.53 (m, 4H), 4.47-4.44 (m, 2H), 3.93-3.87 (m, 3H), 3.74-3.69 (m, 1H), 3.62-3.57 (m, 1H), 2.58-2.57 (m, 2H), 1.92-1.84 (m, 1H), 1.81-1.73 (m, 2H), 1.54-1.45 (m, 1H).

### Example 79

### (S)-N-((2-(((oxetan-2-ylmethyl)amino)methyl)-1H-indol-6-yl)methyl)-9-oxo-2,3-dihydro-9H-[1,4]dioxolo[2',3':4,5]pyrido[1,2-a]pyrimidine-7-carboxamide

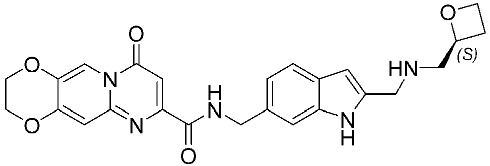

Following the synthetic method of **Example 76,** using Intermediate 8 and (S)-oxetan-2-ylmethanamine as starting materials, the title compound (9.6 mg, white solid) was obtained through similar procedures. MS (ESI): m/z 476.0 [M+H]⁺. ¹H NMR (400 MHz, DMSO-*d*₆) δ 10.90 (s, 1H), 9.05 (t, *J =* 6.0 Hz, 1H), 8.62 (s, 1H), 7.37 (d, *J =* 7.6 Hz, 1H), 7.29 (s, 1H), 7.19 (s, 1H), 6.95 (dd, *J* = 8.8 Hz, 1.6 Hz, 1H), 6.67 (s, 1H), 6.22 (s, 1H), 4.80-4.73 (m, 1H), 4.57-4.53 (m, 4H), 4.49-4.45 (m, 3H), 4.40-4.33 (m, 1H), 3.83 (s, 2H), 2.77-2.66 (m, 2H), 2.43-2.32 (m, 2H).

### Example 80

### N-((2-((((1-Methoxycyclobutyl)methyl)amino)methyl)-1H-indol-6-yl)methyl)-9-oxo-2,3-dihydro-9H-[1,4]dioxino[2',3':4,5]pyrido[1,2-a]pyrimidine-7-carboxamide

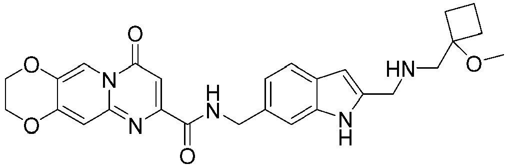

### (1) Methyl 1-methoxycyclobutane-1-carboxylate

1-Hydroxycyclobutane-1-carboxylic acid (800 mg, 6.89 mmol) was dissolved in *N,N-*dimethylformamide (10 mL), sodium hydride (1.10 g, 27.56 mmol, 60%) was added at 0°C, and the mixture was stirred at room temperature for 2 hours. Iodomethane (2.94 g, 20.67 mmol) was then added, and the mixture was stirred at room temperature for 16 hours. After the reaction was completed, saturated ammonium chloride solution (50 mL) was added for quenching, and the aqueous phase was extracted with ethyl acetate (30 mL x 3). The combined organic phases were washed with saturated sodium chloride solution (50 mL), dried over anhydrous sodium sulfate, and concentrated to obtain the title compound (500 mg, yellow oil), yield: 50%. ¹H NMR (400 MHz, DMSO-*d*₆) δ 3.70 (s, 3H), 3.09 (s, 3H), 2.37-2.30 (m, 2H), 2.16-2.08 (m, 2H), 1.82-1.71 (m, 2H).

### (2) 1-Methoxycyclobutane-1-carboxamide

Methyl 1-methoxycyclobutane-1-carboxylate (300 mg, 2.08 mmol) was dissolved in 7M ammonia in methanol solution (5 mL) and stirred at 60°C for 16 hours. After the reaction was completed, the reaction solution was concentrated to obtain the title compound (200 mg, white oil), yield: 74%. ¹H NMR (400 MHz, CDCl₃) δ 6.26 (s, 1H), 5.28 (s, 1H), 3.24 (s, 3H), 2.49-2.42 (m, 2H), 2.24-2.16 (m, 2H), 1.95-1.82 (m, 2H).

### (3) (1-Methoxycyclobutyl)methanamine

1-Methoxycyclobutane-1-carboxamide (50 mg, 0.39 mmol) and lithium aluminum hydride (44.04 mg, 1.16 mmol) were dissolved in tetrahydrofuran (3 mL) and stirred at 70°C for 1 hour. The reaction was detected as complete by LCMS, and the reaction solution was concentrated to obtain the title compound (44 mg, yellow oil), yield: 99%. MS (ESI): m/z 116.2 [M+H]⁺.

### (4) N-((2-((((1-Methoxycyclobutyl)methyl)amino)methyl)-1H-indol-6-yl)methyl)-9-oxo-2,3-dihydro-9H-[1,4]dioxino[2',3':4,5]pyrido[1,2-a]pyrimidine-7-carboxamide

Intermediate 8 (7.5 mg, 0.02 mmol) and (1-methoxycyclobutyl)methanamine (10.65 mg, 0.09 mmol) were dissolved in methanol/acetic acid (3 mL/0.3 mL) and stirred at 50°C for 1 hour. sodium cyanoborohydride (5.81 mg, 0.09 mmol) was then added, and the reaction was continued to stir at 50°C for 16 hours. The reaction solution was concentrated, and the residue was purified by Prep-HPLC to obtain the title compound (4 mg, white solid), yield: 43%. MS (ESI): m/z 504.3 [M+H]⁺. ¹H NMR (400 MHz, DMSO-*d*₆) δ 10.93 (s, 1H), 9.08 (t, *J* = 5.6 Hz, 1H), 8.62 (s, 1H), 7.37 (d, *J* = 8.4 Hz, 1H), 7.29 (s, 1H), 7.19 (s, 1H), 6.95 (d, *J* = 8.4 Hz, 1H), 6.67 (s, 1H), 6.23 (s, 1H), 4.56-4.53 (m, 4H), 4.46-4.42 (m, 2H), 3.84 (s, 2H), 2.99 (s, 3H), 2.62 (s, 2H), 2.00-1.92 (m, 2H), 1.87-1.82 (m, 2H) , 1.68-1.56 (m, 2H).

### Example 81

### N-((2-((5-oxa-8-azaspiro[3.5]nonan-8-yl)methyl)-1H-indol-6-yl)methyl)-9-oxo-2,3-dihydro-9H-[1,4]dioxino[2',3':4,5]pyrido[1,2-a]pyrimidine-7-carboxamide

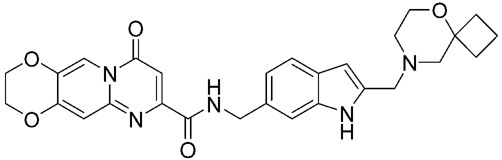

Following the synthetic method of **Example 76,** using Intermediate 8 and 5-oxa-8-azaspiro[3.5]nonane as starting materials, the title compound (6.0 mg, white solid) was obtained through similar procedures. MS (ESI): m/z 516.1 [M+H]⁺. ¹H NMR (400 MHz, DMSO-*d*₆) δ 10.92 (s, 1H), 9.07 (t, *J =* 7.8 Hz, 1H), 8.62 (s, 1H), 7.38 (d, *J* = 8.4 Hz, 1H), 7.32 (s, 1H), 7.19 (s, 1H), 6.96 (d, *J =* 8.8 Hz, 1H), 6.67 (s, 1H), 6.24 (s, 1H), 4.59-4.53 (m, 4H), 4.47-4.46 (m, 2H), 3.59 (s, 2H), 3.51-3.48 (m, 2H), 2.36-2.30 (m, 4H), 2.00-1.82 (m, 4H), 1.75-1.64 (m, 1H), 1.52-1.41 (m, 1H).

### Example 82

### N-((2-((((5-Fluorothiophen-2-yl)methyl)amino)methyl)-1H-indol-6-yl)methyl)-8-oxo-8H-[1,3]dioxolo[2',3':4,5]pyrido[1,2-a]pyrimidine-6-carboxamide

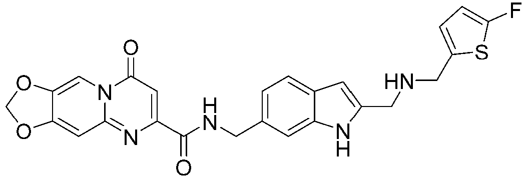

Intermediate 7 (15 mg, 0.04 mmol), (5-fluorothiophen-2-yl)methanamine hydrochloride (6.7 mg, 0.04 mmol) and acetic acid (0.1 mL) were dissolved in methanol/*N*,*N*-dimethylformamide (5 mL/1 mL) and stirred at room temperature for 0.5 hours. Sodium cyanoborohydride (5 mg, 0.08 mmol) was then added, and the reaction was stirred at 50°C overnight. The reaction solution was concentrated, and the residue was purified by Prep-HPLC to obtain the title compound (5.3 mg, yellow solid), yield: 28%. MS (ESI): m/z 506.0 [M+H]⁺. ¹H NMR (400 MHz, DMSO-*d*₆) δ 10.89 (s, 1H), 9.06 (t, *J =* 6.0 Hz, 1H), 8.59 (s, 1H), 7.39 (d, *J =* 8.0 Hz, 1H), 7.30 (s, 1H), 7.14 (s, 1H), 6.96 (dd, *J =* 8.0, 1.2 Hz, 1H), 6.73 (s, 1H), 6.61 (t, *J =* 3.6 Hz, 1H), 6.52-6.50 (m, 1H), 6.38 (s, 2H), 6.24 (s, 1H), 4.55 (d, *J =* 6.4 Hz, 2H), 3.81 (s, 2H), 3.74 (d, *J =* 2.4 Hz, 2H).

### Example 83

### N-((2-((((1-Methoxycyclobutyl)methyl)amino)methyl)-1H-indol-6-yl)methyl)-8-oxo-8H-[1,3]dioxolo[2',3':4,5]pyrido[1,2-a]pyrimidine-6-carboxamide

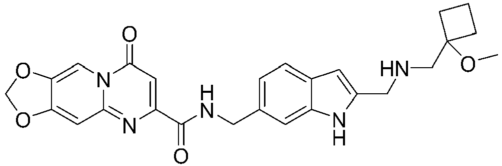

Following the synthetic method of **Example 80,** using Intermediate 7 and (1-methoxycyclobutyl)methanamine as raw materials in Step 4, the title compound (8.3 mg, white solid) was obtained through similar procedures. MS (ESI): m/z 490.1 [M+H]⁺. ¹H NMR (400 MHz, DMSO-*d*₆) δ 10.91 (s, 1H), 9.04 (t, *J =* 6.0 Hz, 1H), 8.59 (s, 1H), 7.38 (d, *J* = 8.0 Hz, 1H), 7.29 (s, 1H), 7.14 (s, 1H), 6.95 (dd, *J* = 8.0, 1.6 Hz, 1H), 6.73 (s, 1H), 6.38 (s, 2H), 6.23 (s, 1H), 4.54 (d, *J* = 6.4 Hz, 2H), 3.84 (s, 2H), 2.99 (s, 3H), 2.63 (s, 2H), 2.00-1.92 (m, 2H), 1.88-1.82 (m, 2H), 1.67-1.59 (m, 1H), 1.49-1.41 (m, 1H).

### Example 84

### N-((2-((((2-Oxabicyclo[2.1.1]hexan-1-yl)methyl)amino)methyl)-1H-indol-6-yl)methyl)-8-oxo-8H-[1,3]dioxolo[2',3':4,5]pyrido[1,2-a]pyrimidine-6-carboxamide

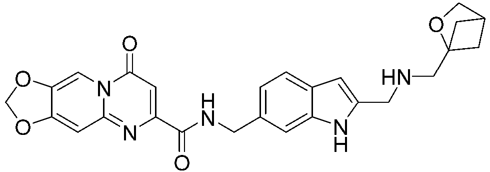

Intermediate 7 (15 mg, 0.04 mmol), (2-oxabicyclo[2.1.1]hexan-1-yl)methanamine hydrochloride (6.0 mg, 0.04 mmol) and acetic acid (0.1 mL) were dissolved in methanol/*N,N*-dimethylformamide (5 mL/1 mL) and stirred at room temperature for 0.5 hours. sodium cyanoborohydride (5 mg, 0.08 mmol) was then added, and the reaction was stirred at 50°C overnight. The reaction solution was concentrated, and the residue was purified by Prep-HPLC to obtain the title compound (4.3 mg, white solid), yield: 23%. MS (ESI): m/z 488.1 [M+H]⁺. ¹H NMR (400 MHz, DMSO-*d*₆) δ 10.91 (s, 1H), 9.05 (t, *J =* 6.4 Hz, 1H), 8.59 (s, 1H), 7.38 (d, *J =* 8.0 Hz, 1H), 7.29 (s, 1H), 7.15 (s, 1H), 6.95 (dd, *J =* 8.0, 1.2 Hz, 1H), 6.73 (s, 1H), 6.38 (s, 2H), 6.22 (s, 1H), 4.54 (d, *J =* 6.0 Hz, 2H), 3.83 (s, 2H), 3.61 (s, 2H), 2.83 (t, *J =* 2.8 Hz, 1H), 2.77 (s, 2H), 1.72-1.70 (m, 2H), 1.33-1.30 (m, 2H).

### Example 85

### N-((2-(((Cyclobutylmethyl)amino)methyl)-5-fluoro-1H-indol-6-yl)methyl)-9-oxo-2,3-dihydro-9H-[1,4]dioxino[2',3':4,5]pyrido[1,2-a]pyrimidine-7-carboxamide

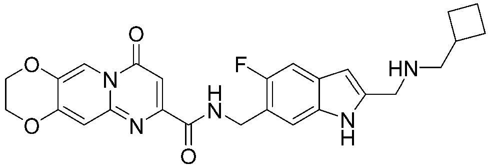

### (1) N-((2-(Diethoxymethyl)-5-fluoro-1H-indol-6-yl)methyl)-9-oxo-2,3-dihydro-9H-[1,4]dioxino[2',3':4,5]pyrido[1,2-a]pyrimidine-7-carboxamide

Following the synthetic method of Step 4 in **Example 73,** using Intermediate 4 (50 mg, 0.2 mmol) and (2-(diethoxymethyl)-5-fluoro-1H-indol-6-yl)methanamine (80 mg, 0.3 mmol) as raw materials, the title compound (60 mg, yellow oil) was obtained through similar procedures, yield: 60%. MS (ESI): m/z 519.0 [M+Na]⁺.

### (2) N-((5-Fluoro-2-formyl-1H-indol-6-yl)methyl)-9-oxo-2,3-dihydro-9H-[1,4]dioxino[2',3':4,5]pyrido[1,2-a]pyrimidine-7-carboxamide

Following the synthetic method of Step 5 in **Example 73,** using *N*-((2-(diethoxymethyl)-5-fluoro-1H-indol-6-yl)methyl)-9-oxo-2,3-dihydro-9H-[1,4]dioxino[2',3':4,5]pyrido[1,2-a]pyrimidine-7-carboxamide (60 mg, 0.12 mmol) as the raw material, the title compound (50 mg, white solid) was obtained through similar procedures, yield: 98%. MS (ESI): m/z 422.8 [M+H]⁺.

### (3)N-((2-(((Cyclobutylmethyl)amino)methyl)-5-fluoro-1H-indol-6-yl)methyl)-9-oxo-2,3-dihydro-9H-[1,4]dioxino[2',3':4,5]pyrido[1,2-a]pyrimidine-7-carboxamide

N-((5-Fluoro-2-formyl-1H-indol-6-yl)methyl)-9-oxo-2,3-dihydro-9H-[1,4]dioxino[2',3':4,5]pyrido[1,2-a]pyrimidine-7-carboxamide (25 mg, 0.06 mmol) and cyclobutylmethanamine (15 mg, 0.18 mmol) were dissolved in methanol/acetic acid/*N,N-*dimethylformamide (3 mL/0.3 mL/1.5 mL) and stirred at 50°C for 1 hour. sodium cyanoborohydride (19 mg, 0.30 mmol) was then added, and the reaction was continued to stir at 50°C for 16 hours. The reaction solution was concentrated, and the residue was purified by Prep-HPLC to obtain the title compound (13.3 mg, white solid), yield: 46%. MS (ESI): m/z 492.1 [M+H]⁺. ¹H NMR (400 MHz, DMSO-*d*₆) δ 10.95 (s, 1H), 9.01 (t, *J =* 6.4 Hz, 1H), 8.62 (s, 1H), 7.28 (d, *J =* 6.8 Hz, 1H), 7.22 (s, 1H), 7.18 (d, *J =* 11.2 Hz, 1H), 6.66 (s, 1H), 6.21 (s, 1H), 4.59-4.56 (m, 4H), 4.48-4.46 (m, 2H), 3.76 (s, 2H), 2.49-2.46 (m, 2H), 2.40-2.34 (m, 1H), 2.00-1.92 (m, 2H), 1.82-1.75 (m, 2H), 1.65-1.58 (m, 2H).

### Example 86

### N-((2-((((2-Oxabicyclo[2.1.1]hexan-1-yl)methyl)amino)methyl)-5-fluoro-1H-indol-6-yl)methyl)-9-oxo-2,3-dihydro-9H-[1,4]dioxino[2',3':4,5]pyrido[1,2-a]pyrimidine-7-carboxamide

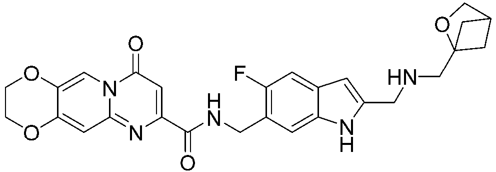

Following the synthetic method of Step 3 in **Example 85,** using N-((5-fluoro-2-formyl-1H-indol-6-yl)methyl)-9-oxo-2,3-dihydro-9H-[1,4]dioxino[2',3':4,5]pyrido[1,2-a]pyrimidine-7-carboxamide and 2-oxabicyclo[2.1.1]hexan-1-ylmethanamine hydrochloride as raw materials, the title compound (8.6 mg, white solid) was obtained through similar procedures. MS (ESI): m/z 520.0 [M+H]⁺. ¹H NMR (400 MHz, DMSO-*d*₆) δ 10.94 (s, 1H), 8.94 (t, *J =* 6.4 Hz, 1H), 8.52 (s, 1H), 7.20 (d, *J =* 6.4 Hz, 1H), 7.12-7.10 (m, 2H), 6.57 (s, 1H), 6.17 (s, 1H), 4.50-4.46 (m, 4H), 4.38-4.36 (m, 2H), 3.76 (s, 2H), 3.52 (s, 2H), 2.74 (t, *J =* 3.2 Hz, 1H), 2.70 (s, 2H), 1.64-1.59 (m, 2H), 1.23-1.22 (m, 2H).

### Example 87

### N-((2-((((2-Oxabicyclo[2.1.1]hexan-1-yl)methyl)amino)methyl)-5-fluoro-1H-indol-6-yl)methyl)-8-oxo-8H-[1,3]dioxolo[2',3':4,5]pyrido[1,2-a]pyrimidine-6-carboxamide

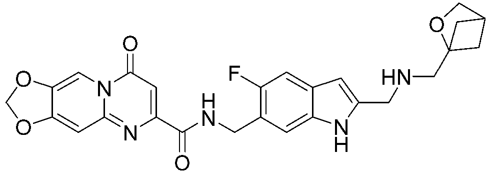

Following the synthetic method of Step 6 in **Example 73,** replacing cyclobutylmethanamine with 2-oxabicyclo[2.1.1]hexan-1-ylmethanamine hydrochloride, the title compound (11.8 mg, white solid) was obtained through similar procedures. MS (ESI): m/z 506.1 [M+H]⁺. ¹H NMR (400 MHz, DMSO-*d*₆) δ 10.98 (s, 1H), 8.99 (t, *J =* 6.0 Hz, 1H), 8.60 (s, 1H), 7.29 (d, *J =* 6.4 Hz, 1H), 7.20 (d, *J* = 11.2 Hz, 1H), 7.17 (s, 1H), 6.73 (s, 1H), 6.38 (s, 2H), 6.24 (s, 1H), 4.59 (d, *J =* 6.0 Hz, 2H), 3.83-3.82 (m, 2H), 3.61 (s, 2H), 2.83 (t, *J =* 3.2 Hz, 1H), 2.77 (s, 2H), 1.72-1.69 (m, 2H), 1.32-1.31 (m, 2H).

### Example 88

### N-((2-(((Cyclobutylmethyl-d₂)amino)methyl)-5-fluoro-1H-indol-6-yl)methyl)-8-oxo-8H-[1,3]dioxolo[2',3':4,5]pyrido[1,2-a]pyrimidine-6-carboxamide

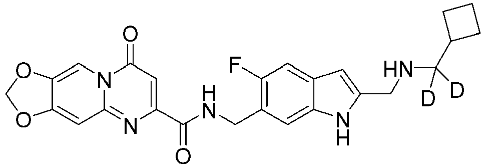

Following the synthetic method of Step 6 in **Example 73,** replacing cyclobutylmethanamine with cyclobutylmethane-*d*₂-amine hydrochloride (Step 1 of **Example 71**), the title compound (43.0 mg, white solid) was obtained through similar procedures. MS (ESI): m/z 480.1 [M+H]⁺. ¹H NMR (400 MHz, DMSO-*d*₆) δ 11.20 (s, 1H), 9.04 (t, *J =* 6.0 Hz, 1H), 8.60 (s, 1H), 7.31 (d, *J =* 5.6 Hz, 1H), 7.23 (d, *J* = 11.2 Hz, 1H), 7.17 (s, 1H), 6.73 (s, 1H), 6.38 (s, 2H), 6.31 (s, 1H), 4.59 (d, *J* = 6.0 Hz, 2H), 3.89 (s, 2H), 2.45-2.43 (m, 1H), 1.99-1.96 (m, 2H), 1.83-1.75 (m, 2H), 1.66-1.62 (m, 2H).

### Example 89

### N-((5-Fluoro-2-((((3-fluorobicyclo[1.1.1]pentan-1-yl)methyl-d₂)amino)methyl)-1H-indol-6-yl)methyl)-8-oxo-8H-[1,3]dioxolo[2',3':4,5]pyrido[1,2-a]pyrimidine-6-carboxamide

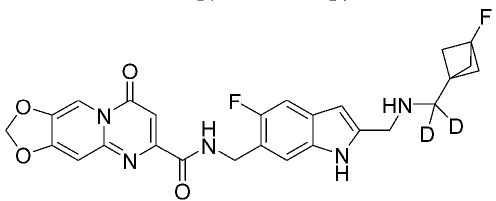

Following the synthetic method of Step 6 in **Example 73,** replacing cyclobutylmethanamine with (3-fluorobicyclo[1.1.1]pentan-1-yl)methane-*d*₂-amine **(Example 72),** the title compound (9.5 mg, white solid) was obtained through similar procedures. MS (ESI): m/z 510.0 [M+H]⁺. ¹H NMR (400 MHz, DMSO-*d*₆) δ 10.96 (s, 1H), 9.01 (t, *J* = 6.4 Hz, 1H), 8.60 (s, 1H), 7.28 (d, *J* = 6.4 Hz, 1H), 7.21-7.17 (m, 2H), 6.73 (s, 1H), 6.38 (s, 2H), 6.23 (s, 1H), 4.59 (d, *J =* 6.0 Hz, 2H), 3.79 (s, 2H), 1.92-1.91(m, 6H).

### Example 90

### N-((2-(((Cyclobutylmethyl-d₂)amino)methyl)-5-fluoro-1H-indol-6-yl)methyl)-9-oxo-2,3-dihydro-9H-[1,4]dioxino[2',3':4,5]pyrido[1,2-a]pyrimidine-7-carboxamide

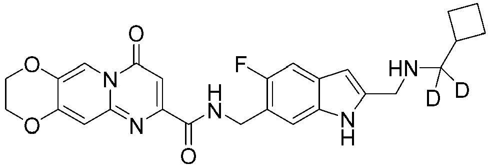

Following the synthetic method of Step 3 in **Example 85,** using N-((5-fluoro-2-formyl-1H-indol-6-yl)methyl)-9-oxo-2,3-dihydro-9H-[1,4]dioxino[2',3':4,5]pyrido[1,2-a]pyrimidine-7-carboxamide and cyclobutylmethane-*d*₂-amine hydrochloride (Step 1 of **Example 71**) as raw materials, the title compound (3.5 mg, white solid) was obtained through similar procedures. MS (ESI): m/z 494.1 [M+H]⁺. ¹H NMR (400 MHz, DMSO-*d*₆) δ 10.95 (s, 1H), 9.01 (t, *J* = 5.6 Hz, 1H), 8.63 (s, 1H), 7.28 (d, *J* = 6.4 Hz, 1H), 7.22-7.17 (m, 2H), 6.66 (s, 1H), 6.21 (s, 1H), 4.59-4.58 (m, 4H), 4.48-4.46 (m, 2H), 3.76 (s, 2H), 2.39-2.36 (m, 1H), 1.99-1.93 (m, 2H), 1.81-1.74 (m, 2H), 1.63-1.58 (m, 2H).

### Example 91

### N-((2-((((2-Oxabicyclo[2.1.1]hexan-1-yl)methyl-d₂)amino)methyl)-1H-indol-6-yl)methyl)-9-oxo-2,3-dihydro-9H-[1,4]dioxino[2',3':4,5]pyrido[1,2-a]pyrimidine-7-carboxamide

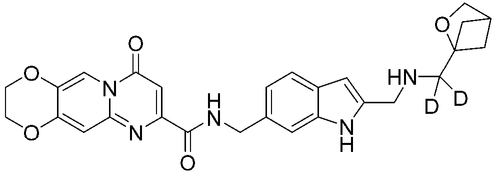

Following the synthetic method of **Example 77,** using Intermediate 8 and (2-oxabicyclo[2.1.1]hexan-1-yl)methane-*d*₂-amine as raw materials, the title compound (11.7 mg, white solid) was obtained through similar procedures. MS (ESI): m/z 504.1 [M+H]⁺. ¹H NMR (400 MHz, DMSO-*d*₆) δ 10.91 (s, 1H), 9.04 (t, *J* = 6.4 Hz, 1H), 8.62 (s, 1H), 7.37 (d, *J* = 8.0 Hz, 1H), 7.29 (s, 1H), 7.19 (s, 1H), 6.95 (d, *J* = 7.6 Hz, 1H), 6.67 (s, 1H), 6.21 (s, 1H), 4.57-4.53 (m, 4H), 4.47-4.45 (m, 2H), 3.82 (s, 2H), 3.61 (s, 2H), 2.83 (t, *J* = 2.8 Hz, 1H), 1.71-1.68 (m, 2H), 1.33-1.29 (m, 2H).

(2-Oxabicyclo[2.1.1]hexan-1-yl)methane-*d*₂-aminewas synthesized as follows:

### (1) 1-(Iodomethyl)-2-oxabicyclo[2.1.1]hexane

(3-Methylenecyclobutyl)methanol (2.0 g, 20.38 mmol), sodium bicarbonate (5.14 g, 61.13 mmol) and iodine (10.34 g, 40.76 mmol) were added to a mixed solution of methyl tert-butyl ether and water (100 mL, 1:1), and the reaction was stirred at 25°C for 16 hours. Methyl tert-butyl ether and water (300 mL, 1:1) were added to the reaction solution, the organic phase was concentrated under reduced pressure, and the residue was purified by silica gel column chromatography (petroleum ether/ethyl acetate = 95/5) to obtain the title compound (3.5 g, yellow oil), yield: 77%. MS (ESI): m/z 225.0 [M+H]⁺.

### (2) Methyl (2-oxabicyclo[2.1.1]hexan-1-yl)acetate

1-(Iodomethyl)-2-oxabicyclo[2.1.1]hexane (1.5 g, 6.7 mmol) and potassium acetate (1.97 g, 20.09 mmol) were dissolved in dimethyl sulfoxide (50 mL) and stirred at 90°C for 16 hours. Ethyl acetate and water (200 mL, 1:1) were added for extraction, and the organic phase was concentrated under reduced pressure to obtain the title compound (1.1 g, yellow oil), yield: 100%. MS (ESI): m/z 157.0 [M+H]⁺.

### (3) (2-Oxabicyclo[2.1.1]hexan-1-yl)methanol

Methyl (2-oxabicyclo[2.1.1]hexan-1-yl)acetate (1.1 g, 7.04 mmol) and sodium ethoxide (958 mg, 14.09 mmol) were dissolved in anhydrous ethanol (50 mL) and stirred at 25°C for 12 hours. The reaction solution was concentrated, and the residue was purified by silica gel column chromatography (petroleum ether/ethyl acetate = 50/50) to obtain the title compound (450 mg, yellow oil), yield: 56%. MS (ESI): m/z 137.0 [M+Na]⁺.

### (4) 2-Oxabicyclo[2.1.1]hexane-1-carboxylic acid

(2-Oxabicyclo[2.1.1]hexan-1-yl)methanol (450 mg, 3.94 mmol), 2,2,6,6-tetramethylpiperidine oxide (924 mg, 5.91 mmol) and iodobenzene diacetate (1.9 g, 5.91 mmol) were added to a mixed solution of acetonitrile and water (30 mL, 2:1), and the reaction was stirred at 25°C for 12 hours. The reaction solution was concentrated, ethyl acetate and water (100 mL, 1:1) were added, and the aqueous phase was freeze-dried to obtain the title compound (400 mg, yellow oil), yield: 79%. MS (ESI): m/z 129.0 [M+H]⁺.

### (5) N-Benzyl-2-oxabicyclo[2.1.1]hexane-1-carboxamide

2-Oxabicyclo[2.1.1]hexane-1-carboxylic acid (400 mg, 3.12 mmol), benzylamine (1.0 g, 9.37 mmol), 1-hydroxybenzotriazole (633 mg, 4.68 mmol), 1-(3-dimethylaminopropyl)-3-ethylcarbodiimide hydrochloride (898 mg, 4.68 mmol) and *N,N*-diisopropylethylamine (2.03 g, 15.16 mmol)were dissolved in *N,N*-dimethylformamide (50 mL) and stirred at 25°C for 16 hours.

The reaction solution was extracted three times with ethyl acetate and water (100 mL, 1:1), the organic phase was concentrated, and the residue was purified by silica gel column chromatography (petroleum ether/ethyl acetate = 65/35) to obtain the title compound (270 mg, yellow solid), yield: 40%. MS (ESI): m/z 218.0 [M+H]⁺.

### (6) N-Benzyl-1-(2-oxabicyclo[2.1.1]hexan-1-yl)methane-d₂-amine

N-Benzyl-2-oxabicyclo[2.1.1]hexane-1-carboxamide (230 mg, 1.06 mmol) and lithium aluminum deuteride (133 mg, 3.18 mmol) were added to 1,4-dioxane (20 mL) and stirred at 100°C for 12 hours. Ethyl acetate and water (50 mL, 1:1) were added to the reaction solution at 0°C, stirred vigorously for 0.5 hours, filtered through Celite, the filter cake was rinsed with ethyl acetate, extracted separated, the organic phase was concentrated, and the residue was purified by silica gel column chromatography (petroleum ether/ethyl acetate = 40/60) to obtain the title compound (110 mg, colorless oil), yield: 51%. MS (ESI): m/z 206.1 [M+H]⁺.

### (7) (2-Oxabicyclo[2.1.1]hexan-1-yl)methane-d₂-amine

N-Benzyl-1-(2-oxabicyclo[2.1.1]hexan-1-yl)methane-*d*₂-amine (30 mg, 0.15 mmol) and palladium on carbon (6 mg, 20% w/w) were added to anhydrous methanol (5 mL) and stirred at 50°C for 12 hours. The reaction solution was filtered to obtain the crude methanol solution of the title compound (5 mL, white liquid), yield: 100%. MS (ESI): m/z 116.0 [M+H]⁺.

### Example 92

### N-((2-((((2-Oxabicyclo[2.1.1]hexan-1-yl)methyl-d₂)amino)methyl)-1H-indol-6-yl)methyl)-8-oxo-8H-[1,3]dioxolo[4',5':4,5]pyrido[1,2-a]pyrimidine-6-carboxamide

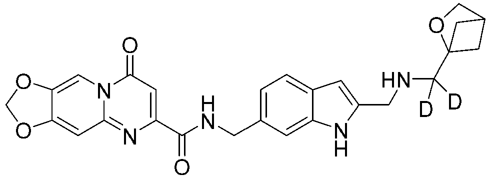

Following the synthetic method of **Example 84,** using Intermediate 7 and (2-oxabicyclo[2.1.1]hexan-1-yl)methane-*d*₂-amine as raw materials, the title compound (10.5 mg, white solid) was obtained through similar procedures. MS (ESI): m/z 490.1 [M+H]⁺. ¹H NMR (400 MHz, DMSO-*d*₆) δ 10.89 (s, 1H), 9.03 (t, *J* = 6.0 Hz, 1H), 8.60 (s, 1H), 7.37 (d, *J* = 8.4 Hz, 1H), 7.29 (s, 1H), 7.14 (s, 1H), 6.94 (d, *J* = 8.0 Hz, 1H), 6.73 (s, 1H), 6.37 (s, 2H), 6.21 (s, 1H), 4.57-4.53 (m, 2H), 3.82 (s, 2H), 3.61 (s, 2H), 2.83 (t, *J* = 3.2 Hz, 1H), 1.71-1.69 (m, 2H), 1.32-1.30 (m, 2H).

### Example 93

### N-((2-((((2-Oxabicyclo[2.1.1]hexan-1-yl)methyl-d₂)amino)methyl)-5-fluoro-1H-indol-6-yl)methyl)-8-oxo-8H-[1,3]dioxolo[4',5':4,5]pyrido[1,2-a]pyrimidine-6-carboxamide

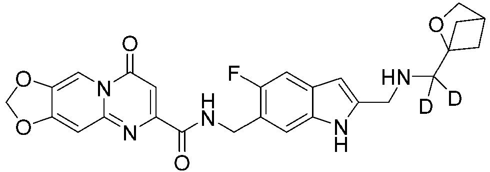

Following the synthetic method of **Example 84,** using *N*-((5-fluoro-2-formyl-1H-indol-6-yl)methyl)-8-oxo-8H-[1,3]dioxolo[4',5':4,5]pyrido[1,2-a]pyrimidine-6-carboxamide (Step 5 of **Example 73**) and (2-oxabicyclo[2.1.1]hexan-1-yl)methane-*d*₂-amine as raw materials, the title compound (5.0 mg, white solid) was obtained through similar procedures. MS (ESI): m/z 508.1 [M+H]⁺. ¹H NMR (400 MHz, DMSO-*d*₆) δ 10.98 (s, 1H), 8.99 (t, *J =* 6.0 Hz, 1H), 8.60 (s, 1H), 7.28 (d, *J* = 6.4 Hz, 1H), 7.21-7.13 (m, 2H), 6.73 (s, 1H), 6.38 (s, 2H), 6.23 (s, 1H), 4.58 (d, *J =* 6.0 Hz, 2H), 3.81 (s, 2H), 3.61 (s, 2H), 2.83 (t, *J* = 3.2 Hz, 1H), 1.71-1.69 (m, 2H), 1.31-1.30 (m, 2H).

### Example 94

### N-((5-fluoro-2-((((3-fluorobicyclo[1.1.1]pentan-1-yl)methyl-d₂)amino)methyl)-1H-indol-6-yl)methyl)-9-oxo-2,3-dihydro-9H-[1,4]dioxino[2',3':4,5]pyrido[1,2-a]pyrimidine-7-carboxamide

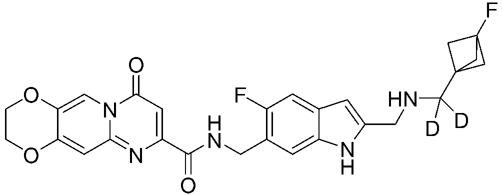

Following the synthetic method of Step 3 in **Example 85,** using *N*-((5-fluoro-2-formyl-1H-indol-6-yl)methyl)-9-oxo-2,3-dihydro-9H-[1,4]dioxino[2',3':4,5]pyrido[1,2-a]pyrimidine-7-carboxamide and (3-fluorobicyclo[1.1.1]pentan-1-yl)methane-*d*₂-amine as raw materials, the title compound (2.4 mg, white solid) was obtained through similar procedures. MS (ESI): m/z 524.1 [M+H]⁺. ¹H NMR (400 MHz, DMSO-*d*₆) δ 10.99 (s, 1H), 9.02 (t, *J* = 6.4 Hz, 1H), 8.63 (s, 1H), 8.43 (s, 2H), 7.28 (d, *J =* 6.4 Hz, 1H), 7.22 (s, 1H), 7.19 (d, *J =* 11.2 Hz, 1H), 6.66 (s, 1H), 6.22 (s, 1H), 4.59-4.56 (m, 4H), 4.48-4.47 (m, 2H), 3.79 (s, 2H), 1.92 (d, *J* = 2.8 Hz, 6H).

### Example 95

### N-((2-((((3-fluorobicyclo[1.1.1]pentan-1-yl)methyl-d₂)amino)methyl)-1H-indol-6-yl)methyl)-9-oxo-2,3-dihydro-9H-[1,4]dioxino[2',3':4,5]pyrido[1,2-a]pyrimidine-7-carboxamide

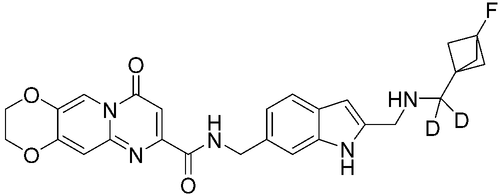

Following the synthetic method of **Example 77,** using Intermediate 8 and (3-fluorobicyclo[1.1.1]pentan-1-yl)methane-*d*₂-amineas raw materials, the title compound (21.3mg, white solid) was obtained through similar procedures. MS (ESI): m/z 506.1 [M+H]⁺. ¹H NMR (400 MHz, DMSO-*d*₆) δ 10.87 (s, 1H), 9.06 (t, *J =* 6.0 Hz, 1H), 8.61 (s, 1H), 7.37 (d, *J =* 8.0 Hz, 1H), 7.29 (s, 1H), 7.19 (s, 1H), 6.95 (d, *J* = 8.0 Hz, 1H), 6.67 (s, 1H), 6.20 (s, 1H), 4.56-4.53 (m, 4H), 4.47-4.46 (m, 2H), 3.80 (s, 2H), 1.92 (d, *J =* 2.8 Hz, 6H).

The reference drug STC-15 used in the present invention was prepared according to the method of **Example 2** in Patent WO2021111124.

### Test Example 1: METTL3/14 Methyltransferase Activity Assay

The final initial concentration of the compound was 10 µM, with 3-fold serial dilution to give a total of 10 concentrations. 100 nL of the compound was transferred to a 384-well plate and centrifuged at 1000 RPM. 2.5 µL of METTL3/14 (active motif, Cat No. 31570) was added to the test wells and incubated at 25°C for 10 min. 2.5 µL of METTL3-Biotin & SAM (GenScript) was added to the test wells andincubated at 25°C for 60 min. 2.5 µL of YTHDF1 (active motif, Cat No. 31608) was added to the test wells and centrifuged at 1000 RPM. 2.5 µL of Streptavidin-Tb (Perkin Elmer Cat No. 61SATLB) & Flag-mede (Perkin Elmer Cat No. 61FG2DLA) was added to the test wells and centrifuged at 1000 RPM. Incubated at 25°C for 60 min. HTRF was measured using a Plate reader. The results of each compound are shown in Table 1, where A+indicates <10nM, A indicates 10-100nM, B indicates 100-1000nM, and C indicates >1000nM.

**Table 1**

| **Example** | METTL3/14 HTRF IC50 (nM) | **Example** | METTL3/14 HTRF IC50 (nM) |
|---|---|---|---|
| 1 | A | 54 | A+ |
| 2 | A | 56 | A |
| 3 | A | 58 | A |
| 8 | B | 59 | B |
| 11 | A | 60 | A+ |
| 13 | A | 61 | B |
| 14 | A | 62 | A+ |
| 15 | B | 63 | A+ |
| 20 | A | 71 | A+ |
| 21 | B | 72 | A+ |
| 24 | A+ | 73 | A+ |
| 25 | A+ | 74 | A+ |
| 26 | A | 75 | A |
| 27 | A | 76 | A |
| 28 | B | 77 | A+ |
| 29 | A | 78 | A |
| 30 | A | 79 | A |
| 31 | A | 80 | A |
| 32 | A | 81 | B |
| 34 | A | 82 | A |
| 36 | A+ | 83 | A+ |
| 37 | A | 84 | A+ |
| 38 | A+ | 85 | A |
| 39 | A | 86 | A |
| 40 | A | 87 | A |
| 41 | A+ | 88 | A+ |
| 42 | B | 89 | A+ |
| 43 | A | 90 | A+ |
| 44 | A | 91 | A |
| 45 | A | 92 | A |
| 46 | A+ | 93 | A |
| 51 | A+ | 94 | A+ |
| 52 | A+ | 95 | A+ |
| 53 | A+ | STC-15 | A |

### Test Example 2:Kasumi-1 Cell Inhibitory Activity

Test compounds were dissolved in DMSO to prep are a 250×final concentration solution. The initial concentration was 10 µM with 3-fold dilution, resulting in a total of ten concentration points. 4 µL of the compound solution was added to 96 µL of cell culture medium to dilute it to 10×the final concentration. On the first day, Kasumi-1 cells (purchased from Precedo) were suspended in cell culture medium at the desired density, 90 µL of the cell suspension was added to a 96-well plate, and 10 µL of 10×compound solution was added for incubation, with a final DMSO concentration of 0.4% in each well. Incubated at 37 °C, 5% CO₂ for 5 days. Before measurement after 5 days, the test plate was placed at room temperature, 50 µL of CellTiter-Glo^{®} reagent (purchased from Promega, Cat. No. G7573) was added, mixed on a shaker for 2 minutes to lyse the cells, and incubated at room temperature for 10 minutes to stabilize the fluorescent signal. Luminescence values were recorded using Paradigm, and the response-concentration curve was fitted with Graphpad Pr ism 8.0 to calculate IC₅₀values. The results of each compound are shown in Table2, where A indicates <100nM, B indicates 100-1000nM, and C indicates >1000nM. 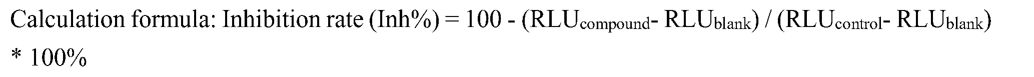Inhibition rate (Inh%) = 100 - (RLUcompound- RLUblank) / (RLUcontrol- RLUblank) * 100%

**Table 2**

| **Example** | IC50 (nM) | **Example** | IC50 (nM) |
|---|---|---|---|
| 20 | B | 72 | A |
| 24 | B | 73 | B |
| 25 | B | 77 | B |
| 26 | B | 84 | B |
| 32 | B | 85 | A |
| 36 | A | 86 | B |
| 38 | B | 87 | B |
| 41 | B | 88 | A |
| 44 | B | 89 | A |
| 45 | B | 90 | A |
| 46 | A | 91 | B |
| 51 | B | 92 | B |
| 52 | B | 93 | B |
| 53 | B | 94 | A |
| 54 | B | 95 | A |
| 71 | A | STC-15 | B |

### Test Example 3: Rat Pharmacokinetic Test

3.1 Experimental Animals: Male SD rats, weighing 190-230 g, 3 rats per compound
3.2 Experimental Design: All animals were fasted overnight (10-14 hours) the day before admin istration and fed 4 hours after admin istration. For intravenous injection, the drug vehicle was 5% DMA + 5% Solutol + 90% Saline (DMA: dimethylacetamide; Solutol: polyethyleneglycol-15-hydroxystearate; Saline: normal saline), with a dosage of 2 mg/kg and an admin istration concentration of 1 mg/mL. Blood samples were collected via the jugular vein at 5 min, 15 min, 30 min, 1 h, 2 h, 4 h, 6 h, 8 h, and 24 h after admin istration. Approximately 0.25 mL of each sample was collected, anticoagulated with EDTA-K2, placed on ice after collection, and centrifuged to separate plasma within 1 hour (6000 g, 3 min, 2-8°C). Plasma samples were stored in a - 80°Crefrigerator before analys is.

3.3 Result Analys is:Collected samples were detected using LC-MS/MS method. Pharmacokinetic parameters were calculated from blood drug concentration data at different time points using Phoenix WinNonlin 8.2.0 to obtain the average values of in vivo clearance (CL) and AUC₀₋ₜ.

**Table 3 CL and AUC of test compounds after intravenous injection in rats**

| Test Compound | CL (mL/kg/min) | AUC₀₋ₜ(h*ng/mL) |
|---|---|---|
| **STC-15** | 40.3 | 808 |
| **Example 24** | 15.3 | 2179 |
| **Example 25** | 22.7 | 1459 |
| **Example 73** | 23.0 | 1443 |
| **Example 88** | 19.1 | 1713 |
| **Example 89** | 28.1 | 1214 |
| **Example 94** | 28.5 | 1125 |

## Claims

1. A compound of formula (I): 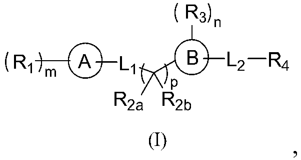
or a deuterated, a stereoisomer, a pharmaceutically acceptable salt, or a pharmaceutically acceptable solvate thereof;
wherein,
Ring A is a 5- to 15-membered heterocyclyl, preferably a 5- to 6-membered monocyclic heterocyclyl, a 9- to 10-membered bicyclic heterocyclyl, or a 13- to 14-membered tricyclic heterocyclyl, containing heteroatoms selected from O, N, S, P, and optionally having 1, 2, or 3 atoms oxo-substituted;
L₁ is 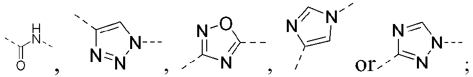
m is selected from 0, 1, 2, 3, 4 or 5;
R₁ is oxo, or
R₁ is -Z-Rₐ;
Z is selected from a chemical bond, C₁₋₃ alkylene, -NH-C₁₋₃ alkylene, -C(=O)-, -O-, -S-, -S(=O)-, - SO₂-;
Rₐ is selected from halogen, cyano, hydroxy, C₁₋₃ alkyl, C₁₋₃ alkoxy, halo-C₁₋₃ alkyl, halo-C₁₋₃ alkoxy, -NH₂, nitro, formyl, -NH-C₁₋₃ alkyl, -N(-C₁₋₃ alkyl)₂, optionally substituted 3- to 6-membered cycloalkyl, optionally substituted 4- to 6-membered heterocycloalkyl, optionally substituted phenyl, optionally substituted 5- to 7-membered heteroaryl;
When two R₁ are both attached to the same carbon atom of Ring A, the two R₁ together with the carbon atom to which they are attached may form an optionally substituted 3- to 6-membered cycloalkyl or a 3- to 6-membered heterocyclyl containing 1-2 heteroatoms selected from N, O, S; Ring B is an 8- to 10-membered heterocyclyl, preferably an 8- to 10-membered heteroaryl;
n, p are selected from 0, 1, 2, or 3;
R₂ₐ and R_{2b} are independently selected from H, halogen, cyano, hydroxyl, C₁₋₃ alkyl, C₁₋₃ alkoxy, halo-C₁₋₃ alkyl, halo-C₁₋₃ alkoxy, nitro, formyl, or
R₂ₐ and R_{2b} together with the carbon atom to which they are attached form a 3- to 6-membered cycloalkyl or a 3- to 6-membered heterocycloalkyl containing 1-2 heteroatoms selected from N, O, S;
R₃ is selected from H, halogen, cyano, hydroxyl, C₁₋₃ alkyl, C₁₋₃ alkoxy, halo-C₁₋₃ alkyl, halo-C₁₋₃ alkoxy, nitro, formyl;
L₂ is selected from C₁₋₃ alkylene, halo-C₁₋₃ alkylene;
R₄ is 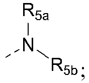
R₅ₐ and R_{5b} are selected from H or -L₃-R₆, where L₃ is selected from a chemical bond, C₁₋₃ alkylene, halo-C₁₋₃ alkylene, and R₆ is selected from C₁₋₆ alkyl, C₂₋₆ alkynyl, optionally substituted phenyl, optionally substituted 5- to 10-membered heterocyclyl, optionally substituted 3- to 8-membered cycloalkyl, optionally substituted 4- to 8-membered heterocycloalkyl, or
R₅ₐ and R_{5b} together with the nitrogen atom to which they are attached form an optionally substituted 4- to 10-membered heterocyclyl, preferably a 4- to 10-membered heterocycloalkyl.

2. The compound, or the deuteride, the stereo isomer, or the pharmaceutically acceptable salt, or the pharmaceutically acceptable solvate thereof according to claim 1, wherein:
Ring A is selected from one of the following:
i) 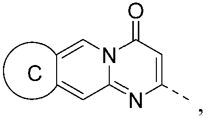 wherein Ring C is a 5- to 7-membered heterocyclyl containing 1-3 heteroatoms selected from N, O, S; more preferably 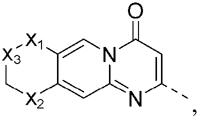 wherein X₁ and X₂ are independently selected from O, S, CH₂, S(=O)₂, C(=O), and X₃ is selected from CH₂, NH or a chemical bond;
ii) 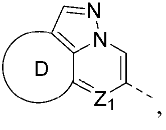 wherein Ring D is a 6- to 8-membered heterocyclyl containing 1-3 heteroatoms selected from N, O, S, and Z₁ is selected from N or CH; more preferably 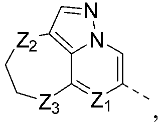 wherein Z₁ is selected from N or CH, Z₂ and Z₃ are independently selected from CH₂, NH, O, S;
iii) 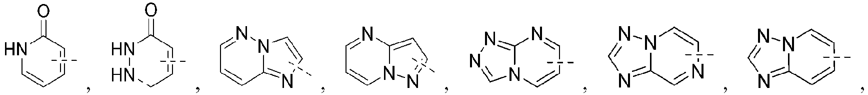 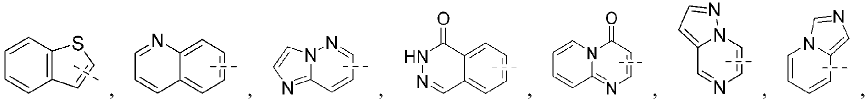 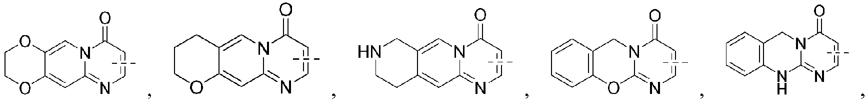 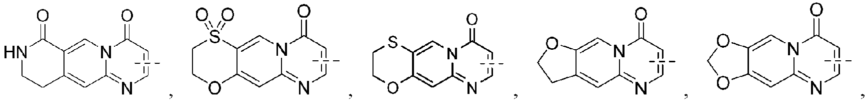 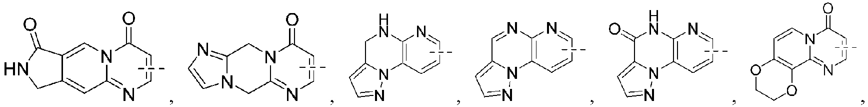 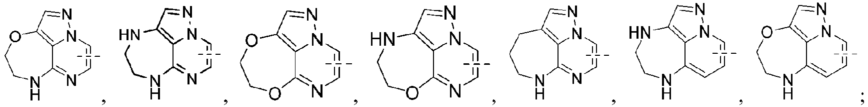
Ring A is preferably: 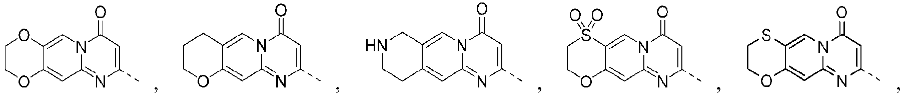 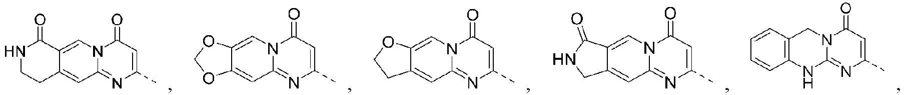 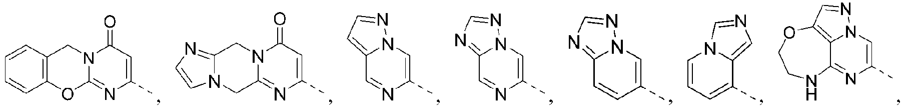 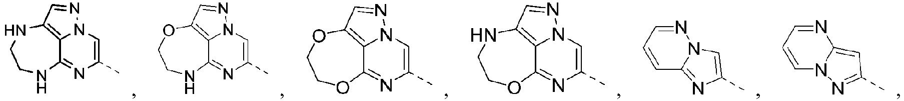 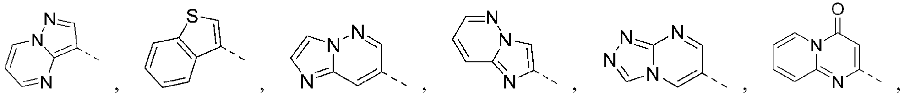 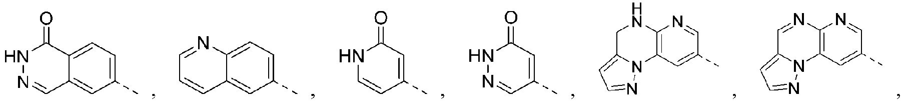 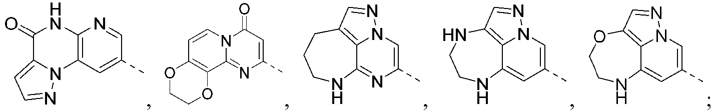
m is selected from 0, 1, 2 or 3;
R₁ is oxo, or
R₁ is -Z-Rₐ;
Z is selected from a chemical bond, methylene, -NH-CH₂-, -C(=O)-, -O-, -S-, -S(=O)-, -SO₂-;
Rₐ is selected from halogen, cyano, hydroxyl, C₁₋₃ alkyl, C₁₋₃ alkoxy, halo-C₁₋₃ alkyl, halo-C₁₋₃ alkoxy, -NH₂, nitro, formyl, -NH-C₁₋₃ alkyl, -N(-C₁₋₃ alkyl)₂, and the following groups optionally substituted by 1, 2 or 3 R_{b} groups: 3- to 6-membered cycloalkyl, 4- to 6-membered heterocycloalkyl, phenyl, 5- to 7-membered heteroaryl;
R_{b} is selected from halogen, cyano, hydroxyl, C₁₋₃ alkyl, C₁₋₃ alkoxy, halo-C₁₋₃ alkyl, halo-C₁₋₃ alkoxy, -NH₂, nitro, formyl, -NH-C₁₋₃ alkyl, -N(-C₁₋₃ alkyl)₂, preferably F, methyl, methoxy, amino, methylamino;
Rₐ is preferably selected from F, methyl, trifluoromethyl, difluoromethyl, methoxy, trifluoromethoxy, amino, methylamino, and the following groups optionally substituted by 1, 2 or 3 R_{b}: 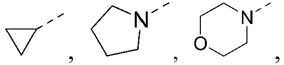 phenyl, 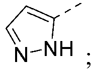 more preferably F, methyl, trifluoromethyl, difluoromethyl, methoxy, trifluoromethoxy, amino, methylamino, 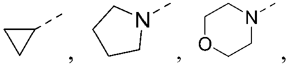 phenyl, 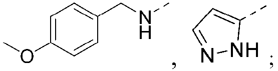
R₁ is more preferably selected from F, methyl, trifluoromethyl, difluoromethyl, methoxy, trifluoromethoxy, amino, methylamino, 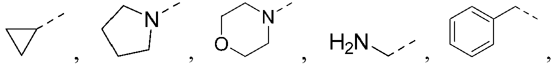 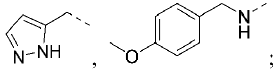
When two R₁ are both substituted on the same carbon atom of Ring A, the two R₁ may form an optionally substituted 3- to 6-membered cycloalkyl or a 3- to 6-membered heterocyclyl containing 1-2 heteroatoms selected from N, O, S, preferably cyclopropyl, cyclobutyl, oxiranyl, oxetanyl, azetidinyl.

3. The compound, or the deuteride, the stereo isomer, or the pharmaceutically acceptable salt, or the pharmaceutically acceptable solvate thereof according to claim 2, wherein:
Ring A and R₁ form the following structures:

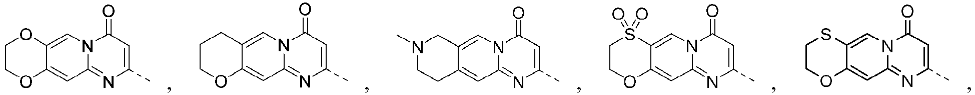

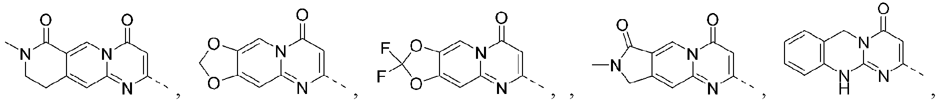

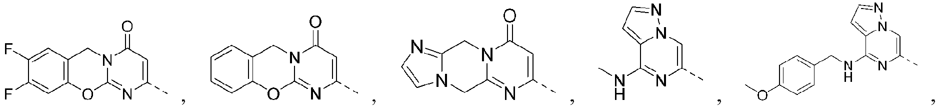

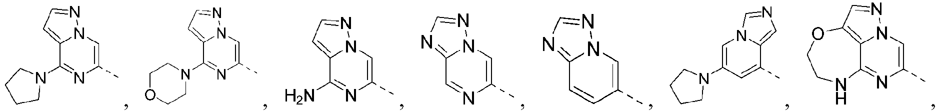

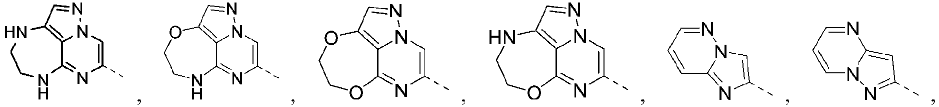

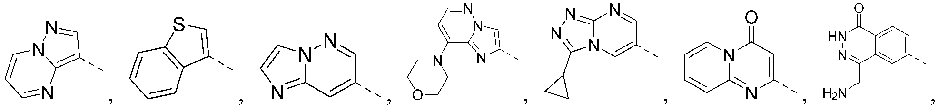

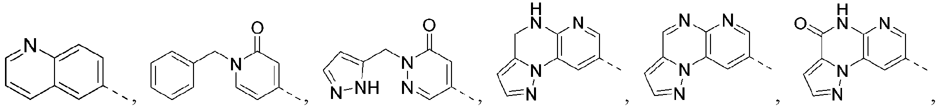

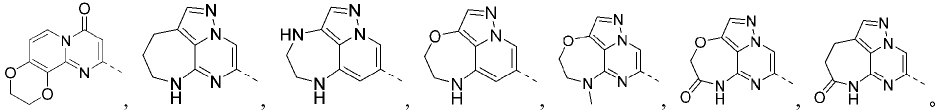

4. The compound, or the deuteride, the stereo isomer, or the pharmaceutically acceptable salt, or the pharmaceutically acceptable solvate thereof according to claim 1, wherein;
Ring B is selected from: 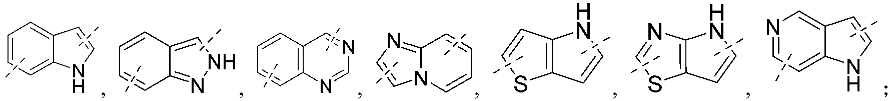
Ring B is preferably selected from: 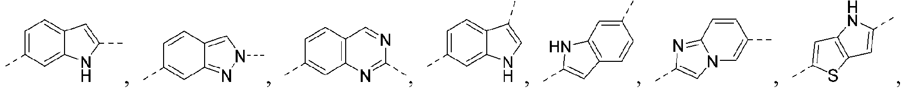 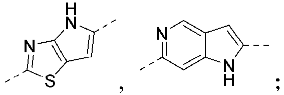
n is selected from 0, 1, 2 or 3;
R₃ is selected from H, halogen, C₁₋₃ alkyl, C₁₋₃ alkoxy, halo-C₁₋₃ alkyl, halo-C₁₋₃ alkoxy, preferably H, F, methyl, trifluoromethyl, difluoromethyl, methoxy, trifluoromethoxy.

5. The compound, or the deuteride, the stereo isomer, or the pharmaceutically acceptable salt, or the pharmaceutically acceptable solvate thereof according to claim 1, wherein;
L₂ is selected from methylene, ethylidene;
R₄ is 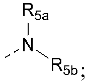
R₅ₐ and R_{5b} are selected from H or -L₃-R₆, where L₃ is selected from a chemical bond, methylene, ethylidene, propylidene, 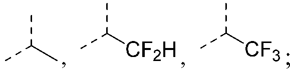 R₆ is selected from the following groups optionally substituted with 1, 2 or 3 R₇: ethynyl, isopropyl, tert-butyl, phenyl, 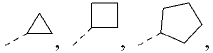 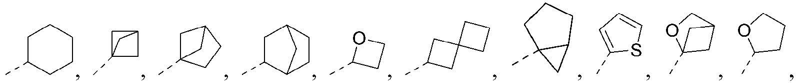 or
R₅ₐ and R_{5b} together with the N atom to which they are attached form 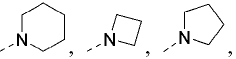 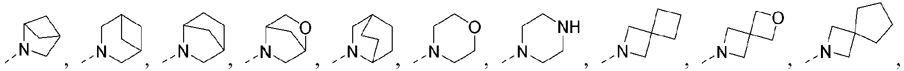 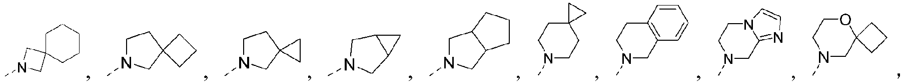
optionally substituted with 1, 2 or 3 R₇;
R₇ is selected from halogen, cyano, hydroxyl, C₁₋₃ alkyl, C₁₋₃ alkoxy, halo-C₁₋₃ alkyl, halo-C₁₋₃ alkoxy, =CF₂, hydroxy-C₁₋₃ alkyl, preferably F, hydroxyl, methyl, trifluoromethyl, difluoromethyl, methoxy, trifluoromethoxy, =CF₂, hydroxymethyl.

6. The compound, or the deuteride, the stereo isomer, or the pharmaceutically acceptable salt, or the pharmaceutically acceptable solvate thereof according to claim 5, wherein:
R₄ is selected from

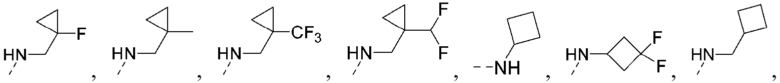

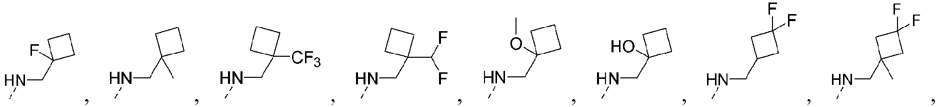

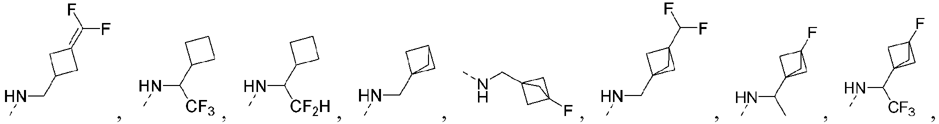

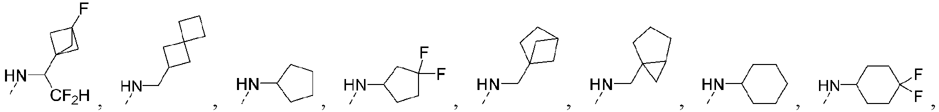

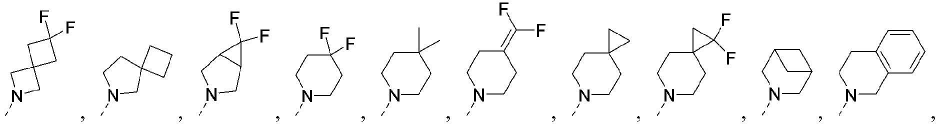

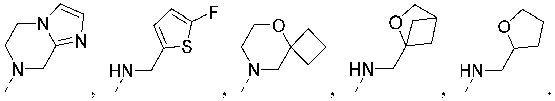

7. The compound, or the deuteride, the stereo isomer, or the pharmaceutically acceptable salt, or the pharmaceutically acceptable solvate thereof according to claim 5, wherein:
L₂ and R₄ form the following structures:

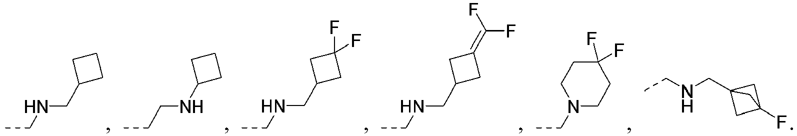

8. The compound, or the deuteride, the stereo isomer, or the pharmaceutically acceptable salt, or the pharmaceutically acceptable solvate thereof according to claim 1, wherein:
L₁ is

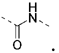

9. The compound, or the deuteride, the stereo isomer, or the pharmaceutically acceptable salt, or the pharmaceutically acceptable solvate thereof according to claim 1, wherein:
n and p are 1;
R₂ₐ, R_{2b} and R₃ are H.

10. The compound, or the deuteride, the stereo isomer, or the pharmaceutically acceptable salt, or the pharmaceutically acceptable solvate thereof according to claim 1, wherein, it has the structure of formula (II): 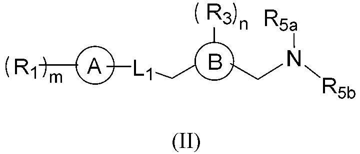 wherein,
Ring A is selected from one of the following:
i) 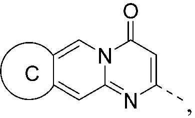 wherein Ring C is selected from 5- to 7-membered heterocyclyl containing 1-3 heteroatoms selected from N, O, S; more preferably 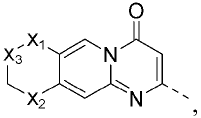 wherein X₁ and X₂ are independently selected from O, S, CH₂, S(=O)₂, C(=O), and X₃ is selected from CH₂, NH or a chemical bond;
ii) 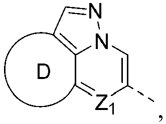 wherein Ring D is selected from 6- to 8-membered heterocyclyl containing 1-3 heteroatoms selected from N, O, S, and Z₁ is selected from N or CH; more preferably 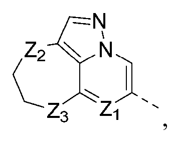 Z₁ is selected from N or CH, and Z₂ and Z₃ are independently selected from CH₂, NH, O, S;
iii) 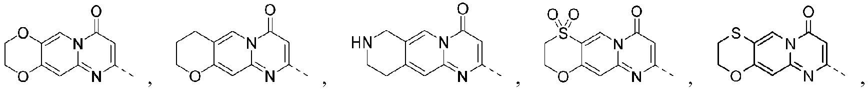 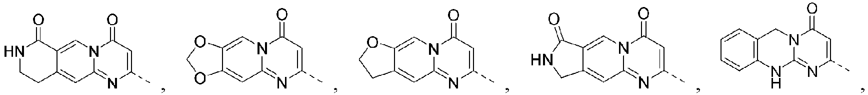 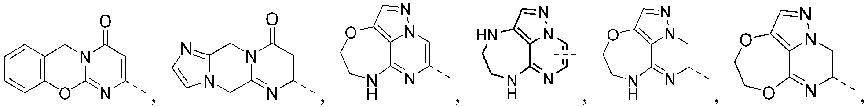 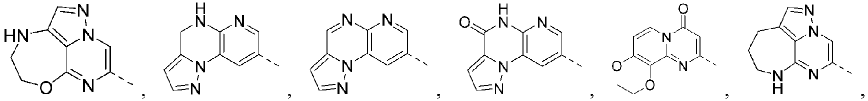 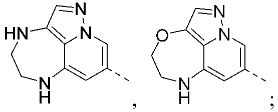
m is selected from 0, 1, 2 or 3;
R₁ is oxo, or
R₁ is -Z-Rₐ;
Z is selected from a chemical bond, C₁₋₃ alkylene, -NH-C₁₋₃ alkylene, -C(=O)-, -O-, -S-, -S(=O)-, - SO₂-, preferably methylene, -NH-CH₂-, -C(=O)-, -SO₂-;
Rₐ is selected from halogen, cyano, hydroxyl, C₁₋₃ alkyl, C₁₋₃ alkoxy, halo-C₁₋₃ alkyl, halo-C₁₋₃ alkoxy, -NH₂, nitro, formyl, -NH-C₁₋₃ alkyl, -N(-C₁₋₃ alkyl)₂, and the following groups optionally substituted with 1, 2 or 3 R_{b}: 3- to 6-membered cycloalkyl, 4- to 6-membered heterocycloalkyl, phenyl, 5- to 7-membered heteroaryl;
R_{b} is selected from halogen, cyano, hydroxyl, C₁₋₃ alkyl, C₁₋₃ alkoxy, halo-C₁₋₃ alkyl, halo-C₁₋₃ alkoxy, -NH₂, nitro, formyl, -NH-C₁₋₃ alkyl, -N(-C₁₋₃ alkyl)₂, preferably F, methyl, methoxy, amino, methylamino;
Rₐ is preferably selected from F, methyl, amino, methylamino;
When two R₁ are both substituted on the same carbon atom of Ring A, the two R₁ together with the carbon atom to which they are attached may form an optionally substituted 3- to 6-membered cycloalkyl or 3- to 6-membered heterocyclyl containing 1-2 heteroatoms selected from N, O, S;
L₁ is 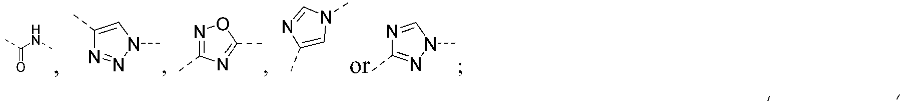
Ring B is selected from: 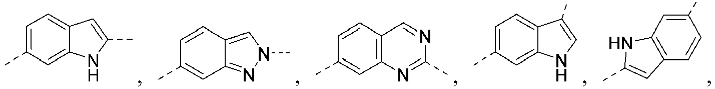 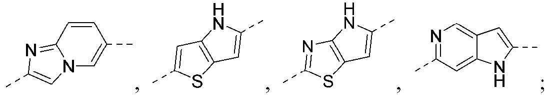
n is selected from 0, 1, 2 or 3;
R₃ is independently selected from halogen, cyano, hydroxyl, C₁₋₃ alkyl, C₁₋₃ alkoxy, halo-C₁₋₃ alkyl, halo-C₁₋₃ alkoxy;
R₅ₐ and R_{5b} are selected from H or -L₃-R₆; L₃ is selected from a chemical bond, C₁₋₃ alkylene, halo-C₁₋₃ alkylene, preferably a chemical bond, methylene, 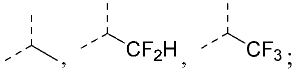 R₆ is selected from C₁₋₆ alkyl, C₂₋₆ alkynyl, optionally substituted phenyl, optionally substituted 5- to 10-membered heterocyclyl, optionally substituted 3- to 8-membered cycloalkyl, optionally substituted 4- to 8-membered heterocycloalkyl, preferably ethynyl, isopropyl, tert-butyl, and the following groups optionally substituted with 1, 2 or 3 R₇: phenyl, 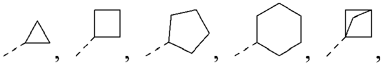 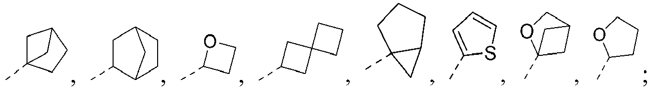 or
R₅ₐ and R_{5b} together with the attached N atom form an optionally substituted 4- to 10-membered heterocyclyl, preferably a 4- to 10-membered heterocycloalkyl, preferably the following groups optionally substituted with 1, 2 or 3 R₇:. 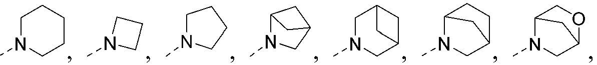 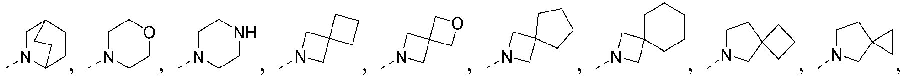 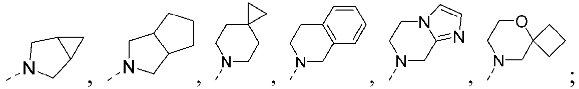
R₇ is selected from halogen, cyano, hydroxyl, C₁₋₃ alkyl, C₁₋₃ alkoxy, halo-C₁₋₃ alkyl, halo-C₁₋₃ alkoxy, =CF₂, hydroxy-C₁₋₃ alkyl, preferably F, hydroxyl, methyl, trifluoromethyl, difluoromethyl, methoxy, trifluoromethoxy, =CF₂, hydroxymethyl.

11. The compound, or the deuteride, the stereo isomer, or the pharmaceutically acceptable salt, or the pharmaceutically acceptable solvate thereof according to claim 10, wherein:
Ring A is selected from: 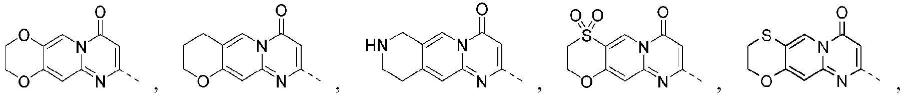 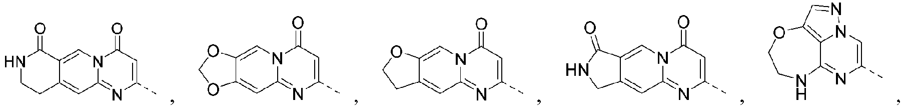 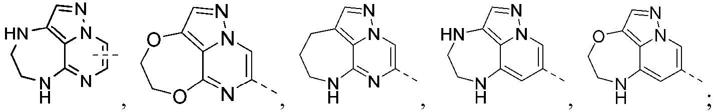 preferably 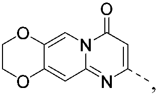 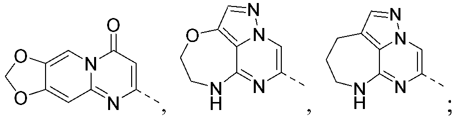 more preferably 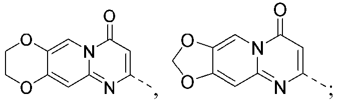
R₁ is oxo, or
R₁ is -Z-Rₐ;
Z is selected from a chemical bond;
Rₐ is selected from halogen, C₁₋₃ alkyl, C₁₋₃ alkoxy, halo-C₁₋₃ alkyl, halo-C₁₋₃ alkoxy; preferably F, methyl.

12. The compound, or the deuteride, the stereo isomer, or the pharmaceutically acceptable salt, or the pharmaceutically acceptable solvate thereof according to claim 10 or 11, wherein:
L₁ is 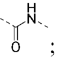
More preferably:
Ring B is selected from 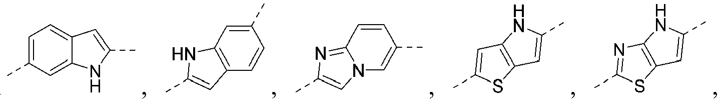 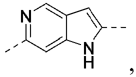 preferably 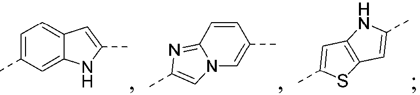
R₃ is independently selected from F, Cl, methyl, methoxy, trifluoromethyl, difluoromethyl, trifluoromethoxy, difluoromethoxy;
More preferably:
R₆ is selected from the following groups optionally substituted with 1, 2 or 3 R₇: 3- to 8-membered cycloalkyl, 4- to 8-membered heterocycloalkyl containing 1-2 heteroatoms selected from N, O, S, thienyl; preferably 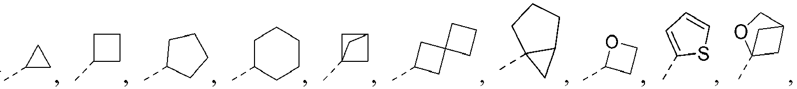 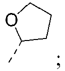
R₇ is selected from halogen, hydroxyl, C₁₋₃ alkyl, C₁₋₃ alkoxy, halo-C₁₋₃ alkyl, halo-C₁₋₃ alkoxy, =CF₂, preferably F, hydroxyl, methyl, trifluoromethyl, difluoromethyl, methoxy, trifluoromethoxy, =CF₂, more preferably F, methyl, trifluoromethyl, difluoromethyl, methoxy, trifluoromethoxy;
or more preferably:
R₅ₐ and R_{5b} together with the attached N atom form the following groups optionally substituted with 1, 2 or 3 R₇: 4- to 10-membered heterocycloalkyl containing 1-3 heteroatoms selected from N, O, S, 4- to 10-membered heterocyclyl containing 1-3 heteroatoms selected from N, O, S;
preferably 4- to 10-membered heterocycloalkyl containing 1-3 heteroatoms selected from N, O, S, 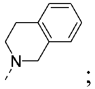 more preferably 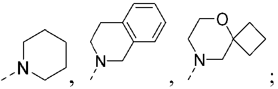
R₇ is selected from halogen, hydroxyl, C₁₋₃ alkyl, C₁₋₃ alkoxy, halo-C₁₋₃ alkyl, halo-C₁₋₃ alkoxy, =CF₂, preferably F, hydroxyl, methyl, trifluoromethyl, difluoromethyl, methoxy, trifluoromethoxy, =CF₂, more preferably F, methyl, trifluoromethyl, difluoromethyl, methoxy, trifluoromethoxy.

13. The compound, or the deuteride, the stereo isomer, or the pharmaceutically acceptable salt, or the pharmaceutically acceptable solvate thereof according to claim 10, wherein:
it has the structure of formula (III): 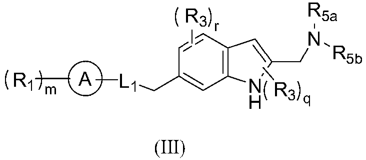
r and q are selected from 0, 1 or 2.

14. The compound, or the deuteride, the stereo isomer, or the pharmaceutically acceptable salt, or the pharmaceutically acceptable solvate thereof according to claim 10, wherein: 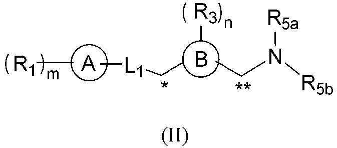 the deuterated sites are located at the C atoms marked with "*", "**" or L₃; more preferably, the deuterated sites are located at L₃.

15. The compound, or the deuteride, the stereo isomer, or the pharmaceutically acceptable salt, or the pharmaceutically acceptable solvate thereof according to claim 1, wherein:
it includes the following compounds:

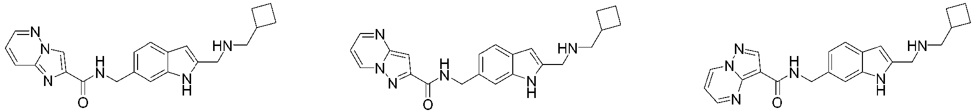

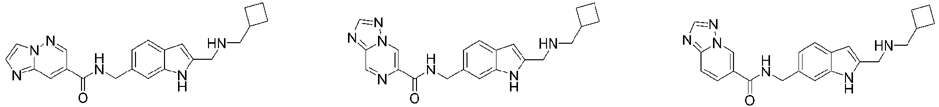

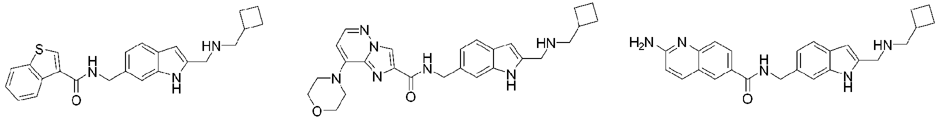

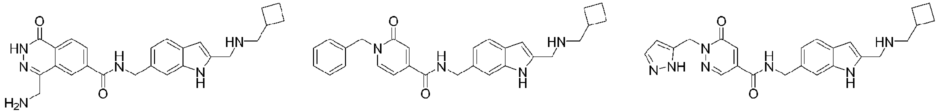

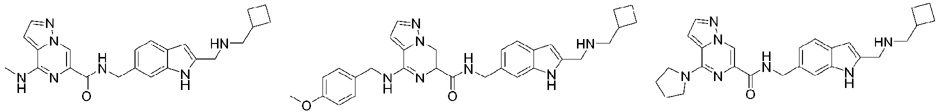

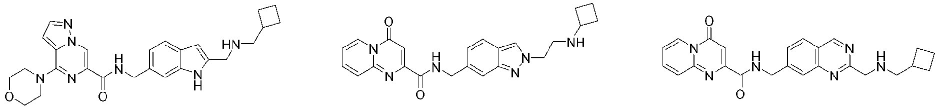

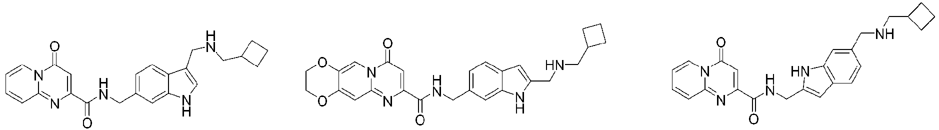

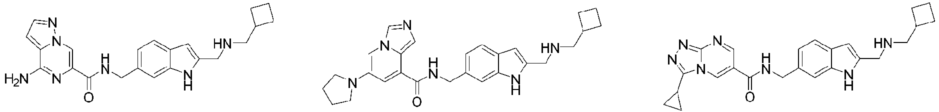

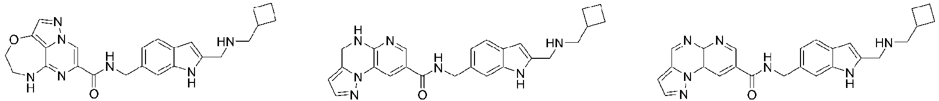

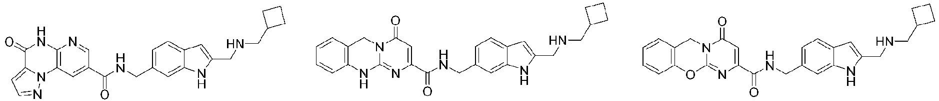

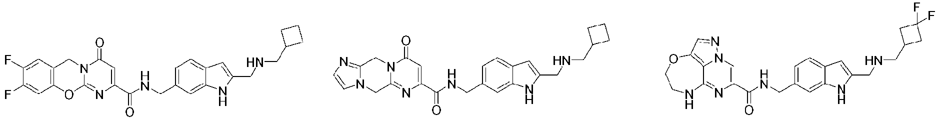

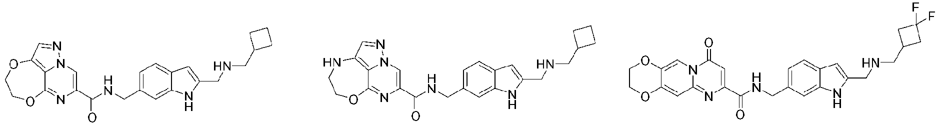

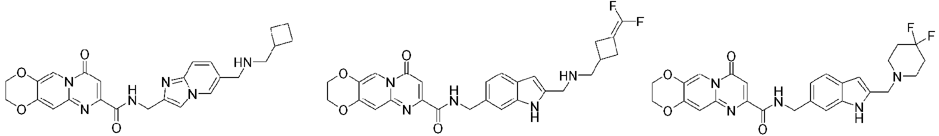

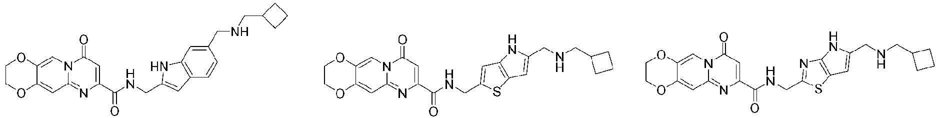

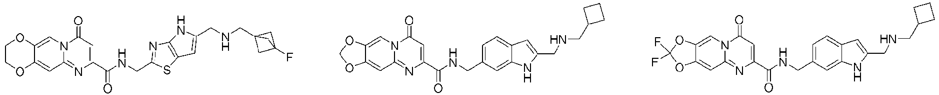

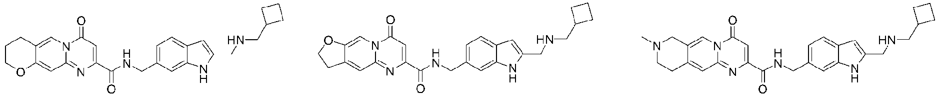

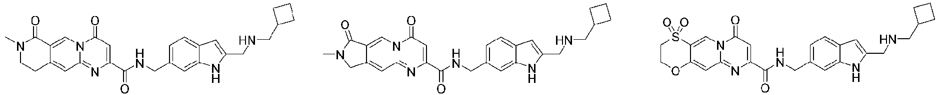

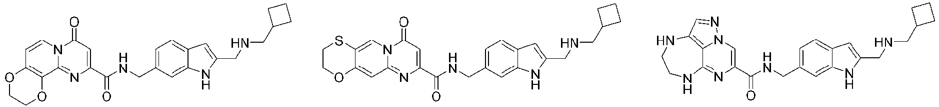

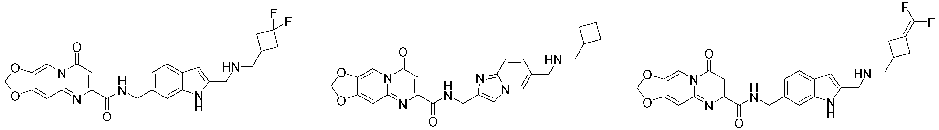

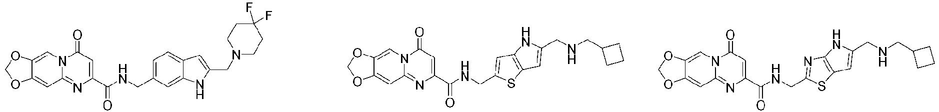

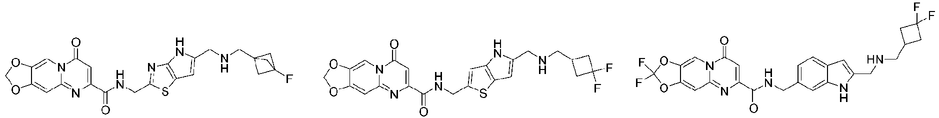

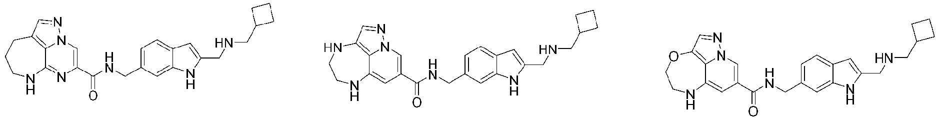

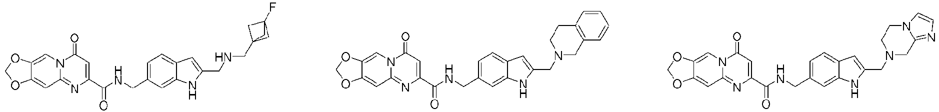

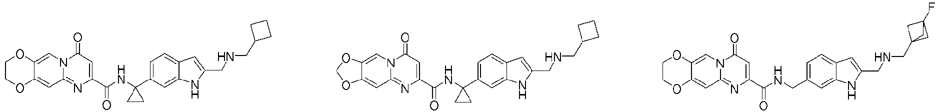

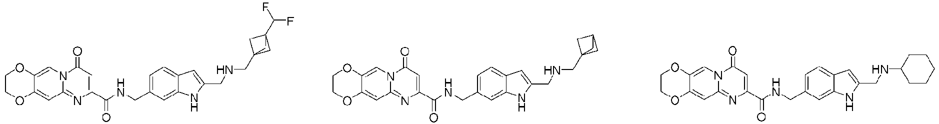

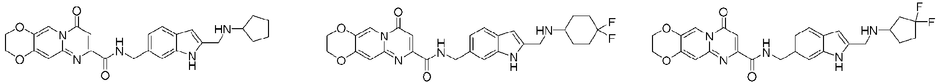

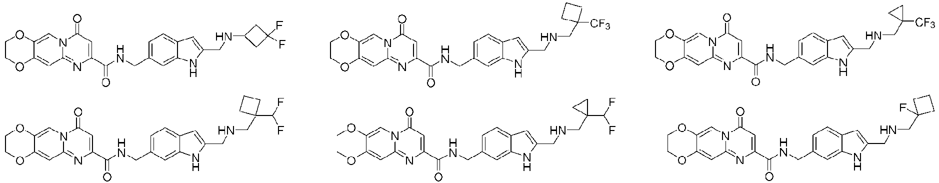

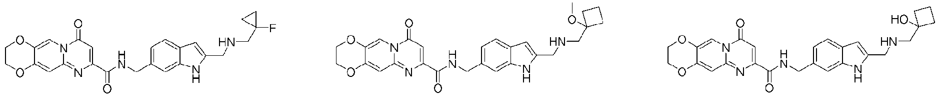

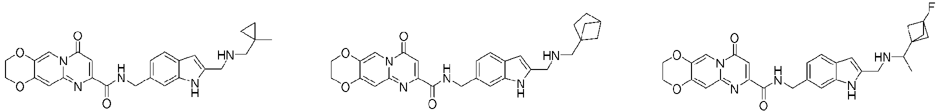

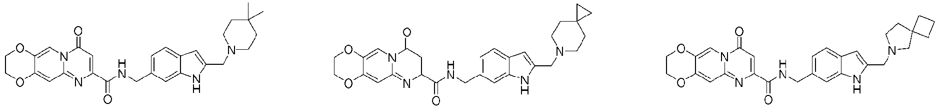

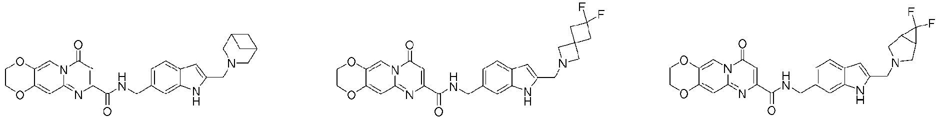

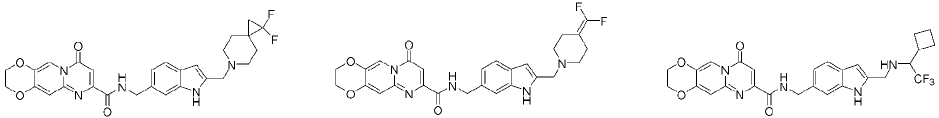

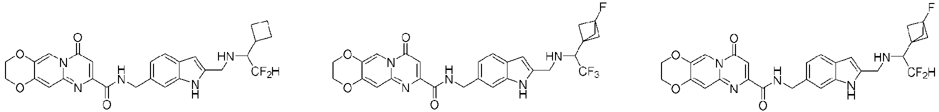

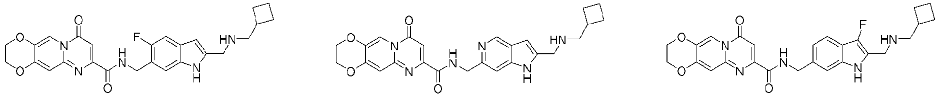

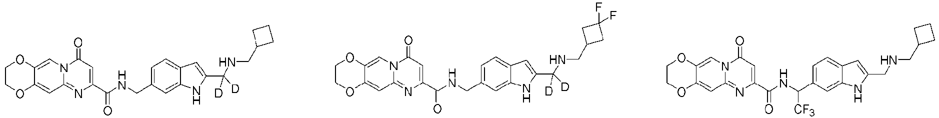

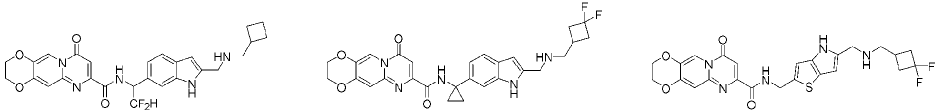

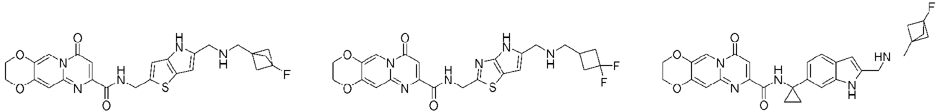

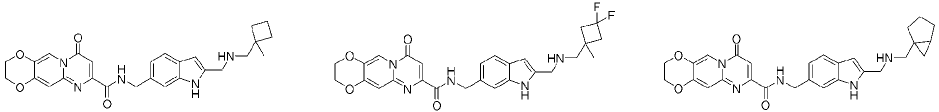

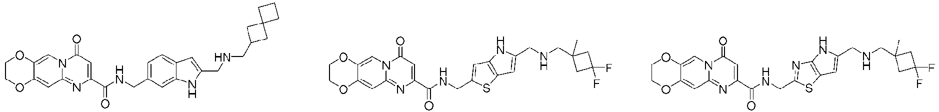

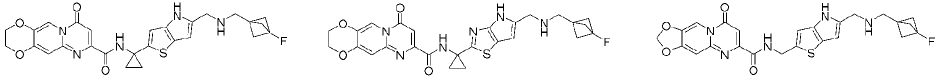

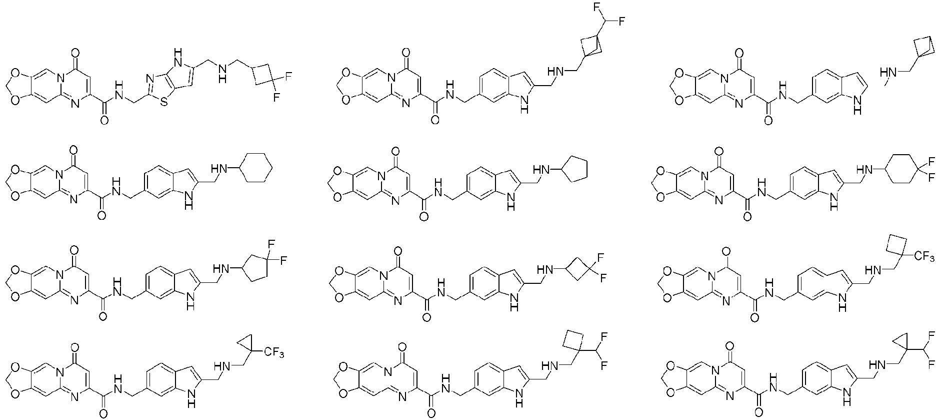

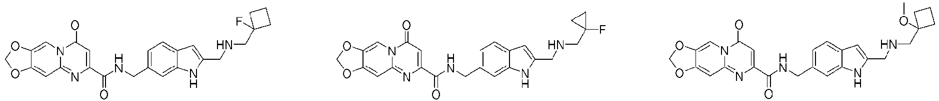

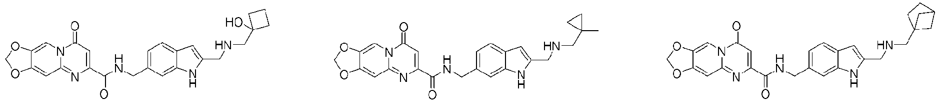

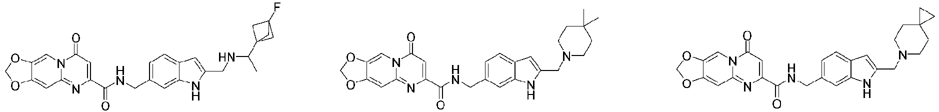

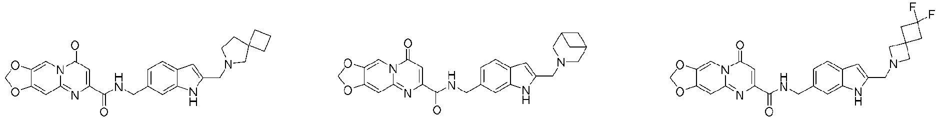

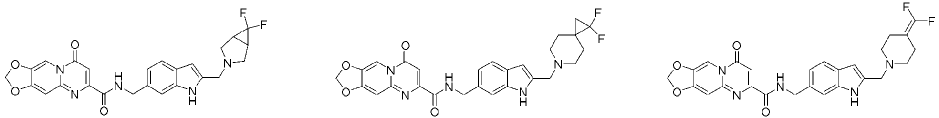

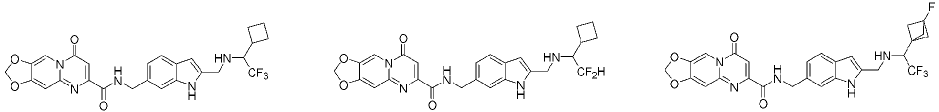

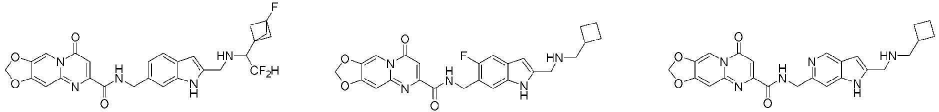

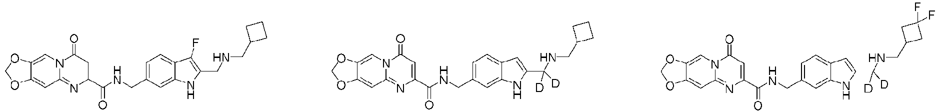

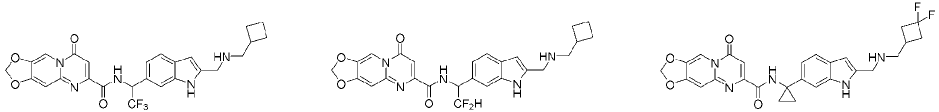

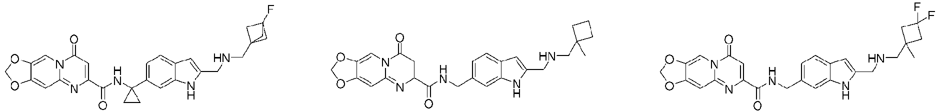

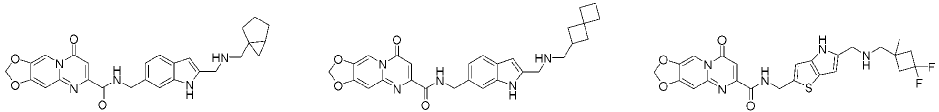

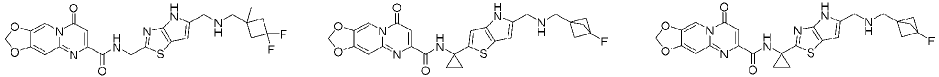

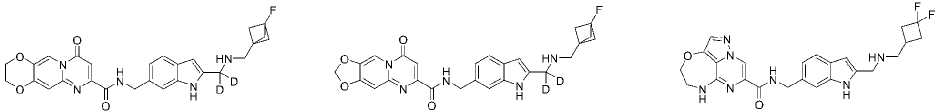

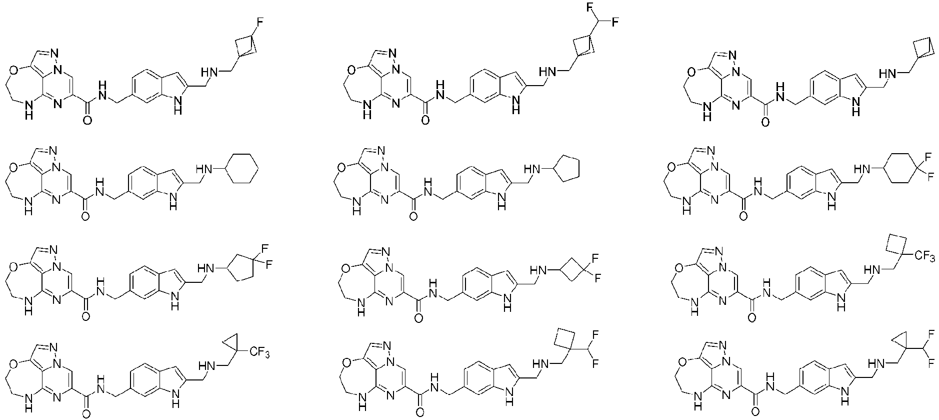

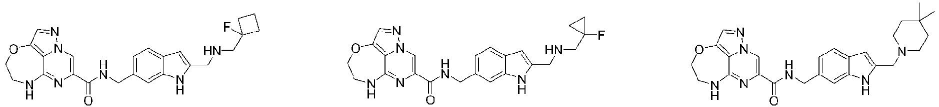

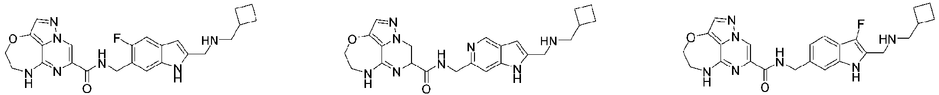

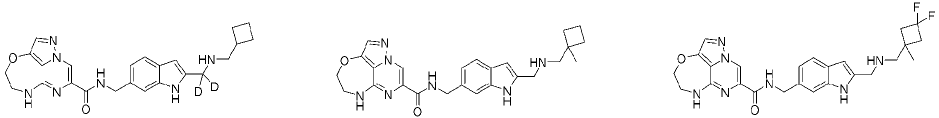

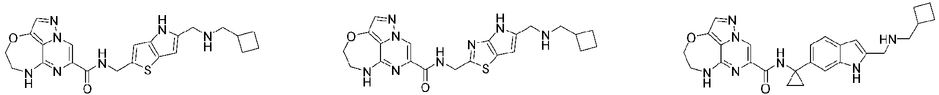

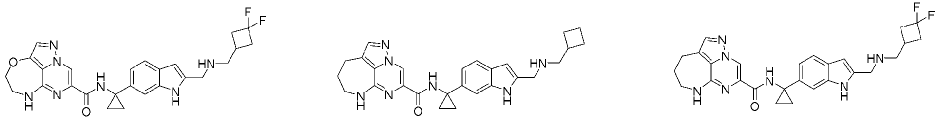

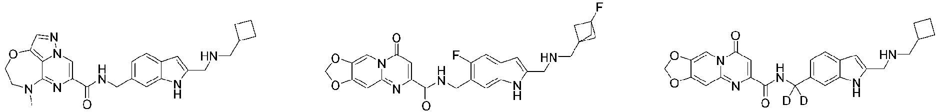

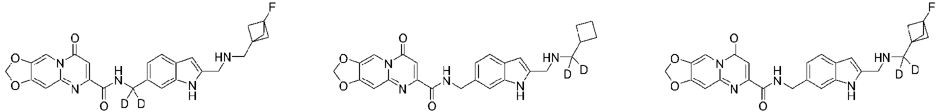

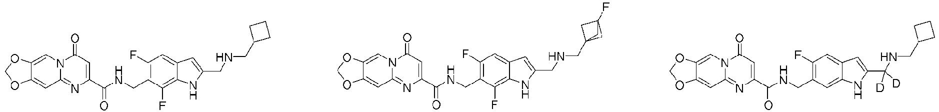

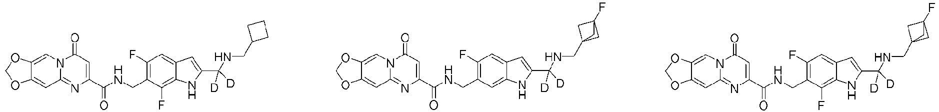

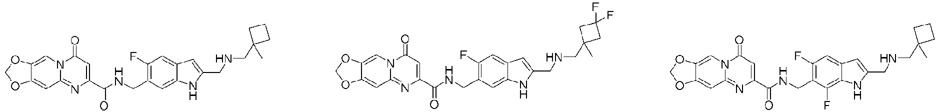

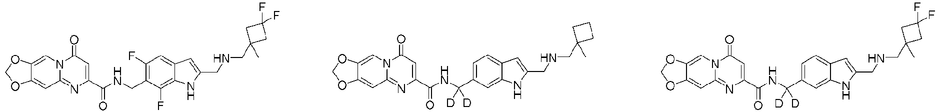

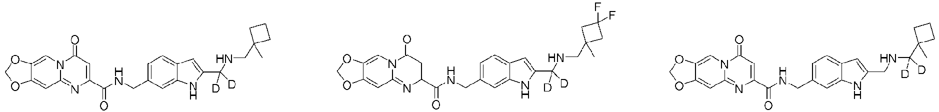

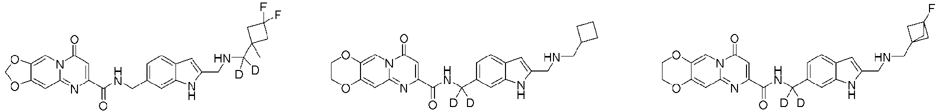

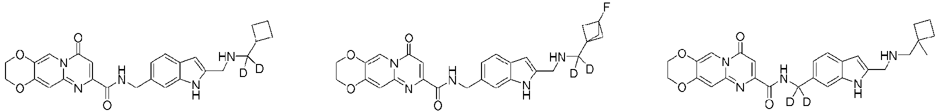

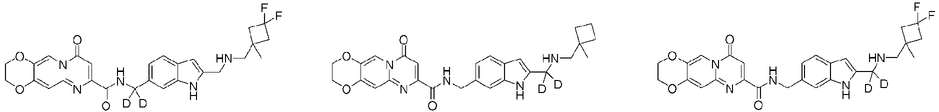

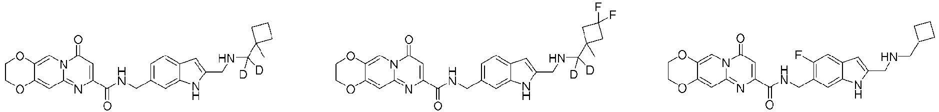

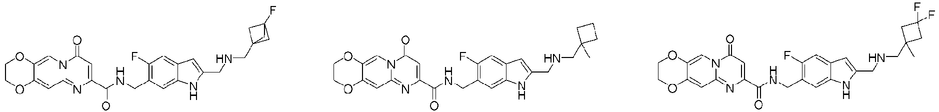

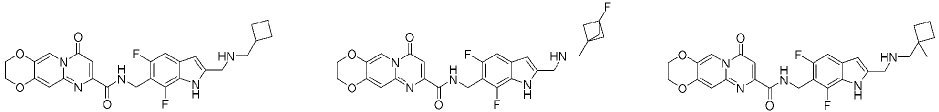

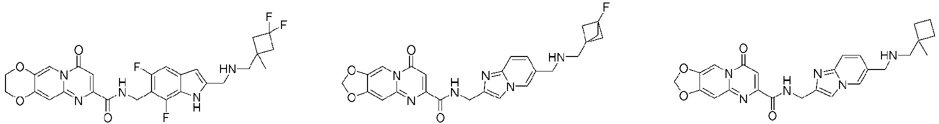

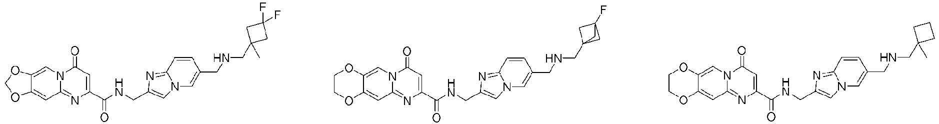

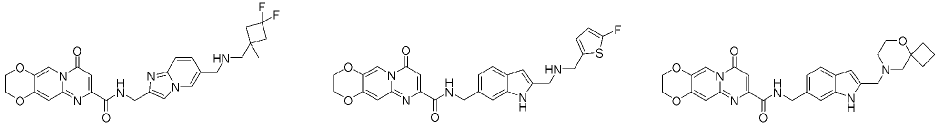

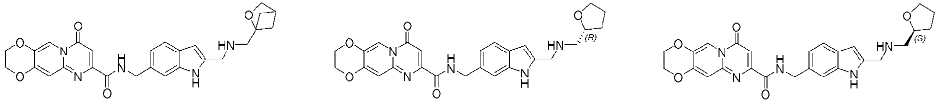

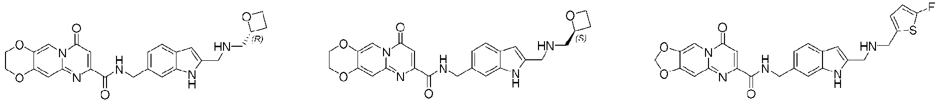

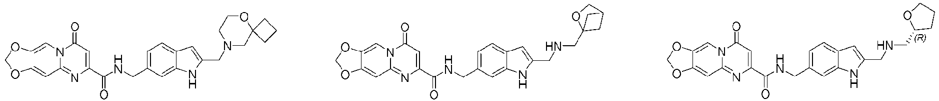

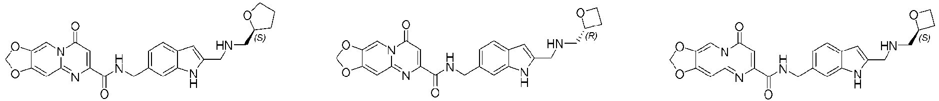

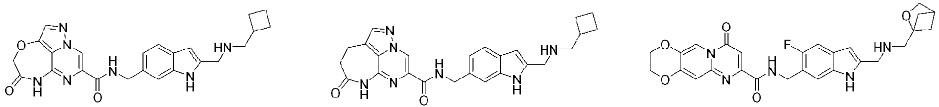

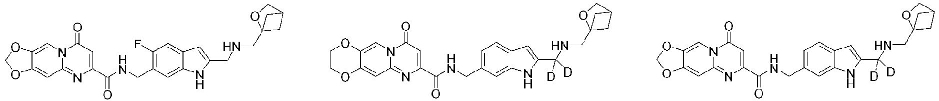

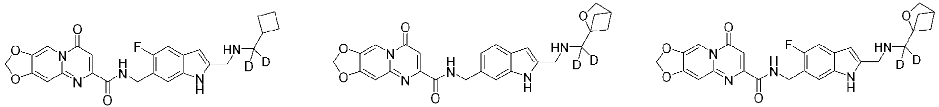

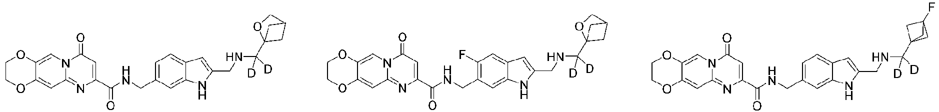

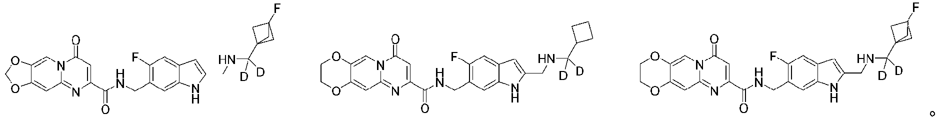

16. A pharmaceutical composition comprising the compound, or the deuteride, the stereo isomer, or the pharmaceutically acceptable salt, or the pharmaceutically acceptable solvate thereof according to any one of claims 1-15, and a pharmaceutically acceptable carrier thereof.

17. Use of the compound, or the deuteride, the stereo isomer, or the pharmaceutically acceptable salt, or the pharmaceutically acceptable solvate thereof according to any one of claims 1-15, or the pharmaceutical composition according to claim 16 in the preparation of a medicament for treating and/or preventing METTL3-related diseases.

18. The use according to claim 17, wherein, the METTL3-related diseases are selected from autoimmune diseases, neurological diseases, infectious diseases, and tumors.

19. The use according to claim 18, wherein, the METTL3-related diseases are selected from AML, myeloid leukemia, solid tumors such as hepatocellular carcinoma, colorectal cancer, and prostate cancer.
